(19) Europäisches Patentamt / European Patent Office / Office européen des brevets

(11) EP 1 374 891 B1

(12) **EUROPEAN PATENT SPECIFICATION**

(45) Date of publication and mention of the grant of the patent:
**10.09.2008 Bulletin 2008/37**

(51) Int Cl.:
***A61K 38/57*** *(2006.01)* ***A61P 11/12*** *(2006.01)*

(21) Application number: **03018789.2**

(22) Date of filing: **22.12.1999**

(54) **Method for accelerating the rate of mucociliary clearance**

Methode zur Beschleunigung der Schleimauflöserate

Technique permettant d'accélérer le débit de clairance mucociliaire

(84) Designated Contracting States:
**AT BE CH CY DE DK ES FI FR GB GR IE IT LI LU MC NL PT SE**
Designated Extension States:
**AL LT LV MK RO SI**

(30) Priority: **22.12.1998 US 218913**
**17.11.1999 US 441966**

(43) Date of publication of application:
**02.01.2004 Bulletin 2004/01**

(62) Document number(s) of the earlier application(s) in accordance with Art. 76 EPC:
**99963636.8 / 1 140 150**

(73) Proprietor: **Bayer Aktiengesellschaft**
**51368 Leverkusen (DE)**

(72) Inventors:
- **Hall, Roderick**
  **Woodham**
  **Surrey KT15 3SJ (GB)**
- **Poll, Christopher T.**
  **Slough SL1 9JY (GB)**
- **Newton, Benjamin B.**
  **Reading**
  **Berkshire RG1 3QA (GB)**
- **Taylor, William J.A.**
  **Windsor SL4 5UB (GB)**

(74) Representative: **Webber, Philip Michael**
**Frank B. Dehn & Co.**
**St Bride's House**
**10 Salisbury Square**
**London**
**EC4Y 8JD (GB)**

(56) References cited:
**EP-A- 0 563 389** **WO-A-92/15605**
**WO-A-97/33996**

- **RASCHE B ET AL: "Uber die Wirkung des Proteaseinhibitors Aprotinin auf die Lungenfunktion sowie die inhibitorische Aktivität des Sputums bei Patienten mit chronisch-obstruktiver Bronchitis" ARZNEIMITTEL-FORSCHUNG, vol. 25, no. 1, January 1975 (1975-01), pages 110-116, XP000929292**
- **SCHMIDT O.P.: "[Drug therapy in cases of bronchosecretory disorders]. MEDIKAMENTOSE THERAPIE BRONCHOSEKRETORISCHER STORUNGEN." MEDIZINISCHE KLINIK, vol. 72, no. 5, 1977, pages 145-160, XP000929302**
- **LINDBERG S ET AL: "Tissue kallikrein stimulates mucociliary activity in the rabbit maxillary sinus." ACTA OTO-LARYNGOLOGICA, (1991) 111 (6) 1126-32., XP000929352**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> Bayer Aktiengesellschaft

<120> Method For Accelerating The Rate Of Mucociliary Clearance

<130> 89.71942/001.cpp

<140> 99963636.8
<141> 1998-12-22

<150> PCT/GB99/04381
<151> 1999-12-22

<150> US 09/441,966
<151> 1999-11-17

<150> US 09/218,913
<151> 1998-12-22

<160> 105

<170> PatentIn version 3.1

<210> 1
<211> 179
<212> PRT
<213> Homo sapiens

<400> 1

Ala Asp Arg Glu Arg Ser Ile His Asp Phe Cys Leu Val Ser Lys Val
1 5 10 15

Val Gly Arg Cys Arg Ala Ser Met Pro Arg Trp Trp Tyr Asn Val Thr
20 25 30

Asp Gly Ser Cys Gln Leu Phe Val Tyr Gly Gly Cys Asp Gly Asn Ser
35 40 45

Asn Asn Tyr Leu Thr Lys Glu Glu Cys Leu Lys Lys Cys Ala Thr Val
50 55 60

Thr Glu Asn Ala Thr Gly Asp Leu Ala Thr Ser Arg Asn Ala Ala Asp
65 70 75 80

Ser Ser Val Pro Ser Ala Pro Arg Arg Gln Asp Ser Glu Asp His Ser
85 90 95

Ser Asp Met Phe Asn Tyr Glu Glu Tyr Cys Thr Ala Asn Ala Val Thr
100 105 110

Gly Pro Cys Arg Ala Ser Phe Pro Arg Trp Tyr Phe Asp Val Glu Arg
115 120 125

Asn Ser Cys Asn Asn Phe Ile Tyr Gly Gly Cys Arg Gly Asn Lys Asn
130 135 140

Ser Tyr Arg Ser Glu Glu Ala Cys Met Leu Arg Cys Phe Arg Gln Gln
145 150 155 160

Glu Asn Pro Pro Leu Pro Leu Gly Ser Lys Val Val Val Leu Ala Gly
165 170 175

Ala Val Ser

<210> 2
<211> 197
<212> PRT
<213> Homo sapiens

<220>
<221> SIGNAL
<222> (1)..(18)
<223>

<400> 2

Ala Gly Ser Phe Leu Ala Trp Leu Gly Ser Leu Leu Leu Ser Gly Val
1               5                   10                  15

Leu Ala Ala Asp Arg Glu Arg Ser Ile His Asp Phe Cys Leu Val Ser
            20                  25                  30

Lys Val Val Gly Arg Cys Arg Ala Ser Met Pro Arg Trp Trp Tyr Asn
        35                  40                  45

Val Thr Asp Gly Ser Cys Gln Leu Phe Val Tyr Gly Gly Cys Asp Gly
    50                  55                  60

Asn Ser Asn Asn Tyr Leu Thr Lys Glu Glu Cys Leu Lys Lys Cys Ala
65                  70                  75                  80

Thr Val Thr Glu Asn Ala Thr Gly Asp Leu Ala Thr Ser Arg Asn Ala
                85                  90                  95

Ala Asp Ser Ser Val Pro Ser Ala Pro Arg Arg Gln Asp Ser Glu Asp
            100                 105                 110

His Ser Ser Asp Met Phe Asn Tyr Glu Glu Tyr Cys Thr Ala Asn Ala
        115                 120                 125

Val Thr Gly Pro Cys Arg Ala Ser Phe Pro Arg Trp Tyr Phe Asp Val
    130                 135                 140

Glu Arg Asn Ser Cys Asn Asn Phe Ile Tyr Gly Gly Cys Arg Gly Asn
145                 150                 155                 160

Lys Asn Ser Tyr Arg Ser Glu Glu Ala Cys Met Leu Arg Cys Phe Arg
                165                 170                 175

Gln Gln Glu Asn Pro Pro Leu Pro Leu Gly Ser Lys Val Val Val Leu
            180                 185                 190

Ala Gly Ala Val Ser
        195

<210> 3
<211> 153
<212> PRT
<213> Homo sapiens

<400> 3

Ile His Asp Phe Cys Leu Val Ser Lys Val Val Gly Arg Cys Arg Ala
1 5 10 15

Ser Met Pro Arg Trp Trp Tyr Asn Val Thr Asp Gly Ser Cys Gln Leu
20 25 30

Phe Val Tyr Gly Gly Cys Asp Gly Asn Ser Asn Asn Tyr Leu Thr Lys
35 40 45

Glu Glu Cys Leu Lys Lys Cys Ala Thr Val Thr Glu Asn Ala Thr Gly
50 55 60

Asp Leu Ala Thr Ser Arg Asn Ala Ala Asp Ser Ser Val Pro Ser Ala
65 70 75 80

Pro Arg Arg Gln Asp Ser Glu Asp His Ser Ser Asp Met Phe Asn Tyr
85 90 95

Glu Glu Tyr Cys Thr Ala Asn Ala Val Thr Gly Pro Cys Arg Ala Ser
100 105 110

Phe Pro Arg Trp Tyr Phe Asp Val Glu Arg Asn Ser Cys Asn Asn Phe
115 120 125

Ile Tyr Gly Gly Cys Arg Gly Asn Lys Asn Ser Tyr Arg Ser Glu Glu
130 135 140

Ala Cys Met Leu Arg Cys Phe Arg Gln
145 150

<210> 4
<211> 58
<212> PRT
<213> Homo sapiens

<400> 4

```
Ile His Asp Phe Cys Leu Val Ser Lys Val Val Gly Arg Cys Arg Ala
1               5                   10                  15

Ser Met Pro Arg Trp Trp Tyr Asn Val Thr Asp Gly Ser Cys Gln Leu
            20                  25                  30

Phe Val Tyr Gly Gly Cys Asp Gly Asn Ser Asn Asn Tyr Leu Thr Lys
        35                  40                  45

Glu Glu Cys Leu Lys Lys Cys Ala Thr Val
    50                  55
```

<210> 5
<211> 51
<212> PRT
<213> Homo sapiens

<400> 5

```
Cys Leu Val Ser Lys Val Val Gly Arg Cys Arg Ala Ser Met Pro Arg
1               5                   10                  15

Trp Trp Tyr Asn Val Thr Asp Gly Ser Cys Gln Leu Phe Val Tyr Gly
            20                  25                  30

Gly Cys Asp Gly Asn Ser Asn Asn Tyr Leu Thr Lys Glu Glu Cys Leu
        35                  40                  45

Lys Lys Cys
    50
```

<210> 6
<211> 58
<212> PRT
<213> Homo sapiens

<400> 6

```
Tyr Glu Glu Tyr Cys Thr Ala Asn Ala Val Thr Gly Pro Cys Arg Ala
1               5                   10                  15

Ser Phe Pro Arg Trp Tyr Phe Asp Val Glu Arg Asn Ser Cys Asn Asn
            20                  25                  30

Phe Ile Tyr Gly Gly Cys Arg Gly Asn Lys Asn Ser Tyr Arg Ser Glu
        35                  40                  45

Glu Ala Cys Met Leu Arg Cys Phe Arg Gln
    50                  55
```

<210> 7
<211> 51
<212> PRT
<213> Homo sapiens

<400> 7

```
Cys Thr Ala Asn Ala Val Thr Gly Pro Cys Arg Ala Ser Phe Pro Arg
1               5                   10                  15

Trp Tyr Phe Asp Val Glu Arg Asn Ser Cys Asn Asn Phe Ile Tyr Gly
            20                  25                  30

Gly Cys Arg Gly Asn Lys Asn Ser Tyr Arg Ser Glu Glu Ala Cys Met
        35                  40                  45

Leu Arg Cys
    50
```

<210> 8
<211> 92
<212> PRT
<213> Homo sapiens

<400> 8

```
Ala Asp Arg Glu Arg Ser Ile His Asp Phe Cys Leu Val Ser Lys Val
1               5                   10                  15

Val Gly Arg Cys Arg Ala Ser Met Pro Arg Trp Trp Tyr Asn Val Thr
            20                  25                  30

Asp Gly Ser Cys Gln Leu Phe Val Tyr Gly Gly Cys Asp Gly Asn Ser
        35                  40                  45

Asn Asn Tyr Leu Thr Lys Glu Glu Cys Leu Lys Lys Cys Ala Thr Val
    50                  55                  60

Thr Glu Asn Ala Thr Gly Asp Leu Ala Thr Ser Arg Asn Ala Ala Asp
65                  70                  75                  80

Ser Ser Val Pro Ser Ala Pro Arg Arg Gln Asp Ser
                85                  90
```

<210> 9
<211> 708
<212> DNA
<213> Artificial Sequence

<220>
<223> Consensus DNA sequence of human Bikunin (Fig. 3).

<220>
<221> misc_feature
<222> (622)..(622)
<223> "n" is any nucleotide.

<220>
<221> misc_feature
<222> (679)..(679)
<223> "n" is any nucleotide.

<220>
<221> misc_feature
<222> (707)..(707)
<223> "n" is any nucleotide.

<400> 9

ggccgggtcg tttctcgcct ggctgggatc gctgctcctc tctggggtcc tggcggccga 60
ccgagaacgc agcatccacg acttctgcct ggtgtcgaag gtggtgggca gatgccgggc 120
ctccatgcct aggtggtggt acaatgtcac tgacggatcc tgccagctgt ttgtgtatgg 180
gggctgtgac ggaaacagca ataattacct gaccaaggag gagtgcctca agaaatgtgc 240
cactgtcaca gagaatgcca cgggtgacct ggccaccagc aggaatgcag cggattcctc 300
tgtcccaagt gctcccagaa ggcaggattc tgaagaccac tccagcgata tgttcaacta 360
tgaagaatac tgcaccgcca acgcagtcac tgggccttgc cgtgcatcct tcccacgctg 420
gtactttgac gtggagagga actcctgcaa taacttcatc tatggaggct gccggggcaa 480
taagaacagc taccgctctg aggaggcctg catgctccgc tgcttccgcc agcaggagaa 540
tcctcccctg ccccttggct caaaggtggt ggttctggcc ggggctgttt cgtgatggtg 600
ttgatccttt tcctggggag cntccatggt cttactgatt ccgggtggca aggaggaacc 660
aggagcgtgc cctgcgganc gtctggagct tcggagatga caagggnt 708

<210> 10
<211> 197
<212> PRT
<213> Artificial Sequence

<220>
<223> Amino acids -18 to 179 of the translation of the consensus DNA sequence in Fig. 3.

<400> 10

```
Ala Gly Ser Phe Leu Ala Trp Leu Gly Ser Leu Leu Leu Ser Gly Val
1               5                   10                  15

Leu Ala Ala Asp Arg Glu Arg Ser Ile His Asp Phe Cys Leu Val Ser
            20                  25                  30

Lys Val Val Gly Arg Cys Arg Ala Ser Met Pro Arg Trp Trp Tyr Asn
        35                  40                  45

Val Thr Asp Gly Ser Cys Gln Leu Phe Val Tyr Gly Gly Cys Asp Gly
    50                  55                  60

Asn Ser Asn Asn Tyr Leu Thr Lys Glu Glu Cys Leu Lys Lys Cys Ala
65                  70                  75                  80

Thr Val Thr Glu Asn Ala Thr Gly Asp Leu Ala Thr Ser Arg Asn Ala
                85                  90                  95

Ala Asp Ser Ser Val Pro Ser Ala Pro Arg Arg Gln Asp Ser Glu Asp
            100                 105                 110

His Ser Ser Asp Met Phe Asn Tyr Glu Glu Tyr Cys Thr Ala Asn Ala
        115                 120                 125

Val Thr Gly Pro Cys Arg Ala Ser Phe Pro Arg Trp Tyr Phe Asp Val
    130                 135                 140

Glu Arg Asn Ser Cys Asn Asn Phe Ile Tyr Gly Gly Cys Arg Gly Asn
145                 150                 155                 160

Lys Asn Ser Tyr Arg Ser Glu Glu Ala Cys Met Leu Arg Cys Phe Arg
                165                 170                 175

Gln Gln Glu Asn Pro Pro Leu Pro Leu Gly Ser Lys Val Val Val Leu
            180                 185                 190

Ala Gly Ala Val Ser
        195
```

<210> 11
<211> 179
<212> PRT
<213> Artificial Sequence

<220>
<223> Variants of human Bikunin.

<220>
<221> MISC_FEATURE
<222> (8)..(8)
<223> Each "Xaa" independently represents a naturally occurring amino acid residue except Cys, with the proviso that at least one "Xaa" in SEQ ID NO:11 is different from the corresponding amino acid residue of the native sequence.

<220>
<221> MISC_FEATURE
<222> (17)..(17)
<223> Each "Xaa" independently represents a naturally occurring amino acid residue except Cys, with the proviso that at least one "Xaa" in SEQ ID NO:11 is different from the corresponding amino acid residue of the native sequence.

<220>
<221> MISC_FEATURE
<222> (19)..(19)
<223> Each "Xaa" independently represents a naturally occurring amino acid residue except Cys, with the proviso that at least one "Xaa" in SEQ ID NO:11 is different from the corresponding amino acid residue of the native sequence.

<220>
<221> MISC_FEATURE
<222> (21)..(26)
<223> Each "Xaa" independently represents a naturally occurring amino acid residue except Cys, with the proviso that at least one "Xaa" in SEQ ID NO:11 is different from the corresponding amino acid residue of the native sequence.

<220>
<221> MISC_FEATURE
<222> (40)..(40)
<223> Each "Xaa" independently represents a naturally occurring amino acid residue except Cys, with the proviso that at least one "Xaa" in SEQ ID NO:11 is different from the corresponding amino acid residue of the native sequence.

<220>
<221> MISC_FEATURE
<222> (42)..(42)
<223> Each "Xaa" independently represents a naturally occurring amino acid residue except Cys, with the proviso that at least one "Xaa" in SEQ ID NO:11 is different from the corresponding amino acid residue of the native sequence.

<220>
<221> MISC_FEATURE
<222> (45)..(47)
<223> Each "Xaa" independently represents a naturally occurring amino acid residue except Cys, with the proviso that at least one "Xaa" in SEQ ID NO:11 is different from the corresponding amino acid residue of the native sequence.

<220>
<221> MISC_FEATURE
<222> (52)..(52)
<223> Each "Xaa" independently represents a naturally occurring amino acid residue except Cys, with the proviso that at least one "Xaa" in SEQ ID NO:11 is different from the corresponding amino acid residue of the native sequence.

<220>
<221> MISC_FEATURE
<222> (64)..(64)
<223> Each "Xaa" independently represents a naturally occurring amino acid residue except Cys, with the proviso that at least one "Xaa" in SEQ ID NO:11 is different from the corresponding amino acid residue of the native sequence.

<220>
<221> MISC_FEATURE
<222> (103)..(103)
<223> Each "Xaa" independently represents a naturally occurring amino acid residue except Cys, with the proviso that at least one "Xaa" in SEQ ID NO:11 is different from the corresponding amino acid residue of the native sequence.

<220>
<221> MISC_FEATURE
<222> (112)..(112)
<223> Each "Xaa" independently represents a naturally occurring amino acid residue except Cys, with the proviso that at least one "Xaa" in SEQ ID NO:11 is different from the corresponding amino acid residue of the native sequence.

<220>
<221> MISC_FEATURE
<222> (114)..(114)
<223> Each "Xaa" independently represents a naturally occurring amino acid residue except Cys, with the proviso that at least one "Xaa" in SEQ ID NO:11 is different from the corresponding amino acid residue of the native sequence.

<220>
<221> MISC_FEATURE
<222> (116)..(121)
<223> Each "Xaa" independently represents a naturally occurring amino acid residue except Cys, with the proviso that at least one "Xaa" in SEQ ID NO:11 is different from the corresponding amino acid residue of the native sequence.

<220>
<221> MISC_FEATURE
<222> (135)..(135)
<223> Each "Xaa" independently represents a naturally occurring amino acid residue except Cys, with the proviso that at least one "Xaa" in SEQ ID NO:11 is different from the corresponding amino acid residue of the native sequence.

<220>
<221> MISC_FEATURE
<222> (137)..(137)
<223> Each "Xaa" independently represents a naturally occurring amino acid residue except Cys, with the proviso that at least one "Xaa" in SEQ ID NO:11 is different from the corresponding amino acid residue of the native sequence.

<220>
<221> MISC_FEATURE
<222> (140)..(142)
<223> Each "Xaa" independently represents a naturally occurring amino acid residue except Cys, with the proviso that at least one "Xaa" in SEQ ID NO:11 is different from the corresponding amino acid residue of the native sequence.

<220>
<221> MISC_FEATURE
<222> (147)..(147)
<223> Each "Xaa" independently represents a naturally occurring amino acid residue except Cys, with the proviso that at least one "Xaa" in SEQ ID NO:11 is different from the corresponding amino acid residue of the native sequence.

<220>
<221> MISC_FEATURE
<222> (159)..(159)
<223> Each "Xaa" independently represents a naturally occurring amino acid residue except Cys, with the proviso that at least one "Xaa" in SEQ ID NO:11 is different from the corresponding amino acid residue of the native sequence.

<400> 11

```
Ala Asp Arg Glu Arg Ser Ile Xaa Asp Phe Cys Leu Val Ser Lys Val
1               5                   10                  15

Xaa Gly Xaa Cys Xaa Xaa Xaa Xaa Xaa Xaa Trp Trp Tyr Asn Val Thr
            20                  25                  30

Asp Gly Ser Cys Gln Leu Phe Xaa Tyr Xaa Gly Cys Xaa Xaa Xaa Ser



        35                  40                  45

Asn Asn Tyr Xaa Thr Lys Glu Glu Cys Leu Lys Lys Cys Ala Thr Xaa
    50                  55                  60

Thr Glu Asn Ala Thr Gly Asp Leu Ser Thr Ser Arg Asn Ala Ala Asp
65                  70                  75                  80

Ser Ser Val Pro Ser Ala Pro Arg Arg Gln Asp Ser Glu His Asp Ser
                85                  90                  95

Ser Asp Met Phe Asn Tyr Xaa Glu Tyr Cys Thr Ala Asn Ala Val Xaa
            100                 105                 110

Gly Xaa Cys Xaa Xaa Xaa Xaa Xaa Xaa Trp Tyr Phe Asp Val Glu Arg
        115                 120                 125

Asn Ser Cys Asn Asn Phe Xaa Tyr Xaa Gly Cys Xaa Xaa Xaa Lys Asn
    130                 135                 140

Ser Tyr Xaa Ser Glu Glu Ala Cys Met Leu Arg Cys Phe Arg Xaa Gln
145                 150                 155                 160

Glu Asn Pro Pro Leu Pro Leu Gly Ser Lys Val Val Val Leu Ala Gly
                165                 170                 175

Ala Val Ser
```

<210> 12
<211> 393
<212> DNA
<213> Homo sapiens

<220>
<221> misc_feature

<222> (361)..(361)
<223> "n" is any nucleotide.

<220>
<221> misc_feature
<222> (367)..(367)
<223> "n" is any nucleotide.

<220>
<221> misc_feature
<222> (384)..(384)
<223> "n" is any nucleotide.

<220>
<221> misc_feature
<222> (390)..(390)
<223> "n" is any nucleotide.

<400> 12

```
ggccgggtcg tttctcgcct ggctgggatc gctgctcctc tctggggtcc tggccggccg      60
accgagaacg cagcatccac gacttctgcc tggtgtcgaa ggtggtgggc agattccggg     120
cctccatgcc taggtggtgg tacaatgtca ctgacggatc ctgccagctg tttgtgtatg     180
ggggctgtga cggaaacagc aataattacc tgaccaagga ggagtgcctc aagaaatgtg     240
ccactgtcac agagaatgcc acgggtgacc tggccaccag caggaatgca gcggattcct     300
ctgtcccaag tgctcccaga aggcaggatt cttgaagacc acttcagcga tatgtttcaa     360
ntattgnaag aataattgca ccgncaacgn att                                  393
```

<210> 13
<211> 110
<212> PRT
<213> Homo sapiens

<220>
<221> SIGNAL
<222> (1)..(18)
<223>

<400> 13

```
Pro Gly Arg Phe Ser Pro Gly Trp Asp Arg Cys Ser Ser Leu Gly Ser
1               5                   10                  15

Trp Pro Ala Asp Arg Glu Arg Ser Ile His Asp Phe Cys Leu Val Ser
            20                  25                  30

Lys Val Val Gly Arg Phe Arg Ala Ser Met Pro Arg Trp Trp Tyr Asn
        35                  40                  45

Val Thr Asp Gly Ser Cys Gln Leu Phe Val Tyr Gly Gly Cys Asp Gly
    50                  55                  60

Asn Ser Asn Asn Tyr Leu Thr Lys Glu Glu Cys Leu Lys Lys Cys Ala
65                  70                  75                  80

Thr Val Thr Glu Asn Ala Thr Gly Asp Leu Ala Thr Ser Arg Asn Ala
                85                  90                  95

Ala Asp Ser Ser Val Pro Ser Ala Pro Arg Arg Gln Asp Ser


            100                 105                 110
```

<210> 14
<211> 510
<212> DNA
<213> Homo sapiens

<220>
<221> misc_feature
<222> (424)..(424)
<223> "n" is any nucleotide.

<220>
<221> misc_feature
<222> (481)..(481)
<223> "n" is any nucleotide.

<220>
<221> misc_feature
<222> (509)..(509)
<223> "n" is any nucleotide.

<400> 14

```
gcaataatta cctgaccaag gaggagtgcc tcaagaaatg tgccactgtc acagagaatg      60
ccacgggtga cctggccacc agcaggaatg cagcggattc ctctgtccca agtctcccag     120
aaggcaggat tctgaagacc actccagcga tatgttcaac tatgaagaat actgcaccgc     180
caacgcagtc actgggcctt gccgtgcatc cttcccacgc tggtactttg acgtggagag     240
gaactcctgc aataacttca tctatggagg ctgccggggc aataagaaca gctaccgctc     300
tgaggaggcc tgcatgctcc gctgcttccg ccagcaggag aatcctcccc tgccccttgg     360
ctcaaaggtg gtggttctgg ccggggctgt ttcgtgatgg tgttgatcct tttcctgggg     420
agcntccatg gtcttactga ttccgggtgg caaggaggaa ccaggagcgt gccctgcgga     480
ncgtctggag cttcggagat gacaagggnt                                      510
```

<210> 15
<211> 20
<212> PRT
<213> Homo sapiens

<400> 15

```
Leu Pro Asp Gln Gly Gly Val Pro Gln Glu Met Cys His Cys His Arg
1               5                   10                  15

Glu Cys His Gly
            20
```

<210> 16
<211> 427
<212> DNA
<213> Homo sapiens

<220>
<221> misc_feature
<222> (3)..(3)
<223> "n" is any nucleotide.

<220>
<221> misc_feature
<222> (11)..(12)
<223> "n" is any nucleotide.

<220>
<221> misc_feature
<222> (17)..(17)
<223> "n" is any nucleotide.

<220>
<221> misc_feature
<222> (48)..(48)

<223> "n" is any nucleotide.

<220>
<221> misc_feature
<222> (425)..(425)
<223> "n" is any nucleotide.

<400> 16

```
gcngcgcgtt nntcgcntgc tgggatcgct gcacctctct ggggtcgngg cggccgaccg      60
agaacgcagc atccacgact tctgcctggt gtcgaaggtg gtgggcagat gccgggcctc     120
catgcctagg tggtggtaca atgtcactga cggatcctgc cagctgtttg tgtatggggg     180
ctgtgacgga aacagcaata attacctgac caaggaggag tgcctcaaga aatgtgccac     240
tgtcacagag aatgccacgg gtgacctggc caccagcagg aatgcagcgg attcctctgt     300
cccaagtgct cccagaaggc aggattctga agaccactcc agcgatatgt tcaactatga     360
agaatactgg caccgccaac gcattcactg ggcctgcgtg catccttccc acgctggtac     420
tttgncg                                                               427
```

<210> 17
<211> 423
<212> DNA
<213> Homo sapiens

<220>
<221> misc_feature
<222> (6)..(6)
<223> "n" is any nucleotide.

<220>
<221> misc_feature
<222> (401)..(401)
<223> "n" is any nucleotide.

<220>
<221> misc_feature
<222> (407)..(407)
<223> "n" is any nucleotide.

<400> 17

```
tgggantcgc tgctcctctc tggggtcctg gcggccgacc gagaacgcag catccacgac     60
ttctgcctgg tgtcgaaggt ggtgggcaga tgccgggcct ccatgcctag gtggtggtac    120
aatgtcactg acggatcctg ccagctgttt gtgtatgggg gctgtgacgg aaacagcaat    180
aattacctga ccaaggagga gtgcctcaag aaatgtgcca ctgtcacaga gaatgccacg    240
ggtgacctgg ccaccagcag gaatgcagcg gattcctctg tcccaagtgc tcccagaagg    300
caggattctg aagaccactc cagcgatatg ttcaactatg aagaatactg caccgccaac    360
gcagtcactg ggccttgcgt ggaatccttt cccacgctgg naatttngac gttgagaagg    420
aac                                                                  423
```

<210> 18
<211> 57
<212> PRT
<213> Unknown

<220>
<223> Kunitz-like domain of tissue factor pathway inhibitor precursor 1.

<400> 18

```
His Ser Phe Cys Ala Phe Lys Ala Asp Asp Gly Pro Cys Lys Ala Ile
1               5                   10                  15

Met Lys Arg Phe Phe Phe Asn Ile Phe Thr Arg Gln Cys Glu Glu Phe
            20                  25                  30

Ile Tyr Gly Gly Cys Glu Gly Asn Gln Asn Arg Phe Glu Ser Leu Glu
        35                  40                  45

Glu Cys Lys Lys Met Cys Thr Arg Asp
    50                  55
```

<210> 19
<211> 57
<212> PRT
<213> Unknown

<220>
<223> Kunitz-like domain of tissue factor pathway inhibitor precursor 1.

<400> 19

Pro Asp Phe Cys Phe Leu Glu Glu Asp Pro Gly Ile Cys Arg Gly Tyr
1 5 10 15

Ile Thr Arg Tyr Phe Tyr Asn Asn Gln Thr Lys Gln Cys Glu Arg Phe
20 25 30

Lys Tyr Gly Gly Cys Leu Gly Asn Met Asn Asn Phe Glu Thr Leu Glu
35 40 45

Glu Cys Lys Asn Ile Cys Glu Asp Gly
50 55

<210> 20
<211> 57
<212> PRT
<213> Unknown

<220>
<223> Kunitz-like domain of tissue factor pathway inhibitor precursor.

<400> 20

Pro Ser Trp Cys Leu Thr Pro Ala Asp Arg Gly Leu Cys Arg Ala Asn
1 5 10 15

Glu Asn Arg Phe Tyr Tyr Asn Ser Val Ile Gly Lys Cys Arg Pro Phe
20 25 30

Lys Tyr Ser Gly Cys Gly Gly Asn Glu Asn Asn Phe Thr Ser Lys Gln
35 40 45

Glu Cys Leu Arg Ala Cys Lys Lys Gly
50 55

<210> 21
<211> 57
<212> PRT
<213> Unknown

<220>
<223> Kunitz-like domain of tissue factor pathway inhibitor precursor 2.

<400> 21

Ala Glu Ile Cys Leu Leu Pro Leu Asp Tyr Gly Pro Cys Arg Ala Leu

1 5 10 15

Leu Leu Arg Tyr Tyr Tyr Arg Tyr Arg Thr Gln Ser Cys Arg Gln Phe
20 25 30

Leu Tyr Gly Gly Cys Glu Gly Asn Ala Asn Asn Phe Tyr Thr Trp Glu
35 40 45

Ala Cys Asp Asp Ala Cys Trp Arg Ile
50 55

<210> 22
<211> 57
<212> PRT
<213> Unknown

<220>
<223> Kunitz-like domain of tissue factor pathway inhibitor precursor 2.

<400> 22

Pro Ser Phe Cys Tyr Ser Pro Lys Asp Glu Gly Leu Cys Ser Ala Asn
1 5 10 15

Val Thr Arg Tyr Tyr Phe Asn Pro Arg Tyr Arg Thr Cys Asp Ala Phe
20 25 30

Thr Tyr Thr Gly Cys Gly Gly Asn Asp Asn Asn Phe Val Ser Arg Glu
35 40 45

Asp Cys Lys Arg Ala Cys Ala Lys Ala
50 55

<210> 23
<211> 57
<212> PRT
<213> Unknown

<220>
<223> Kunitz-like domain of amyloid precursor protein homologue.

<400> 23

Lys Ala Val Cys Ser Gln Glu Ala Met Thr Gly Pro Cys Arg Ala Val
1               5                   10                  15

Met Pro Arg Thr Thr Phe Asp Leu Ser Lys Gly Lys Cys Val Arg Phe
            20                  25                  30

Ile Thr Gly Gly Cys Gly Gly Asn Arg Asn Asn Phe Glu Ser Glu Asp
        35                  40                  45

Tyr Cys Met Ala Val Cys Lys Ala Met
    50                  55

<210> 24
<211> 58
<212> PRT
<213> Unknown

<220>
<223> Kunitz-like domain of aprotinin.

<400> 24

Arg Pro Asp Phe Cys Leu Glu Pro Pro Tyr Thr Gly Pro Cys Lys Ala
1               5                   10                  15

Arg Ile Ile Arg Tyr Phe Tyr Asn Ala Lys Ala Gly Leu Cys Gln Thr
            20                  25                  30

Phe Val Tyr Gly Gly Cys Arg Ala Lys Arg Asn Asn Phe Lys Ser Ala
        35                  40                  45

Glu Asp Cys Met Arg Thr Cys Gly Gly Ala
    50                  55

<210> 25
<211> 51
<212> PRT
<213> Unknown

<220>
<223> Kunitz-like domain of inter-alpha-trypsin inhibitor precursor.

<400> 25

Cys Gln Leu Gly Tyr Ser Ala Gly Pro Cys Met Gly Met Thr Ser Arg
1               5                   10                  15

Tyr Phe Tyr Asn Gly Thr Ser Met Ala Cys Glu Thr Phe Gln Tyr Gly
            20                  25                  30

Gly Cys Met Gly Asn Gly Asn Asn Phe Val Thr Glu Lys Glu Cys Leu
        35                  40                  45

Gln Thr Cys
    50

<210> 26
<211> 57
<212> PRT
<213> Unknown

<220>
<223> Kunitz-like domain of inter-alpha-trypsin inhibitor precursor.

<400> 26

Val Ala Ala Cys Asn Leu Pro Ile Val Arg Gly Pro Cys Arg Ala Phe
1               5                   10                  15

Ile Gln Leu Trp Ala Phe Asp Ala Val Lys Gly Lys Cys Val Leu Phe
            20                  25                  30

Pro Tyr Gly Gly Cys Gln Gly Asn Gly Asn Lys Phe Tyr Ser Glu Lys
        35                  40                  45

Glu Cys Arg Glu Tyr Cys Gly Val Pro
    50                  55

<210> 27
<211> 57
<212> PRT
<213> Unknown

<220>
<223> Kunitz-like domain of amyloid precursor protein.

<400> 27

Glu Val Cys Cys Ser Glu Gln Ala Glu Thr Gly Pro Cys Arg Ala Met
1 5 10 15

Ile Ser Arg Trp Tyr Phe Asp Val Thr Glu Gly Lys Cys Ala Pro Phe
20 25 30

Phe Tyr Gly Gly Cys Gly Gly Asn Arg Asn Asn Phe Asp Thr Glu Glu
35 40 45

Tyr Cys Met Ala Val Cys Gly Ser Ala
50 55

<210> 28
<211> 51
<212> PRT
<213> Unknown

<220>
<223> Kunitz-like domain of collagen alpha-3(VI) precursor.

<400> 28

Cys Lys Leu Pro Lys Asp Glu Gly Thr Cys Arg Asp Phe Ile Leu Lys
1 5 10 15

Trp Tyr Tyr Asp Pro Asn Thr Lys Ser Cys Ala Arg Phe Trp Tyr Gly
20 25 30

Gly Cys Gly Gly Asn Glu Asn Lys Phe Gly Ser Gln Lys Glu Cys Glu
35 40 45

Lys Val Cys
50

<210> 29
<211> 57
<212> PRT
<213> Unknown

<220>
<223> Kunitz-like domain of HKI-B9.

<400> 29

```
Pro Asn Val Cys Ala Phe Pro Met Glu Lys Gly Pro Cys Gln Thr Tyr
1               5                   10                  15

Met Thr Arg Trp Phe Phe Asn Phe Glu Thr Gly Glu Cys Glu Leu Phe
            20                  25                  30

Ala Tyr Gly Gly Cys Gly Gly Asn Ser Asn Asn Phe Leu Arg Lys Glu
        35                  40                  45

Lys Cys Glu Lys Phe Cys Lys Phe Thr
    50                  55
```

<210> 30
<211> 46
<212> DNA
<213> Artificial Sequence

<220>
<223> 5' sense oligonucleotide used in Example 6.

<400> 30
gccaagcttg gataaaagat atgaagaata ctgcaccgcc aacgca 46

<210> 31
<211> 35
<212> DNA
<213> Artificial Sequence

<220>
<223> 3' antisense oligonucleotide used in Example 6.

<400> 31
ggggatcctc actgctggcg gaagcagcgg agcat 35

<210> 32
<211> 206
<212> DNA
<213> Artificial Sequence

<220>
<223> Cloned bikunin cDNA fragment in Example 6.

<400> 32

```
ccaagcttgg ataaaagata tgaagaatac tgcaccgcca acgcagtcac tgggccttgc      60
cgtgcatcct tcccacgctg gtactttgac gtggagagga actcctgcaa taacttcatc     120
tatggaggct gccggggcaa taagaacagc taccgctctg aggaggcctg catgctccgc     180
tgcttccgcc agcagtgagg atcccc                                          206
```

<210> 33

<211> 28
<212> DNA
<213> Artificial Sequence

<220>
<223> 3' PCR primer used to amplify EST R74593.

<400> 33
cgaagcttca tctccgaagc tccagacg 28

<210> 34
<211> 31
<212> DNA
<213> Artificial Sequence

<220>
<223> 5' PCR primer used to amplify EST R74593.

<400> 34
aggatctaga caataattac ctgaccaagg a 31

<210> 35
<211> 36
<212> DNA
<213> Artificial Sequence

<220>
<223> 5' PCR primer used to amplify EST R35464.

<400> 35
ggtctagagg ccgggtcgtt tctcgcctgg ctggga 36

<210> 36
<211> 19
<212> DNA
<213> Artificial Sequence

<220>
<223> 5' PCR primer used to amplify EST R34808.

<400> 36
cacctgatcg cgagacccc 19

<210> 37
<211> 19
<212> DNA
<213> Artificial Sequence

<220>
<223> Vector specific DNA sequencing primer (SP6).

<400> 37
gatttaggtg acactatag 19

<210> 38
<211> 20
<212> DNA
<213> Artificial Sequence

<220>
<223> Vector specific DNA sequencing primer (T7).

<400> 38
taatacgact cactataggg 20

<210> 39
<211> 22
<212> DNA
<213> Artificial Sequence

<220>
<223> Gene specific DNA sequencing primer.

<400> 39
ttacctgacc aaggaggagt gc 22

<210> 40
<211> 23
<212> DNA
<213> Artificial Sequence

<220>
<223> Gene specific DNA sequencing primer.

<400> 40
aatccgctgc attcctgctg gtg 23

<210> 41
<211> 20
<212> DNA
<213> Artificial Sequence

<220>
<223> Gene specific DNA sequencing primer.

<400> 41
cagtcactgg gccttgccgt 20

<210> 42
<211> 105
<212> DNA
<213> Artificial Sequence

<220>
<223> 5' sense oligonucleotide used in Example 5.

<400> 42

gaaggggtaa gcttggataa aagatatgaa gaatactgca ccgccaacgc agtcactggg 60

ccttgccgtg catccttccc acgctggtac tttgacgtgg agagg 105

<210> 43
<211> 129
<212> DNA
<213> Artificial Sequence

<220>
<223> 3' antisense oligonucleotide used in Example 5.

<400> 43

cgcggatccc tactggcgga agcagcggag catgcaggcc tcctcagagc ggtagctgtt 60

cttattgccc cggcagcctc catagatgaa gttattgcag gagttcctct ccacgtcaaa 120

gtaccagcg 129

<210> 44
<211> 207
<212> DNA
<213> Artificial Sequence

<220>
<223> Cloned bikunin fragment in Example 5.

<400> 44

gaaggggtaa gcttggataa aagatatgaa gaatactgca ccgccaacgc agtcactggg 60

ccttgccgtg catccttccc acgctggtac tttgacgtgg agaggaactc ctgcaataac 120

ttcatctatg gaggctgccg gggcaataag aacagctacc gctctgagga ggcctgcatg 180

ctccgctgct tccgccagta gggatcc 207

<210> 45
<211> 248
<212> PRT
<213> Artificial Sequence

<220>
<223> EST derived consensus sequence of human Bikunin (Figs. 4D and 4G).

<220>
<221> SIGNAL
<222> (1)..(23)
<223>

<400> 45

Met Leu Arg Ala Glu Ala Asp Gly Val Ser Arg Leu Leu Gly Ser Leu
1 5 10 15

Leu Leu Ser Gly Val Leu Ala Ala Asp Arg Glu Arg Ser Ile His Asp
20 25 30

Phe Cys Leu Val Ser Lys Val Val Gly Arg Cys Arg Ala Ser Met Pro
35 40 45

Arg Trp Trp Tyr Asn Val Thr Asp Gly Ser Cys Gln Leu Phe Val Tyr
50 55 60

Gly Gly Cys Asp Gly Asn Ser Asn Asn Tyr Leu Thr Lys Glu Glu Cys
65 70 75 80

Leu Lys Lys Cys Ala Thr Val Thr Glu Asn Ala Thr Gly Asp Leu Ala
85 90 95

Thr Ser Arg Asn Ala Ala Asp Ser Ser Val Pro Ser Ala Pro Arg Arg
100 105 110

Gln Asp Ser Glu Asp His Ser Ser Asp Met Phe Asn Tyr Glu Glu Tyr
115 120 125

Cys Thr Ala Asn Ala Val Thr Gly Pro Cys Arg Ala Ser Phe Pro Arg
130 135 140

Trp Tyr Phe Asp Val Glu Arg Asn Ser Cys Asn Asn Phe Ile Tyr Gly
145 150 155 160

Gly Cys Arg Gly Asn Lys Asn Ser Tyr Arg Ser Glu Glu Ala Cys Met
165 170 175

Leu Arg Cys Phe Arg Gln Gln Glu Asn Pro Pro Leu Pro Leu Gly Ser
180 185 190

Lys Val Val Val Leu Ala Gly Leu Phe Val Met Val Leu Ile Leu Phe
195 200 205

Leu Gly Ala Ser Met Val Tyr Leu Ile Arg Val Ala Arg Arg Asn Gln 210 215 220 Glu Arg Ala Leu Arg Thr Val Trp Ser Ser Gly Asp Asp Lys Glu Gln 225 230 235 240 Leu Val Lys Asn Thr Tyr Val Leu 245

<210> 46
<211> 782
<212> DNA
<213> Homo sapiens

<400> 46

```
acctgatcgc gagaccccaa cggctggtgg cgtcgcctgc gcgtctcggc tgagctggcc      60
atggcgcagc tgtgcgggct gaggcggagc cgggcgtttc tcgccctgct gggatcgctg     120
ctcctctctg gggtcctggc ggccgaccga gaacgcagca tccacgactt ctgcctggtg     180
tcgaaggtgg tgggcagatg ccgggcctcc atgcctaggt ggtggtacaa tgtcactgac     240
ggatcctgcc agctgtttgt gtatgggggc tgtgacggaa acagcaataa ttacctgacc     300
aaggaggagt gcctcaagaa atgtgccact gtcacagaga atgccacggg tgacctggcc     360
accagcagga atgcagcgga ttcctctgtc ccaagtgctc ccagaaggca ggattctgaa     420
gaccactcca gcgatatgtt caactatgaa gaatactgca ccgccaacgc agtcactggg     480
ccttgccgtg catccttccc acgctggtac tttgacgtgg agaggaactc ctgcaataac     540
ttcatctatg gaggctgccg gggcaataag aacagctacc gctctgagga ggcctgcatg     600
ctccgctgct tccgccagca ggagaatcct cccctgcccc ttggctcaaa ggtggtggtt     660
ctggcggggc tgttcgtgat ggtgttgatc ctcttcctgg gagcctccat ggtctacctg     720
atccgggtgg cacggaggaa ccaggagcgt gccctgcgca ccgtctggag cttcggagat     780
ga                                                                    782
```

<210> 47
<211> 240
<212> PRT
<213> Homo sapiens

<220>
<221> SIGNAL
<222> (1)..(27)
<223>

<400> 47

Met Ala Gln Leu Cys Gly Leu Arg Arg Ser Arg Ala Phe Leu Ala Leu
1               5                   10                  15

Leu Gly Ser Leu Leu Leu Ser Gly Val Leu Ala Ala Asp Arg Glu Arg
            20                  25                  30

Ser Ile His Asp Phe Cys Leu Val Ser Lys Val Val Gly Arg Cys Arg
        35                  40                  45

Ala Ser Met Pro Arg Trp Trp Tyr Asn Val Thr Asp Gly Ser Cys Gln
    50                  55                  60

Leu Phe Val Tyr Gly Gly Cys Asp Gly Asn Ser Asn Asn Tyr Leu Thr
65                  70                  75                  80

Lys Glu Glu Cys Leu Lys Lys Cys Ala Thr Val Thr Glu Asn Ala Thr
                85                  90                  95

Gly Asp Leu Ala Thr Ser Arg Asn Ala Ala Asp Ser Ser Val Pro Ser
            100                 105                 110

Ala Pro Arg Arg Gln Asp Ser Glu Asp His Ser Ser Asp Met Phe Asn
        115                 120                 125

Tyr Glu Glu Tyr Cys Thr Ala Asn Ala Val Thr Gly Pro Cys Arg Ala
    130                 135                 140

Ser Phe Pro Arg Trp Tyr Phe Asp Val Glu Arg Asn Ser Cys Asn Asn
145                 150                 155                 160

Phe Ile Tyr Gly Gly Cys Arg Gly Asn Lys Asn Ser Tyr Arg Ser Glu
                165                 170                 175

Glu Ala Cys Met Leu Arg Cys Phe Arg Gln Gln Glu Asn Pro Pro Leu
            180                 185                 190

Pro Leu Gly Ser Lys Val Val Val Leu Ala Gly Leu Phe Val Met Val
        195                 200                 205

Leu Ile Leu Phe Leu Gly Ala Ser Met Val Tyr Leu Ile Arg Val Ala
    210                 215                 220

Arg Arg Asn Gln Glu Arg Ala Leu Arg Thr Val Trp Ser Phe Gly Asp
225                 230                 235                 240

<210> 48
<211> 1544
<212> DNA
<213> Homo sapiens

<220>
<221> misc_feature
<222> (1358)..(1358)
<223> "n" is any nucleotide.

<400> 48

gcacgagttg ggaggtgtag cgcggctctg aacgcgctga gggccgttga gtgtcgcagg 60
cggcgagggc gcgagtgagg agcagaccca ggcatcgcgc gccgagaagg ccgggcgtcc 120
ccacactgaa ggtccggaaa ggcgacttcc gggggctttg gcacctggcg gaccctcccg 180
gagcgtcggc acctgaacgc gaggcgctcc attgcgcgtg cgcgttgagg ggcttcccgc 240
acctgatcgc gagaccccaa cggctggtgg cgtcgcctgc gcgtctcggc tgagctggcc 300
atggcgcagc tgtgcgggct gaggcggagc cgggcgtttc tcgccctgct gggatcgctg 360
ctcctctctg gggtcctggc ggccgaccga gaacgcagca tccacgactt ctgcctggtg 420
tcgaaggtgg tgggcagatg ccgggcctcc atgcctaggt ggtggtacaa tgtcactgac 480
ggatcctgcc agctgtttgt gtatgggggc tgtgacggaa acagcaataa ttacctgacc 540
aaggaggagt gcctcaagaa atgtgccact gtcacagaga atgccacggg tgacctggcc 600
accagcagga atgcagcgga ttcctctgtc ccaagtgctc ccagaaggca ggattctgaa 660
gaccactcca gcgatatgtt caactatgaa gaatactgca ccgccaacgc agtcactggg 720
ccttgccgtg catccttccc acgctggtac tttgacgtgg agaggaactc ctgcaataac 780
ttcatctatg gaggctgccg gggcaataag aacagctacc gctctgagga ggcctgcatg 840
ctccgctgct tccgccagca ggagaatcct cccctgcccc ttggctcaaa ggtggtggtt 900
ctggcggggc tgttcgtgat ggtgttgatc ctcttcctgg gagcctccat ggtctacctg 960
atccgggtgg cacggaggaa ccaggagcgt gccctgcgca ccgtctggag ctccggagat 1020
gacaaggagc agctggtgaa gaacacatat gtcctgtgac cgccctgtcg ccaagaggac 1080
tggggaaggg aggggagact atgtgtgagc ttttttaaa tagagggatt gactcggatt 1140
tgagtgatca ttagggctga ggtctgtttc tctgggaggt aggacggctg cttcctggtc 1200
tggcagggat gggtttgctt tggaaatcct ctaggaggct cctcctcgca tggcctgcag 1260
tctggcagca gccccgagtt gtttcctcgc tgatcgattt ctttcctcca ggtagagttt 1320
tctttgctta tgttgaattc cattgcctcc ttttctcnat cacagaagtg atgttggaat 1380
cgtttctttt gtttgtctga tttatggttt ttttaagtat aaacaaaagt tttttattag 1440
cattctgaaa gaaggaaagt aaaatgtaca agtttaataa aaaggggcct tccccttta g 1500

aataaatttc cagcatgttg ctttcaaaaa aaaaaaaaa aaaa 1544

<210> 49
<211> 252
<212> PRT
<213> Homo sapiens

<220>
<221> SIGNAL
<222> (1)..(27)
<223>

<400> 49

```
Met Ala Gln Leu Cys Gly Leu Arg Arg Ser Arg Ala Phe Leu Ala Leu
1               5                   10                  15

Leu Gly Ser Leu Leu Leu Ser Gly Val Leu Ala Ala Asp Arg Glu Arg
            20                  25                  30

Ser Ile His Asp Phe Cys Leu Val Ser Lys Val Val Gly Arg Cys Arg
        35                  40                  45

Ala Ser Met Pro Arg Trp Trp Tyr Asn Val Thr Asp Gly Ser Cys Gln
    50                  55                  60

Leu Phe Val Tyr Gly Gly Cys Asp Gly Asn Ser Asn Asn Tyr Leu Thr
65                  70                  75                  80

Lys Glu Glu Cys Leu Lys Lys Cys Ala Thr Val Thr Glu Asn Ala Thr
                85                  90                  95

Gly Asp Leu Ala Thr Ser Arg Asn Ala Ala Asp Ser Ser Val Pro Ser
            100                 105                 110

Ala Pro Arg Arg Gln Asp Ser Glu Asp His Ser Ser Asp Met Phe Asn
        115                 120                 125

Tyr Glu Glu Tyr Cys Thr Ala Asn Ala Val Thr Gly Pro Cys Arg Ala
    130                 135                 140

Ser Phe Pro Arg Trp Tyr Phe Asp Val Glu Arg Asn Ser Cys Asn Asn
145                 150                 155                 160

Phe Ile Tyr Gly Gly Cys Arg Gly Asn Lys Asn Ser Tyr Arg Ser Glu
                165                 170                 175

Glu Ala Cys Met Leu Arg Cys Phe Arg Gln Gln Glu Asn Pro Pro Leu
```

180 185 190

Pro Leu Gly Ser Lys Val Val Val Leu Ala Gly Leu Phe Val Met Val
195 200 205

Leu Ile Leu Phe Leu Gly Ala Ser Met Val Tyr Leu Ile Arg Val Ala
210 215 220

Arg Arg Asn Gln Glu Arg Ala Leu Arg Thr Val Trp Ser Ser Gly Asp
225 230 235 240

Asp Lys Glu Gln Leu Val Lys Asn Thr Tyr Val Leu
245 250

<210> 50
<211> 146
<212> PRT
<213> Homo sapiens

<400> 50

```
Cys Leu Val Ser Lys Val Val Gly Arg Cys Arg Ala Ser Met Pro Arg
1               5                   10                  15

Trp Trp Tyr Asn Val Thr Asp Gly Ser Cys Gln Leu Phe Val Tyr Gly
            20                  25                  30

Gly Cys Asp Gly Asn Ser Asn Asn Tyr Leu Thr Lys Glu Glu Cys Leu
        35                  40                  45

Lys Lys Cys Ala Thr Val Thr Glu Asn Ala Thr Gly Asp Leu Ala Thr
    50                  55                  60

Ser Arg Asn Ala Ala Asp Ser Ser Val Pro Ser Ala Pro Arg Arg Gln
65                  70                  75                  80

Asp Ser Glu Asp His Ser Ser Asp Met Phe Asn Tyr Glu Glu Tyr Cys
                85                  90                  95

Thr Ala Asn Ala Val Thr Gly Pro Cys Arg Ala Ser Phe Pro Arg Trp
            100                 105                 110

Tyr Phe Asp Val Glu Arg Asn Ser Cys Asn Asn Phe Ile Tyr Gly Gly
        115                 120                 125

Cys Arg Gly Asn Lys Asn Ser Tyr Arg Ser Glu Glu Ala Cys Met Leu
    130                 135                 140

Arg Cys
145
```

<210> 51
<211> 1530
<212> DNA
<213> Artificial Sequence

<220>
<223> Consensus bikunin sequence of Fig. 4C.

<220>
<221> misc_feature
<222> (46)..(46)
<223> "n" is any nucleotide.

<220>
<221> misc_feature
<222> (117)..(117)
<223> "n" is any nucleotide.

<220>
<221> misc_feature
<222> (313)..(313)
<223> "n" is any nucleotide.

<400> 51

```
gcgacctccg cgcgttggga ggtgtagcgc ggctctgaac gcgtgnaggg ccgttgagtg      60
tcgcaggcgg cgagggcgcg agtgaggagc agacccaggc atcgcgcgcc gagaagncgg     120
gcgtccccac actgaaggtc cggaaaggcg acttccgggg gctttggcac ctggcggacc     180
ctcccggagc gtcggcacct gaacgcgagg cgctccattg cgcgtgcgtt tgaggggctt     240
cccgcacctg atcgcgagac cccaacggct ggtggcgtcg ctgcgcgtct cggctgagct     300
ggccatggcg cantgttgcg ggctgaggcg gacggcgttt ctcgcctgct gggatcgctg     360
ctcctctctg gggtcctggc ggccgaccga gaacgcagca tccacgactt ctgcctggtg     420
tcgaaggtgg tgggcagatg ccgggcctcc atgcctaggt ggtggtacaa tgtcactgac     480
ggatcctgcc agctgtttgt gtatggggggc tgtgacggaa acagcaataa ttacctgacc     540
aaggaggagt gcctcaagaa atgtgccact gtcacagaga atgccacggg tgacctggcc     600
accagcagga atgcagcgga ttcctctgtc ccaagtgctc ccagaaggca ggattctgaa     660
gaccactcca gcgatatgtt caactatgaa gaatactgca ccgccaacgc agtcactggg     720
ccttgccgtg catccttccc acgctggtac tttgacgtgg agaggaactc ctgcaataac     780
ttcatctatg gaggctgccg gggcaataag aacagctacc gctctgagga ggcctgcatg     840
ctccgctgct tccgccagca ggagaatcct cccctgcccc ttggctcaaa ggtggtggtt     900
ctggcggggc tgttcgtgat ggtgttgatc ctcttcctgg gagcctccat ggtctacctg     960
atccgggtgg cacggaggaa ccaggagcgt gccctgcgca ccgtctggag ctccggagat    1020
gacaaggagc agctggtgaa gaacacatat gtcctgtgac cgccctgtcg ccaagaggac    1080
tggggaaggg aggggagact atgtgtgagc tttttttaaa tagagggatt gactcggatt    1140
tgagtgatca ttagggctga ggtctgtttc tctgggaggt aggacggctg cttcctggtc    1200
tggcagggat gggtttgctt tggaaatcct ctaggaggct cctcctcgca tggcctgcag    1260
tctggcagca gccccgagtt gtttcctcgc tgatcgattt ctttcctcca ggtagagttt    1320
tctttgctta tgttgaattc cattgcctct tttctcatca cagaagtgat gttggaatcg    1380
tttcttttgt ttgtctgatt tatggttttt ttaagtataa acaaaagttt tttattagca    1440
ttctgaaaga aggaaagtaa aatgtacaag tttaataaaa aggggccttc ccctttagaa    1500
taaaaaaaaa aaaaaaaaaa aaaaaaaaaa                                     1530
```

<210> 52
<211> 170
<212> PRT
<213> Homo sapiens

<400> 52

```
Ala Asp Arg Glu Arg Ser Ile His Asp Phe Cys Leu Val Ser Lys Val
1               5                   10                  15

Val Gly Arg Cys Arg Ala Ser Met Pro Arg Trp Trp Tyr Asn Val Thr
            20                  25                  30

Asp Gly Ser Cys Gln Leu Phe Val Tyr Gly Gly Cys Asp Gly Asn Ser
        35                  40                  45

Asn Asn Tyr Leu Thr Lys Glu Glu Cys Leu Lys Lys Cys Ala Thr Val
    50                  55                  60

Thr Glu Asn Ala Thr Gly Asp Leu Ala Thr Ser Arg Asn Ala Ala Asp
65                  70                  75                  80

Ser Ser Val Pro Ser Ala Pro Arg Arg Gln Asp Ser Glu Asp His Ser
                85                  90                  95

Ser Asp Met Phe Asn Tyr Glu Glu Tyr Cys Thr Ala Asn Ala Val Thr
            100                 105                 110

Gly Pro Cys Arg Ala Ser Phe Pro Arg Trp Tyr Phe Asp Val Glu Arg
        115                 120                 125

Asn Ser Cys Asn Asn Phe Ile Tyr Gly Gly Cys Arg Gly Asn Lys Asn
    130                 135                 140

Ser Tyr Arg Ser Glu Glu Ala Cys Met Leu Arg Cys Phe Arg Gln Gln
145                 150                 155                 160

Glu Asn Pro Pro Leu Pro Leu Gly Ser Lys
                165                 170
```

<210> 53
<211> 27
<212> PRT
<213> Homo sapiens

<400> 53

Met Ala Gln Leu Cys Gly Leu Arg Arg Ser Arg Ala Phe Leu Ala Leu
1               5                   10                  15

Leu Gly Ser Leu Leu Leu Ser Gly Val Leu Ala
            20                  25

<210> 54
<211> 23
<212> PRT
<213> Homo sapiens

<400> 54

Met Leu Arg Ala Glu Ala Asp Gly Val Ser Arg Leu Leu Gly Ser Leu
1               5                   10                  15

Leu Leu Ser Gly Val Leu Ala
            20

<210> 55
<211> 102
<212> DNA
<213> Artificial Sequence

<220>
<223> 5' sense oligonucleotide used for construct #2 in Example 5.

<400> 55

gaaggggtaa gcttggataa aagagaagaa tactgtactg ctaatgctgt tactggtcca 60

tgtagagctt cttttccaag atggtacttt gatgttgaaa ga 102

<210> 56
<211> 129
<212> DNA
<213> Artificial Sequence

<220>
<223> 3' antisense oligonucleotide used for construct #2 in Example 5.

<400> 56

actggatcct cattggcgaa aacatctcaa catacaggct tcttcagatc tgtaagaatt 60

tttattacct ctacaaccac cgtaaataaa attattacaa gaatttcttt caacatcaaa 120

gtaccatct 129

<210> 57
<211> 108
<212> DNA
<213> Artificial Sequence

<220>
<223> 5' sense oligonucleotide used for construct #3 in Example 5.

<400> 57

gaaggggtaa gcttggataa aagaaattac gaagaatact gtactgctaa tgctgttact 60

ggtccatgta gagcttcttt tccaagatgg tactttgatg ttgaaaga 108

<210> 58
<211> 117
<212> DNA
<213> Artificial Sequence

<220>
<223> 5' sense oligonucleotide used for construct #4 in Example 5.

<400> 58

gaaggggtaa gcttggataa aagagatatg tttaattacg aagaatactg tactgctaat 60

gctgttactg gtccatgtag agcttctttt ccaagatggt actttgatgt tgaaaga 117

<210> 59
<211> 19
<212> DNA
<213> Artificial Sequence

<220>
<223> Sense oligonucleotide used in PCR in Example 8.

<400> 59
cacctgatcg cgagacccc 19

<210> 60
<211> 23
<212> DNA
<213> Artificial Sequence

<220>
<223> Antisense oligonucleotide used in PCR in Example 8.

<400> 60
ctggcggaag cagcggagca tgc 23

<210> 61
<211> 45
<212> DNA
<213> Artificial Sequence

<220>
<223> Oligonucleotide used in in vitro mutagenesis in Example 9.

<400> 61
cgcgtctcgg ctgacctggc cctgcagatg gcgcacgtgt gcggg 45

<210> 62
<211> 60
<212> DNA
<213> Artificial Sequence

<220>
<223> Oligonucleotide used in in vitro mutagenesis in Example 9.

<400> 62
ctgccccttg gctcaaagta ggaagatctt ccccccgggg gggtggttct ggcggggctg 60

<210> 63
<211> 14
<212> PRT
<213> Homo sapiens

<400> 63

```
Leu Arg Cys Phe Arg Gln Gln Glu Asn Pro Pro Pro Leu Gly
1               5                   10
```

<210> 64
<211> 20
<212> PRT
<213> Homo sapiens

<400> 64

```
Ala Asp Arg Glu Arg Ser Ile His Asp Phe Cys Leu Val Ser Lys Val
1               5                   10                  15

Val Gly Arg Cys
            20
```

<210> 65
<211> 20
<212> PRT
<213> Homo sapiens

<400> 65

Phe Asn Tyr Glu Glu Tyr Cys Thr Ala Asn Ala Val Thr Gly Pro Cys
1               5                   10                  15

Arg Ala Ser Phe
            20

<210> 66
<211> 11
<212> PRT
<213> Homo sapiens

<400> 66

Pro Arg Tyr Val Asp Gly Ser Gln Phe Tyr Gly
1               5                   10

<210> 67
<211> 55
<212> PRT
<213> Homo sapiens

<400> 67

Val Val Val Leu Ala Gly Leu Phe Val Met Val Leu Ile Leu Phe Leu
1               5                   10                  15

Gly Ala Ser Met Val Tyr Leu Ile Arg Val Ala Arg Arg Asn Gln Glu
            20                  25                  30

Arg Ala Leu Arg Thr Val Trp Ser Ser Gly Asp Asp Lys Glu Gln Leu
        35                  40                  45

Val Lys Asn Thr Tyr Val Leu
    50                  55

<210> 68
<211> 43
<212> PRT
<213> Homo sapiens

<400> 68

Val Val Val Leu Ala Gly Leu Phe Val Met Val Leu Ile Leu Phe Leu
1 5 10 15

Gly Ala Ser Met Val Tyr Leu Ile Arg Val Ala Arg Arg Asn Gln Glu
20 25 30

Arg Ala Leu Arg Thr Val Trp Ser Phe Gly Asp
35 40

<210> 69
<211> 55
<212> PRT
<213> Homo sapiens

<400> 69

Val Val Val Leu Ala Gly Leu Phe Val Met Val Leu Ile Leu Phe Leu
1 5 10 15

Gly Ala Ser Met Val Tyr Leu Ile Arg Val Ala Arg Arg Asn Gln Glu
20 25 30

Arg Ala Leu Arg Thr Val Trp Ser Ser Gly Asp Asp Lys Glu Gln Leu
35 40 45

Val Lys Asn Thr Tyr Val Leu
50 55

<210> 70
<211> 213
<212> PRT
<213> Homo sapiens

<400> 70

Ala Asp Arg Glu Arg Ser Ile His Asp Phe Cys Leu Val Ser Lys Val
1 5 10 15

Val Gly Arg Cys Arg Ala Ser Met Pro Arg Trp Trp Tyr Asn Val Thr
20 25 30

Asp Gly Ser Cys Gln Leu Phe Val Tyr Gly Gly Cys Asp Gly Asn Ser
35 40 45

Asn Asn Tyr Leu Thr Lys Glu Glu Cys Leu Lys Lys Cys Ala Thr Val
50 55 60

Thr Glu Asn Ala Thr Gly Asp Leu Ala Thr Ser Arg Asn Ala Ala Asp
65 70 75 80

Ser Ser Val Pro Ser Ala Pro Arg Arg Gln Asp Ser Glu Asp His Ser
85 90 95

Ser Asp Met Phe Asn Tyr Glu Glu Tyr Cys Thr Ala Asn Ala Val Thr
100 105 110

Gly Pro Cys Arg Ala Ser Phe Pro Arg Trp Tyr Phe Asp Val Glu Arg
115 120 125

Asn Ser Cys Asn Asn Phe Ile Tyr Gly Gly Cys Arg Gly Asn Lys Asn
130 135 140

Ser Tyr Arg Ser Glu Glu Ala Cys Met Leu Arg Cys Phe Arg Gln Gln
145 150 155 160

Glu Asn Pro Pro Leu Pro Leu Gly Ser Lys Val Val Val Leu Ala Gly
165 170 175

Leu Phe Val Met Val Leu Ile Leu Phe Leu Gly Ala Ser Met Val Tyr
180 185 190

Leu Ile Arg Val Ala Arg Arg Asn Gln Glu Arg Ala Leu Arg Thr Val
195 200 205

Trp Ser Phe Gly Asp
210

<210> 71

<211> 225
<212> PRT
<213> Homo sapiens

<400> 71

```
Ala Asp Arg Glu Arg Ser Ile His Asp Phe Cys Leu Val Ser Lys Val
1               5                   10                  15

Val Gly Arg Cys Arg Ala Ser Met Pro Arg Trp Trp Tyr Asn Val Thr
            20                  25                  30

Asp Gly Ser Cys Gln Leu Phe Val Tyr Gly Gly Cys Asp Gly Asn Ser
        35                  40                  45

Asn Asn Tyr Leu Thr Lys Glu Glu Cys Leu Lys Lys Cys Ala Thr Val
    50                  55                  60

Thr Glu Asn Ala Thr Gly Asp Leu Ala Thr Ser Arg Asn Ala Ala Asp
65                  70                  75                  80

Ser Ser Val Pro Ser Ala Pro Arg Arg Gln Asp Ser Glu Asp His Ser
                85                  90                  95

Ser Asp Met Phe Asn Tyr Glu Glu Tyr Cys Thr Ala Asn Ala Val Thr
            100                 105                 110

Gly Pro Cys Arg Ala Ser Phe Pro Arg Trp Tyr Phe Asp Val Glu Arg
```

115 120 125

Asn Ser Cys Asn Asn Phe Ile Tyr Gly Gly Cys Arg Gly Asn Lys Asn
130 135 140

Ser Tyr Arg Ser Glu Glu Ala Cys Met Leu Arg Cys Phe Arg Gln Gln
145 150 155 160

Glu Asn Pro Pro Leu Pro Leu Gly Ser Lys Val Val Val Leu Ala Gly
165 170 175

Leu Phe Val Met Val Leu Ile Leu Phe Leu Gly Ala Ser Met Val Tyr
180 185 190

Leu Ile Arg Val Ala Arg Arg Asn Gln Glu Arg Ala Leu Arg Thr Val
195 200 205

Trp Ser Ser Gly Asp Asp Lys Glu Gln Leu Val Lys Asn Thr Tyr Val
210 215 220

Leu
225

<210> 72
<211> 19
<212> PRT
<213> Homo sapiens

<220>
<221> MISC_FEATURE
<222> (9)..(9)
<223> "Xaa" is Ile, Thr, Asn, or Ser.

<220>
<221> MISC_FEATURE
<222> (11)..(11)
<223> "Xaa" is Val, Ala, Glu, or Gly.

<220>
<221> MISC_FEATURE
<222> (17)..(17)
<223> "Xaa" is Ser, Pro, Thr, or Ala.

<220>
<221> MISC_FEATURE
<222> (19)..(19)
<223> "Xaa" is Tyr, His, Asn, or Asp.

<400> 72

Arg Pro Leu Gln Arg Tyr Val Ser Xaa Ile Xaa Arg Ile Ile Ala Pro
1 5 10 15

Xaa Thr Xaa

<210> 73
<211> 108
<212> PRT
<213> Homo sapiens

<400> 73

Pro Gly His Gln Gln Glu Cys Ser Gly Phe Leu Cys Pro Lys Ser Pro
1 5 10 15

Arg Arg Gln Asp Ser Glu Asp His Ser Ser Asp Met Phe Asn Tyr Glu
20 25 30

Glu Tyr Cys Thr Ala Asn Ala Val Thr Gly Pro Cys Arg Ala Ser Phe
35 40 45

Pro Arg Trp Tyr Phe Asp Val Glu Arg Asn Ser Cys Asn Asn Phe Ile
50 55 60

Tyr Gly Gly Cys Arg Gly Asn Lys Asn Ser Tyr Arg Ser Glu Glu Ala
65 70 75 80

Cys Met Leu Arg Cys Phe Arg Gln Gln Glu Asn Pro Pro Leu Pro Leu
85 90 95

Gly Ser Lys Val Val Val Leu Ala Gly Ala Val Ser
100 105

<210> 74
<211> 31
<212> PRT
<213> Homo sapiens

<220>
<221> MISC_FEATURE
<222> (25)..(25)
<223> "Xaa" is Asp or Glu.

<400> 74

Ser Phe Ser Trp Gly Ala Ser Met Val Leu Leu Ile Pro Gly Gly Lys
1 5 10 15

Glu Glu Pro Gly Ala Cys Pro Ala Xaa Arg Leu Glu Leu Arg Arg
20 25 30

<210> 75
<211> 511
<212> DNA
<213> Artificial Sequence

<220>
<223> Corrected version of EST R74593 (Fig. 3).

<220>
<221> misc_feature
<222> (425)..(425)
<223> "n" is any nucleotide.

<220>
<221> misc_feature
<222> (482)..(482)
<223> "n" is any nucleotide.

<220>
<221> misc_feature
<222> (510)..(510)
<223> "n" is any nucleotide.

<400> 75

gcaataatta cctgaccaag gaggagtgcc tcaagaaatg tgccactgtc acagagaatg 60

ccacgggtga cctggccacc agcaggaatg cagcggattc ctctgtccca agtgctccca 120

gaaggcagga ttctgaagac cactccagcg atatgttcaa ctatgaagaa tactgcaccg 180

ccaacgcagt cactgggcct tgccgtgcat ccttcccacg ctggtacttt gacgtggaga 240

ggaactcctg caataacttc atctatggag gctgccgggg caataagaac agctaccgct 300

ctgaggaggc ctgcatgctc cgctgcttcc gccagcagga gaatcctccc ctgccccttg 360

gctcaaaggt ggtggttctg gccggggctg tttcgtgatg gtgttgatcc ttttcctggg 420

gagcntccat ggtcttactg attccgggtg gcaaggagga accaggagcg tgccctgcgg 480

ancgtctgga gcttcggaga tgacaagggn t 511

<210> 76
<211> 31
<212> PRT
<213> Artificial Sequence

<220>
<223> Amino acids 184-214 of the translation of the consensus DNA sequence in Fig. 3.

<220>
<221> MISC_FEATURE
<222> (25)..(25)
<223> "Xaa" is Asp or Glu.

<400> 76

```
Ser Phe Ser Trp Gly Ala Ser Met Val Leu Leu Ile Pro Gly Gly Lys
1               5                   10                  15

Glu Glu Pro Gly Ala Cys Pro Ala Xaa Arg Leu Glu Leu Arg Arg
            20                  25                  30
```

<210> 77
<211> 312
<212> DNA
<213> Homo sapiens

<220>
<221> misc_feature
<222> (45)..(45)
<223> "n" is any nucleotide.

<220>
<221> misc_feature
<222> (49)..(49)
<223> "n" is any nucleotide.

<220>
<221> misc_feature
<222> (118)..(118)
<223> "n" is any nucleotide.

<220>
<221> misc_feature
<222> (231)..(231)
<223> "n" is any nucleotide.

<220>
<221> misc_feature
<222> (305)..(305)
<223> "n" is any nucleotide.

<400> 77

gcgacctccg cgcgttggga ggtgtagcgc ggctctgaac gcgtngagng gccgttgagt 60

gtcgcaggcg gcgagggcgc gagtgaggag cagacccagg catcgcgcgc cgagaagncg 120

ggcgtcccca cactgaaggt ccggaaaggc gacttccggg ggctttggca cctggcggac 180

cctcccggag cgtcggcacc tgaacgcgag gcgctccatt gcgcgtgcgt ntgaggggct 240

tcccgcacct gatcgcgaga ccccaacggc tggtggcgtc gcctgcgcgt ctcggctgag 300

ctggncatgt cg 312

<210> 78
<211> 330
<212> DNA
<213> Homo sapiens

<220>
<221> misc_feature
<222> (117)..(117)
<223> "n" is any nucleotide.

<220>
<221> misc_feature
<222> (123)..(123)
<223> "n" is any nucleotide.

<220>
<221> misc_feature
<222> (321)..(321)
<223> "n" is any nucleotide.

<400> 78

gcgacctccg cgcgttggga ggtgtagcgc ggctctgaac gcgtgcaggg ccgttgagtg 60

tcgcaggcgg cgagggcgcg agtgaggagc agacccaggc atcgcgcgcc gagaagncgg 120

gcntccccac actgaaggtc cggaaaggcg acttccgggg gctttggcac ctggcggacc 180

ctcccggagc gtggcacctg aacgcgaggc gctccattgc gcgtgcgttt gaggggcttc 240

ccgcacctga tcgcgagacc ccaacggctg gtggcgtcgc ctgcgcgtct cggctgagct 300

ggccatggcg cactgtgcgg ngctgaggcg 330

<210> 79
<211> 283
<212> DNA
<213> Homo sapiens

<220>
<221> misc_feature
<222> (9)..(9)
<223> "n" is any nucleotide.

<220>
<221> misc_feature
<222> (11)..(11)
<223> "n" is any nucleotide.

<220>
<221> misc_feature
<222> (222)..(222)
<223> "n" is any nucleotide.

<220>
<221> misc_feature
<222> (231)..(231)
<223> "n" is any nucleotide.

<220>
<221> misc_feature
<222> (262)..(262)
<223> "n" is any nucleotide.

<220>
<221> misc_feature
<222> (267)..(267)
<223> "n" is any nucleotide.

<220>
<221> misc_feature
<222> (274)..(274)
<223> "n" is any nucleotide.

<400> 79

ttgagtgtng naggcggcga gggcgcgagt gaggagcaga cccaggcatc gcgcgccgag 60

aaggccgggc gtccccacac tgaaggtccg gaaaggcgac ttccgggggc tttggcacct 120

ggcggaccct cccggagcgt cggcacctga acgcgaggcg ctccattgcg cgtgcgtttg 180

aggggcttcc cgcacctgat cgcgagaccc caacggctgg tngcgtcgct ncgcgtctcg 240

gctgagcttg gccatggcgc antgttncgg gctnaggcgg acg 283

<210> 80
<211> 423
<212> DNA
<213> Homo sapiens

<220>
<221> misc_feature
<222> (44)..(44)
<223> "n" is any nucleotide.

<220>
<221> misc_feature
<222> (46)..(46)
<223> "n" is any nucleotide.

<220>
<221> misc_feature
<222> (76)..(76)
<223> "n" is any nucleotide.

<220>
<221> misc_feature
<222> (114)..(114)
<223> "n" is any nucleotide.

<220>
<221> misc_feature
<222> (187)..(187)
<223> "n" is any nucleotide.

<220>
<221> misc_feature
<222> (268)..(268)
<223> "n" is any nucleotide.

<220>
<221> misc_feature
<222> (309)..(309)
<223> "n" is any nucleotide.

<220>
<221> misc_feature
<222> (317)..(317)
<223> "n" is any nucleotide.

<220>
<221> misc_feature
<222> (332)..(332)
<223> "n" is any nucleotide.

<220>
<221> misc_feature
<222> (370)..(370)
<223> "n" is any nucleotide.

<400> 80

```
ggcgacctcc gcgcgttggg aggtgtagcg cgctctgaac gggnangggc cgttgagtgt     60
cgcaggcggc agggcngagt gaggagcaga cccaggcatc gcgcgccgag aagncgggcg    120
tccccacact gaaggtccgg aaaggcgact tccgggggct ttggcacctg gcggacgtcc    180
cggagcnggc acctgaacgc gaggcgctcc attgcgcgtg cgtttgaggg gcttcccgca    240
cctgatcgcg agaccccaac ggctggtngc gtcgctggcg cgttctcggc tgagctggcc    300
atggcgcant gttgcgngct gaggcggacc gncgtttttc ttcgccttgc tgggattcgc    360
ttgcttcctn tctgggggtt cctgggcggc cgaccgagaa cgcagcatcc aagaattttt    420
gcc                                                                  423
```

<210> 81
<211> 344
<212> DNA
<213> Homo sapiens

<220>
<221> misc_feature
<222> (35)..(35)
<223> "n" is any nucleotide.

<220>
<221> misc_feature
<222> (148)..(148)
<223> "n" is any nucleotide.

<220>
<221> misc_feature
<222> (235)..(235)
<223> "n" is any nucleotide.

<220>
<221> misc_feature
<222> (261)..(261)
<223> "n" is any nucleotide.

<220>
<221> misc_feature
<222> (272)..(272)
<223> "n" is any nucleotide.

<220>
<221> misc_feature
<222> (293)..(293)
<223> "n" is any nucleotide.

<220>
<221> misc_feature
<222> (300)..(300)
<223> "n" is any nucleotide.

<220>
<221> misc_feature
<222> (313)..(313)
<223> "n" is any nucleotide.

<220>
<221> misc_feature
<222> (320)..(320)
<223> "n" is any nucleotide.

<400> 81

ggaggagcag acccaggcat cgcgcgccga gaagncgggc gtccccacac tgaaggtccg 60

gaaaggcgac ttccgggggc tttggcacct ggcggaccct cccggagcgt cggcacctga 120

acgcgaggcg ctccattgcg cgtgcgtntg gaggggcttc ccgcacctga tcgcgagacc 180

ccaacggctg gtgggcgtcg ctgcgcgtct tcggctgagc tgggccatgg cgcanttgtt 240

gcgggctgag gcggacgcgg ncgtttttc gnccttgctg ggattcgttg ttnctctctn 300

ggggttctgg ggnggccgan cgagaacgca agcattcacg attt 344

<210> 82
<211> 253
<212> DNA
<213> Homo sapiens

<220>
<221> misc_feature
<222> (56)..(56)
<223> "n" is any nucleotide.

<220>
<221> misc_feature
<222> (137)..(137)
<223> "n" is any nucleotide.

<220>
<221> misc_feature
<222> (145)..(145)
<223> "n" is any nucleotide.

<220>
<221> misc_feature
<222> (159)..(159)
<223> "n" is any nucleotide.

<220>
<221> misc_feature
<222> (233)..(233)
<223> "n" is any nucleotide.

<400> 82

ggaccctccc ggagcgtcgg cacctgaacg cgaggcctcc attgcggtgc gtgtgnaggg 60

gcttcccgca cctgatcgcg agaccccaac ggctggtggc gtcgctgcgc gtctcggctg 120

agctggccat ggcgcantgt tgcgngctga ggcggcggnc gttttctcgc ctgctgggat 180

cgctgctcct ctctggggtc ctggcggccg accgagaacg cagcatccac ganttcttcc 240

tggtgttcga agg 253

<210> 83
<211> 419
<212> DNA
<213> Homo sapiens

<220>

<221> misc_feature
<222> (20)..(20)
<223> "n" is any nucleotide.

<220>
<221> misc_feature
<222> (26)..(26)
<223> "n" is any nucleotide.

<220>
<221> misc_feature
<222> (95)..(95)
<223> "n" is any nucleotide.

<220>
<221> misc_feature
<222> (292)..(292)
<223> "n" is any nucleotide.

<220>
<221> misc_feature
<222> (313)..(315)
<223> "n" is any nucleotide.

<400> 83

ttagcgcggc tctgaacgcn agaagnggcc gttgagtgtc gcaggcggcg agggcgcgag 60

tgaggagcag acccaggcat cgcgcgccga gaagncgggc gtccccacac tgaaggtccg 120

gaaaggcgac ttccgggggc tttggcacct ggcggaccct cccggagcgt cggcacctga 180

acgcgaggcg ctccattgcg cgtgcgtttg aggggcttcc cgcacctgat cgcgagaccc 240

caacggctgg tggcgtcgcc tgcgcgtctc ggctgagctg gccatggcgc antggtgcgg 300

gcttgaggcg gannngccgt ttctcgcctg ctgggatcgc tgctcctctc tggggtcctg 360

gcggccgacc gagaacgcag catccacgac ttctgcctgg tgtcgaaggt ggtgggcag 419

<210> 84
<211> 477
<212> DNA
<213> Homo sapiens

<220>
<221> misc_feature
<222> (27)..(27)
<223> "n" is any nucleotide.

<220>
<221> misc_feature
<222> (139)..(139)
<223> "n" is any nucleotide.

<220>
<221> misc_feature

<222> (223)..(223)
<223> "n" is any nucleotide.

<220>
<221> misc_feature
<222> (232)..(232)
<223> "n" is any nucleotide.

<220>
<221> misc_feature
<222> (302)..(302)
<223> "n" is any nucleotide.

<220>
<221> misc_feature
<222> (310)..(310)
<223> "n" is any nucleotide.

<220>
<221> misc_feature
<222> (322)..(322)
<223> "n" is any nucleotide.

<220>
<221> misc_feature
<222> (328)..(328)
<223> "n" is any nucleotide.

<220>
<221> misc_feature
<222> (357)..(357)
<223> "n" is any nucleotide.

<220>
<221> misc_feature
<222> (375)..(375)
<223> "n" is any nucleotide.

<220>
<221> misc_feature
<222> (392)..(392)
<223> "n" is any nucleotide.

<220>
<221> misc_feature
<222> (398)..(398)
<223> "n" is any nucleotide.

<220>
<221> misc_feature
<222> (405)..(405)
<223> "n" is any nucleotide.

<220>
<221> misc_feature
<222> (427)..(427)
<223> "n" is any nucleotide.

<220>
<221> misc_feature
<222> (437)..(437)
<223> "n" is any nucleotide.

<220>
<221> misc_feature
<222> (449)..(449)
<223> "n" is any nucleotide.

<220>
<221> misc_feature
<222> (458)..(458)
<223> "n" is any nucleotide.

<220>
<221> misc_feature
<222> (474)..(474)
<223> "n" is any nucleotide.

<400> 84

```
agacccaggc atcgcgcgcc gagaagncgg gcgtccccac actgaaggtc cggaaaggcg     60
acttccgggg gctttggcac ctggcggacc ctcccggagc gtcggcacct gaacgcgagg    120
cctccattgc cgtgcgttng aggggcttcc cggaacttga tcgcgagacc ccaacggctg    180
gtggcgtcgc tgcgcgtcct cggctgagct ggccatggcg cantggtgcc gngctgaggc    240
cggagggccg gtttctcgcc ttgctgggat cgctgctcct ctctggggtc ctggcggccg    300
ancgaagaan gcagcaatcc angaattnct gcctggtgtt cgaaagttgg tgggcanatt    360
ccggggcctt catgnctaag gttggttggt anaatgtnaa ttaangattc ttgcaactgt    420
ttgtgtnatt ggggctntta aacggaaana caataatnac ctgaccaaag aagnaat       477
```

<210> 85
<211> 393
<212> DNA
<213> Homo sapiens

<220>
<221> misc_feature
<222> (361)..(361)
<223> "n" is any nucleotide.

<220>
<221> misc_feature
<222> (367)..(367)
<223> "n" is any nucleotide.

<220>
<221> misc_feature
<222> (384)..(384)
<223> "n" is any nucleotide.

<220>
<221> misc_feature
<222> (390)..(390)
<223> "n" is any nucleotide.

<400> 85

```
ggccgggtcg tttctcgcct ggctgggatc gctgctcctc tctggggtcc tggccggccg      60
accgagaacg cagcatccac gacttctgcc tggtgtcgaa ggtggtgggc agattccggg     120
cctccatgcc taggtggtgg tacaatgtca ctgacggatc ctgccagctg tttgtgtatg     180
ggggctgtga cggaaacagc aataattacc tgaccaagga ggagtgcctc aagaaatgtg     240
ccactgtcac agagaatgcc acgggtgacc tggccaccag caggaatgca gcggattcct     300
ctgtcccaag tgctcccaga aggcaggatt cttgaagacc acttcagcga tatgtttcaa     360
ntattgnaag aataattgca ccgncaacgn att                                  393
```

<210> 86
<211> 428
<212> DNA
<213> Homo sapiens

<220>
<221> misc_feature
<222> (3)..(3)
<223> "n" is any nucleotide.

<220>
<221> misc_feature
<222> (11)..(12)
<223> "n" is any nucleotide.

<220>
<221> misc_feature
<222> (17)..(17)
<223> "n" is any nucleotide.

<220>
<221> misc_feature
<222> (48)..(48)
<223> "n" is any nucleotide.

<220>
<221> misc_feature
<222> (425)..(425)
<223> "n" is any nucleotide.

<400> 86

gcngcgcgtt nntcgcntgc tgggatcgct gcacctctct ggggtcgngg cggccgaccg 60
agaacgcagc atccacgact tctgcctggt gtcgaaggtg gtgggcagat gccgggcctc 120
catgcctagg tggtggtaca atgtcactga cggatcctgc cagctgtttg tgtatggggg 180
ctgtgacgga aacagcaata attacctgac caaggaggag tgcctcaaga aatgtgccac 240
tgtcacagag aatgccacgg gtgacctggc caccagcagg aatgcagcgg attcctctgt 300
cccaagtgct cccagaaggc aggattctga agaccactcc agcgatatgt tcaactatga 360
agaatactgg caccgccaac gcattcactg ggcctgcgtg catccttccc acgctggtac 420
tttgncgt 428

<210> 87
<211> 425
<212> DNA
<213> Homo sapiens

<220>
<221> misc_feature
<222> (7)..(7)
<223> "n" is any nucleotide.

<220>
<221> misc_feature
<222> (403)..(403)
<223> "n" is any nucleotide.

<220>
<221> misc_feature
<222> (409)..(409)
<223> "n" is any nucleotide.

<400> 87

ctgggantcg ctgctcctct ctggggtcct ggcggccgac cgagaacgca gcatccacga 60
cttctgcctg gtgtcgaagg tggtgggcag atgccgggcc tccatgccta ggtggtggta 120
caatgtcact gacggatcct gccagctgtt tgtgtatggg ggctgtgacg gaaacagcaa 180

taattacctg accaaggagg agtgcctcaa gaaatgtgcc actgtcacag agaatgccac 240
gggtgacctg gccaccagca ggaatgcagc ggattcctct gtcccaagtg ctcccagaag 300
gcaggattct gaagaccact ccagcgatat gttcaactat gaagaatact gcaccgccaa 360
cgcagtcact ggggccttgc gtggaatcct ttcccacgct ggnaatttng acgttgagaa 420
ggaac 425

<210> 88
<211> 343
<212> DNA
<213> Homo sapiens

<220>
<221> misc_feature
<222> (48)..(48)
<223> "n" is any nucleotide.

<220>
<221> misc_feature
<222> (62)..(62)
<223> "n" is any nucleotide.

<220>
<221> misc_feature
<222> (211)..(211)
<223> "n" is any nucleotide.

<220>
<221> misc_feature
<222> (232)..(232)
<223> "n" is any nucleotide.

<220>
<221> misc_feature
<222> (245)..(245)
<223> "n" is any nucleotide.

<220>
<221> misc_feature
<222> (309)..(309)
<223> "n" is any nucleotide.

<220>
<221> misc_feature
<222> (318)..(318)
<223> "n" is any nucleotide.

<400> 88

gattcggcac aggggaaaca gcaataatta cctgaccaag gaggagtncc tcaagaaatg 60

tnccactgtc acagagaatg ccacgggtga cctggccacc agcaggaatg cagcggattc 120

ctctgtccca agtgctccca gaaggcagga ttctgaagac cactccagcg atatgttcaa 180

ctatgaagaa tactgcaccg ccaacgcagt ncactgggcc ttgcgtggca tnccttccca 240

cgctngtact ttgacgtgga gaggaactcc tggcaataac ttcatctatg gaggcttgcc 300

ggggcaatna agaacagntt accgctcttt aggaggcctg cat 343

<210> 89

<211> 510
<212> DNA
<213> Homo sapiens

<220>
<221> misc_feature
<222> (424)..(424)
<223> "n" is any nucleotide.

<220>
<221> misc_feature
<222> (481)..(481)
<223> "n" is any nucleotide.

<220>
<221> misc_feature
<222> (509)..(509)
<223> "n" is any nucleotide.

<400> 89

gcaataatta cctgaccaag gaggagtgcc tcaagaaatg tgccactgtc acagagaatg 60

ccacgggtga cctggccacc agcaggaatg cagcggattc ctctgtccca agtctcccag 120

aaggcaggat tctgaagacc actccagcga tatgttcaac tatgaagaat actgcaccgc 180

caacgcagtc actgggcctt gccgtgcatc cttcccacgc tggtactttg acgtggagag 240

gaactcctgc aataacttca tctatggagg ctgccggggc aataagaaca gctaccgctc 300

tgaggaggcc tgcatgctcc gctgcttccg ccagcaggag aatcctcccc tgccccttgg 360

ctcaaaggtg gtggttctgg ccggggctgt ttcgtgatgg tgttgatcct tttcctgggg 420

agcntccatg gtcttactga ttccgggtgg caaggaggaa ccaggagcgt gccctgcgga 480

ncgtctggag cttcggagat gacaagggnt 510

<210> 90
<211> 293
<212> DNA
<213> Homo sapiens

<220>
<221> misc_feature
<222> (257)..(257)
<223> "n" is any nucleotide.

<400> 90

gctaccgctc tgaggaggcc tgcatgctcc gctgcttccg ccagcaggag aatcctcccc 60

tgccccttgg ctcaaaggtg gtggttctgg cggggctgtt cgtgatggtg ttgatcctct 120

tcctggggag cctccatggt ctacctgatc cgggtggcac ggagggaacc agggagcgtg 180

ccctgcgcac cgtctgggag ctccggagat gacaagggag cagctgggtg aagaacacat 240

atgttcctgt tgaccgncct gttcgccaag aggattgggg gaagggaggg gga 293

<210> 91
<211> 282
<212> DNA
<213> Homo sapiens

<220>
<221> misc_feature
<222> (19)..(19)
<223> "n" is any nucleotide.

<220>
<221> misc_feature
<222> (147)..(147)
<223> "n" is any nucleotide.

<400> 91

ttccgccaag caggaaaant cctcccctcc cccttggctc aaaggtggtg gttcctggcg 60

gggctgttcg tgatggtgtt gatccctcct tcccgggagc ctcccatggt cctaccctga 120

tccgggtggc acggaggaac ccaggancgt gccctgcgca ccgtctggag ctccggagat 180

gacaaggagc agctggtgaa gaacacatat gtcctgtgac cgccctgtcg ccaagaggac 240

tggggaaggg aggggagact atgtgtgagc ttttttaaa ta 282

<210> 92
<211> 390
<212> DNA
<213> Homo sapiens

<220>
<221> misc_feature
<222> (33)..(33)
<223> "n" is any nucleotide.

<220>
<221> misc_feature
<222> (55)..(55)
<223> "n" is any nucleotide.

<220>
<221> misc_feature
<222> (118)..(118)
<223> "n" is any nucleotide.

<220>
<221> misc_feature
<222> (213)..(213)
<223> "n" is any nucleotide.

<220>
<221> misc_feature
<222> (228)..(228)
<223> "n" is any nucleotide.

<220>
<221> misc_feature
<222> (259)..(259)
<223> "n" is any nucleotide.

<220>
<221> misc_feature
<222> (267)..(267)
<223> "n" is any nucleotide.

<220>
<221> misc_feature
<222> (324)..(324)
<223> "n" is any nucleotide.

<220>
<221> misc_feature
<222> (333)..(333)
<223> "n" is any nucleotide.

<220>
<221> misc_feature
<222> (344)..(344)
<223> "n" is any nucleotide.

<220>
<221> misc_feature
<222> (387)..(387)
<223> "n" is any nucleotide.

<400> 92

gagaggaact cctgcaataa cttcatctat ggnggctgcc ggggaataag aacanctacc 60

gctctgagga ggcctgcgtg ctccgctgct tccgctgtgt gttctcttcc aggccagcag 120

gagaatcctc ccctgcccct tggctcaaag gtggtggttc tggcggggct gttcgtgatg 180

gtgttgatcc tcttcctggg agcctccatg gtntacctga tccgggtngc acggaggaac 240

cagggagcgt gccctgcgna ccgtctngga gctccggaga tgacaaggag cagctggtga 300

agaacacata tgtcctgtga ccgncctgtt cgncaagagg actnggggaa aggggagggg 360

agattatgtg ttgagttttt tttaaantag 390

<210> 93
<211> 406
<212> DNA
<213> Homo sapiens

<220>
<221> misc_feature
<222> (306)..(306)
<223> "n" is any nucleotide.

<220>
<221> misc_feature
<222> (328)..(328)
<223> "n" is any nucleotide.

<220>
<221> misc_feature
<222> (342)..(342)
<223> "n" is any nucleotide.

<220>
<221> misc_feature
<222> (365)..(365)
<223> "n" is any nucleotide.

<220>
<221> misc_feature
<222> (370)..(370)
<223> "n" is any nucleotide.

<220>
<221> misc_feature
<222> (377)..(377)
<223> "n" is any nucleotide.

<220>
<221> misc_feature
<222> (382)..(382)
<223> "n" is any nucleotide.

<220>
<221> misc_feature
<222> (402)..(402)
<223> "n" is any nucleotide.

<400> 93

```
gattcggaac gaggagccgg ggcaataaga acagctaccg ctctgaggag gcctgcatgc      60
tccgctgctt ccgccagcag gagaatcctc ccctgcccct tggctcaaag gtggtggttc     120
tggcggggct gttcgtgatg gtgttgatcc tcttcctggg agcctccatg gtctacctga     180
tccgggtggc acggaggaac cagggagcgt gccctgcgca ccgtctggga gctccggaga     240
tgacaaggga gcagctggtg aagaacacat atgttcctgt tgaccgccct gttcgccaag     300
agggantggg ggaaggggag ggggaganta ttgttgttga gntttttttt aaaattagga     360
ggggnttgan ttcgggnttt tnagttgatc catttagggg gntgag                    406
```

<210> 94
<211> 360
<212> DNA
<213> Homo sapiens

<220>
<221> misc_feature
<222> (1)..(1)
<223> "n" is any nucleotide.

<220>
<221> misc_feature
<222> (142)..(142)
<223> "n" is any nucleotide.

<220>
<221> misc_feature
<222> (339)..(339)
<223> "n" is any nucleotide.

<220>
<221> misc_feature
<222> (347)..(347)
<223> "n" is any nucleotide.

<400> 94

```
nggccttgca gtgctccgct gcttccgcca gcaggagaat cctcccctgc cccttggctc      60
aaaggtggtg gttctggcgg ggctgttcgt gatggtgttg atcctcttcc tgggagcctc     120
catggtctac ctgatccggg tngcacggag gaaccaggag cgtgccctgc gcaccgtctg     180
gagctccgga gatgacaagg agcagctggt gaagaacaca tatgtcctgt gaccgccctg     240
tcgccaagag gactggggaa gggaggggag actatgtgtg agcttttttt aaatagaggg     300
attgactcgg atttgagtga tcattagggc tgaggtctnt ttctctngga ggtaggacga     360
```

<210> 95
<211> 438
<212> DNA
<213> Homo sapiens

<220>
<221> misc_feature
<222> (334)..(334)
<223> "n" is any nucleotide.

<220>
<221> misc_feature
<222> (368)..(368)
<223> "n" is any nucleotide.

<220>
<221> misc_feature
<222> (376)..(376)
<223> "n" is any nucleotide.

<400> 95

cggggctgtt cgtgatggtg ttgatcctct tcctgggagc ctccatggtc tacctgatcc 60

gggtggcacg gaggaaccag gagcgtgccc tgcgcaccgt ctggagctcc ggagatgaca 120

aggagcagct ggtgaagaac acatatgtcc tgtgaccgcc ctgtcgccaa gaggactggg 180

gaagggaggg gagactatgt gtgagctttt tttaaataga gggattgact cggatttgag 240

tgatcattag ggctgaggtc tgtttctctg ggaggtagga cggctgcttc ctgggtcttg 300

gcagggatgg ggtttgcttt gggaaatcct cttnggaggc tcctccttcg catgggcctt 360

gcagtctngg cagcancccc cgagtttttt tccttcgctg atccgatttc ttttcctcca 420

ggtaagaatt tttctttt 438

<210> 96
<211> 448
<212> DNA
<213> Homo sapiens

<220>
<221> misc_feature
<222> (108)..(108)
<223> "n" is any nucleotide.

<220>
<221> misc_feature
<222> (261)..(261)
<223> "n" is any nucleotide.

<400> 96

gggaaccagg agcgtgccct gcgcaccggt ctggagctcc ggagatgaca aggagcagct 60

ggtgaagaac acatatgtcc tgtgaccgcc ctgtcgccaa gaggactngg gaagggaggg 120
gagactatgt gtgagctttt tttaaataga gggattgact cggatttgag tgatcattag 180
ggctgaggtc tgtttctctg ggaggtagga cggctgcttc ctggtctggc agggatgggt 240
ttgctttgga gaatcctcta ngaggctcct cctcgcatgg cctgcagtct ggcagcagcc 300
ccgagttgtt tcctcgctga tcgatttctt tcctccaggt agagttttct ttgcttatgt 360
tgaattccat tgcctctttt ctcatcacag aagtgatgtt ggaatcgttt cttttgtttt 420
gtctgattta tgggtttttt ttaagtat 448

<210> 97
<211> 331
<212> DNA
<213> Homo sapiens

<220>
<221> misc_feature
<222> (20)..(20)
<223> "n" is any nucleotide.

<220>
<221> misc_feature
<222> (30)..(30)
<223> "n" is any nucleotide.

<400> 97

attagggctg aggtctgttn ctctgggagn taggacggct gccttcctgg tctggcaggg 60
atgggtttgc tttggaaatc ctctaggagg ctcctcctcg catggcctgc agttctgcag 120
cagccccgag ttgtttcctc gctgatcgat ttctttcctc caggtagagt ttctttgct 180
tatgttgaat tccattgcct cttttctcat cacagaagtg atgttggaat cgtttctttt 240
gtttgtctga tttatggttt ttttaagtat aaacaaaagt tttttattag cattctgaaa 300
gaaggaaagt aaaatgtaca agtttaataa a 331

<210> 98
<211> 373
<212> DNA
<213> Homo sapiens

<220>
<221> misc_feature
<222> (45)..(45)
<223> "n" is any nucleotide.

<220>
<221> misc_feature

<222> (102)..(102)
<223> "n" is any nucleotide.

<220>
<221> misc_feature
<222> (105)..(105)
<223> "n" is any nucleotide.

<220>
<221> misc_feature
<222> (159)..(159)
<223> "n" is any nucleotide.

<220>
<221> misc_feature
<222> (174)..(174)
<223> "n" is any nucleotide.

<220>
<221> misc_feature
<222> (213)..(213)
<223> "n" is any nucleotide.

<220>
<221> misc_feature
<222> (337)..(337)
<223> "n" is any nucleotide.

<400> 98

```
gattgactcg gatttgagtg atcattaggg ctgaggtctg tttcnctggg aggtaggacg     60
gctgctcccc tggtctggca gggatgggtt tgctttggaa anccnctagg aggctcctcc    120
tcgcatggcc tgcagtctgg cagcagcccc gagttgttnc ctcgctgatc gatntctttc    180
ccccaggtag agttttcttt gcttatgttg aantccattg cctcttttct catcacagaa    240
gtgatgttgg aatcgtttct tttgtttgtc tgatttatgg ttttttaag tataaacaaa    300
agttttttat tagcattctg aaagaaggaa agtaaantgt acaagtttaa taaaaagggg    360
ccttcccctt taa                                                       373
```

<210> 99
<211> 380
<212> DNA
<213> Homo sapiens

<400> 99

```
gattgactcg gatttggagt gatcattagg gctgaggtct gtttctctgg gaggtaggac     60
ggctgcttcc tggtctggca gggatgggtt tgctttggaa atcctctagg aggctcctcc    120
ttcgcatggc ctgcagtctg gcagcagccc cgagttgttt cctcgctgat cgatttcttt    180
```

cctccaggta gagttttctt tgcttatgtt gaattccatt gcctcttttc tcatcacaga 240

agtgatgttg gaatcgtttc ttttgtttgt ctgatttatg gtttttttaa gtataaacaa 300

aagtttttta ttagcattct gaaagaagga aagtaaaatg tacaagttta ataaaaaggg 360

gccttcccct ttagaataaa 380

<210> 100
<211> 320
<212> DNA
<213> Homo sapiens

<220>
<221> misc_feature
<222> (304)..(304)
<223> "n" is any nucleotide.

<220>
<221> misc_feature
<222> (309)..(309)
<223> "n" is any nucleotide.

<400> 100

tctggcaggg atgggtttgc tttggaaatc ctctaggagg ctcctcctcg catggcctgc 60

agtctggcag cagcccgagt tgtttcctcg ctgatcgatt tctttcctcc aggtagagtt 120

ttctttgctt atgttgaatt ccattgcctc ttttctcatc acagaagtga tgttggaatc 180

gtttcttttg tttgtctgat ttatggtttt tttaagtata aacaaaagtt ttttattagc 240

attctgaaag aaggaaagta aaatgtacaa gtttaataaa aaggggcctt cccctttagg 300

aatnaaaana aaaaagggtg 320

<210> 101
<211> 397
<212> DNA
<213> Homo sapiens

<220>
<221> misc_feature
<222> (24)..(24)
<223> "n" is any nucleotide.

<400> 101

gattgactcg gatttgagtg atcnattagg gctgaggtct gtttctctgg gaggtaggac 60
ggctgcttca tggtctggca gggatgggtt tgctttggaa atcctctagg aggctcctcc 120
tcgcatggcc tgcagtctgc agcagccccg agttgtttcc tcgctgatcg atttctttcc 180
tccaggtaga gttttctttg cttatgttga attccattgc ctcttttctc atcacagaag 240
tgatgttgga atcgtttctt ttgtttgtct gatttatggt ttttttaagt ataaacaaaa 300

gttttttatt agcattctga aagaaggaaa gtaaaatgta caagtttaat aaaaaggggc 360
cttccccttt agaataaatt tcagcatgtg ctttcaa 397

<210> 102
<211> 289
<212> DNA
<213> Homo sapiens

<220>
<221> misc_feature
<222> (61)..(61)
<223> "n" is any nucleotide.

<220>
<221> misc_feature
<222> (74)..(74)
<223> "n" is any nucleotide.

<220>
<221> misc_feature
<222> (122)..(122)
<223> "n" is any nucleotide.

<220>
<221> misc_feature
<222> (184)..(184)
<223> "n" is any nucleotide.

<400> 102

gaggctcctc ctcgcatggc ctgcagtctt ggcagcagcc ccgagttgtt tcctcgctga 60
ncgatttctt tccnccaggt agagttttct ttgcttatgt tgaattccat tgcctctttt 120
cncatcacag aagtgatgtt ggaatcgttt cttttgtttg tctgatttat ggttttttta 180
agtntaaaca aaagtttttt attagcattc tgaaagaagg aaagtaaaat gtacaagttt 240
aataaaaagg ggccttcccc tttagaataa aaaaaaaaaa aaaaaaaaa 289

<210> 103
<211> 311
<212> DNA

<213> Homo sapiens

<220>
<221> misc_feature
<222> (7)..(7)
<223> "n" is any nucleotide.

<400> 103

```
cttttgnaaa tcctctagga ggctcctcct cgcatggcct gcagtctgca gcagccccga      60

gttgtttcct cgctgatcgg atttctttcc tccaggtaga gttttctttg cttatgttga     120

attccattgc ctcttttctc atcacagaag tgatgttgga atcgtttctt ttgtttgtct     180

gatttatggt ttttttaagt ataaacaaaa gtttttttatt agcattctga aagaaggaaa     240

gtaaaatgta caagtttaat aaaaaggggc cttccccttt agaataaatt tcagcatgtg     300

ctttcaaaaa a                                                          311
```

<210> 104
<211> 338
<212> DNA
<213> Homo sapiens

<220>
<221> misc_feature
<222> (32)..(32)
<223> "n" is any nucleotide.

<220>
<221> misc_feature
<222> (67)..(67)
<223> "n" is any nucleotide.

<220>
<221> misc_feature
<222> (136)..(136)
<223> "n" is any nucleotide.

<400> 104

```
ggtctggcag ggatgggttt gcctttggaa ancctctagg aggctcctcc tcgcatggcc     60
tgcagtnctg gcagcagacc ccgagttgtt tcctcgctga tcgatttctt tacccccagg    120
tagagttttc ctttgnctta tgttgaattc cattgcctct tttactcatc acagaagtga    180
tgttggaatc gtttcttttg tttgtctgat ttatggtttt tttaagtata aacaaaagtt    240
ttttattagc attctgaaag aaggaaagta aaatgtacaa gtttaataaa aaggggcctt    300
cccctttaga ataaaaaaaa aaaaaaaaaa aaaaaaaa                            338
```

<210> 105
<211> 343
<212> DNA
<213> Homo sapiens

<220>
<221> misc_feature
<222> (13)..(13)
<223> "n" is any nucleotide.

<220>
<221> misc_feature
<222> (19)..(19)
<223> "n" is any nucleotide.

<220>
<221> misc_feature
<222> (107)..(107)
<223> "n" is any nucleotide.

<400> 105

```
ccctgggtcc tgncaaggna tggggtttgc tttggaaatc ctcttaggag gctcctcctc     60
gcatggcctg cagtctggca gcagccccga gttgtttcct cgctgancga tttctttcct    120
ccaggtagag ttttctttgc ttatgttgaa ttccattgcc tcttttctca tcacagaagt    180
gatgttggaa tcgtttcttt tgtttgtctg atttatggtt tttttaagta taaacaaaag    240
tttttttatta gcattctgaa agaaggaaag taaaatgtac aagtttaata aaaaggggcc    300
ttcccccttta gaataaaaaa aaaaaaaaaa aaaaaaaaaa aaa                     343
```

## Description

## Cross Reference

This application is a continuation-in-part of US. Patent application serial no. 09/218,913, filed December 22,1998.

## Field of the Invention

The present invention relates to compositions comprising serine protease inhibitor proteins as specified herein in the production of medicaments which stimulate the rate of mucociliary clearance of mucus and sputum in lung airways.

## Background of the Invention

### *Problem Addressed*

Mucociliary dysfunction, characterized by the inability of ciliated epithelium to clear mucus and sputum in lung airways, is a serious complication of chronic obstructive lung diseases such as Chronic Bronchitis (CB), Bronchiectasis (BE), asthma and, especially, Cystic Fibrosis (CF). Patients suffering from mucociliary dysfunction are particularly vulnerable to secondary bacterial infections. Treatment and maintenance modalities for CF and other respiratory diseases associated with mucociliary dysfunction and the need for improved treatments have been described. See, for instance, Braga "Drugs in Bronchial Mucology, Raven Press, New York, 1989; Lethem et al, Am Rev. Respir. Dis. 142:1053-1058, 1990; U.S. Patent No. 5,830,436..

### *Cystic Fibrosis*

Cystic fibrosis (CF) is an autosomal recessive disease that causes abnormalities in fluid and electrolyte transport in exocrine epithelia. Mutations within the DNA coding for a protein termed the cystic fibrosis transmembrane conductance regulator (CFTR) have been found in virtually all CF patients. Cells of the lung are particularly affected. Di Santagrese et al, Am J. Med. 66: 121-132 (1979).

In CF, the luminal border of the airway mucosal cell is unresponsive to cAMP-dependant protein kinase activation of membrane chloride ion channels. The cell permeability to Cl- is impaired and $Na^+$ absorption across the cell membrane is accelerated. Both of these electrolyte imbalances tend to reduce the level of hydration of the airway mucus thus contributing to the viscous lung secretions characteristic of CF. Knowles, Clin. Chest. Med.11: 75 (1986). Adventitious bacteria and mycoplasmas enter the lung airways and establish colonies within the mucus. The thick mucus associated with CF isolates these pathogens from the immune system. Since mucociliary clearance is reduced in CF patients, bacterial clearance is also reduced. Lung congestion and infection are thus common. The prolonged presence of these pathogenic agents invariably initiates inflammatory reactions that compromise lung function. Bedrossian et al., Human Pathol. 7:195-204,1976.

Mucus viscosity in CF lungs is in part due to the decreased hydration of the mucus as related to Cl- channel malfunction and modification of sodium (Na~) ion concentration in the airway surface liquid (ASL) that change the rate of airway mucociliary clearance (MCC). The mechanisms involved in mucus transport have been studied in vitro and in vivo. CB, CF, and BE sputa are transported slowly by the mammalian ciliated epithelium of the mucus depleted bovine trachea (MDBT) (Wills et al, J. Clin. Invest. 97(1): 9-13,1995). Slow transportability of diseased sputum on the MDBT may be linked to its low electrolyte/osmolyte content (Wills et al, J. Resp. Crit. Care Med. 151(4): 1255-1258,1997). Indeed, diseased sputum is known to have low electrolyte content relative to plasma (Matthews et al, Am. Rev. Resp. Dis. 88: 199-204, 1963 ; Potter et al, Am. Rev. Resp. Dis. 67(1): 83-87,1967 ; Tomkiewicz et al, Am. Rev. Resp. Dis. 148(4, Pt. 1): 1002-1007, 1993).

Further studies on the MDBT have shown that transportability of diseased mucus is markedly improved following treatment with sodium chloride (Wills et al 1995). Furthermore, clinical studies have shown that inhalation of hypertonic saline, or of the epithelial sodium channel (ENaC) blocker amiloride can significantly increase MCC in diseased patients (Robinson et al , Thorax 52(10): 900-903,1997; App et al, Am. Rev. Resp. Dis.141, 605-612,1990). Recently, the relationship between mucus clearance and its ionic composition in vivo in the guinea-pig model of tracheal mucus velocity (TMV) has been elucidated. In vivo studies showed that a 5 minute aerosol of hypertonic saline transiently increased TMV. An increase in TMV was observed 1 min after hypertonic saline (14.4%) aerosol. TMV was $5.1 \pm 1.0$ mm.min$^{-1}$ (n=9) in 0.9% saline-exposed animals compared to $11.3 \pm 1.3$ mm.min$^{-1}$ in hypertonic saline exposed animals (n=9; $p \leq 0.001$)(Newton & Hall, 1997). Inhaled amiloride also caused an increase in TMV. A significant increase in TMV was observed 15 minutes after a 20 minute aerosol of amiloride (10mM). TMV was $3.2 \pm 2.5$ mm.min-1 (n=9) in water-exposed animals compared to 8.1$\pm$0.3 $mm.min^{-1}$ in amiloride-exposed animals (n=8; p≤0.05) Newton et al, Ped. Pulm. S17, Abs. 364,1998). These agents would appear to act by increasing the ionic content of airway surface liquid (ASL).

Clinical genetic evidence from subjects with systemic pseudohypo-aldosteronism (SPHA) also supports the view that down-regulation of the activity of airway epithelial sodium channels will increase mucociliary clearance in the lung. SPHA patients with loss of function mutations in the genes for the epithelial sodium channel subunits had no sodium absorption from airways surfaces, they had increased sodium ion concentration in nasal surface liquid compared to normal subjects, and they had 4-fold increased lung mucociliary clearance rate compared to normal subjects (Kerem et al, New England J. Med. 341, 156-162, 1999).

Recently, a serine protease termed channel activating protease-1 (CAP-1) has been found in the apical membrane of amphibian Xenopus kidney epithelial cells (A6 cells) (Vallet et al, Nature 389(6651): 607-610,1997). CAP-1 appears to modulate $Na^+$ channel activity in these cells. Exposure of the apical membrane to the prototypical bovine Kunitz inhibitor, aprotinin, reduced transepithelial $Na^+$ transport (Vallet et al 1997 : Chraibi et al, J. Gen. Physio. 111(1): 127-138, 1998). The effect of Bukinin (1-170), a two Kunitz domain human homologue of bovine aprotinin (Delaria et al, J. Biol. Chem. 272(18): 12209-12214, 1997; Marlor et al, J. Biol. Chem. 272(18): 12202-12208, 1997), was evaluated using normal cultured human bronchial epithelial cell (HBE) short circuit current (Isc) in vitro (McAulay et al, Ped. Pulm. S17, Abs. 141, 1998). Bikunin (1.5ug.$ml^{-1}$: 70nM) significantly inhibited 54% $Na^+$ Isc in normal HBE cells (n=5-8 ; p≤0.05). Overall, Bikunin (70nM) inhibited 58% of the baseline Isc in 90 minutes. In a further study, Bikunin (5ug.$^{ml}$-1) significantly inhibited 84% $Na^+$ Isc in normal HBE cells (n=6; p≤0.01) whilst the serpin-family serine protease inhibitor alpha(1)-protease inhibitor ($\alpha_1$-PI)(50 ug.$ml^{-1}$) was without a significant effect. In cultured human cystic fibrosis airway epithelial cells in vitro, Isc was inhibited by bikunin (1-170) (1 ug/ mL), and was inhibited by aprotinin (20 ug/mL).

Two recent studies by a single research group have demonstrated a protease inhibitor induced effect on TMV. $\alpha_1$-PI (10mg) given either 30 min before antigen challenge, or 1 h after challenge, attenuated antigen-induced reduction in TMV in allergic sheep, 6h after challenge (O'Riordan et al, Am. J. Resp. Crit. Care Med. 97(5):1522-1528,1997). In Fig 1 in the O'Riordan et al 1997 paper, the authors showed that $\alpha_1$-PI administered on its own (no antigen challenge) to the airways of allergic sheep, had no effect on baseline TMV over a 6h period. In the second study, $\alpha_1$-PI was given 6 h after antigen challenge and caused only a significant reversal of the antigen-induced fall in TMV at 24 h after challenge (O'Riordan et al, J. App. Physio. 85(3): 1086-1091, 1998). The authors argue that the mechanism for the effect of $\alpha_1$-PI is associated with its anti-neutrophil elastase property, where neutrophil elastase is believed to be the enzyme responsible for the reduced rate of mucociliary clearance in their model. They reasoned that $\alpha_1$-PI could be used to treat mucociliary dysfunction brought about by allergy-induced neutrophil elastase release in asthma (O'Riordan et al 1998); they did not speculate on a potential role in other respiratory diseases.

## Brief Summary of the Invention

The instant invention is directed to the use of Kunitz-family serine protease inhibitors as specified herein in the manufacture of medicaments that stimulate the rate of mucociliary clearance (MCC) of mucus and sputum in the airways of the lung. Kunitz-serine protease inhibitors can be used to treat lung diseases such as Cystic Fibrosis (CF), Chronic Bronchitis (CB) and Bronchiectasis (BE) where the retention and accumulation of mucus is a major clinical problem. Until now, prior art has not associated protease inhibitors with the ability to increase the rate of MCC above baseline rate. Kunitz-type serine protease inhibitors can also be used to treat chronic sinusitis and glue ear where the retention and accumulation of mucus is a clinical problem.

Examples of human serine protease inhibitors include Bikunin and variants and fragments thereof such as the ones described in WO 97/33996, published September 18,1997 (Bayer Corp.), and U.S. Patent No. 5,407,915, issued April 18,1995 (Bayer AG).

In one embodiment, the invention provides the use of a human Kunitz-type serine protease inhibitor for preparing a medicament for treating a lung disease associated with the retention and accumulation of mucus, selected from the group of diseases consisting of cystic fibrosis, chronic obstructive lung disease, chronic bronchitis, bronchietasis, chronic sinusitis and glue ear, the medicament comprising an effective amount of a human Kunitz-type serine protease inhibitor and physiologically acceptable carrier therefor, wherein the Kunitz-type serine protease inhibitor comprises an amino acid sequence as specified in claim 1.

The invention will be better understood from a consideration of the following detailed description and claims, taken in conjunction with the drawings, in which:

Figure 1 depicts the nucleotide sequence of EST R35464 (SEQ ID NO:12) and the translation of this DNA sequence (labeled "ORF") which yielded an open reading frame with some sequence similarity to aprotinin. Amino acids 1-110 of the translation correspond to SEQ ID NO:13; amino acids 112-130 correspond to SEQ ID NO:72. The translation product contains 5 of the 6 cysteines in the correct spacing that is characteristic for Kunitz-like inhibitor domains (indicated in bold). The position normally occupied by the remaining cysteine (at codon 38) contained instead a phenylalanine (indicated by an asterisk).

Figure 2 depicts the nucleotide sequence of EST R74593 (SEQ ID NO:14), and the translation of this DNA sequence (labeled "ORF") which yielded an open reading frame with homology to the Kunitz class of serine protease inhibitor domains. Amino acids 3-22 of the translation correspond to SEQ ID NO:15; amino acids 24-131 correspond to SEQ ID NO:73; amino acids 136-166 correspond to SEQ ID NO:74. The translation product contained 6 cysteines in the correct spacing that is characteristic for Kunitz-like inhibitor domains (indicated in bold). However, the reading frame sequence includes stop codons at codon 3 and 23.

Figure 3 depicts a deduced nucleic acid sequence of human placental bikunin (SEQ ID NO:9) labeled "consensus" and matched with the translated protein amino acid sequence labeled "translated". Amino acids -18-179 of the translation correspond to SEQ ID NO:10; amino acids 184-214 correspond to SEQ ID NO:76. Also as comparison are shown the nucleic acid sequence for ESTs H94519 (SEQ ID NO:16), N39798 (SEQ ID NO:17), R74593 (corrected by the insertion of G at position 114) (SEQ ID NO:75) and R35464 (SEQ ID NO:12). The underlined nucleotides in the consensus sequence correspond to the site of PCR primers described in the Examples. Underlined amino acids in the translated consensus sequence are residues whose identity have been confirmed by amino acid sequencing of purified native human placental bikunin. Nucleotide and amino acid code are standard single letter code, "N" in the nucleic acid code indicates an unassigned nucleic acid, and "*" indicates a stop codon or unassigned amino acid in the amino acid sequence.

Figure 4A depicts the original overlay of a series of ESTs with some nucleic acid sequence homology to ESTs encoding human placental bikunin, or portions thereof. Shown for reference are the relative positions of bikunin (7-64) and bikunin (102-159), labeled KID1 and KID2 respectively.

Figure 4B depicts a subsequent more comprehensive EST overlay incorporating additional ESTs. Numbers on the upper X-axis refer to length in base pairs, starting at the first base from the most 5' EST sequence. The length of each bar is in proportion to the length in base pairs of the individual ESTs including gaps. The EST accession numbers are indicated to the right of their respective EST bars.

Figure 4C depicts the corresponding alignment of the oligonucleotide sequences of each of the overlapping ESTs shown schematically in Figure 4B. The upper sequence (SEQ ID NO:51) labeled bikunin represents the consensus oligonucleotide sequence derived from the overlapping nucleotides at each position. The numbers refer to base-pair position within the EST map. The oligonucleotides in EST R74593 (SEQ ID NO:89) that are bold underlined (at map positions 994 and 1005) are base insertions observed in R74593 that were consistently absent in each of the other overlapping ESTs. In Figure 4C, N40851 corresponds to SEQ ID NO:77; N39876 corresponds to SEQ ID NO: 78; R87894 corresponds to SEQ ID NO:79; H16866 corresponds to SEQ ID NO:80; R34808 corresponds to SEQ ID NO:81; T66058 corresponds to SEQ ID NO:82; N57450 corresponds to SEQ ID NO:83; N57374 corresponds to SEQ ID NO:84; R35464 corresponds to SEQ ID NO:85; H94519 corresponds to SEQ ID NO:86; N39798 corresponds to SEQ ID NO:87; H87300 corresponds to SEQ ID NO:88; R74593 corresponds to SEQ ID NO:89; R31730 corresponds to SEQ ID NO:90; R34701 corresponds to SEQ ID NO:91; H02982 corresponds to SEQ ID NO:92; R32676 corresponds to SEQ ID NO:93; T47439 corresponds to SEQ ID NO:94; R73968 corresponds to SEQ ID NO:95; H39840 corresponds to SEQ ID NO:96; H95233 corresponds to SEQ ID NO:97; H39841 corresponds to SEQ ID NO:98; N30199 corresponds to SEQ ID NO:99; T52966 corresponds to SEQ ID NO:100; N29508 corresponds to SEQ ID NO:101; N26919 corresponds to SEQ ID NO:102; N26910 corresponds to SEQ ID NO:103; H16757 corresponds to SEQ ID NO: 104; and N27732 corresponds to SEQ ID NO:105.

Figure 4D depicts the amino acid translation of the consensus oligonucleotide sequence for bikunin depicted in Figure 4C (SEQ ID NO.: 45).

Figure 4E depicts the nucleotide sequence (SEQ ID NO.: 46) and corresponding amino acid translation (SEQ ID NO.: 47) of a placental bikunin encoding sequence that was derived from a human placental cDNA library by PCR-based amplification.

Figure 4F depicts the nucleotide sequence (SEQ ID NO.: 48) and corresponding amino acid translation (SEQ ID NO.: 49) of a native human placental bikunin encoding clone that was isolated from a human placental lambda cDNA library by colony hybridization.

Figure 4G compares the alignment of the amino acid translated oligonucleotide sequences for placental bikunin obtained by EST overlay (SEQ ID NO.: 45), PCR based cloning (SEQ ID NO.: 47), and conventional lambda colony hybridization (SEQ ID NO.: 49).

Figure 5 shows a graph of purification of human placental bikunin from placental tissue after Superdex 75 Gel-Filtration. The plot is an overlay of the protein elution profile as measured by OD 280 nm (solid line), activity of eluted protein in a trypsin inhibition assay (% inhibition shown by circles), and activity of eluted protein in a kallikrein inhibition assay (% inhibition shown by squares).

Figure 6 shows a graph which plots the purification of human placental bikunin from placental tissue using C18 Reverse-Phase Chromatography. The plot is an overlay of the protein elution profile as measured by OD 215 nm (solid line), activity of eluted protein in a trypsin inhibition assay (% inhibition shown by circles), and activity of eluted protein in a kallikrein inhibition assay (% inhibition shown by squares).

Figure 7 depicts a silver stained SDS-PAGE gel of highly purified placental bikunin (lane 2), and a series of molecular size marker proteins (lane 1) of the indicated sizes in kilodaltons. Migration was from top to bottom.

Figure 8 shows the amount of trypsin inhibitory activity present in the cell-free fermentation broth from the growth of yeast strains SC101 (panel 8A) or WHL341 (panel 8B) that were stably transformed with a plasmid (pS604) that directs the expression of placental bikunin (102-159).

Figure 9 shows both a silver stained SDS-PAGE (Figure 9A) and a Western blot with anti-placental bikunin (102-159) pAb (Figure 9B) of cell-free fermentation broth from the growth of yeast strain SC101 (recombinants 2.4 and 2.5) that was stably transformed with a plasmid directing the expression of either bovine aprotinin, or placental bikunin (102-159). Migration was from top to bottom.

Figure 10 is a photograph which shows a silver stained SDS-PAGE of highly purified placental bikunin (102-159) (lane 2) and a series of molecular size marker proteins (lane 1) of the indicated sizes in Kilodaltons. Migration was from top to bottom.

Figure 11 is a photograph which shows the results of Northern blots of mRNA from various human tissues that was hybridized to a $^{32}P$ labeled cDNA probe encoding either placental bikunin (102-159) (Figure 11A) or encoding placental bikunin (1-213) (Figure 11B). Migration was from top to bottom. The numbers to the right of each blot refer to the size in kilobases of the adjacent RNA markers. The organs from which mRNA was derived is described under each lane of the blot.

Figure 12 depicts an immunoblot of placental derived placental bikunin with rabbit antiserum raised against either synthetic reduced placental bikunin (7-64) (Figure 12A) or 102-159 (Figure 12b). For each panel, contents were: molecular size markers (lanes 1); native placental bikunin isolated from human placenta (lanes 2); synthetic placental bikunin (7-64) (lanes 3) and synthetic placental bikunin (102-159) (lanes 4). Tricine 10-20% SDS-PAGE gels were blotted and developed with protein A-purified primary polyclonal antibody (8 ug IgG in 20 ml 0.1% BSA/Tris-buffered saline (pH 7.5), followed by alkaline phosphatase-conjugated goat anti-rabbit secondary antibody. Migration was from top to bottom.

Figure 13 depicts a Coomassie Blue stained 10-20% Tricine SDS-PAGE gel of 3 micrograms of highly purified placental bikunin (1-170) derived from a baculovirus / Sf9 expression system (lane 2). Lane 1 contains molecular size markers. Migration was from top to bottom.

Figure 14 depicts a comparison of the effect of increasing concentrations of either Sf9-derived human placental bikunin (1-170) (filled circles), synthetic placental bikunin (102-159) (open circles), or aprotinin (open squares) on the activated partial thromboplastin time of human plasma. Clotting was initiated with $CaCl_2$. The concentration of proteins are plotted versus the -fold prolongation in clotting time. The uninhibited clotting time was 30.8 seconds.

Figure 15 illustrates the effect of Bikunin at dosage levels of 2 uM and 0.2 uM relative to amiloride (100 uM) and Hank's Balanced salt solution (HBSS) vehicle (control) on potential differences in guinea pig trachea 3 hours post-treatment.

Figure 16 illustrates (a) the postioning of the instillment syringe and beta probe relative to the guinea pig trachea; (b) a representive graph for measurement of trachea mean velocity (TMV) using $^{32}P$-labelled *S.cerevisae*; and (c) the sustained increase in TMV in vivo in guinea pig in response to Bikunin (5 ug) relative to HBSS vehicle control at 1.5, 1.75, 2.0, 2.25 and 2.5 hours following tracheal instillment.

Figure 17 illustrates that Bikunin (70 nM)decreases sodium current in cultured human bronchial epithelial cells in vitro relative to amiloride (10 uM).

Figure 18 illustrates the effect of a 5 min aerosol of hypertonic saline (14.4%) on increasing TMV, following aerosol treatment in guinea pig trachea.

Figure 19 illustrates the effect of a 20 minute aerosol of amiloride (10 mM) on TMV, following aerosol treatment in guinea pig trachea.

Figure 20 illustrates that Bikunin (5ug/mL), aprotinin (5 ug/mL), and aprotinin double mutein (0.5 ug/mL, 1.5 ug/ mL and 5 ug/ mL) decrease sodium short circuit current in cultured human bronchial cells in vitro.

Figure 21 illustrates that Aprotinin (1 mg/mL) inhibited Isc in vitro in human CF bronchial epithelial cells.

Figure 22 illustrates that Bikunin aerosol (3 mL of 3 mg/mL) significantly increased TMV in sheep relative to PBS control.

Figure 23 illustrates that Bikunin (50 ug/mL) inhibited sodium current in vitro in guinea pig tracheal epithelial cells over a 30 minute period.

Figure 24 illustrates that Bikunin (100 ug/mL) significantly inhibited sodium current in vitro in ovine trachael epithelial cells over a 90 minute period.

Figure 25 illustrates that (a) exposure to LPS caused a significant PMN influx and that (b) Bikunin significantly inhibited potential difference in guinea pigs pre-exposed to LPS.

Figure 26 illustrates that Aprotinin double mutein (10 ug) increased TMV in vivo in the guinea pig relative to HBSS over a sustained period of 1.5 to 2.5 hours following administration.

Figure 27 represents a plasmid map of pBC-BK (CMV-IE = cytomegalovirus immediate early; DHFR = dihydrofolate reductase; AMP-r = ampicillin resistance)

Figure 28 illustrates that (a) CHO expressed Bikunin (1-170) (10 ug/mL) decreases sodium current in vitro in human CF bronchial epithelial cells over a 90 minute period and (b) CHO Bikunin (1-170) at 1, 5, and 10 ug/mL and Aprotinin at 20 ug/mL decreases sodium current at 90 minutes after apical application to human CF bronchial epithelial cells in vitro.

Figure 29 illustrates the process steps for purifying Bikunin (1-170) from a CHO cell expression system.

Figure 30A shows a graph of the migration of the isoforms of purified CHO bikunin (1-170) using C18 Reverse-Phase Chromatography. The plot is an overlay of the protein elution profile as measured by Absorbance at 280 nm (solid line) and the percentage of acetonitrile in 0.1% Trifluoroacetic acid used to elute the protein (diamonds).

Figure 30B is a photograph which shows a silver stained SDS-PAGE of purified bikunin (1-170) glycosylated isoforms (lanes 45-55) expressed from a CHO cell expression system and a series of molecular size marker proteins (between lanes 54 and 55) of the indicated sizes in Kilodaltons. Migration was from top to bottom.

Figure 31 is a photograph which shows a silver stained SDS-PAGE of N-Glycosidase F treated (lanes 2 and 4) and untreated (lanes 1 and 3) of CHO purified bikunin (1-170) isoforms and a series of molecular size marker proteins of the indicated sizes in Kilodaltons. Migration was from top to bottom.

**Detailed Description of the Invention**

The present invention relates to the use of a human Kunitz-type serine protease inhibitor for preparing a medicament for treating a lung disease associated with the retention and accumulation of mucus, selected from the group of diseases consisting of cystic fibrosis, chronic obstructive lung disease, chronic bronchitis, bronchietasis, chronic sinusitis and glue ear, the medicament comprising an effective amount of a human Kunitz-type serine protease inhibitor and physiologically acceptable carrier therefor, wherein the Kunitz-type serine protease inhibitor comprises an amino acid sequence as specified in claim 1.

The uses also encompass the use of a newly identified human protein herein called human placental bikunin that contains two serine protease inhibitor domains of the Kunitz class.

A preferred application for placental bikunin, isolated domains, and other variants is for stimulating mucociliary clearance in CF patients as part of disease therapy and management. These medicaments reduce or eliminate mucus and sputum buildup in lung airways in patients with chronic obstructive lung disease, thereby reducing the risk of secondary lung infections and other adverse side effects, as well as avoiding or delaying the need for lung transplant surgery in CF patients.

A protinin has been shown to reduce transepithelial Na+ transport in the apical membrane of amphibian Xenopus kidney epithelial cells (A6 cells) (Vallet et al 1997 : Chraibi et al 1998). The mechanism of aprotinin action has been proposed to involve inhibition of CAP-1, a protease involved in modulating $Na^+$ channel activity in A6 cells. Bikunin (1-170), a two Kunitz domain human homologue of bovine aprotinin (Delaria et al 1997 : Marlor et al 1997), was also shown to significantly inhibit normal cultured human bronchial epithelial cell (HBE) short circuit current (Isc) in vitro (McAulay et al 1998). Bikunin (1.5ug.$ml^{-1}$ : 70nM) significantly inhibited 54% $Na^+$ Isc in normal HBE cells (n=5-8 ; p≤0.05). Overall, Bikunin (70nM) inhibited 58% of the baseline Isc in 90 minutes. In a further study, Bikunin (5ug.$^{ml-}$1) significantly inhibited 84% $Na^+$ Isc in normal HBE cells (n=6; p≤0.01) whilst the serpin-family serine protease inhibitor alpha(1)-protease inhibitor ($\alpha_1$-PI)(50ug.$mL^{-1}$) was without a significant effect. In cultured human cystic fibrosis airway epithelial cells in vitro, Isc was inhibited by bikunin (1-170) (1 ug/mL), and was inhibited by aprotinin (20 ug/mL).

In light of these observations, Kunitz-type serine inhibitors such as aprotinin, placental bikunin and fragments thereof are contemplated as therapeutics for treating mucocililiary dysfunction, including cystic fibrosis.

By "Kunitz inhibitor" is meant an inhibitor of proteases; structurally, a "Kunitz inhibitor" or "Kunitz domain" is a protein, or protein domain, typically of about 60 amino acids in length and containing three disulfide bonds. (See Laskowske & Kato, Ann. Rev. Biochem. 49, 593-626, 1980).

A significant advantage of the Kunitz domains of the serine protease inhibitor Bikunin and fragments and analogs thereof of the present invention is that they are human proteins, and also less positively charged than Trasylol® (Example 1), thereby reducing the risk of kidney damage on administration of large doses of the proteins. Being of human origin, the protein of the instant invention can thus be administered to human patients with significantly reduced risk of undesired immunological reactions as compared to administration of similar doses of Trasylol®. Furthermore, it was found that bikunin(102-159), bikunin(7-64), and bikunin(1-170) are significantly more potent inhibitors of plasma kallikrein than Trasylol® *in vitro* (Example 3, 4 and 10). Thus bikunin and fragments thereof are expected to be more effective *in vivo* relative to aprotinin.

The amount of the pharmaceutical composition to be employed will depend on the recipient and the condition being treated. The requisite amount may be determined without undue experimentation by protocols known to those skilled in the art. Alternatively, the requisite amount may be calculated, based on a determination of the amount of target protease such as plasmin, kallikrein or prostasin which must be inhibited in order to treat the condition. As the active materials contemplated in this invention are deemed to be nontoxic, treatment preferably involves administration of an excess of the optimally required amount of active agent.

For stimulating the rates of mucociliary clearance in patients with chronic obstructive lung disease, the proteins of the instant invention may be used like aprotinin Trasylol® while taking into account the differences in potency. The use of Trasylol® is outlined in the Physicians Desk Reference, 1995, listing for Trasylol® supplement A. Briefly, with the patient in a supine position, the loading dose of placental bikunin, isolated domain or other variant is given by infusion slowly over about 20 to 30 minutes. In general, a total dose of between about $2x10^6$ KIU (kallikrein inhibitory units) and $8 X10^6$ KIU will be used, depending on such factors as patient weight and condition. Preferred loading doses are those that contain a total of 1 to 2 million kallikrein inhibitory units (KIU).

The proteins of the instant invention are employed in pharmaceutical compositions formulated in the manner known to the art. Such compositions contain active ingredient(s) plus one or more pharmaceutically acceptable carriers, diluents, fillers, binders, and other excipients, depending on the administration mode and dosage form contemplated. Examples of therapeutically inert inorganic or organic carriers known to those skilled in the art include, but are not limited to, lactose, corn starch or derivatives thereof, talc, vegetable oils, waxes, fats, polyols such as polyethylene glycol, water, saccharose, alcohols, glycerin and the like. Various preservatives, emulsifiers, dispersants, flavorants, wetting agents, antioxidants, sweeteners, colorants, stabilizers, salts, buffers and the like can also be added, as required to assist in the stabilization of the formulation or to assist in increasing bioavailability of the active ingredient(s) or to yield a formulation of acceptable flavor or odor in the case of oral, nasal or pulmonary dosing. The inhibitor employed in such compositions may be in the form of the original compound itself, or optionally, in the form of a pharmaceutically acceptable salt. The compositions so formulated are selected as needed for administration of the inhibitor by any suitable mode known to those skilled in the art.

Parenteral administration modes include intravenous (i.v.), subcutaneous (s.c.), intraperitoneal (i.p.), and intramuscular (i.m.) routes. Intravenous administration can be used to obtain acute regulation of peak plasma concentrations of the drug as might be needed. Alternatively, the drug can be administered at a desired rate continuously by i.v. catheter. Suitable vehicles include sterile, non-pyrogenic aqueous diluents, such as sterile water for injection, sterile-buffered solutions or sterile saline. The resulting composition is administered to the patient prior to and/or during surgery by intravenous injection or infusion.

Improved half life and targeting of the drug to phagosomes such as neutrophils and macrophage involved in inflammation may be aided by entrapment of the drug in liposomes. It should be possible to improve the selectivity of liposomal targeting by incorporating into the outside of the liposomes ligands that bind to macromolecules specific to target organs/ tissues such as the GI tract and lungs. Alternatively, i.m. or s.c. deposit injection with or without encapsulation of the drug into degradable microspheres (e.g., comprising poly-DL-lactide-co-glycolide) or protective formulations containing collagen can be used to obtain prolonged sustained drug release. For improved convenience of the dosage form it is possible to use an i.p. implanted reservoir and septum such as the percuseal system. Improved convenience and patient compliance may also be achieved by use of either injector pens (e.g., the Novo Pin or Q-pen) or needle-free jet injectors (e.g., from Bioject, Mediject or Becton Dickinson). Precisely controlled release can also be achieved using implantable pumps with delivery to the desired site via a cannula. Examples include the subcutaneously implanted osmotic pumps available from ALZA such as the ALZET osmotic pump.

Oral delivery may be achieved by incorporating the drug into tablets, coated tablets, dragées, hard and soft gelatin capsules, solutions, emulsions, suspensions or enteric coated capsules designed to release the drug into the colon where digestive protease activity is low. Examples of the latter include the OROS-CT/Osmet™ system of ALZA, and the PULSINCAP™ system of Scherer Drug Delivery Systems. Other systems use azo-crosslinked polymers that are degraded by colon-specific bacterial azoreductases, or pH sensitive polyacrylate polymers that are activated by the rise in pH in the colon. The above systems may be used in conjunction with a wide range of available absorption enhancers. Rectal delivery may be achieved by incorporating the drug into suppositories.

Nasal delivery may be achieved by incorporating the drug into bioadhesive particulate carriers (<200 mm) such as those comprising cellulose, polyacrylate or polycarbophil, in conjunction with suitable absorption enhancers such as phospholipids or acylcarnitines. Commercially available systems include those developed by Dan Biosys and Scios Nova.

For stimulating the rate of mucociliary clearance, the preferred mode of administration of the placental bikunin variants of the present invention is pulmonary delivery. The Kunitz-type serine protease inhibitors disclosed herein may be administered to the lungs of a subject by any suitable means, but are preferably administered by administering an aerosol suspension of respirable particles comprised of the active compound, which the subject inhales. The respirable particles may be liquid or solid. Micron-sized dry powders containing the medicament in a suitable carrier such as mannitol, sucrose or lactose may be delivered to the lung airway surface using dry powder inhalers such as those of Inhale™, Dura™, Fisons (Spinhaler™), and Glaxo (Rotahaler™), or Astra (Turbohaler™) propellant based metered dose inhalers. Solution formulations with or without liposomes may be delivered using nebulizers.

Aerosols of liquid particles comprising the proteins may be produced by any suitable means, such as with a pressure-driven aerosol nebulizer or an ultrasonic nebulizer. See, e.g., U.S. Pat. No. 4,501,729. Nebulizers are commercially available devices which transform solutions or suspensions of the active ingredient into a therapeutic aerosol mist either by means of acceleration of compressed gas, typically air or oxygen, through a narrow venturi orifice or by means of ultrasonic agitation. Suitable formulations for use in nebulizers consist of the active ingredient in a liquid carrier. The carrier is typically water (and most preferably sterile, pyrogen-free water) or a dilute aqueous alcoholic solution, preferably made isotonic with body fluids by the addition of, for example, sodium chloride. Optional additives include preservatives if the formulation is not made sterile, for example, methyl hydroxybenzoate, antioxidants, flavoring agents, volatile oils, buffering agents and surfactants.

Aerosols of solid particles comprising the protein may likewise be produced with any solid particulate medicament aerosol generator. Aerosol generators for administering solid particulate medicaments to a subject produce particles which are respirable, as explained above, and generate a volume of aerosol containing a predetermined metered dose of a medicament at a rate suitable for human administration. One illustrative type of solid particulate aerosol generator is an insufflator. Suitable formulations for administration by insufflation include finely comminuted powders which may be delivered by means of an insufflator or taken into the nasal cavity in the manner of a snuff. In the insufflator, the powder (e.g., a metered dose thereof effective to carry out the treatments described herein) is contained in capsules or cartridges, typically made of gelatin or plastic, which are either pierced or opened in situ and the powder delivered by air drawn through the device upon inhalation or by means of a manually-operated pump. The powder employed in the insufflator consists either solely of the protein or of a powder blend comprising the protein, a suitable powder diluent, such as lactose, and an optional surfactant. A second type of illustrative aerosol generator comprises a metered dose inhaler. Metered dose inhalers are pressurized aerosol dispensers, typically containing a suspension or solution formulation of the active ingredient in a liquified propellant. During use these devices discharge the formulation through a valve adapted to deliver a metered volume, typically from 10 to 200 uL, to produce a fine particle spray containing the protein. Suitable propellants include certain chlorofluorocarbon compounds, for example, dichlorodifluoromethane, trichlorofluoromethane, dichlorotetrafluoroethane and mixtures thereof. The formulation may additionally contain one or more co-solvents, for example, ethanol, surfactants, such as oleic acid or sorbitan trioleate, antioxidants and suitable flavoring agents.

For metered dose inhaler or dry powder inhaler devices, the aerosol, whether formed from solid or liquid particles, may be produced by the aerosol generator at a rate of from about 5 to 150 liters per minute, more preferably from about 10 to 100 liters per minute, and most preferably for metered dose inhalers from about 10 to 50 liters per minute, and most perferably for dry powder inhalers about 60 liters per minute. Aerosols generated by nebulizer, jet or ultrasonic, may be produced by the aerosol generator at a rate of from about 1 to 100 liters per minute, more preferably from about 4 to 10 liters per minute. Aerosols containing greater amounts of protein may be administered more rapidly.

The dosage of the protease inhibitor will vary depending on the condition being treated and the state of the subject. The daily dose may be divided among one or several unit dose administrations. The daily dose by weight may range from about 0.01 to 20 milligrams of respirable particles for a human subject, depending upon the age and condition of the subject.

Solid or liquid particulate pharmaceutical formulations containing protease inhibitors of the present invention should include particles of respirable size: that is, particles of a size sufficiently small to pass through the mouth and larynx upon inhalation and into the bronchi and alveoli of the lungs. In general, particles ranging from about 1 to 8 microns in size (more particularly, less than about 6 microns in size) are respirable. Particles of non-respirable size which are included in the aerosol tend to be deposited in the throat and swallowed, and the quantity of non-respirable particles in the aerosol is preferably minimized. For nasal administration, a particle size in the range of 10-500 microns is preferred to ensure retention in the nasal cavity.

In the manufacture of a formulation according to the invention, the protease inhibitor is typically admixed with, inter alia, an acceptable carrier. The carrier must, of course, be acceptable in the sense of being compatible with any other ingredients in the formulation and must not be deleterious to the patient. The carrier may be a solid or a liquid, or both, and is preferably formulated with the compound as a unit-dose formulation, for example, a capsule, which may contain from 0.5% to 99% by weight of the active compound. One or more active compounds may be incorporated in the formulations of the invention, which formulations may be prepared by any of the well-known techniques of pharmacy consisting essentially of admixing the components.

Compositions containing respirable dry particles of protease inhibitor may be prepared by grinding the inhibitor with a mortar and pestle, and then passing the micronized composition through a 400 mesh screen to break up or separate out large agglomerates.

The pharmaceutical composition may optionally contain a dispersant which serves to facilitate the formation of an aerosol. A suitable dispersant is lactose, which may be blended with the active agent in any suitable ratio (e.g., a 1 to 1 ratio by weight).

If desired, general ex vivo and in vivo gene therapy strategies may employed to deliver nucleic acid constructs encoding Kunitz-type serine protease inhibitor proteins such as Bikunin, Aprotinin or fragments and variants thereof such as the ones described in WO 97/33996 (Bayer Corp.) and U.S. Patent No. 5,407,915.(Bayer AG). Gene therapy techniques that are primarily virus-based have been used to transform pulmonary cells as a means for treating the manifestations of CF in the lung and associated extrapulmonary tissues. See WO 93/03709, published March 3,1993 which describes the use of retroviral and non-retroviral vectors (e.g., adenoviruses and adeno-associated viruses) for the stable expression of the CFTR gene in CF patients. Alternatively, non-viral methods for delivery of exogenous nucleic acids are also known . See WO 93/12240, published June 24, 1993 and references cited therein, describing a transcription or expression cassettes including the cod ing sequence for a CFTR molecule operably joined to regulatory sequences functional in a mammal. The nucleic acid constructs are then supplied to the airways and alveoli of the lung in a number of ways including aerosolized delivery alone or in combination with lipid-based complexes, e.g., Lipofectin.™ WO 95/26356, published October 5,1995 describes representative examples of lipids useful for transfection.

### Searching Human Sequence Data

The existence of a distinct human protein homologous in function to aprotinin, was deduced following a unique analysis of sequence entries to the expressed-sequence-tag data-base (hereafter termed dbEST) at the NCBI (National Center for Biological Information, Maryland). Using the TBlastN algorithm (BLAST, or Basic Local Alignment Search Tool uses the method of Altschul et a., (1990) J. Mol Biol 215, 00 403-410, to search for similarities between a query sequence and all the sequences in a data-base, protein or nucleic acid in any combination), the data-base was examined for nucleotide sequences bearing homology to the sequence of bovine pre-pro-aprotinin, Trasylol®. This search of numerous clones was selectively narrowed to two particular clones which could possibly encode for a deduced amino acid sequence that would correspond to a human protein homologous in function to aprotinin. The selected nucleic acid sequences were R35464 (SEQ ID NO: 12) and R74593 (SEQ ID NO: 14) that were generated from a human placental nucleic acid library. The translated protein sequence in the longest open reading frame for R35464 (SEQ ID NO: 13) was missing one of the 6 cysteines that are critical for formation of the Kunitz-domain covalent structure, meaning that the nucleic acid sequence of R35464 could not yield a functional inhibitor. Similarly, the longest translated open reading frame from clone R74593 (SEQ ID NO: 15) contained a stop codon 5' to the region encoding the Kunitz like sequence, meaning that this sequence, could not be translated to yield a functional secreted Kunitz domain. The significance of these sequences alone was unclear. It was possible that they represented a) the products of pseudogenes, b) regions of untranslated mRNA, or c) the products of viable mRNA which had been sequenced incorrectly.

### Discovery of Human Bikunin

To specifically isolate and determine the actual human sequence, cDNA primers were designed to be capable of hybridizing to sequences located 5' and 3' to the segment of cDNA encoding our proposed Kunitz like sequences found within R35464 and R74593. The primers used to amplify a fragment encoding the Kunitz like sequence of R74593 were CGAAGCTTCATCTCCGAAGCTCCAGACG (the 3'primer with a HindIII site; SEQ ID NO.:33) and AGGATCTAGACAATAATTACCTGACCAAGGA (the 5'primer with an XbaI site; SEQ ID NO.:34).

These primers were used to amplify by PCR (30 cycles) a 500 base pair product from a human placental cDNA library from Clontech (MATCHMAKER, Cat #HL4003AB, Clontech Laboratories, Palo Alto, CA), which was subcloned into Bluescript-SK+ and sequenced with the T3 primer with a Sequenase™ kit version 2.0. Surprisingly, the sequence of the fragment obtained using our primers was different from the sequence listed in the dbEST data base for clone R74593. In particular, our new sequence contained an additional guanosine base inserted 3' to the putative stop codon, but 5' to the segment encoding the Kunitz-like sequence (Figure 3). The insertion of an additional G shifted the stop codon out of the reading frame for the Kunitz-like domain (G at base pair 114 of the corrected sequence for R74593; Figure 3).

Subsequent query of the dbEST for sequences homologous to the Kunitz-like peptide sequence of R74593 yielded H94519 derived from human retina library and N39798. These sequences contained a Kunitz-like sequence that was almost identical to the Kunitz-like domain encoded in R35464 except that it contained all six of the characteristic cysteines. Overlay of each of the nucleotide sequences with that of R74593 (corrected by the insertion of G at b,p, 114) and R35464 was used to obtain a consensus nucleotide sequence for a partial human placental bikunin (SEQ ID NO.: 9; Figure 3). The translated consensus sequence yielded an open reading frame extending from residue -18 to +179 (Figure 3; full translation SEQ ID NO.: 10) that contained two complete Kunitz-like domain sequences, within the region of amino acid residues 17-64 and 102-159 respectively.

Further efforts attempted to obtain additional 5' sequence by querying dbEST with the sequence of R35464. Possible matches from such searches, that possessed additional 5' sequence were then in turn used to re-query the dbEST. In such an iterative fashion, a series of overlapping 5' sequences were identified which included clones H16866, T66058, R34808, R87894, N40851 and N39876 (Figure 4). Alignment of some of these sequences suggested the presence of a 5' ATG which might serve as a start site for synthesis of the consensus translated protein sequence. From this selected information, it was now possible to selectively screen for, and determine the nucleic acid and polypeptide sequences of a human protein with homologous function to aprotinin.

Re-interrogation of the dbEST revealed a number of new EST entries shown schematically in Figure 4B. Overlap with these additional ESTs allowed us to construct a much longer consensus oligonucleotide sequence (Figure 4C) that extended both 5' and 3' beyond the original oligonucleotide sequence depicted in Figure 3. In fact, the new sequence of total length 1.6 kilobases extended all the way to the 3' poly-A tail. The increased number of overlapping ESTs at each base-pair position along the sequence improved the level of confidence in certain regions such as the sequence overlapping with the 3' end of EST R74593 (Figure 3). Several overlapping ESTs in this region corroborated two critical base deletions relative to R74593 (located as bold underlined in Figure 4C, map positions 994 and 1005). Translation of the new consensus sequence (Figure 4D) in the bikunin encoding frame yielded a form of placental bikunin that was larger (248 amino acids) than the mature sequence (179 amino acids) encoded from the original consensus (SEQ ID NO.: 1), and was terminated by an in-frame stop codon within the oligonucleotide consensus. The size increase was due to a frame shift in the 3' coding region resulting from removal of the two base insertions unique to EST R74593. The frame shift moved the stop codon of the original consensus (Figure 3) out of frame enabling read through into a new frame encoding the additional amino acid sequence. The new translation product (Figure 4D) was identical to the original protein consensus sequence (SEQ ID NO.: 1) between residues +1 to +175 (encoding the Kunitz domains), but contained a new C-terminal extension exhibiting a putative 24 residue long transmembrane domain (underlined in Figure 4D) followed by a short 31 residue cytoplasmic domain. The precise sequence around the initiator methionine and signal peptide was somewhat tentative due to considerable heterogeneity amongst the overlapping ESTs in this region.

Analysis of the protein sequence by Geneworks™, highlighted asparagine residues at positions 30 and 67 as consensus sites for putative N-linked glycosylation. Asparagine 30 was not observed during N-terminal sequencing of the full length protein isolated from human placenta, consistent with it being glycosylated.

**Cloning of Human Bikunin**

The existence of a human mRNA corresponding to the putative human bikunin nucleotide sequence inferred from the analysis of Figure 3, was confirmed as follows. The nucleic acid primer hybridizing 5' to the Kunitz-encoding cDNA sequence of R35464 (b.p. 3-27 of consensus nucleotide sequence in Figure 3): GGTCTAGAGGCCGGGTCGTTTCTCGCCTGGCTGGGA (a 5' primer derived from R35464 sequence with an XbaI site; SEQ ID NO.: 35), and the nucleic acid primer hybridizing 3' to the Kunitz encoding sequence of R74593 (b.p. 680-700 of consensus nucleotide sequence in Figure 3), was used to PCR amplify, from a Clontech human placental library, a fragment of the size (ca. 670 b.p) expected from a cDNA consensus nucleotide sequence encoding the placental bikunin sequence of Figure 3 (Shown schematically in Figure 4A).

Using a 5' primer hybridizing to a sequence in R87894 that is 126 b.p 5' to the putative ATG start site discussed above, (shown schematically in Figure 4A at b.p. 110) plus the same 3' primer to R74593 as used above, it was possible to amplify a fragment from a Clontech human placental library of the expected size (approximately 872 b.p) predicted by EST overlay (Shown schematically in Figure 4).

Sequencing of the 872 b.p. fragment showed it to contain nucleotide segment corresponding to b.p. 110 to 218 of EST R87894 at its 5' end and b.p. 310 to 542 of the consensus sequence for placental bikunin inferred from the EST overlay analysis (of Figure 3), at its 3' end. This 3' nucleotide sequence contained all of the Kunitz-like domain encoded by placental bikunin (102-159).

To obtain a cDNA encoding the entire extracellular region of the protein, the following 5' PCR primer: CACCTGATCGCGAGACCCC (SEQ ID NO.: 36) designed to hybridize to a sequence within EST R34808 was used with the same 3' primer to EST 74593 to amplify (30 cycles) an approximately 780 base-pair cDNA product from the human placental cDNA library. This product was gel purified, and cloned into the TA vector (Invitrogen) for DNA sequencing by the dideoxy method (Sanger F., et al., (1977) Proc. Natl. Acad. Sci (USA), 74, pp 5463-5467) with the following primers:

| | |
|---|---|
| Vector Specific: | GATTTAGGTGACACTATAG (SP6) (SEQ ID NO.: 37) |
| | TAATACGACTCACTATAGGG (T7) (SEQ ID NO.: 38) |
| Gene Specific: | TTACCTGACCAAGGAGGAGTGC (SEQ ID NO.: 39) |
| | AATCCGCTGCATTCCTGCTGGTG (SEQ ID NO.: 40) |
| | CAGTCACTGGGCCTTGCCGT (SEQ ID NO.: 41) |

The resulting cDNA sequence is depicted in Figure 4E together with its translation product. At the nucleotide level, the sequence exhibited only minor differences from the consensus EST sequence (Figure 4D). Translation of the sequence yielded a coding sequence containing an in-frame initiator ATG site, signal peptide and mature placental bikunin sequence and transmembrane domain. The translated sequence of the PCR product was missing the last 12 amino acid residues from the cytoplasmic domain as a consequence of the choice of selection of the 3' primer for PCR amplification. This choice of 3' PCR primer (designed based on the sequence of R74593) was also responsible for the introduction of an artifactual S to F mutation at amino acid position 211 of the translated PCR-derived sequence. The signal peptide deduced from translation of the PCR fragment was somewhat different to that of the EST consensus.

To obtain a full length placental bikunin cDNA, the PCR derived product (Figure 4E) was gel purified and used to isolate a non-PCR based full length clone representing the bikunin sequence. The PCR derived cDNA sequence was labeled with $^{32}$P-CTP by High Prime (Boehringer Mannheim) and used to probe a placental cDNA Library (Stratagene, Unizap™ λ library) using colony hybridization techniques. Approximately 2 X $10^6$ phage plaques underwent 3 rounds of screening and plaque purification. Two clones were deemed full length (~1.5 kilobases) as determined by restriction enzyme analysis and based on comparison with the size of the EST consensus sequence (see above). Sequencing of one of these clone by the dideoxy method yielded the oligonucleotide sequence depicted in Figure 4F. The translation product from this sequence yielded a protein with inframe initiator methionine, signal peptide and mature placental bikunin sequence. The mature placental bikunin sequence was identical to the sequence of the mature protein derived by translation of the EST consensus although the signal peptide sequence lengths and sequences differed. Unlike the PCR derived product, the cDNA derived by colony hybridization contained the entire ectodomain, transmembrane domain, cytoplasmic domain and in-frame stop codon. In fact, the clone extended all the way to the poly-A tail. The initiator methionine was followed by a hydrophobic signal peptide which was identical to the signal peptide encoded in the PCR derived clone. Subsequently, we expressed and purified soluble fragments of placental bikunin, Bikunin (1-170), from Sf9 cells (Example 9) and CHO cells (Example 17), and found them to be functional protease inhibitors (Examples 10 and 18). Furthermore, we isolated from human placenta a soluble fragment of placental bikunin which was also an active protease inhibitor (Example 7).

Based on the above observations, it seems that full length placental bikunin has the capacity to exist as a transmembrane protein on the surface of cells as well as a soluble protein. Other transmembrane proteins that contain Kunitz domains are known to undergo proteolytic processing to yield mixtures of soluble and membrane associated forms. These include two forms of the Amyloid Precursor Protein termed APP751 (Esch F., et al., (1990) Science, 248, pp 1122-1124) and APP 770 (Wang R., et al., (1991), J. Biol Chem, 266, pp16960-16964).

Contact activation is a process which is activated by exposure of damaged vascular surfaces to components of the coagulation cascade. Angiogenesis is a process that involves local activation of plasmin at endothelial surfaces. The specificity of placental bikunin and its putative capacity to anchor to cell surfaces, suggest that the physiologic functions of transmembranous placental bikunin may include regulation of contact activation and angiogenesis.

The amino acid sequences for placental bikunin (7-64), bikunin (102-159), and full length placental bikunin (Figure 4F) were searched against the PIR (Vers. 46.0) and PatchX (Vers. 46.0) protein databases as well as the GeneSeq (Vers. 20.0) protein database of patented sequences using the Genetics Computer Group program FastA. Using the Genetics Computer Group program TFastA (Pearson and Upman, 1988, Proc. Natl. Acad. Sci. USA 85: 2444-2448), these same protein sequences were searched versus the six-frame translations of the GenBank (Vers. 92.0 with updates to 1/26/96) and EMBL (modified Vers. 45.0) nucleotide databases as well as the GeneSeq (Vers. 20.0) nucleotide database of patented sequences. The EST and STS subsets of GenBank and EMBL were not included in this set of searches. The best matches resulting from these searches contained sequences which were only about 50% identical over their full length to the 58-amino acid protein sequence derived from our analysis of clones R74593 and R35464.

### Isolation of Human Bikunin

As mentioned above, synthetic peptides corresponding to bikunin (7-64) and bikunin (102-159) as determined from the translated consensus sequence for bikunin (Figure 3), could be refolded (Examples 2 and 1, respectively) to yield active kallikrein inhibitor protein (Example 4 and 3, respectively). We exploited this unexpected property to devise a purification scheme to isolate native placental bikunin from human tissue.

Using a purification scheme which employed kallikrein-sepharose affinity chromatography as a first step, highly purified native potent kallikrein inhibitor was isolated. The isolated native human bikunin had an identical N-terminus (sequenced for 50 amino acid residues) as the sequence predicted by the translation of the consensus nucleic acid sequence (Figure 3) amino acid residues +1 to +50 (Example 7). This confirmed for the first time the existence of a novel native kallikrein inhibitor isolated from human placenta.

Known Kunitz-like domains are listed below. Residues believed to be making contact with target proteases are highlighted as of special interest (bold/underlined). These particular residues are named positions $Xaa^{1-16}$ for specific reference as shown by label Xaa.

Bikunin (7-64) (SEQ ID NO.: 4)

```
Xaa                                       1 1  111     1          1
 1        2  3 456789                     0 1  234     5          6
IHDFCLVSKVV GRCRASMPRW WYNVTDGSCQ LFVYGGCDGN SNNYLTKEEC LKKCATV
```

Bikunin (102-159) (SEQ ID NO.: 6)
YEEYCTANAVT GPCRASFPRW YFDVERNSCN NFIYGGCRGN KNSYRSEEAC MLRCFRQ
Tissue factor pathway inhibitor precursor 1 (SEQ ID NO.: 18)
-HSFCAFKADD GPCKAIMKRF FFNIFTRQCE EFIYGGCEGN QNRFESLEEC KKMCTRD
Tissue factor pathway inhibitor precursor 1 (SEQ ID NO.: 19)
-PDFCFLEEDP GICRGYITRY FYNNQTKQCE RFKYGGCLGN MNNFETLEEC KNICEDG
Tissue factor pathway inhibitor precursor (SEQ ID NO.: 20)
-PSWCLTPADR GLCRANENRF YYNSVIGKCR PFKYSGCGGN ENNFTSKQEC LRACKKG
Tissue factor pathway inhibitor precursor 2 (SEQ ID NO.: 21)
-AEICLLPLDY GPCRALLLRY YYRYRTQSCR QFLYGGCEGN ANNFYTWEAC DDACWRI
Tissue factor pathway inhibitor precursor 2 (SEQ ID NO.: 22)
-PSFCYSPKDE GLCSANVTRY YFNPRYRTCD AFTYTGCGGN DNNFVSREDC KRACAKA
Amyloid precursor protein homologue (SEQ ID NO: 23)
-KAVCSQEAMT GPCRAVMPRT TFDLSKGKCV RFITGGCGGN RNNFESEDYC MAVCKAM
Aprotinin (SEQ ID NO: 24)
RPDFCLEPPYT GPCKARIIRY FYNAKAGLCQ TFVYGGCRAK RNNFKSAEDC MRTCGGA
Inter-α-trypsin inhibitor precursor (SEQ ID NOs: 25)
----CQLGYSA GPCMGMTSRY FYNGTSMACE TFQYGGCMGN GNNFVTEKEC LQTC
Inter-α-trypsin inhibitor precursor (SEQ ID NOs: 26)VAACNLPIVR GPCRAFIOLW
AFDAVKGKCV LFPYGGCQGN GNKFYSEKEC REYCGVP
Amyloid precursor protein (SEQ ID NO: 27)
-EVCCSEQAET GPCRAMISRW YFDVTEGKCA PFFYGGCGGN RNNFDTEEYC MAVCGSA
Collagen α-3(VI) precursor (SEQ ID NO: 28)
----CKLPKDE GTCRDFILKW YYDPNTKSCA RFWYGGCGGN ENKFGSQKEC EKVC
HKI-B9 (SEQ ID NO: 29)
-PNVCAFPMER GPCQTYMTRW FFNFETGECE LFAYGGCGGN SNNFLRKEKC EKFCKFT

The placental bikunin, isolated domains or other variants of the present invention may be produced by standard solid phase peptide synthesis using either t-Boc chemistry as described by Merrifield R.B. and Barany G., in: The peptides, Analysis, Synthesis, Biology, 2, Gross E. et al., Eds. Academic Press (1980) Chapter 1; or using F-moc chemistry as described by Carpino L.A., and Han G.Y., (1970) J. Amer Chem Soc., 92, 5748-5749, and illustrated in Example 2. Alternatively, expression of a DNA encoding the placental bikunin variant may be used to produce recombinant placental bikunin variants.

The instant invention provides for the use of a purified human serine protease inhibitor which can specifically inhibit kallikrein, that has been isolated from human placental tissue via affinity chromatography. The human serine protein inhibitor, herein called human placental bikunin, contains two serine protease inhibitor domains of the Kunitz class. In one particular embodiment, the use of the instant invention relates to a protein having the amino acid sequence:

```
ADRERSIHDF CLVSKVVGRC RASMPRWWYN VTDGSCQLFV YGGCDGNSNN    50
YLTKEECLKK CATVTENATG DLATSRNAAD SSVPSAPRRQ DSEDHSSDMF   100
NYEEYCTANA VTGPCRASFP RWYFDVERNS CNNFIYGGCR GNKNSYRSEE   150
ACMLRCFRQQ ENPPLPLGSK VVVLAGAVS                          179
```

(SEQ ID NO.: 1)

In a preferred embodiment the use of the instant invention relates to a bikunin protein (Bikunin (1-170)) having the amino acid sequence:

**ADRERSIHDF CLVSKVVGRC RASMPRWWYN VTDGSCQLFV YGGCDGNSNN 50**

**YLTKEECLKK CATVTENATG DLATSRNAAD SSVPSAPRRQ DSEDHSSDMF 100**

**NYEEYCTANA VTGPCRASFP RWYFDVERNS CNNFIYGGCR GNKNSYRSEE 150**

**ACMLRCFRQQ ENPPLPLGSK 170**

(SEQ ID NO.: 52)

In one aspect, the biological activity of the protein useful in practicing the instant invention is that it can bind to and substantially inhibit the biological activity of trypsin, human plasma and tissue kallikreins, human plasmin and Factor XIIa. In a preferred embodiment, the present invention provides for the use of a native human placental bikunin protein, in glycosylated form. In a further embodiment the instant invention encompasses the use of native human bikunin protein which has been formed such that it contains at least one cysteine-cysteine disulfide bond. In a preferred embodiment, the protein contains at least one intra-chain cysteine-cysteine disulfide bond formed between a pair of cysteines selected from the group consisting of CYS11-CYS61, CYS20-CYS44, CYS36-CYS57, CYS106-CYS156, CYS115-CYS139, and CYS131-CYS152, wherein the cysteines are numbered according to the amino acid sequence of native human placental bikunin. One of ordinary skill will recognize that the protein of the use of the instant invention may fold into the proper three-dimensional conformation, such that the biological activity of native human bikunin is maintained, where none, one or more, or all of the native intra-chain cysteine-cysteine disulfide bonds are present. In a most preferred embodiment, the protein of the use of the instant invention is properly folded and is formed with all of the proper native cysteine-cysteine disulfide bonds.

Active protein for use in the instant invention can be obtained by purification from human tissue, such as placenta, or via synthetic protein chemistry techniques, as illustrated by the Examples below. It is also understood that the protein for use in the instant invention may be obtained using molecular biology techniques, where self-replicating vectors are capable of expressing the protein of the instant invention from transformed cells. Such protein can be made as non-secreted, or secreted forms from transformed cells. In order to facilitate secretion from transformed cells, to enhance the functional stability of the translated protein, or to aid folding of the bikunin protein, certain signal peptide sequences may be added to the NH2-terminal portion of the native human bikunin protein.

In one embodiment, the use of the instant invention thus provides for the use of native human bikunin protein with at least a portion of the native signal peptide sequence intact. Thus one embodiment of the use of the invention provides for the use of native human bikunin with at least part of the signal peptide, having the amino acid sequence:

| | | | | | |
|---|---|---|---|---|---|
| A G S F L | A W L G S | L L L S G | V L A | | -1 |
| A D R E R | S I H D F | C L V S K | V V G R C | R A S M P | 25 |
| R W W Y N | V T D G S | C Q L F V | Y G G C D | G N S N N | 50 |
| Y L T K E | E C L K K | C A T V T | E N A T G | D L A T S | 75 |
| R N A A D | S S V P S | A P R R Q | D S E D H | S S D M F | 100 |
| N Y E E Y | C T A N A | V T G P C | R A S F P | R W Y F D | 125 |
| V E R N S | C N N F I | Y G G C R | G N K N S | Y R S E E | 150 |
| A C M L R | C F R Q Q | E N P P L | P L G S K | V V V L A | 175 |
| G A V S | | | | | 179 |

(SEQ ID NO.: 2)

In a preferred embodiment, the use of the instant invention provides for the use of a native human placental bikunin protein with part of the leader sequence intact, having the amino acid sequence of SEQ ID NO.: 52 with an intact leader segment having the amino acid sequence:

MAQLCGL RRSRAFLALL GSLLLSGVLA -1

(SEQ ID NO.: 53)

In another embodiment, the use of the instant invention provides for the use of bikunin protein with part of the leader sequence intact, having the amino acid sequence of SEQ ID NO.: 52 with the intact leader segment having the amino acid sequence:

MLR AEADGVSRLL GSLLLSGVLA -1

(SEQ ID NO.: 54)

In a preferred numbering system used herein the amino acid numbered +1 is assigned to the NH2-terminus of the amino acid sequence for native human placental bikunin. One will readily recognize that functional protein fragments can be derived from native human placental bikunin, which will maintain at least part of the biological activity of native human placental bikunin, and act as serine protease inhibitors.

In one embodiment, the protein for use in the use of the instant invention comprises a fragment of native human placental bikunin, which contains at least one functional Kunitz-like domain, having the amino acid sequence of native human placental bikunin amino acids 7-159, hereinafter called "bikunin (7-159)". Thus the use of instant invention embodies a use that employs a protein having the amino acid sequence:

```
      I H D F  C L V S K  V V G R C  R A S M P    25
R W W Y N  V T D G S  C Q L F V  Y G G C D  G N S N N    50
Y L T K E  E C L K K  C A T V T  E N A T G  D L A T S    75
R N A A D  S S V P S  A P R R Q  D S E D H  S S D M F   100
N Y E E Y  C T A N A  V T G P C  R A S F P  R W Y F D   125
V E R N S  C N N F I  Y G G C R  G N K N S  Y R S E E   150
A C M L R  C F R Q                                      159
```

(SEQ ID NO.: 3)

where the amino acid numbering corresponds to that of the amino acid sequence of native human placental bikunin. Another functional variant of this embodiment can be the fragment of native human placental bikunin, which contains at least one functional Kunitz-like domain, having the amino acid sequence of native human placental bikunin amino acids 11-156, bikunin (11-156)

```
                   C L V S K  V V G R C  R A S M P    25
R W W Y N  V T D G S  C Q L F V  Y G G C D  G N S N N    50
Y L T K E  E C L K K  C A T V T  E N A T G  D L A T S    75
R N A A D  S S V P S  A P R R Q  D S E D H  S S D M F   100
N Y E E Y  C T A N A  V T G P C  R A S F P  R W Y F D   125
V E R N S  C N N F I  Y G G C R  G N K N S  Y R S E E   150
A C M L R  C                                            156
```

(SEQ ID NO.: 50).

One can recognize that the individual Kunitz-like domains are also fragments of the native placental bikunin. In particular, the use of the instant invention contemplates the use of a protein having the amino acid sequence of a first Kunitz-like domain consisting of the amino acid sequence of native human placental bikunin amino acids 7-64, hereinafter called "bikunin (7-64)". Thus in one embodiment the use of the instant invention encompasses the use of a protein which contains at least one Kunitz-like domain having the amino acid sequence:

```
      I H D F  C L V S K  V V G R C  R A S M P    25

R W W Y N  V T D G S  C Q L F V  Y G G C D  G N S N N    50
Y L T K E  E C L K K  C A T V                            64
```

(SEQ ID NO.: 4)

where the amino acid numbering corresponds to that of the amino acid sequence of native human placental bikunin. Another form of the protein used in the instant invention can be a first Kunitz-like domain consisting of the amino acid sequence of native human placental bikunin amino acids 11-61, "bikunin (11-61)" having the amino acid sequence:

```
                  C L V S K  V V G R C  R A S M P     25
R W W Y N  V T D G S  C Q L F V  Y G G C D  G N S N N     50
Y L T K E  E C L K K  C                               61
```

(SEQ ID NO.: 5)

The use of the instant invention also provides for the use of a protein having the amino acid sequence of a Kunitz-like domain consisting of the amino acid sequence of native human placental bikunin amino acids 102-159, hereinafter called "bikunin (102-159)". Thus one embodiment the use of the instant invention encompasses the use of a protein which contains at least one Kunitz-like domain having the amino acid sequence:

```
  Y E E Y  C T A N A  V T G P C  R A S F P  R W Y F D    125
V E R N S  C N N F I  Y G G C R  G N K N S  Y R S E E    150
A C M L R  C F R Q                                       159
```

(SEQ ID NO.: 6)

where the amino acid numbering corresponds to that of the amino acid sequence of native human placental bikunin. Another form of this domain can be a Kunitz-like domain consisting of the amino acid sequence of native human placental bikunin amino acids 106-156, "bikunin (106-156)" having the amino acid sequence:

```
           C T A N A  V T G P C  R A S F P  R W Y F D    125
V E R N S  C N N F I  Y G G C R  G N K N S  Y R S E E    150
A C M L R  C                                             156
```

(SEQ ID NO.: 7)

Thus one of ordinary skill will recognize that fragments of the native human bikunin protein can be made which will retain at least some of the native protein biological activity. Such fragments can also be combined in different orientations or multiple combinations to provide for alternative proteins which retain some of, the same, or more biological activity of the native human bikunin protein.

One will readily recognize that biologically active protein employed in the use of the instant invention may comprise one or more of the instant Kunitz-like domains in combination with additional Kunitz-like domains from other sources. Biologically active protein of the use of the instant invention may comprise one or more of the instant Kunitz-like domains in combination with additional protein domains from other sources with a variety of biological activities. The biological activity of the protein useful in practicing the instant invention can be combined with that of other known protein or proteins to provide for multifunctional fusion proteins having predictable biological activity.

An open reading frame which terminates at an early stop codon can still code for a functional protein. The use of the instant invention encompasses such alternative termination, and in one embodiment provides for the use of a protein of the amino acid sequence:

```
A D R E R   S I H D F   C L V S K   V V G R C   R A S M P   25
R W W Y N   V T D G S   C Q L>F V   Y G G C D   G N S N N   50
Y L T K E   E C L K K   C A T V T   E N A T G   D L A T S   75
R N A A D   S S V P S   A P R R Q   D S                     92
```

(SEQ ID NO.: 8)

One embodiment of the use of the invention provides for the use of native human bikunin, with an intact leader sequence, and with at least part of the transmembrane domain (underlined), having an amino acid sequence selected from:

```
EST                                           MLR AEADGVSRLL GSLLLSGVLA  -1
PCR                                       MAQLCGL RRSRAFLALL GSLLLSGVLA  -1
λcDNA                                     MAQLCGL RRSRAFLALL GSLLLSGVLA  -1

EST    ADRERSIHDF CLVSKVVGRC RASMPRWWYN VTDGSCQLFV YGGCDGNSNN  50
PCR    ADRERSIHDF CLVSKVVGRC RASMPRWWYN VTDGSCQLFV YGGCDGNSNN  50
λcDNA  ADRERSIHDF CLVSKVVGRC RASMPRWWYN VTDGSCQLFV YGGCDGNSNN  50

EST    YLTKEECLKK CATVTENATG DLATSRNAAD SSVPSAPRRQ DSEDHSSDMF 100
PCR    YLTKEECLKK CATVTENATG DLATSRNAAD SSVPSAPRRQ DSEDHSSDMF 100
λcDNA  YLTKEECLKK CATVTENATG DLATSRNAAD SSVPSAPRRQ DSEDHSSDMF 100

EST    NYEEYCTANA VTGPCRASFP RWYFDVERNS CNNFIYGGCR GNKNSYRSEE 150
PCR    NYEEYCTANA VTGPCRASFP RWYFDVERNS CNNFIYGGCR GNKNSYRSEE 150
λcDNA  NYEEYCTANA VTGPCRASFP RWYFDVERNS CNNFIYGGCR GNKNSYRSEE 150

EST    ACMLRCFRQQ ENPPLPLGSK VVVLAGLFVM VLILFLGASM VYLIRVARRN 200
PCR    ACMLRCFRQQ ENPPLPLGSK VVVLAGLFVM VLILFLGASM VYLIRVARRN 200
λcDNA  ACMLRCFRQQ ENPPLPLGSK VVVLAGLFVM VLILFLGASM VYLIRVARRN 200

EST    QERALRTVWS SGDDKEQLVK NTYVL                            225
PCR    QERALRTVWS FGD                                         213
λcDNA  QERALRTVWS SGDDKEQLVK NTYVL                            225
```

where EST is EST derived consensus SEQ ID NO.: 45, PCR is PCR clone SEQ ID NO.:47, and λcDNA is lambda cDNA clone SEQ ID NO.:49. In a preferred embodiment a protein of the use of the instant invention comprises one of the amino add sequence of SEQ ID NO.: 45, 47 or 49 wherein the protein has been cleaved in the region between the end of the last Kunitz domain and the transmembrane region (underlined).

The protein may also be used when the signal peptide is deleted, for example, a protein having the amino add sequence of SEQ ID NO.: 52 continuous with a transmembrane amino add sequence:

```
EST    VVVLAGLFVM VLILFLGASM VYLIRVARRN    200
EST    QERALRTVWS SGDDKEQLVK NTYVL         225
```

(SEQ ID NO.: 69)

a transmembrane amino acid sequence:

```
PCR    VVVLAGLFVM VLILFLGASM VYLIRVARRN    200
PCR    QERALRTVWS FGD                      213
```

(SEQ ID NO.: 68)

or a transmembrane amino acid sequence:

λcDNA VVVLAGLFVM VLILFLGASM VYLIRVARRN 200
λcDNA QERALRTVWS SGDDKEQLVK NTYVL 225

(SEQ ID NO.: 67).

The use of the invention also embodies the use the following proteins:

ADRERSIHDF CLVSKVVGRC RASMPRWWYN VTDGSCQLFV YGGCDGNSNN 50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150

ACMLRCFRQQ ENPPLPLGSK VVVLAGLFVM VLILFLGASM VYLIRVARRN 200
QERALRTVWS SGDDKEQLVK NTYVL 225
(SEQ ID NO.: 71),

or

ADRERSIHDF CLVSKVVGRC RASMPRWWYN VTDGSCQLFV YGGCDGNSNN 50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150
ACMLRCFRQQ ENPPLPLGSK VVVLAGLFVM VLILFLGASM VYLIRVARRN 200
QERALRTVWS FGD 213
(SEQ ID NO.: 70).

The protein amino acid sequences for use in the instant invention clearly teach one skilled in the art the appropriate nucleic acid sequences which can be used in molecular biology techniques to produce the proteins for use in the instant invention. The specification discloses be use of a nucleic acid sequence which encodes for a human bikunin having the consensus DNA sequence of Figure 3 (SEQ ID NO.: 9), which translates into the amino acid sequence for native human placental bikunin sequence of Figure 3 (SEQ ID NO.: 10). The specification also discloses a consensus nucleic acid sequence of Figure 4C (SEQ ID NO.: 51) which encodes for an amino acid sequence of Figure 4D (SEQ ID NO.: 45).

Also disclosed is the use of a nucleic acid sequence which encodes for native human placental bikunin having the DNA sequence of Figure 4F (SEQ ID NO.: 48) which encodes for the protein sequence of SEQ ID NO.: 49, and a nucleic acid sequence of Figure 4E (SEQ ID NO.: 46) which encodes for a protein sequence of SEQ ID NO.: 47.

Also disclosed are methods for stimulating MCC in a patient suffering from mucociliary dysfunction, wherein an effective amount of the disclosed human serine protease inhibitors of the present invention in a biologically compatible vehicle is administered to the patient.

The present specification also discloses for a method for stimulating MCC that employs variants of placental bikunin, and the specific Kunitz domains described above, that contain amino acid substitutions that alter the protease specificity. Preferred sites of substitution are indicated below as positions $Xaa^1$ through $Xaa^{32}$ in the amino acid sequence for native placental bikunin. Substitutions at $Xaa^1$ through $Xaa^{16}$ are also preferred for variants of bikunin (7-64), while substitutions at $Xaa^{17}$ through $Xaa^{32}$ are preferred for variants of bikunin (102-159).

Thus the specification discloses the use of a protein having an amino acid sequence:

Ala Asp Arg Glu Arg Ser Ile $Xaa^1$ Asp Phe 10

Cys Leu Val Ser Lys Val $Xaa^2$ Gly $Xaa^3$ Cys 20
$Xaa^4$ $Xaa^5$ $Xaa^6$ $Xaa^7$ $Xaa^8$ $Xaa^9$ Trp Trp Tyr Asn 30
Val Thr Asp Gly Ser Cys Gln Leu Phe $Xaa^{10}$ 40
Tyr $Xaa^{11}$ Gly Cys $Xaa^{12}$ $Xaa^{13}$ $Xaa^{14}$ Ser Asn Asn 50
Tyr $Xaa^{15}$ Thr Lys Glu Glu Cys Leu Lys Lys 60
Cys Ala Thr $Xaa^{16}$ Thr Glu Asn Ala Thr Gly 70
Asp Leu Ser Thr Ser Arg Asn Ala Ala Asp 80
Ser Ser Val Pro Ser Ala Pro Arg Arg Gln 90
Asp Ser Glu His Asp Ser Ser Asp Met Phe 100
Asn Tyr $Xaa^{17}$ Glu Tyr Cys Thr Ala Asn Ala 110
Val $Xaa^{18}$ Gly $Xaa^{19}$ Cys $Xaa^{20}$ $Xaa^{21}$ $Xaa^{22}$ $Xaa^{23}$ $Xaa^{24}$ 120
$Xaa^{25}$ Trp Tyr Phe Asp Val Glu Arg Asn Ser 130
Cys Asn Asn Phe $Xaa^{26}$ Tyr $Xaa^{27}$ Gly Cys $Xaa^{28}$ 140
$Xaa^{29}$ $Xaa^{30}$ Lys Asn Ser Tyr $Xaa^{31}$ Ser Glu Glu 150
Ala Cys Met Leu Arg Cys Phe Arg $Xaa^{32}$ Gln 160
Glu Asn Pro Pro Leu Pro Leu Gly Ser Lys 170
Val Val Val Leu Ala Gly Ala Val Ser 179
(SEQ ID NO: 11).

where $Xaa^1$- $Xaa^{32}$ each independently represents a naturally occurring amino acid residue except Cys, with the proviso that at least one of the amino acid residues $Xaa^1$-$Xaa^{32}$ is different from the corresponding amino acid residue of the native sequence.

In the present context, the term "naturally occurring amino acid residue" is intended to indicate any one of the 20 commonly occurring amino acids, i.e., Ala, Arg, Asn, Asp, Cys, Gin, Glu, Gly, His, Ile, Leu, Lys, Met, Phe, Pro, Ser, Thr, Trp, Tyr and Val.

By substituting one or more amino acids in one or more of the positions indicated above, it may be possible to change the inhibitor specificity profile of native placental bikunin or that of the individual Kunitz-like domains, bikunin (7-64) or bikunin (102-159) so that it preferentially inhibits other serine proteases such as, but not limited to, the enzymes of the complement cascade, TF/FVIIa, FXa, prostasin, thrombin, neutrophil elastase, cathepsin G or proteinase-3.

Examples of preferred variants of placental bikunin include those wherein $Xaa^1$ is an amino acid residue selected from the group consisting of His, Glu, Pro, Ala, Val or Lys, in particular wherein $Xaa^1$ is His or Pro; or wherein $Xaa^2$ is an amino acid residue selected from the group consisting of Val, Thr, Asp, Pro, Arg, Tyr, Glu, Ala, Lys, in particular wherein $Xaa^2$ is Val or Thr; or wherein $Xaa^3$ is an amino acid residue selected from the group consisting of Arg, Pro, Ile, Leu, Thr, in particular wherein $Xaa^3$ is Arg or Pro; or wherein $Xaa^4$ is an amino acid residue selected from the group consisting of Arg, Lys and Ser, Gln, in particular wherein $Xaa^4$ is Arg or Lys; or wherein $Xaa^5$ is an amino acid residue selected from the group consisting of Ala, Gly, Asp, Thr, in particular wherein $Xaa^5$ is Ala; or wherein $Xaa^6$ is an amino acid residue selected from the group consisting of Ser, Ile, Tyr, Asn, Leu, Val, Arg, Phe, in particular wherein $Xaa^6$ is Ser or Arg; or wherein $Xaa^7$ is an amino acid residue selected from the group consisting of Met, Phe, Ile, Glu, Leu, Thr and Val, in particular wherein $Xaa^7$ is Met or Ile; or wherein $Xaa^8$ is an amino acid residue selected from the group consisting of Pro, Lys, Thr, Gln, Asn, Leu, Ser or Ile, in particular wherein $Xaa^8$ is Pro or Ile; or wherein $Xaa^9$ is an amino acid residue selected from the group consisting of Arg, Lys or Leu, in particular wherein $Xaa^9$ is Arg: or wherein $Xaa^{10}$ is an amino acid residue selected from the group consisting of Val, Ile, Lys, Ala, Pro, Phe, Trp, Gin, Leu and Thr, in particular wherein $Xaa^{10}$ is Val; or wherein $Xaa^{11}$ is an amino acid residue selected from the group consisting of Gly, Ser and Thr, in particular wherein $Xaa^{11}$ is Gly; or wherein $Xaa^{12}$ is an amino acid residue selected from the group consisting of Asp, Arg, Glu, Leu, Gln, Gly, in particular wherein $Xaa^{12}$ is Asp or Arg; or wherein $Xaa^{13}$ is an amino acid residue selected from the group consisting of Gly and Ala; or wherein $Xaa^{14}$ is an amino acid residue selected from the group consisting of Asn or Lys; or wherein $Xaa^{15}$ is an amino acid residue selected from the group consisting of Gly, Asp, Leu, Arg, Glu, Thr, Tyr, Val, and Lys, in particular wherein $Xaa^{15}$ is Leu or Lys; or wherein $Xaa^{16}$ is an amino acid residue selected from the group consisting of Val, Gln, Asp, Gly, Ile, Ala, Met, and Val, in particular wherein $Xaa^{16}$ is Val or Ala; or wherein $Xaa^{17}$ is an amino acid residue selected from the group consisting of His, Glu, Pro, Ala, Lys and Val, in particular wherein $Xaa^{17}$ is Glu or Pro; or wherein $Xaa^{18}$ is an amino acid residue selected from the group consisting of Val, Thr, Asp, Pro, Arg, Tyr, Glu, Ala or Lys, in particular wherein $Xaa^{18}$ is Thr; or wherein $Xaa^{19}$ is an amino acid residue selected from the group consisting of Arg, Pro, Ile, Leu or Thr, in particular wherein $Xaa^{19}$ is Pro; or wherein $Xaa^{20}$ is an amino acid residue selected from the group consisting of Arg, Lys, Gln and Ser, in particular wherein $Xaa^{20}$ is Arg or Lys; or wherein $Xaa^{21}$ is an amino acid residue selected from the group consisting of Ala, Asp, Thr or Gly; in particular wherein $Xaa^{21}$ is Ala; or wherein $Xaa^{22}$ is an amino acid residue selected from the group consisting of Ser, Ile, Tyr, Asn, Leu, Val, Arg or Phe, in particular wherein $Xaa^{22}$ is Ser or Arg ; or wherein $Xaa^{23}$ is an amino acid residue selected from the group consisting of Met, Phe, Ile, Glu, Leu, Thr and Val, in particular wherein $Xaa^{23}$ is Phe or Ile; or wherein $Xaa^{24}$ is an amino acid residue selected from the group consisting of Pro, Lys, Thr, Asn, Leu, Gln, Ser or Ile, in particular wherein $Xaa^{24}$ is Pro or Ile; or wherein $Xaa^{25}$ is an amino acid residue selected from the group consisting of Arg, Lys or Leu, in particular wherein $Xaa^{25}$ is Arg: or wherein $Xaa^{26}$ is an amino acid residue selected from the group consisting of Val, Ile, Lys, Leu, Ala, Pro, Phe, Gln, Trp and Thr, in particular wherein $Xaa^{26}$ is Val or Ile; or wherein $Xaa^{27}$ is an amino acid residue selected from the group consisting of Gly, Ser and Thr, in particular wherein $Xaa^{27}$ is Gly; or wherein $Xaa^{28}$ is an amino acid residue selected from the group consisting of Asp, Arg, Glu, Leu, Gly or Gln, in particular wherein $Xaa^{28}$ is Arg; or wherein $Xaa^{29}$ is an amino acid residue selected from the group consisting of Gly and Ala; or wherein $Xaa^{30}$ is an amino acid residue selected from the group consisting of Asn or Lys; or wherein $Xaa^{31}$ is an amino acid residue selected from the group consisting of Gly; Asp, Leu, Arg, Glu, Thr, Tyr, Val, and Lys, in particular wherein $Xaa^{31}$ is Arg or Lys; or wherein $Xaa^{32}$ is an amino acid residue selected from the group consisting of Val, Gln, Asp, Gly, Ile, Ala, Met, and Thr, in particular wherein $Xaa^{32}$ is Gln or Ala.

Also disclosed are DNA constructs that encode the Placental bikunin protein variants of the present invention. These constructs may be prepared by synthetic methods such as those described in Beaucage S.L. and Caruthers M.H., (1981) Tetrahedron Lett, 22, pp1859-1862; Matteucci M.D and Caruthers M.H., (1981), J. Am. Chem. Soc. 103, p 3185; or from genomic or cDNA which may have been obtained by screening genomic or cDNA libraries with cDNA probes designed to hybridize with placental bikunin encoding DNA sequence. Genomic or cDNA sequence can be modified at one or more sites to obtain cDNA encoding any of the amino acid substitutions or deletions described in this disclosure.

Also disclosed are expression vectors containing the DNA constructs encoding the placental bikunin, isolated domains or other variants used in the present invention that can be used for the production of recombinant placental bikunin variants. The cDNA should be connected to a suitable promoter sequence which shows transcriptional activity in the host cell of choice, possess a suitable terminator and a poly-adenylation signal. The cDNA encoding the placental bikunin variant can be fused to a 5' signal peptide that will result in the protein encoded by the cDNA to undergo secretion. The signal peptide can be one that is recognized by the host organism. In the case of a mammalian host cell, the signal peptide can also be the natural signal peptide present in full length placental bikunin. The procedures used to prepare such vectors for expression of placental bikunin variants are well known in the art and are for example described in Sambrook et al., Molecular Cloning: A laboratory Manual, Cold Spring Harbor, New York, (1989).

Also disclosed are transformed cells containing the DNA constructs encoding the placental bikunin, isolated domains or other variants used in the present invention that can be used for the production of recombinant placental bikunin variants. A variety of combinations of expression vector and host organism exist which can be used for the production of the placental bikunin variants. Suitable host cells include baculovirus infected Sf9 insect cells, mammalian cells such as BHK, CHO, Hela and C-127, bacteria such as E. coli, and yeasts such as Saccharomyces cervisiae. Methods for the use of mammalian, insect and microbial expressions systems needed to achieve expression of placental bikunin are well known in the art and are described, for example, in Ausubel F.M et al., Current Protocols in Molecular Biology, John Wiley & Sons (1995), Chapter 16. For fragments of placental bikunin containing a single Kunitz inhibitor domain such as bikunin (7-64) and (102-159), yeast and E. coli expression systems are preferable, with yeast systems being most preferred. Typically, yeast expression would be carried out as described in US patent 5,164,482 for aprotinin variants and adapted in Example 5 of the present specification for placental bikunin (102-159). E.coli expression could be carried out using the methods described in US patent 5,032,573. Use of mammalian and yeast systems are most preferred for the expression of larger placental bikunin variants containing both inhibitor domains such as the variant bikunin(7-159).

DNA encoding variants of placental bikunin that possess amino acid substitution of the natural amino sequence can be prepared for expression of recombinant protein using the methods of Kunkel T.A., (1985) Proc. Natl. Acad. Sci USA 82, pp 488-492. Briefly, the DNA to be mutagenized is cloned into a single stranded bacteriophage vector such as M13. An oligonucleotide spanning the region to be changed and encoding the substitution is hybridized to the single stranded DNA and made double stranded by standard molecular biology techniques. This DNA is then transformed into an appropriate bacterial host and verified by dideoxynucleotide sequencing. The correct DNA is then cloned into the expression plasmid. Alternatively, the target DNA may be mutagenized by standard PCR techniques, sequenced, and inserted into the appropriate expression plasmid.

The following particular examples are offered by way of illustration, and not limitation, of certain aspects and preferred embodiments of the instant invention.

**Example 1**

**Preparation of synthetic placental bikunin (102-159)**

***Materials and methods/Reagents used.*** The fluorogenic substrate Tos-Gly-Pro-Lys-AMC was purchased from Bachem BioScience Inc (King of Prussia, PA). PNGB, Pro-Phe-Arg-AMC, Ala-Ala-Pro-Met-AMC, bovine trypsin (type III), human plasma kallikrein, and human plasmin were from Sigma (St. Louis, MO).

Recombinant aprotinin (Trasylol®) was from Bayer AG (Wuppertal, Germany). Pre-loaded Gln Wang resin was from Novabiochem (La Jolla, CA). Thioanisole, ethanedithiol and t-butyl methyl ether was from Aldrich (Milwaukee, WI).

***Quantification of functional placental bikunin (7-64) (SEQ ID NO:* 4) *and (102-159)***

The amount of trypsin inhibitory activity present in the refolded sample at various stages of purification was measured using GPK-AMC as a substrate. Bovine trypsin (200 pmoles) was incubated for 5 min at 37%C with bikunin (7-64) or (102-159), from various stages of purification, in buffer A (50 mM Hepes, pH 7.5, 0.1 M NaCl, 2 mM $CaCl_2$ and 0.01 % triton X-100). GPK-AMC was added (20 μM final) and the amount of coumarin produced was determined by measuring the fluorescence (ex = 370 nm, em = 432 nm) on a Perkin-Elmer LS-50B fluorimeter over a 2 min. period. For samples being tested the % inhibition for each was calculated according to equation 1; where $R_O$ is the rate of fluorescence increase in the presence of inhibitor and $R_1$ is the rate determined in the absence of added sample. One unit of activity for the inhibitor is defined as the amount needed to achieve 50% inhibition in the assay using the conditions as described.

$$\%\ \text{inhibition} = 100 \times [1 - R_O/R_1] \qquad (1)$$

*Synthesis*. Placental bikunin (102-159)(SEQ ID NO: 6) was synthesized on an Applied Biosystems model 420A peptide synthesizer using NMP-HBTU Fmoc chemistry. The peptide was synthesized on pre loaded Gln resin with an 8-fold excess of amino acid for each coupling. Cleavage and deprotection was performed in 84.6% trifluoroacetic acid (TFA), 4.4% thioanisole, 2.2% ethanedithiol, 4.4% liquified phenol, and 4.4% $H_2O$ for 2 hours at room temperature. The crude peptide was precipitated, centrifuged and washed twice in t-butyl methyl ether. The peptide was purified on a Dynamax 60A C18 reverse-phase HPLC column using a TFA/acetonitrile gradient. The final preparation (61.0 mg) yielded the correct amino acid composition and molecular mass by Electrospray mass spectroscopy (MH+ =6836.1; calcd = 6835.5) for the predicted sequence:

YEEYCTANAV TGPCRASFPR WYFDVERNSC NNFIYGGCRG NKNSYRSEEA
CMLRCFRQ (SEQ ID NO.: 6)

***Purification.*** Refolding of placental bikunin (102-159) was performed according to the method of Tam et al., (J. Am. Chem. Soc. 1991,113; 6657-62). A portion of the purified peptide (15.2 mg) was dissolved in 4.0 ml of 0.1 M Tris, pH 6.0, and 8 M urea. Oxidation of the disulfides was accomplished by dropwise addition of a solution containing 23% DMSO, and 0.1 M Tris, pH 6.0 to obtain a final concentration of 0.5 mg/ml peptide in 20% DMSO, 0.1 M Tris, pH 6.0, and 1 M urea. The solution was allowed to stir for 24 hr at 25°C after which it was diluted 1:10 in buffer containing 50 mM Tris, pH 8.0, and 0.1 M NaCl. The material was purified using a kallikrein affinity column made by covalently attaching 30 mg of bovine pancreatic kallikrein (Bayer AG) to 3.5 mls of CNBr activated Sepharose (Pharmacia) according to the manufacturers instructions. The refolded material was loaded onto the affinity column at a flow rate of 1 ml/min and washed with 50 mM Tris, pH 8.0, and 0.1 M NaCl until absorbance at 280 nm of the wash could no longer be detected. The column was eluted with 3 volumes each of 0.2 M acetic acid, pH 4.0 and 1.7. Active fractions were pooled (see below) and the pH of the solution adjusted to 25. The material was directly applied to a Vydac C18 reverse-phase column (5 micron, 0.46 x 25 cm) which had been equilibrated in 22.5% acetonitrile in 0.1% TFA. Separation was achieved using a linear gradient of 22.5 to 40% acetonitrile in 0.1 % TFA at 1.0 ml/min over 40 min. Active fractions were pooled, lyophilized, redissolved in 0.1 % TFA, and stored at -20°C until needed.

***Results.*** Synthetic placental bikunin (102-159) was refolded using 20% DMSO as the oxidizing agent as described above, and purified by a 2-step purification protocol as shown below, to yield an active trypsin inhibitor (Table 1 below).

**Table 1 Purification table for the isolation of synthetic placental bikunin (102-159)**

TABLE 1

| Purification Step | Vol (ml) | mg/ml | mg | Units[c] (U) | SpA (U/mg) | Yield |
|---|---|---|---|---|---|---|
| 8.0 M Urea | 4.0 | 3.75[a] | 15.0 | 0 | 0 | - |
| 20% DMSO | 32.0 | 0.47[a] | 15.0 | 16,162 | 1,078 | 100 |
| Kallikrein affinity | 9.8 | 0.009[b] | 0.09 | 15,700 | 170,000 | 97 |
| C18 | 3.0 | 0.013[ab] | 0.04 | 11,964 | 300,000 | 74 |

[a]Protein determined by AAA.
[b]Protein determined by OD280 nm using the extinction coefficient determined for the purified protein ($1.7 \times 10^4$ $Lmol^{-1}$ $cm^{-1}$).
[c]One Unit is defined as the amount of material required to inhibit 50% of trypsin activity in a standard assay.

Chromatography of the crude refolded material over an immobilized bovine pancreatic kallikrein column selectively isolated 6.0% of the protein and 97% of the trypsin inhibitory activity present. Subsequent chromatography using C18 reverse-phase yielded a further purification of 2-fold, with an overall recovery of 74%. On RPHPLC, the reduced and refolded placental bikunin (102-159), exhibited elution times of 26.3 and 20.1 minutes, respectively. Mass spectroscopy analysis of the purified material revealed a molecular mass of 6829.8; a loss of 6 mass units from the starting material. This demonstrates the complete formation of the 3 disulfides predicted from the peptide sequence.

The isoelectric points of the purified, refolded synthetic placental bikunin (102-159) was determined using a Multiphor II Electrophoresis System (Pharmacia) run according to the manufacturers suggestions, together with pI standards, using a precast Ampholine® PAGplate (pH 3.5 to 9.5) and focused for 1.5 hrs. After staining, the migration distance from the cathodic edge of the gel to the different protein bands was measured. The pI of each unknown was determined by using a standard curve generated by a plot of the migration distance of standards versus the corresponding pI's. With this technique, the pI of placental bikunin (102-159) was determined to be 8.3, in agreement with the value predicted from the amino acid sequence. This is lower than the value of 10.5 established for the pI of aprotinin. (Tenstad et al., 1994, Acta Physiol. Scand. 152:33-50).

### Example 2

### Preparation of synthetic placental bikunin (7-64)

Placental bikunin (7-64) (SEQ ID NO: 4) was synthesized, refolded and purified essentially as described for placental bikunin (102-159) (SEQ ID NO:6) in Example 1 but with the following modifications: during refolding, the synthetic peptide was stirred for 30 hr as a solution in 20% DMSO at 25°C; purification by C18 RP-HPLC was achieved with a linear gradient of 25 to 45% acetonitrile in 0.1 % TFA over 40 min (1ml/min). Active fractions from the first C18 run were reapplied to the column and fractionated with a linear gradient (60 min, 1 ml/min) of 20 to 40% acetonitrile in 0.1 % TFA.

***Results.*** The final purified reduced peptide exhibited an MH+ = 6563, consistent with the sequence:

IHDFCLVSKV VGRCRASMPR WWYNVTDGSC QLFVYGGCDG NSNNYLTKEE
CLKKCATV (SEQ ID NO.: 4)

The refolding and purification yielded a functional Kunitz domain that was active as an inhibitor of trypsin (Table 2 below).

**Table 2A** **Purification table for the isolation of synthetic placental bikunin (7-64)**

| TABLE 2A | | | | | | |
|---|---|---|---|---|---|---|
| Purification Step | Vol (ml) | mg/ml | mg | Units (U) | SpA (U/mg) | Yield |
| 8.0 M Urea | 8.0 | 2.5 | 20.0 | 0 | 0 | - |
| 20% DMSO | 64.0 | 0.31 | 20.0 | 68,699 | 3,435 | 100 |
| Kall affinity pH 4.0 | 11.7 | 0.10 | 1.16 | 43,333 | 36,110 | 62 |
| Kall affinity pH 1.7 | 9.0 | 0.64 | 5.8 | 4972 | 857 | 7.2 |
| C18-1 | 4.6 | 0.14 | 0.06 | 21,905 | 350,143 | 31.9 |
| C18-2 | 1.0 | 0.08 | 0.02 | 7,937 | 466,882 | 11.5 |

The purified refolded protein exhibited an MH+ = 6558, i.e. $5\pm1$ mass units less than for the reduced peptide. This demonstrates that refolding caused the formation of at least one appropriate disulfide bond.

The pI of placental bikunin (7-64) was determined using the methods employed to determine the pI of placental bikunin (102-159). Placental bikunin (7-64) exhibited a pI that was much higher than the predicted value (pI = 7.9). Refolded placental bikunin (7-64) migrated to the cathodic edge of the gel (pH 9.5) and an accurate pI could not be determined under these conditions.

***Continued Preparation of synthetic placental bikunin (7-64)***

Because the synthetic placental bikunin (7-64) may not have undergone complete deprotection prior to purification and refolding, refolding was repeated using protein which was certain to be completely deprotected. Placental bikunin (7-64) was synthesized, refolded and purified essentially as described for placental bikunin (102-159) but with the following modifications: during refolding, the synthetic peptide (0.27 mg/ml) was stirred for 30 hr as a solution in 20% DMSO at 25 C; purification by C18 RP-HPLC was achieved with a linear gradient of 22.5 to 50% acetonitrile in 0.1% TFA over 40 min (1 ml/min).

***Results.*** The final purified reduced peptide exhibited an MH+ = 6567.5 , consistent with the sequence:

IHDFCLVSKV VGRCRASMPRW WYNVTDGSC QLFVYGGCDG NSNNYLTKEE
CLKKCATV (SEQ ID NO.: 4)

The refolding and purification yielded a functional Kunitz domain that was as active as an inhibitor of trypsin (Table 2B below).

**Table 2B** **Purification table for the isolation of synthetic placental bikunin (7-64)**

| TABLE 2B | | | | | | |
|---|---|---|---|---|---|---|
| Purification Step | Vol (ml) | mg/ml | mg | Units (U) | SpA (U/mg) | Yield |
| 8.0 M Urea | 4.9 | 2.1 | 10.5 | 0 | 0 | - |
| 20% DMSO | 39.0 | 0.27 | 10.5 | 236,000 | 22,500 | 100 |
| Kallikrein Affinity (pH 2) | 14.5 | 0.3 | 0.43 | 120,000 | 279,070 | 50.9 |
| C18 Reverse-Phase | 0.2 | 1.2 | 0.24 | 70,676 | 294,483 | 30.0 |

The purified refolded protein exhibited an MH+ = 6561.2, i.e. 6.3 mass units less than for the reduced peptide. This demonstrates that refolding caused the formation of the expected three disulfide bonds.

The pI of refolded placental bikunin (7-64) was determined using the methods employed to determine the pI of placental bikunin (102-159). Refolded placental bikunin (7-64) exhibited a pI of 8.85, slightly higher than the predicted value (pI = 7.9).

**Example 3**

**In vitro specificity of functional placental bikunin fragment (102-159)**

***Proteases.*** Bovine trypsin, human plasmin, and bovine pancreatic kallikrein quantitation was carried out by active site titration using p-nitrophenyl p'-guanidinobenzoate HCl as previously described (Chase,T., and Shaw, E., (1970) Methods Enzmol. 19, 20-27). Human kallikrein was quantitated by active site titration using bovine aprotinin as a standard and PFR-AMC as a substrate assuming a 1:1 complex formation. The $K_m$ for GPK-AMC with trypsin and plasmin under the conditions used for each enzyme was 29 $\mu$M and 726 $\mu$M, respectively; the $K_m$ for PFR-AMC with human plasma kallikrein and bovine pancreatic kallikrein was 457 $\mu$M and 81.5 $\mu$M, respectively; the $K_m$ for AAPR-AMC with elastase was 1600 $\mu$M. Human tissue kallikrein (Bayer, Germany) quantification was carried out by active site titration using p'nitrophenyl p'-guanidinobenzoate HCl as previously described (Chase, T., and Shaw, E., (1970) Methods Enzmol. 19,20-27).

***Inhibition Kinetics:*** The inhibition of trypsin by placental bikunin (102-159) (described in Example 1) or aprotinin was measured by the incubation of 50 pM trypsin with placental bikunin (102-159) (0-2 nM) or aprotinin (0-3 nM) in buffer A in a total volume of 1.0 ml. After 5 min. at 37°C, 15 $\mu$l of 2 mM GPK-AMC was added and the change in fluorescence (as above) was monitored. The inhibition of human plasmin by placental bikunin (102-159) and aprotinin was determined with plasmin (50 pM) and placental bikunin (102-159) (0-10 nM) or aprotinin (0-4 nM) in buffer containing 50 mM Tris-HCl (pH 7.5), 0.1 M NaCl, and 0.02% triton x-100. After 5 min. incubation at 37°C, 25 $\mu$l of 20 mM GPK-AMC was added and the change in fluorescence monitored. The inhibition of human plasma kallikrein by placental bikunin (102-159) or aprotinin was determined using kallikrein (2.5 nM) and placental bikunin (102-159) (0-3 nM) or aprotinin (0-45 nM) in 50 mM Tris-HCl (pH 8.0), 50 mM NaCl, and 0.02% triton x-100. After 5 min. at 37°C 15 $\mu$l of 20 mM PFR-AMC was added and the change in fluorescence monitored. The inhibition of bovine pancreatic kallikrein by placental bikunin (102-159) and aprotinin was determined in a similar manner with kallikrein (92 pM), placental bikunin (102-159) (0-1.6 nM) and aprotinin (0-14 pM) and a final substrate concentration of 100 $\mu$M. The apparent inhibition constant $K_i$* was determined using the nonlinear regression data analysis program Enzfitter software (Biosoft, Cambridge, UK): The kinetic data from each experiment were analyzed in terms of the equation for a tight binding inhibitor:

$$V_i/V_o = 1 - (E_o + I_o + K_i^* - [(E_o + I_o + K_i^*)^2 - 4\,E_oI_o]^{1/2})/2E_o \qquad (2)$$

where $V_i/V_o$ is the fractional enzyme activity (inhibited vs. uninhibited rate), and $E_o$ and $I_o$ are the total concentrations of enzyme and inhibitor, respectively. Ki values were obtained by correcting for the effect of substrate according to the equation:

$$K_i = K_i^*/(1 + [S_o]/K_m) \qquad (3)$$

(Boudier, C., and Bieth, J. G., (1989) Biochim Biophys Acta. 995: 36-41)

For the inhibition of human neutrophil elastase by placental bikunin (102-159) and aprotinin, elastase (19 nM) was incubated with placental bikunin (102-159) (150 nM) or aprotinin (0-7.5 $\mu$M) in buffer containing 0.1 M Tris-HCl (pH 8.0), and 0.05% triton X-100. After 5 min at 37°C, AAPM-AMC (500 $\mu$M or 1000 $\mu$M) was added and the fluorescence measured over a two-minute period. Ki values were determined from Dixon plots of the form 1/V versus [I] performed at two different substrate concentrations (Dixon et al., 1979).

The inhibition of human tissue kallikrein by aprotinin, placental bikunin fragment (7-64) or placental bikunin fragment (102-159) was measured by the incubation of 0.35 nM human tissue kallikrein with placental bikunin (7-64) (0-40 nM) or placental bikunin (102-159) (0-2.5 nM), or aprotinin (0-0.5 nM) in a 1 ml reaction volume containing 50 mM Tris-HCl buffer pH 9.0, 50 mM NaCl, and 0.1 % triton x-100. After 5 min. at 37°C, 5 ul of 2 mM PFR-AMC was added achieving 10 uM final and the change in fluorescence monitored. The Km for PFR-AMC with human tissue kallikrein under the conditions employed was 5.7 uM. The inhibition of human factor Xa (American Diagnostica, Inc, Greenwich, CT) by synthetic placental bikunin (102-159), recombinant placental bikunin, and aprotinin was measured by the incubation of 0.87 nM human factor Xa with increasing amounts of inhibitor in buffer containing 20 mM Tris (pH 7.5), 0.1 M NaCl, and 0.1 % BSA. After 5 min. at 37°C, 30 ul of 20 mM LGR-AMC (Sigma) was added and the change in fluorescence monitored. The inhibition of human urokinase (Sigma) by Kunitz inhibitors was measured by the incubation of urokinase (2.7 ng) with inhibitor in a total volume of 1 ml buffer containing 50 mM Tris-HCl (pH 8.0), 50 mM NaCl, and 0.1% Triton x-100. After 5 min. at 37°C, 35 ul of 20 mM GGR-AMC (Sigma) was added and the change in fluorescence monitored. The inhibition of Factor XIa (from Enzyme Research Labs, Southbend, IN) was measured by incubating FXIa (0.1 nM) with either 0 to 800 nM placental bikunin (7-64), 0 to 140 nM placental bikunin (102-159) or 0 to 40 uM aprotinin in buffer containing 50 mM Hepes pH 7.5, 100 mM NaCl, 2 mM CaCl2, 0.01 % triton x-100, and 1% BSA in a total volume of 1 ml. After 5 min at 37 C, 10 ul of 40 mM Boc-Glu(OBzl)-Ala-Arg-AMC (Bachem Biosciences, King of Prussia, PA) was added and the change in fluorescence monitored.

***Results:*** A direct comparison of the inhibition profiles of placental bikunin (102-159) and aprotinin was made by measuring their inhibition constants with various proteases under identical conditions. The Ki values are listed in Table 3 below.

**Table 3 Ki values for the inhibition of various proteases by bikunin (102-159)**

TABLE 3

| Protease (concentration) | Bikunin (102-159) Ki (nM) | Aprotinin Ki (nM) | Substrate (concentration) | Km (mM) |
|---|---|---|---|---|
| Trypsin (48.5 pM) | 0.4 | 0.8 | GPK-AMC (0.03 mM) | 0.022 |
| Chymotrypsin (5 nM) | 0.24 | 0.86 | AAPF-pNA (0.08 mM) | 0.027 |
| Bovine Pancreatic Kallikrein (92.0 pM) | 0.4 | 0.02 | PFR-AMC (0.1 mM) | 0.08 |
| Human Plasma Kallikrein (2.5 nM) | 0.3 | 19.0 | PFR-AMC (0.3 mM) | 0.46 |
| Human Plasmin (50 pM) | 1.8 | 1.3 | GPK-AMC (0.5 mM) | 0.73 |
| Human Neutrophil Elastase (19 nM) | 323.0 | 8500.0 | AAPM-AMC (1.0 μM) | 1.6 |
| Factor XIIa | >300.0 | 12,000.0 | PFR-AMC (0.2 μM) | 0.35 |
| Human Tissue Kallikrein (0.35 nM) | 0.13 | 0.004 | PFR-AMC (10 μM) | 0.0057 |
| factor Xa (0.87 nM) | 274 | N.I. at 3 μM | LGR-AMC (0.6 mM) | N.D. |
| Urokinase | 11000 | 4500 | GGR-AMC (0.7 mM) | N.D. |
| factor Xia (0.1 nM) | 15 | 288 | E(OBz)AR-AMC (0.4 mM) | 0.46 |

Placental bikunin (102-159) and aprotinin inhibit bovine trypsin and human plasmin to a comparable extent under the conditions employed. Aprotinin inhibited elastase with a Ki of 8.5 μM. Placental bikunin (102-159) inhibited elastase with a Ki of 323nM. The $K_i$ value for the placental bikunin (102-159) inhibition of bovine pancreatic kallikrein was 20-fold higher than that of aprotinin inhibition. In contrast, placental bikunin (102-159) is a more potent inhibitor of human plasma kallikrein than aprotinin and binds with a 56-fold higher affinity.

Because placental bikunin (102-159) is greater than 50 times more potent than Trasylol® as an inhibitor of kallikrein, smaller amounts of human placental bikunin, or fragments thereof (i.e. placental bikunin (102-159)) are needed than Trasylol® in order to maintain the effective patient doses of inhibitor in KIU. This reduces the cost per dose of the drug and reduces the likelihood of adverse nephrotoxic effects upon re-exposure of the medicament to patients. Furthermore, the protein is human derived, and thus much less immunogenic in man than aprotinin which is derived from cows. This results in significant reductions in the risk of incurring adverse immunologic events upon re-exposure of the medicament to patients.

**Example 4**

**In vitro specificity of functional placental bikunin fragment (7-64)**

In vitro specificity of the functional human placental bikunin (7-64) described in Example 2 was determined using the materials and methods as described in the Examples above.

***Results:*** The table below shows the efficacy of placental bikunin (7-64) as an inhibitor of various serine proteases *in vitro.* Data is shown compared against data obtained for screening inhibition using either placental bikunin (102-159), or aprotinin (Trasylol®).

**Table 4 A Ki values for the inhibition of various proteases by bikunin(7-64)**

TABLE 4A

| Protease (concentration) | bikunin(7-64) Ki (nM) | Aprotinin Ki (nM) | Bikunin (102-159) $K_i$ (nM) |
|---|---|---|---|
| Trypsin (48.5 pM) | 0.17 | 0.8 | 0.4 |
| Bovine Pancreatic Kallikrein (920 pM) | 0.4 | 0.02 | 0.4 |
| Human Plasma Kallikrein (2.5 nM) | 2.4 | 19.0 | 0.3 |
| Human Plasmin (50 pM) | 3.1 | 1.3 | 1.8 |
| Bovine chymotrypsin (5 nM) | 0.6 | 0.9 | 0.2 |
| Factor XIIa | >300 | 12000 | >300 |
| elastase | >100 | 8500 | 323 |

The results show that the amino acid sequence encoding placental bikunin (7-64) can be refolded to obtain an active serine protease inhibitor that is effective against at least four trypsin-like serine proteases.

Table 4B below also shows the efficacy of refolded placental bikunin (7-64) as an inhibitor of various serine proteases *in vitro*. Refolded placental bikunin (7-64) was prepared from protein that was certain to be completely deprotected prior to purification and refolding. Data is shown compared against data obtained for screening inhibition using either placental bikunin (102-159), or aprotinin (Trasylol®).

**Table 4B Ki values for the inhibition of various proteases by refolded bikunin (7-64)**

TABLE 4B

| Protease (concentration) | bikunin (7-64) Ki (nM) | Aprotinin Ki (nM) | bikunin (102-159) Ki (nM) |
|---|---|---|---|
| Trypsin (50 pM) | 0.2 | 0.8 | 0.3 |
| Human Plasma Kallikrein (0.2 nM) | 0.7 | 19.0 | 0.7 |
| Human Plasmin (50 pM) | 3.7 | 1.3 | 1.8 |
| Factor XIIa | not done | 12,000 | 4,500 |
| Factor XIa (0.1 nM) | 200 | 288 | 15 |
| Human Tissue Kallikrein | 2.3 | 0.004 | 0.13 |

Suprisingly, placental bikunin (7-64) was more potent than aprotinin at inhibiting human plasma kallikrein, and at least similar in efficacy as a plasmin inhibitor. These data show that placental bikunin (7-64) is at least as effective as aprotinin, using *in vitro* assays, and that one would expect better or similar potency *in vivo*.

**Example 5**

**Expression of placental bikunin variant (102-159) in yeast**

The DNA sequence encoding placental bikunin 102-159 (SEQ ID NO.: 6) was generated using synthetic oligonucleotides. The final DNA product consisted (5' to 3') of 15 nucleotides from the yeast $\alpha$-mating factor propeptide sequence fused to the in-frame cDNA sequence encoding placental bikunin (102-159), followed by an in-frame stop codon. Upon cloning into a yeast expression vector pS604, the cDNA would direct the expression of a fusion protein comprising an N-terminal yeast $\alpha$-mating factor propeptide fused to the 58 amino acid sequence of placental bikunin (102-159). Processing of this fusion protein at a KEX-2 cleavage site at the junction between the $\alpha$-mating factor and Kunitz domain was designed to liberate the Kunitz domain at its native N-terminus.

A 5' sense oligonucleotide of the following sequence and containing a HindIII site for cloning was synthesized:

GAA GGG GTA AGC TTG GAT AAA AGA TAT GAA GAA TAC TGC ACC GCC AAC GCA GTC ACT GGG CCT TGC CGT GCA TCC TTC CCA CGC TGG TAC TTT GAC GTG GAG AGG (SEQ ID NO.: 42)

A 3' antisense oligonucleotide of the following sequence and containing both a BamHI site for cloning and a stop codon was synthesized:

CGC GGA TCC CTA CTG GCG GAA GCA GCG GAG CAT GCA GGC CTC CTC AGA GCG GTA GCT GTT CTT ATT GCC CCG GCA GCC TCC ATA GAT GAA GTT ATT GCA GGA GTT CCT CTC CAC GTC AAA GTA CCA GCG (SEQ ID NO.: 43)

The oligonucleotides were dissolved in 10 mM Tris buffer pH 8.0 containing 1 mM EDTA, and 12 ug of each oligo were added combined and brought to 0.25M NaCl. To hybridize, the oligonucleotides were denatured by boiling for 5 minutes and allowed to cool from 65°C to room temp over 2 hrs. Overlaps were extended using the Klenow fragment and digested with HindIII and BamHI. The resulting digested double stranded fragment was cloned into pUC19 and sequence confirmed. A clone containing the fragment of the correct sequence was digested with BamHI/HindIII to liberate the bikunin containing fragment with the following + strand sequence:

GAA GGG GTA AGC TTG GAT AAA AGA TAT GAA GAA TAC TGC ACC GCC AAC GCA GTC ACT GGG CCT TGC CGT GCA TCC TTC CCA CGC TGG TAC TTT GAC GTG GAG AGG AAC TCC TGC AAT AAC TTC ATC TAT GGA GGC TGC CGG GGC AAT AAG AAC AGC TAC CGC TCT GAG GAG GCC TGC ATG CTC CGC TGC TTC CGC CAG TAG GGA TCC (SEQ ID.: 44)

which was then gel purified and ligated into BamHI/HindIII cut pS604. The ligation mixture was extracted into phenol/chloroform and purified over a S-200 minispin column. The ligation product was directed transformed into yeast strains SC101 and WHL341 and plated on ura selection plates. Twelve colonies from each strain were re-streaked on ura drop out plates. A single colony was inoculated into 2 ml of ura DO media and grown over night at 30°C. Cells were pelleted for 2 minutes at 14000x g and the supernatants evaluated for their content of placental bikunin (102-159).

***Detection of expression of placental bikunin (102-159) in transformed yeast***

Firstly, the supernatants (50 ul per assay) were evaluated for their capacity to inhibit the *in vitro* activity of trypsin using the assay methods as described in Example 1 (1 ml assay volume). An un-used media only sample as well as a yeast clone expressing an inactive variant of aprotinin served as negative controls. A yeast clone expressing natural aprotinin served as a positive control and is shown for comparison.

The second method to quantify placental bikunin (102-159) expression exploited use of polyclonal antibodies (pAbs) against the synthetic peptide to monitor the accumulation of the recombinant peptide using Western blots. These studies were performed only with recombinants derived from strain SC101, since these produced greater inhibitory activity than recombinants derived from strain HL341.

To produce the pAb, two 6-8 week old New Zealand White female rabbits (Hazelton Research Labs, Denver, Pa) were immunized on day zero with 250 ug of purified reduced synthetic placental bikunin (102-159), in Complete Freund's adjuvant, followed by boosts on days 14, 35 and 56 and 77 each with 125 ug of the same antigen in Incomplete Freund's adjuvant. Antiserum used in the present studies was collected after the third boost by established procedures. Polyclonal antibodies were purified from the antiserum over protein A.

Colonies 2.4 and 2.5 from transformation of yeast SC101 (Figure 8) as well as an aprotinin control were grown overnight in 50 ml of ura DO media at 30°C. Cells were pelleted and the supernatant concentrated 100-fold using a Centriprep 3 (Amicon, Beverly, MA) concentrator. Samples of each (30 μl) were subjected to SDS-PAGE on 10-20% tricine buffered gels (Novex, San Diego, CA) using the manufacturers procedures. Duplicate gels were either developed with a silver stain kit (Integrated Separation Systems, Nantick, MA) or transferred to nitrocellulose and developed with the purified polyclonal antibody elicited to synthetic bikunin (102-159). Alkaline-phosphatase conjugated goat anti-rabbit antibody was used as the secondary antibody according to the manufacturer's directions (Kirkegaard and Perry, Gaithersburg, MD).

***Purification of placental bikunin (102-159) from a transformed strain of SC101***

Fermentation broth from a 1L culture of SC101 strain 2.4 was harvested by centrifugation (4,000 g x 30 min.) then applied to a 1.0 ml column of anhydrochymotrypsin-sepharose (Takara Biochemical Inc., CA), that was previously equilibrated with 50 mM Hepes buffer pH 7.5 containing 0.1M NaCl, 2 mM $CaCl_2$ and 0.01% (v/v) triton X-100. The column was washed with the same buffer but containing 1.0 M NaCl until the A280nm declined to zero, whereupon the column was eluted with 0.1M formic acid pH 2.5. Eluted fractions were pooled and applied to a C18 column (Vydac, 5um, 4.6 x 250 mm) previously equilibrated with 0.1 % TFA, and eluted with a 50 min. linear gradient of 20 to 80% acetonitrile in 0.1% TFA. Fractions containing placental bikunin (102-159) were pooled and re-chromatographed on C18 employing elution with a linear 22.5 to 50% acetonitrile gradient in 0.1 % TFA.

***Results.*** Figure 8 shows the percent trypsin activity inhibited by twelve colonies derived from the transformation of each of strains SC101 and WHL341. The results show that all twelve colonies of yeast strain SC101 transformed with the trypsin inhibitor placental bikunin (102-159) had the ability to produce a substantial amount of trypsin inhibitory activity compared to the negative controls both of which showed no ability to inhibit trypsin. The activity is therefore related to the expression of a specific inhibitor in the placental bikunin variant (102-159) transformed cells. The yeast WHL341 samples contained minimal trypsin inhibitory activity. This may be correlated to the slow growth observed with this strain under the conditions employed.

Figure 9 shows the SDS-PAGE and western analysis of the yeast SC101 supernatants. Silver stained SDS-PAGE of supernatants derived from recombinant yeasts 2.4 and 2.5 expressing placental bikunin (102-159) as well as from the yeast expressing aprotinin yielded a protein band running at approximated 6 kDa, corresponding to the size expected for each recombinant Kunitz inhibitor domain. Western analysis showed that the 6 kDa bands expressed by stains 2.4. and 2.5 reacted with the pAb elicited to placental bikunin (102-159). The same 6 kDa band in the aprotinin control did not react with the same antibody, demonstrating the specificity of the antibody for the placental bikunin variant (102-159).

The final preparation of placental bikunin C-terminal domain was highly pure by silver-stained SDS-PAGE (Figure 10). The overall recovery of broth-derived trypsin inhibitory activity in the final preparation was 31%. N-terminal sequencing of the purified inhibitor indicated that 40% of the protein is correctly processed to yield the correct N-terminus for placental bikunin (102-159) while about 60 % of the material contained a portion of the yeast $\alpha$-mating factor. The purified material comprised an active serine protease inhibitor exhibiting an apparent Ki of 0.35 nM for the *in vitro* inhibition of plasma kallikrein.

In conclusion, the accumulation both of a protease inhibitor activity and a protein immunochemically related to synthetic bikunin (102-159) in fermentation broth as well as the isolation of placental bikunin (102-159) from one of the transformed lines provided proof of expression of placental bikunin in the recombinant yeast strains described herein, showing for the first time the utility of yeasts for the production of placental bikunin fragments.

Additional constructs were prepared in an effort to augment the expression level of the Kunitz domain contained within placental bikunin 102-159, as well as to increase the yield of protein with the correct N-terminus. We hypothesized that the N-terminal residues of placental bikunin 102-159 (YEEY-) may have presented a cleavage site that is only poorly recognized by the yeast KEX-2 protease that enzymically removes the yeast a-factor pro-region. Therefore, we prepared yeast expression constructs for the production of placental bikunin 103-159 (N-terminus of EEY...), 101-159 (N-terminus of NYEEY...) and 98-159 (DMFNYEEY..) in order to modify the P' subsites surrounding the KEX-2 cleavage site. To attempt to augment the levels of recombinant protein expression, we also used the yeast preferred codons rather than mammalian preferred codons in preparing some of the constructs described below. The constructs were essentially prepared as described above for placental bikunin 102-159 (defined as construct #1) but with the following modifications:

Construct #2 placental bikunin 103-159, yeast codon usage
A 5' sense oligonucleotide

GAAGGGGTAA GCTTGGATAA AAGAGAAGAA TACTGTACTG CTAATGCTGT
TACTGGTCCA TGTAGAGCTT CTTTTCCAAG ATGGTACTTT GATGTTGAAA GA
(SEQ ID NO.: 55)

and 3' antisense oligonucleotide

ACTGGATCCT CATTGGCGAA AACATCTCAA CATACAGGCT TCTTCAGATC TGTAAGAATT TTTATTACCT CTACAACCAC CGTAAATAAA ATTATTACAA GAATTTCTTT CAACATCAAA GTACCATCT (SEQ ID NO.: 56)

were manipulated as described for the production of an expression construct (construct #1 above) for the expression of placental bikunin 102-159

Construct #3 placental bikunin 101-159, yeast codon usage
A 5' sense oligonucleotide

GAAGGGGTAA GCTTGGATAA AAGAAATTAC GAAGAATACT GTACTGCTAA TGCTGTTACT GGTCCATGTA GAGCTTCTTT TCCAAGATGG TACTTTGATG TTGAAAGA (SEQ ID NO.: 57)

and the same 3' antisense oligonucleotide as used for construct #2, were manipulated as described for the production of an expression construct (construct #1 above) for the expression of placental bikunin 102-159.

Construct #4 placental bikunin 98-159, yeast codon usage A 5' sense oligonucleotide

GAAGGGGTAA GCTTGGATAA AAGAGATATG TTTAATTACG AAGAATACTG TACTGCTAAT GCTGTTACTG GTCCATGTAG AGCTTCTTTT CCAAGATGGT ACTTTGATGT TGAAAGA (SEQ ID NO.: 58)

and the same 3' antisense oligonucleotide as used for construct #2, were manipulated as described for the production of an expression construct (construct #1 above).

Yeast strain SC101 (MAT$\alpha$, ura 3-52, suc 2) was transformed with the plasmids containing each of the above cDNAs, and proteins were expressed using the methods that were described above for the production of placental bikunin 102-159 with human codon usage. Approximately 250 ml of each yeast culture was harvested, and the supernatant from centrifugation (15 min x 3000 RPM) separately subjected to purification over 1 ml columns of kallikrein-sepharose as described above. The relative amount of trypsin inhibitory activity in the applysate, the amount of purified protein recovered and the N-terminal sequence of the purified protein were determined and are listed below in Table 7.

**Table 7 Relative production levels of different proteins containing the C-terminal Kunitz domain of placental bikunin**

| TABLE 7 | | | | |
|---|---|---|---|---|
| Construct | Relative conc. of inhibitor in applysate | N-terminal Amount (pmol) | sequencing sequence | Comments |
| #2 103-159 | none detected | none | none | no expression |
| #3 101-159 | 25 % inhibition | none | none | low expression |

(continued)

| TABLE 7 | | | | |
|---|---|---|---|---|
| Construct | Relative conc. of inhibitor in applysate | N-terminal Amount (pmol) | sequencing sequence | Comments |
| #4 98-159 product | 93 % inhibition | 910 | DMFNYE- | good expression correct |
| #1 102-159 | 82 % inhibition | 480 | AKEEGV- | expression of active incorrectly processed protein |

The results show that placental bikunin fragments of different lengths that contain the C-terminal Kunitz domain show wide variation in capacity to express functional secreted protein. Constructs expressing fragments 101-159 and 103-159 yielded little or low enzymic activity in the supernatants prior to purification, and N-terminal sequencing of 0.05 ml aliquots of each purified fraction yielded undetectable amounts of inhibitor. On the other hand expression either of placental bikunin 102-159 or 98-159 yielded significant amounts of protease activity prior to purification. N-terminal sequencing however showed that the purified protein recovered from expression of 102-159 was once again largely incorrectly processed, exhibiting an N-terminus consistent with processing of the majority of the pre-protein at a site within the yeast α-mating factor pro-sequence. The purified protein recovered from expression of placental bikunin 98-159 however was processed entirely at the correct site to yield the correct N-terminus. Furthermore, nearly twice as much protein was recovered as compared to the recovery of placental bikunin 102-159. Placental bikunin 98-159 thus represents a preferred fragment length for the production of the C-terminal Kunitz domain of placental bikunin by the α-mating factor pre-pro sequence/ KEX-2 processing system of S. *cerevisiae,*

**Example 6**

**Alternative procedure for yeast expression**

The 58 amino acid peptide derived from the R74593 translation product can also be PCR amplified from either the R87894-R74593 PCR product cloned into the TA vector™ (Invitrogen, San Diego, CA) after DNA sequencing or from human placental cDNA. The amplified DNA product will consist of 19 nucleotides from the yeast α-mating factor leader sequence mated to the R74593 sequence which codes for the YEEY-CFRQ (58 residues) so as to make the translation product in frame, constructing an α -mating factor/Kunitz domain fusion protein. The protein sequence also contains a kex 2 cleavage which will liberate the Kunitz domain at its native N-terminus.

The 5' sense oligonucleotide which contains a HindIII site for cloning will contain the following sequence:

GCCAAGCTTG GATAAAAGAT ATGAAGAAT ACTGCACCGC CAACGCA (SEQ ID NO.: 30)

The 3' antisense oligonucleotide contains a BamHI site for cloning as well as a stop codon and is of the following sequence:

GGGGATCCTC ACTGCTGGCG GAAGCAGCGG AGCAT (SEQ ID NO.: 31)

The full 206 nucleotide cDNA sequence to be cloned into the yeast expression vector is of the following sequence:

CCAAGCTTGG ATAAAAGATA TGAAGAATAC TGCACCGCCA ACGCAGTCAC
TGGGCCTTGC CGTGCATCCT TCCCACGCTG GTACTTTGAC GTGGAGAGGA
ACTCCTGCAA TAACTTCATC TATGGAGGCT GCCGGGGCAA TAAGAACAGC
TACCGCTCTG AGGAGGCCTG CATGCTCCGC TGCTTCCGCC AGCAGTGAGG
ATCCCC (SEQ ID NO.: 32)

After PCR amplification, this DNA will be digested with HindIII, BamHI and cloned into the yeast expression vector pMT15 (see US patent 5,164,482, incorporated by reference in the entirety) also digested with HindIII and BamHI. The resulting plasmid vector is used to transform yeast strain SC 106 using the methods described in US patent 5,164,482. The URA 3+ yeast transformants are isolated and cultivated under inducing conditions. The yield of recombinant Placental bikunin variants is determined according to the amount of trypsin inhibitory activity that accumulated in the culture supernatants over time using the *in vitro* assay method described above. Fermentation broths are centrifuged at 9000 rpm for 30 minutes. The supernatant is then filtered through a 0.4 then a 0.2 μm filter, diluted to a conductivity of 7.5 ms, and adjusted to pH 3 with citric acid. The sample is then batch absorbed onto 200 ml of S-sepharose fast flow (Pharmacia) in 50 mM sodium citrate pH 3 and stirred for 60 min. The gel is subsequently washed sequentially with 2 L of each of: 50 mM sodium citrate pH 3.0; 50 mM Tris-HCL pH 9.0; 20 mM HEPES pH 6.0. The washed gel is transferred into a suitable column and eluted with a linear gradient of 0 to 1 M sodium chloride in 20 mM HEPES pH 6.0. Eluted fractions containing *in vitro* trypsin inhibitory activity are then pooled and further purified either by a) chromatography over a column of immobilized anhydrotrypsin (essentially as described in Example 2); b) by chromatography over a column of immobilized bovine kallikrein; or c) a combination of conventional chromatographic steps including gel filtration and /or anion-exchange chromatography.

**Example 7**

**Isolation and characterization of native human placental bikunin from placenta**

Bikunin protein was purified to apparent homogeniety from whole frozen placenta (Analytical Biological Services, Inc, Wilmington, DE). The placenta (740 gm) was thawed to room temperature and cut into 0.5 to 1.0 cm pieces, placed on ice and washed with 600 ml PBS buffer. The wash was decanted and 240 ml of placenta pieces placed into a Waring blender. After adding 300 ml of buffer consisting of 0.1 M Tris (pH 8.0), and 0.1 M NaCl, the mixture was blended on high speed for 2 min, decanted into 750.0 ml centrifuge tubes, and placed on ice. This procedure was repeated until all material was processed. The combined slurry was centrifuged at 4500 x g for 60 minutes at 4°C. The supernatant was filtered through cheese cloth and the placental bikunin purified using a kallikrein affinity column made by covalently attaching 70 mg of bovine pancreatic kallikrein (Bayer AG) to 5.0 mls of CNBr activated Sepharose (Pharmacia) according to manufacturers instruction. The material was loaded onto the affinity column at a flow rate of 2.0 ml/min and washed with 0.1 M Tris (pH 8.0), 0.1 M NaCl until absorbance at 280 nm of the wash could no longer be detected. The column was further washed with 0.1 M Tris (pH 8.0), 0.5 M NaCl and then eluted with 3 volumes of 0.2 M acetic acid, pH 4.0. Fractions containing kallikrein and trypsin inhibitory (see below) activity were pooled, frozen, and lyophilized. Placental bikunin was further purified by gel-filtration chromatography using a Superdex 7510/30 (Pharmacia) column attached to a Beckman System Gold HPLC system. Briefly, the column was equilibrated in 0.1 M Tris, 0.15 M NaCl, and 0.1% Triton X-100 at a flow rate of 0.5 ml/min. The lyophilized sample was reconstituted in 1.0 ml of 0.1 M Tris, pH 8.0 and injected onto the gel-filtration column in 200 μl aliquots. Fractions were collected (0.5 ml) and assayed for trypsin and kallikrein inhibitory activity. Active fractions were pooled, and the pH of the solution adjusted to 2.5 by addition of TFA. The material was directly applied to a Vydac C18 reverse-phase column (5 micron, 0.46 x 25 cm) which had been equilibrated in 20% acetonitrile in 0.1 %TFA. Separation was achieved using a linear gradient of 20 to 80% acetonitrile in 0.1% TFA at 1.0 ml/min over 50 minutes after an initial 20 minute wash at 20% acetonitrile in 0.1% TFA. Fractions (1ml) were collected and assayed for trypsin and kallikrein inhibitory activity. Fractions containing inhibitory activity were concentrated using a speed-vac concentrator (Savant) and subjected to N-terminal sequence analysis.

***Functional assays for Placental Bikunin:***

Identification of functional placental bikunin was achieved by measuring its ability to inhibit bovine trypsin and human plasma kallikrein. Trypsin inhibitory activity was performed in assay buffer (50 mM Hepes, pH 7.5, 0.1 M NaCl, 2.0 mM CaCl2, 0.1 % Triton x-100) at room temperature in a 96-well microtiter plate (Perkin Elmer) using Gly-Pro-Lys-Aminomethylcoumarin as a substrate. The amount of coumarin produced by trypsin was determined by measuring the fluorescence (ex = 370 nm, em = 432 nm) on a Perkin-Elmer LS-50B fluorimeter equipped with a plate reader. Trypsin (23 μg in 100 μl buffer) was mixed with 20 μl of the sample to be tested and incubated for 10 minutes at 25°C. The reaction was started by the addition of 50 μl of the substrate GPK-AMC (33 μM final) in assay buffer. The fluorescence intensity was measured and the % inhibition for each fraction was determined by:

$$\% \text{ inhibition} = 100 \times [1 - Fo/F1]$$

where Fo is the fluorescence of the unknown and F1 is the fluorescence of the trypsin only control. Kallikrein inhibitory activity of the fractions was similarly measured using 7.0 nM kallikrein in assay buffer (50 mM Tris, pH 8.0, 50 mM NaCl, 0.1% triton x-100) and 66.0 μM Pro-Phe-Arg-AMC as a substrate.

**Determination of the *in vitro* specificity of placental bikunin**

The *In vitro* specificity of native human placental bikunin was determined using the materials and methods as described in the preceding examples above. Placental bikunin was quantified by active site titration against a known concentration of trypsin using GPK-AMC as a substrate to monitor the fraction of unbound trypsin.

***Protein Sequencing***

The 1 ml fraction (C18-29 Delaria) was reduced to 300 ml in volume, on a Speed Vac, to reduce the amount of organic solvent. The sample was then loaded onto a Hewlett-Packard miniature biphasic reaction column, and washed with 1 ml of 2% trifluoroacetic acid. The sample was sequenced on a Hewlett-Packard Model G1005A protein sequencing system using Edman degradation. Version 3.0 sequencing methods and all reagents were supplied by Hewlett-Packard. Sequence was confirmed for 50 cycles.

***Results.*** Placental Bikunin was purified to apparent homogeniety by sequential kallikrein affinity, gel-filtration, and reverse-phase chromatography (see purification table below):

**Table 5 Purification table for native Placental Bikunin (1-179)**

| TABLE 5 | | | | | |
|---|---|---|---|---|---|
| Step | Vol (ml) | OD 280 (/ml) | OD 280 | Units[a] (U) | Units/OD 280 |
| Placenta Supernatant | 1800.0 | 41.7 | 75,060 | 3,000,000 | 40.0 |
| Kallikrein Affinity pH 4.0 | 20.0 | 0.17 | 3.36 | 16,000 | 4,880 |
| Kallikrein Affinity pH 1.7 | 10.2 | 0.45 | 4.56 | 12,000 | 2,630 |
| Superdex 75 | 15.0 | 0.0085 | 0.13 | 3,191 | 24,546 |

[a]One Unit is defined as that amount which inhibits 50% of trypsin activity in a standard assay.

The majority of the kallikrein and trypsin inhibitory activity eluted from the kallikrein affinity column in the pH 4.0 elution. Subsequent gel-filtration chromatography (Figure 5) yielded a peak of kallikrein and trypsin inhibitory activity with a molecular weight range of 10 to 40 kDa as judged by a standard curve generated by running molecular weight standards under identical conditions. Reverse-phase C18 chromatography (Figure 6) yielded 4 peaks of inhibitory activity with the most potent eluting at approximately 30 % acetonitrile. The activity associated with the first peak to elute from C18 (fraction 29) exhibited an amino acid sequence starting with amino acid 1 of the predicted amino acid sequence of placental bikunin (ADRER...; SEQ ID NO.:1), and was identical to the predicted sequence for 50 cycles of sequencing (underlined amino acids in Figure 3). Cysteine residues within this sequence stretch were silent as expected for sequencing of oxidized protein. The cysteine residues at amino acid positions 11 and 20 of mature placental bikunin were later identified from sequencing of the S-pyridylethylated protein whereupon PTH-pyridylethyl-cysteine was recovered at cycles 11 and 20.

Interestingly, the asparagine at amino acid residue number 30 of the sequence (Figure 3) was silent showing that this site is likely to be glycosylated. Fraction 29 yielded one major sequence corresponding to that of placental bikunin starting at residue #1 (27 pmol at cycle 1) plus a minor sequence (2 pmol) also derived from placental bikunin starting at residue 6 (SIHD...). This shows that the final preparation sequenced in fraction 29 is highly pure, and most likely responsible for the protease inhibitory activity associated with this fraction (Figure 6).

Accordingly, the final preparation of placental bikunin from C18 chromatography was highly pure based on a silver-stained SDS-PAGE analysis (Figure 7), where the protein migrated with an apparent Mr of 24 kDa on a 10 to 20 % acrylamide tricine gel (Novex, San Diego, CA) calibrated with the following molecular weight markers: insulin (2.9 kDa); bovine trypsin inhibitor (5.8 kDa); lysozyme (14.7 kDa); β-lactaglobulin (18.4 kDa); carbonic anhydrase (29 kDa); and ovalbumin (43 kDa). The above size of placental bikunin on SDS-PAGE is consistent with that predicted from the full length coding sequence (Figure 4F).

As expected based on the N-terminal sequencing results described above, the purified protein reacted with an antibody elicited to placental bikunin (7-64) to yield a band with the same Mr (Figure 12A) as observed for the purified preparation detected on gels by silver stain (Figure 7). However, when the same preparation was reacted with an antibody elicited to synthetic placental bikunin (102-159), a band corresponding to the full length protein was not observed. Rather, a fragment that co-migrated with synthetic bikunin (102-159) of approximately 6 kDa was observed. The simplest interpretation of these results is that the purified preparation had undergone degradation subsequent to purification to yield an N-terminal fragment comprising the N-terminal domain and a C-terminal fragment comprising the C-terminal domain. Assuming that the fragment reactive against antiserum to placental bikunin (7-64) is devoid of the C-terminal end of the full length protein, the size (24 kDa) would suggest a high state of glycosylation.

Table 6. below shows the potency of *in vitro* inhibition of various serine proteases by placental bikunin. Data are compared with that obtained with aprotinin (Trasylol®).

**Table 6 Ki values for the inhibition of various proteases by placental bikunin**

| TABLE 6 | | |
|---|---|---|
| Protease (concentration) | Placental Bikunin Ki (nM) | Aprotinin Ki (nM) |
| Trypsin (48.5 pM) | 0.13 | 0.8 |
| Human Plasmin (50 pM) | 1.9 | 1.3 |

The results show that placental bikunin isolated from a natural source (human placenta) is a potent inhibitor of trypsin-like serine proteases.

**Example 8**

**Expression pattern of placental bikunin amongst different human organs and tissues**

A multiple tissue northern was purchased from Clontech which contained 2 μg of polyA+ RNA from human heart, brain, placenta, lung, liver, skeletal muscle, kidney, and pancreas. Two different cDNA probes were used: 1) a gel purified cDNA encoding placental bikunin (102-159); 2) the 780 base pair PCR-derived cDNA (Figure 4E) liberated from a TA clone by digestion with EcoRI and gel purified. Each probe was labeled using $^{32}$P-dCTP and a random priming labeling kit from Boehringer Mannheim Biochemicals (Indiana), then used to hybridize to the multiple tissue northern according to the manufacturers specifications. Autoradiographs were generated using Biomax film with an 18 hr exposure time, and developed using a Umax Scanner and scanned using Adobe Photoshop.

***Results.*** The pattern of tissue expression observed using a placental bikunin (102-159) probe (Figure 11A) or a larger probe containing both Kunitz domains of placental bikunin (Figure 11B) was essentially the same as might be expected. The placental bikunin mRNA was most abundant in pancreas and placenta. Significant levels were also observed in lung, brain and kidney, while lower levels were observed in heart and liver, and the mRNA was undetectable in skeletal muscle. The transcript size was 1.95 kilobases in all cases, in close agreement with the predicted size of placental bikunin deduced both from EST overlay and cloning of full length cDNA described in preceding sections.

The broad tissue distribution of the mRNA shows that placental bikunin is broadly expressed. Since the protein also contains a leader sequence it would have ample exposure to the human immune system, requiring that it become recognized as a self protein. Additional evidence for a broad tissue distribution of placental bikunin mRNA expression was derived from the fact that some of the EST entries with homology to placental bikunin (Figure 4B) were derived from human adult and infant brain, and human retina, breast, ovary, olfactory epithelium, and placenta. It is concluded therefore that administration of the native human protein to human patients would be unlikely to elicit an immune response.

Interestingly, the expression pattern of placental bikunin is somewhat reminiscent of that for bovine aprotinin which is found in high levels in bovine lung and pancreas. To further elucidate the expression pattern of placental bikunin, RT-PCR of total RNA from the following human cells was determined: un-stimulated human umbilical vein endothelial cells (HUVECs), HK-2 (line derived from kidney proximal tubule), TF-1 (erythroleukemia line) and phorbolester (PMA)-stimulated human peripheral blood leukocytes. The probes used:

CACCTGATCGCGAGACCCC (sense; SEQ ID NO.: 59);
CTGGCGGAAGCAGCGGAGCATGC (antisense; SEQ ID NO.: 60),

were designed to amplify a 600 b.p placental bikunin encoding cDNA fragment. Comparisons were normalized by inclusion of actin primers to amplify an 800 b.p. actin fragment. Whereas the 800 b.p fragment identified on agarose gels with ethidium bromide was of equal intensity in all lanes, the 600 b.p. placental bikunin fragment was absent from the HUVECs but present in significant amounts in each of the other cell lines. We conclude that placental bikunin is not expressed in at least some endothelial cells but is expressed in some leukocyte populations.

**Example 9**

**Purification and properties of Placental Bikunin (1-170) highly purified from a Baculovirus / Sf9 expression system**

A large fragment of Placental bikunin containing both Kunitz domains (Bikunin (1-170) (SEQ ID NO:52) was expressed in Sf9 cells as follows. Placental bikunin cDNA obtained by PCR (Figure 4E) and contained within a TA vector (see previous Examples) was liberated by digestion with HindIII and Xba1 yielding a fragment flanked by a 5' XbaI site and 3' HindIII site. This fragment was gel purified and then cloned into the M13mp19 vector (New England Biolabs, Beverly, MA). In vitro mutagenesis (Kunkel T.A., (1985) Proc. Natl. Acad. Sci. USA, 82: 488-492) was used to generate a Pst1 site 3' to the XbaI site at the 5' end, but 5' to the sequence encoding the ATG start site, natural placental bikunin signal peptide and mature placental bikunin coding sequence. The oligonucleotide used for the mutagenesis had the sequence:

5' CGC GTC TCG GCT GAC CTG GCC CTG CAG ATG GCG CAC GTG TGC GGG 3' (SEQ ID NO.: 61)

A stop codon (TAG) and BglII / XmaI site was similarly engineered at the 3' end of the cDNA using the oligonucleotide:

5' CTG CCC CTT GGC TCA AAG TAG GAA GAT CTT CCC CCC GGG GGG GTG GTT CTG GCG GGG CTG 3' (SEQ ID NO.: 62).

The stop codon was in frame with the sequence encoding placental bikunin and caused termination immediately following the Lysine at amino acid residue 170, thus encoding a truncated placental bikunin fragment devoid of the putative transmembrane domain. The product from digestion with Pst1 and BglII was isolated and cloned into the BacPac8 vector for expression of Placental bikunin fragment (1-170) which contains both Kunitz domains but which is truncated immediately N-terminal to the putative transmembrane segment.

The expression of Bikunin by Sf-9 insect cells was optimal at a multiplicity of infection of 1 to 1 when the medium was harvested at 72 h post infection. After harvesting, the baculovirus cell culture supernatant (2L) was adjusted to pH 8.0 by the addition of Tris-HCl. Bikunin was purified by chromatography using a 5 ml bovine pancreatic kallikrein affinity column as previously described in Example 7 for the purification of native placental bikunin from placenta. Eluted material was adjusted to pH 2.5 with TFA and subjected to chromatography on a C18 reverse-phase column (1.0 x 25 cm) equilibrated in 10% acetonitrile in 0.1% TFA at a flow rate of 1 ml/min. The bikunin was eluted with a linear gradient of 10 to 80% acetonitrile in 0.1% TFA over 40 min. Active fractions were pooled, lyophilized, redissolved in 50 mM Hepes (pH 7.5), 0.1 M NaCl, 2 mM CaCl2, and 0.1% triton x-100, and stored at -20°C until needed. The concentration of recombinant bikunin was determined by amino acid analysis.

***Results.*** Recombinant bikunin was purified from baculovirus cell culture supernatant using a 2-step purification protocol as shown below, to yield an active trypsin inhibitor (Table 8 below).

**Table 8 Purification of recombinant bikunin from transformed culture supernatant**

TABLE 8

| Purification Step | Vol (ml) | OD 280/ml | OD 280 total | Units (U) | Specific activity (U/OD) |
|---|---|---|---|---|---|
| Supernatant | 2300.0 | 9.0 | 20,700 | 6,150,000 | 297 |
| Kallikrein affinity | 23.0 | 0.12 | 2.76 | 40,700 | 14,746 |
| C18 reverse-phase | 0.4 | 3.84 | 1.54 | 11,111 | 72,150 |

Chromatography of the crude material over an immobilized bovine pancreatic kallikrein affinity column selectively isolated 0.013 % of the protein and 0.67 % of the trypsin inhibitory activity present. The majority of the trypsin inhibitory activity present in the starting supernatant did not bind to the immobilized kallikrein and is not related to bikunin (results not shown). Subsequent chromatography using C18 reverse-phase yielded a further purification of 5-fold, with a recovery of 0.2%. The final preparation was highly pure by SDS-PAGE (Figure 13), exhibiting an Mr of 21.3 kDa, and reacted on immunoblots to rabbit anti-placental bikunin 102-159 (not shown). N-terminal sequencing (26 cycles) yielded the expected sequence for mature placental bikunin (Figure 4F) starting at residue +1(ADRER....), showing that the signal peptide was correctly processed in Sf9 cells.

Purified placental bikunin from Sf9 cells (100 pmol) was pyridylethyl-alkylated, CNBr digested and then sequenced without resolution of the resulting fragments. Sequencing for 20 cycles yielded the following N-terminii:

| Sequence | Amount | Placental bikunin residue # |
|---|---|---|
| LRCFrQQENPP-PLG----- | 21 pmol | 154 - 168 (SEQ ID NO.: 63) |
| ADRERSIHDFCLVSKVVGRC | 20 pmol | 1 - 20 (SEQ ID NO.: 64) |
| FNYeEYCTANAVTGPCRASF | 16 pmol | 100 - 119 (SEQ ID NO.: 65) |
| Pr--Y-V-dGS-Q-F-Y-G | 6 pmol | 25 - 43 (SEQ ID NO.: 66) |

Thus N-terminii corresponding to each of the expected four fragments were recovered. This confirms that the Sf9 expressed protein contained the entire ectodomain sequence of placental bikunin (1-170).

**Example 10**

**Inhibition specificity of purified placental bikunin (1-170) derived from Sf9 cells.**

The *in vitro* specificity of recombinant bikunin was determined using the materials and methods as described in Examples 3, 4 and 7. In addition, the inhibition of human tissue kallikrein by bikunin was measured by the incubation of 0.35 nM human tissue kallikrein recombinant bikunin in buffer containing 50 mM Tris (pH 9.0), 50 mM NaCl, and 0.01% triton x-100. After 5 min. at 37°C, 5 μl of 2 mM PFR-AMC was added and the change in fluorescence monitored.

Inhibition of tissue plasminogen activator (tPA) was also determined as follows: tPA (single chain form from human melanoma cell culture from Sigma Chemical Co, St Louis, MO) was pre-incubated with inhibitor for 2 hr at room temperature in 20 mM Tris buffer pH 7.2 containing 150 mM NaCl, and 0.02% sodium azide. Reactions were subsequently initiated by transfer to a reaction system comprising the following initial component concentrations: tPA (7.5 nM), inhibitor 0 to 6.6 μM, DIle-Lpro-Larg-pNitroaniline (1mM) in 28 mM Tris buffer pH 8.5 containing 0.004 % (v/v) triton x-100 and 0.005% (v/v) sodium azide. Formation of pNitroaniline was determined from the A405nm measured following incubation at 37 C for 2hr.

The table below show the efficacy of recombinant bikunin as an inhibitor of various serine proteases *in vitro*. Data is shown compared against data obtained for screening inhibition using either recombinant bikunin, or aprotinin.

**Table 9 Comparisons of Ki values for the inhibition of various proteases by recombinant placental bikunin (1-170) or aprotinin**

| TABLE 9 | | |
|---|---|---|
| Protease (concentration) | Recombinant Bikunin Ki (nM) | Aprotinin Ki (nM) |
| Trypsin (48.5 pM) | 0.064 | 0.8 |
| Human Plasma Kallikrein (2.5 nM) | 0.18 | 19.0 |
| Human Tissue Kallikrein (0.35 nM) | 0.04 | 0.004 |
| Bovine Pancreatic Kallikrein (100 pM) | 0.12 | 0.02 |
| Human Plasmin (50 pM) | 0.23 | 1.3 |
| factor Xa (0.87 nM) | 180 | 5% Inhibition at 31 μM |
| factor XIa (0.1 nM) | 3.0 | 288 |
| tissue plasminogen activator (7.5 nM) | < 60 | no inhibition at 6.6 μM |

(continued)

| Protease (concentration) | Recombinant Bikunin Ki (nM) | Aprotinin Ki (nM) |
|---|---|---|
| Tissue Factor VIIa | 800 | no inhibition at 1 μM |

The results show that recombinant bikunin can be expressed in insect cells to yield an active protease inhibitor that is effective against at least five different serine protease inhibitors. Recombinant bikunin was more potent than aprotinin against human plasma kallikrein, trypsin and plasmin. Surprisingly, the recombinant bikunin was more potent that the synthetically derived bikunin fragments (7-64) and (102-159) against all enzymes tested. These data show that recombinant bikunin is more effective than aprotinin, using *in vitro* assays, and that one would expect better *in vivo* potency.

Besides measuring the potencies against specific proteases, the capacity of placental bikunin (1-170) to prolong the activated partial thromboplastin time (APTT) was evaluated and compared with the activity associated with aprotinin. Inhibitor was diluted in 20 mM Tris buffer pH 7.2 containing 150 mM NaCl and 0.02% sodium azide and added (0.1 ml) to a cuvette contained within an MLA Electra$^R$ 800 Automatic Coagulation Timer coagulometer (Medical Laboratory Automation, Inc., Pleasantville, N.Y.). The instrument was set to APTT mode with a 300 sec. activation time and the duplicate mode. Following addition of 0.1 ml of plasma (Specialty Assayed Reference Plasma lot 1-6-5185, Helena Laboratories, Beaumont, TX), the APTT reagent (Automated APTT-lot 102345, from Organon Teknika Corp., Durhan, NC) and 25 mM CaCl2 were automatically dispensed to initiate clotting, and the clotting time was monitored automatically. The results (Figure 14) showed that a doubling of the clotting time required approximately 2 μM final aprotinin, but only 0.3 μM Sf9 derived placental bikunin. These data show that placental bikunin is an effective anticoagulant, and usefull as a medicament for diseases involving pathologic activation of the intrinsic pathway of coagulation.

## Example 11

### Measurement of Tracheal Potential Difference in the Guinea-pig

The aim of this study was to investigate the effect of the Kunitz serine protease inhibitor Bikunin, and the sodium channel blocker amiloride on guinea-pig tracheal potential difference 3 hours post treatment. These agents were delivered into the cephalad trachea by topical instillation. TPD was monitored 2 hours later for 60 minutes. The procedure used in this Example is described in Newton et al. in "Cilia, Mucus and Mucociliary Interactions," Ed., Baum, G.L. et al., Marcel Dekker, New York, 1998; Newton et al., Ped. Pulm. S17, Abs. 364,1998).

#### *Materials and methods/Reagents used*

Aqueous formulations of Bikunin (1-170)(5 and 50 ug/mL (SEQ ID NO: 52)) (as described in Example 17 below) and amiloride (obtained from Sigma Chemicals, St. Louis, MO, USA)(100 uM) were prepared, sterile filtered and endotoxin tested prior to use. These formulations were prepared in Hank's Balanced salt solution (HBSS) and contained 137 mM NaCl, 3 mM KCl, 3 mM $KH_2PO_4$, 8 mM $Na_2HPO_4$, 0.2% Tween-80, pH 7.1) was prepared, sterile filtered and endotoxin tested for use in this example. HBSS was used as a control solution. Hypnorm® (Fentanyl citrate 0.315 mg/mL and Fluanisone 10 mg/mL) was obtained from Janssen Animal Health and Hypnovel® (Midazolam 5 mg/mL) was obtained from Roche. Male Dunkin-Hartley guinea pigs (550-750 g) were supplied by David Hall, UK. Thermistor probes were obtained from Kane-May Ltd, UK.

#### *Induction of anaethesia and administration of Bikunin into tracheal airway*

Animals were anaesthetised using halothane. Once a satisfactory level of anaesthesia was induced a small incision was made below the lower jaw. The trachea was exposed and 100 ul volume of vehicle, bikunin (0.5 υg or 5 ug) or amiloride (100 uM) was instilled onto the tracheal surface using a needle and syringe. Once injected, the skin incision was sealed using Vetbond® (cyanocacrylate tissue glue). The animals were then allowed to recover.

#### *Preparation of guinea-pig for measurement of tracheal potential difference*

Two hours following agent treatment, guinea-pigs were anaesthetised for a second time with Hypnorm® and Hypnovel® and immobilised in a supine position. Rectal temperature, measured with a thermistor probe was maintained at 37° C by manual adjustment of a heat lamp. A ventral midline incision was made from the lower jaw to the clavicles.

Using blunt dissection a length of trachea was exposed and bisected at the upper edge of the sternum. The external jugular vein was exposed and cannulated. The caudal part of the trachea was then cannulated to allow the animal to spontaneously breath room air. The animal was then placed supine and its body temperature maintained using the heat lamp. 20 min. following induction of i.m. anaesthesia the tracheal agar electrode was inserted into the cephalad trachea and tracheal potential difference was measured for 60 minutes. The reference electrode was placed under cephalad trachea in contact with the trachea cartilage. The wound site was covered to prevent drying.

***Results***

As shown in Figure 15, Bikunin (5 ug) inhibited the potential difference in guinea pig trachea in vivo following three hours of treatment relative to vehicle. The effect of Amiloride (100 uM) and Bikunin (0.5 ug) is shown for comparison.

**Example 12**

**The Effect of Bikunin on Tracheal Mucus Velocity in the Guinea-pig**

The aim of this study was to investigate the effect of the Kunitz family serine protease inhibitor Bikunin on guinea-pig tracheal mucus velocity 1.5 hours post treatment. This agent was delivered into the cephalad trachea by topical instillation. TMV was monitored 1.5 hours later for 60 mins. The procedure used in this Example is described in Newton et al. in "Cila, Mucus and Mucociliary Interactions," Ed., Baum, G.L. et al., Marcel Dekker, New York, 1998; Newton et al., Ped. Pulm. S17, Abs. 364,1998).

***Materials and methods/Reagents used:***

A Bikunin (1-170) formulation (50 ug/mL Bikunin (SEQ ID NO: 52) (as described in Example 17 below) was prepared in HBBS containing 137 mM NaCl, 3 mM KCl, 3 mM $KH_2PO_4$, 8 mM $Na_2HPO_4$, 0.2% Tween-80, pH 7.1). The formulation was sterile filtered and endotoxin tested prior to use in this example. HBSS was used as a control solution. Hypnorm® (Fentanyl citrate 0.315 mg/mLand Fluanisone 10 mg/mL) was obtained from Janssen Animal Health and Hypnovel® (Midazolam 5 mg/mL) was obtained from Roche. Male Dunkin-Hartley guinea pigs (550-750 g) were supplied by David Hall, UK. Thermistor probes were obtained from Kane-May Ltd, UK.

***Induction of anaethesia and administration of Bikunin into tracheal airway***

Animals were anaesthetized using halothane. Once a satisfactory level of anaesthesia was induced, a small incision was made below the lower jaw. The trachea was exposed and 100 ul volume of vehicle or bikunin (5 ug) was instilled onto the tracheal surface using a needle and syringe. Once instilled, the skin incision was sealed using Vetbond® (cyanocacrylate tissue glue). The animals were then allowed to recover.

***Measurement of tracheal mucus velocity(TMV)***

TMV was monitored using a lead collimated miniature Beta particle detector probe arranged to detect the radioactivity emitted from an injected aliquot of $^{32}$P-labelled *Saccharomyces cerevisiae* as it was transported on the tracheal mucociliary layer of an anaesthetized guinea pig (Newton and Hall 1998) Figure 16(a) illustrates the arrangement of the syringe and beta probe. Figure 16(b) illustrates the counts detected by the probe as the $^{32}$P-labelled *S.cerevisiae* is transported along the tracheal mucociliary layer.

70 minutes following instillation of bikunin, each animal was anaesthetized for a second time using Hyponorm® and Hyponovel® and immobilized in a supine position. The first TMV measurement was made 20 minutes afterwards. Subsequent measurements were taken every 15 minutes. The procedure for TMV measurements is described, in detail, in Newton et al., "Cilia. Mucus and Mucociliary Interactions." Ed. Baum, G.L., Preil, Z., Roth, Y., Liron., Ostfield, E., Marcel Dekker. New York, 1990 and Newton et al. in Pediatric Pulmonology S17, Abs 364,1998.

***Results***

As shown in Figure 16(c), Bikunin (5 ug) increased TMV in vivo in guinea pig, relative to saline, over a sustained period of 1.5 to 2.5 hours following administration.

**Example 13**

**Bikunin decreases sodium current in cultured human bronchial epithelial (HBE) cell short circuit current (Isc) in vitro.**

Tertiary HBE cell monolayers grown to confluence were mounted in modified Ussing chambers, immersed in Krebs buffer (KBR) solution and bubbled with 95%$O_2$/5%$CO_2$ warmed to 37C.

Cells were left to equilibrate for 20 minutes before calibrating for background noise and fluid resistance. Transepithelial potential difference was then clamped to 0 mV using a WPI EVC 4000 voltage clamp. Ag/ AgCl electrodes were used to monitor Isc. Once a stable baseline was achieved (typically 10-20 min), cells were treated with amiloride (10 uM). Once a response to amiloride was seen, it was washed out with KBR solution. After return to baseline and equilibration, Bikunin (1-170) (as described in Example 17 below) (0.5-50 ug/mL in PBS) or PBS control was added. 90 minutes following agent treatment, amiloride (10 uM) was added. Once the current was stable forskolin (10 uM) and then bumetanide (100 uM) was added.

**Results**

As shown in Figure 17, Bukinin (70 nM) inhibited sodium current in vitro in human bronchial epithelial cells over a 90 minute period. Forskolin induced cAMP-mediated chloride secretion and monolayer resistance was unaffected.

**Example 14**

**The effect of hypertonic saline (14.4%) on TMV in the guinea pig**

The aim of this comparative study was to investigate the effect of hypertonic saline (14.4% x 5 min) on guinea-pig tracheal mucus velocity. This agent was delivered into the cephalad trachea by aerosol. TMV was monitored immediately and every 15 minutes for 30 minutes. The procedure used in this Example is described in Newton et al. in "Cila, Mucus and Mucociliary Interactions," Ed., Baum, G.L. et al., Marcel Dekker, New York, 1998; Newton et al., Ped. Pulm. S17, Abs. 364,1998).

***Materials and methods/Reagents used***

Hypnorm® (Fentanyl citrate 0.315 mg/mL and Fluanisone 10 mg/mL) was obtained from Janssen Animal Health and Hypnovel® (Midazolam 5 mg/mL) was obtained from Roche. Male Dunkin-Hartley guinea pigs (550-750 g) were supplied by Harlan UK Ltd. Thermistor probes were obtained from Kane-May Ltd, UK.

***Measurement of tracheal mucus velocity:***

Animals were anaesthetized using Hypnorm® and Hypnovel®. TMV was monitored using a lead collimated miniature Beta particle detector probe arranged to detect the radioactivity emitted from an injected aliquot of $^{32}$P-labelled *Saccharomyces cerevisiae* as it was transported on the tracheal mucociliary layer of an anaesthetized guinea pig (Newton and Hall 1998)

The first TMV measurement (run 1) was made 20 minutes after administration. Subsequent measurements were taken every 15 minutes. At a time point 6 minutes before the second run, a 5 minute aerosol of saline (0.9%) or hypertonic saline (14.4%) was administered. The radiolabelled tracer particles were given via the 0.5 um hole made in the trachae. An aerosol of ether saline (0.9%) or hypertonic saline (14.4%) was generated by a Pari pressure nebulizer. The aerosol was switched off one minute before the second run. The procedure for TMV measurements is described, in detail, in Newton et al., "Cilia, Mucus and Mucociliary Interactions." Ed. Baum, G.L., Preil, Z., Roth, Y., Liron., Ostfield, E., Marcel Dekker. New York, 1990 and Newton et al. in Pediatric Pulmonology S17, Abs 364,1998.

***Results***

As shown in Figure 18, hypertonic saline (14.4% x 5 mins) caused a transient increase in TMV immediately after aerosol.

**Example 15**

**Effect of Amiloride on TMV in the Guinea-pig**

The aim of this study was to investigate the effect of amiloride (10mM x 20 min.) on guinea-pig tracheal mucus in the anaesthetized spontaneously breathing guinea pig. This agent was delivered into the cephalad trachea by aerosolization as described in Example 14. The TMV measurement procedure used in this Example is described in Newton et al. in "Cilia, Mucus and Mucociliary Interactions," Ed., Baum, G.L. et al., Marcel Dekker, New York, 1998; Newton et al., Ped. Pulm. S17, Abs. 364, 1998).

***Materials and methods/Reagents used***

An amiloride formulation (10 mM) in water was prepared for this example. Hypnorm® (Fentanyl citrate 0.315 mg/mL and Fluanisone 10 mg/mL) was obtained from Janssen Animal Health and Hypnovel® (Midazolam 5 mg/mL) was obtained from Roche. Male Dunkin-Hartley guinea pigs (550-750 g) were supplied by Harlan UK Ltd. Thermistor probes were obtained from Kane-May Ltd, UK.

***Measurement of trachael mucus velocity***

Animals were anaesthetized using Hypnorm® and Hypnovel®. TMV was monitored using a lead collimated miniature Beta particle detector probe arranged to detect the radioactivity emitted from an injected aliquot of $^{32}$P-labelled *Saccharomyces cerevisiae* as it was transported on the tracheal mucociliary layer of an anaesthetized guinea pig. Guinea pigs were anaesthetized with Hypnorm® and Hyponovel at time 0. Amiloride (10 mM x 20 min) was administered by aerosol. The first TMV measurement was made immediately afterwards and subsequent measurements were taken every 15 minutes.

***Results***

As shown in Figure 19, amiloride (10 mM x 20 mins) caused a statistically significant increase in TMV 15 minutes after aerosol.

**Example 16**

**Aprotinin double mutein decreases sodium current in cultured human bronchial epithelial (HBE) cell short circuit current (Isc)**

The aim of this study was to investigate the effect of the Kunitz family serine protease inhibitor Aprotinin double mutein on Isc in vitro. Tertiary HBE cell monolayers grown to confluence were mounted in modified Ussing chambers, immersed in Krebs buffer (KBR) solution and bubbled with 95% $O_2$/5%$CO_2$ warmed to 37C. Aprotinin double mutein is Des Pro2-Ser10-Arg15-Asp24-Thr26-Glu31-Asn41-Glu53-Aprotinin which is described in Example 1 of EP 821 007, published January 28,1998, incorporated by reference in its entirety.

Cells were left to equilibrate for 20 minutes before calibrating for background noise and fluid resistance. Transepithelial potential difference was then clamped to 0 mV using a WPI EVC 4000 voltage clamp. Ag/AgCl electrodes were used to monitor Isc. Once a stable baseline was achieved (typically 10-20 mins), cells were treated with a amiloride (10uM). Once a response to amiloride was seen, it was washed out with KBR solution. After return to baseline and equilibration, Bikunin (5 ug/mL), Aprotinin double mutein (0.5 to 5 ug/mL), Aprotinin (1.5 to 5 ug/mL) or PBS was added. 90 minutes following agent treatment, amiloride (10uM) was added.

***Results***

As shown in Figure 20, Aprotinin double mutein (0.5 to 5 ug/mL) dose dependently inhibited sodium current in vitro in human bronchial epithelial cells over a 90 minute period.

### Example 17

### Expression, purification and comparative protease inhibitory activity of placental Bikunin (1-170) expressed in Chinese Hamster Ovary (CHO) cells

#### (a) Development of stable, high-producing CHO cell lines that express Bikunin

Stable production cell lines that secrete high quantities of bikunin were developed by transfecting CHO (dhfr-) cells with the expression vector shown in Figure 27. The vector was constructed using standard recombinant DNA techniques. A description of the construction of the expression vector and CHO cell expression system can be found in U.S.S.N. 09/441,654, filed November 12, 1999, entitled "Method of Producing Glycosylated Bikunin, " by Inventor Sam Chan. Briefly, the expression vector pBC-BK was constructed by cloning bikunin cDNA immediately downstream of the cytomegalovirus immediate early promoter and upstream of the polyadenylation signal sequence. The expression vector pBC-BK consists of a transcriptional unit for bikunin, dihydrofolate reductase, and ampicillin resistance. Bikunin cDNA was released from the cloning vector by restriction enzymes, blunt-ended, and ligated to linearized pBC. The linearization of pBC was done by a single restriction enzyme digestion. The orientation of bikunin cDNA was confirmed by sequencing.

About 1 x $10^6$ CHO (Chinese hamster ovary) cells were transfected with 10 $\mu$g of pBC-BK using Lipofectin reagents (Life Technology, Bethesda, Maryland) according to manufacturer's instructions. The cells were then selected in the presence of 50 nM methotrexate and grown in DME/F12 media deficient in thymidine and hypoxanthine plus 5% dialyzed fetal bovine serum. Cell populations were screened for bikunin production with a chromogenic assay. Briefly, bikunin standards or culture fluid was serially diluted and incubated with an equal volume of kallikrein at 37° C for 30 minutes after which a chromogenic substrate, N-benzoyl-Pro-Phe-Arg-pNA, was added. The reaction was incubated for 15 minutes before the addition of 50% acetic acid. The amount of p-nitroanilide released was measured at 405 nM. The high producing populations were further selected in media containing increasing concentrations of methotrexate (100 to 400 nM methotrexate) and screened for the production of bikunin. Limiting dilution cloning was then applied to derive clones with high and stable productivity. The cloning was done in the absence of methotrexate using standard tissue culture techniques by depositing 1 cell/well in 96-well plates. A clone designated FD3-1 was chosen for productivity evaluation in a bioreactor and was deposited on November 12,1999 with the American Type Culture Collection (ATCC), Rockville, MD, and was assigned Patent Deposit Designation PTA-940.

#### (b) Serum-free production of bikunin in a perfusion bioreactor

Continuous production of bikunin was done by continuous perfusion fermentation. A 1.5 liter Wheaton fermenter was inoculated with a stable CHO cell line at 2 x $10^6$ cells/ml and perfused at a medium exchange rate of 0.5 liters/day. The production medium was a DME/F12-based medium supplemented with insulin (10 $\mu$g/ml) and $FeSO_4$ • EDTA(50 $\mu$M). The cell density was maintained at 4 x $10^6$ cells/ml. The average daily yield of the fermenter was ~20 mg/ day. The production of bikunin was stably maintained for 21 days.

#### (c) Purification of bikunin (1-170) produced from a CHO cell expression system

Bikunin produced from CHO cells was purified using standard chromatography techniques involving ion exchange, metal chelate, and size exclusion chromatography as outlined in Figure 29.

The SP column (18 x 10 cm, 2.5 L) was prepared with SP-Sepharose Fast Flow (Pharmacia), and equilibrated. Cold filtered CHO cell harvest (TCF) was diluted 1:2.5 with cold sterile water, and the pH was adjusted to 5.0. Chromatography was performed at ambient temperature with cold buffers. The cold starting material was loaded on the column at 800 mL/ min (189 cm/hr). The amount of bikunin loaded onto the column ranged from 0.888 - 1.938 g (approximately 14 mg/L). After loading, the column was washed with equilibration buffer and the bikunin eluted with elution buffer. The eluate was collected at 2 - 8 °C (in an ice bath) and immediately adjusted to pH 7 with 6 N NaOH. The column was washed, then sanitized with cold (2 - 8 °C) 1 N NaOH, and stored at 2 - 8 °C in 20% ethanol until its next use. The equilibration and wash buffer contained 50 mM NaCl, 30 mM $NaH_2PO_4$, pH 5.0; the elution buffer contained 350 mM NaCl, 30 mM $NaH_2PO_4$, pH 5.0; and the pH adjusting buffer was 1 M citric acid, 1 M $NaH_2PO_4$, pH 2.4.

Thawed SP-Sepharose eluate was concentrated by ultrafiltration (UF) approximately 10-fold to reduce the volume before a 5 to 7-fold diafiltration (DF) was performed in preparation for anion exchange chromatography. All operations were performed at ambient temperature in a horizontal flow hood. UF/DF utilized a Pellicon 2 "mini" filter system from Millipore (Bedford, MA) and two 10 kDa regenerated cellulose cartridges (P2C010C01). Flux rates were approximately 130 $\pm$ 20 mL/min for the two-cartridge system and were maintained by regulating the inlet and outlet pressures between 22 to 26 psi and 12 to 16 psi respectively. Circulation was with a peristaltic pump; recirculation was set to 500 to 600 mL per minute before transmembrane pressure adjustment. Diafiltration was performed with cold 10

mM $NaH_2PO_4$ buffer, pH 8.1.

The Q-Sepharose column chromatography was performed as follows. A 13 x 9 cm, 1.2 L column of Q-Sepharose Fast Flow (Pharmacia) was washed with 5 column volumes (CV) of sterile water and equilibrated with approximately 10 CV's equilibration buffer. Diafiltered SP eluate was adjusted to pH 8.1 and applied on the Q-Sepharose column at 100 mL/min (45 cm/hr). The amount of bikunin loaded onto the column ranged from 1121-2607 mg (approximately 15 mg/mL). After loading, the column was washed with equilibration buffer until the UV absorbance at A280 reached baseline; then the bikunin was eluted. The eluate was collected and used as feed material for Zn-IMAC, zinc immobilized metal ion adsorption chromatography. The column was cleaned with 1 M NaOH, rinsed with sterile water, and stored in 20% ethanol. All operations were performed at 2 - 8 °C. Equilibration and washing buffer for Q-Sepharose column contained 10 mM $NaH_2PO_4$, pH 8.1. The elution buffer contained 10 mM $NaH_2PO_4$, 100 mM NaCl, pH 8.1.

A Zn-IMAC column (5 x 10 cm) containing approximately 200 mL bed volume of Chelating Sepharose Fast Flow (Pharmacia) was charged with 3 volumes of $ZnSO_4$ solution (see below); washed with 2 volumes of sterile water, and equilibrated with 6 volumes of buffer as described below. The Q-Sepharose eluate was adjusted to pH 7.4 and 300 mM NaCl ( by addition of NaCl solid), applied to Zn-IMAC at 30 mL /min (92 cm/hr linear rate), and then the column was washed with equilibration buffer until the UV absorbance reached baseline. The amount of bikunin loaded onto the column ranged from 0.097 -1.681 g (approximately 0.63 mg/mL). The flowthrough and wash were collected for UF. The column was stripped, and sanitized with 0.5 M NaOH. All operations of this step were performed at 2 - 8 °C. Equilibration buffer for Zn-IMAC contained 10 mM $NaH_2PO_4$, 300 mM NaCl, pH 7.4; the strip buffer contained 50 mM EDTA, 10 mM $NaH_2PO_4$, 300 mM NaCl, pH 7.4; charging solution, 2 mg/mL $ZnSO_4 . 7H_2O$.

The Zn-IMAC flow-through was concentrated by ultrafiltration 5-fold with the Pellicon 2 system (Millipore) for Sephacryl S-200 chromatography. Permeate flux rates were approximately 60 to 70 mL/ min and were maintained as described earlier. Recirculation was at 400 to 500 mL/min.

A column (10 x 58.5 cm) containing 4.59 L of Sephacryl S-200 High Resolution (Pharmacia) was equilibrated with 137 mM NaCl, 2.7 mM KCl, 2.9 mM $KH_2PO_4$, 10 mM $NaH_2PO_4$, 8 mM $Na_2HPO_4$, 2 mg/L Tween 80, pH 7.2. Flow rate was 30 mL/ min (23 cm/ hr). In a typical run, 1475 mg of bikunin in a volume of 95 mL was applied to the column.

The post-S-200 pool was treated with Etox resin in batch mode for removal of any potential pyrogen. ActiClean Etox, 2705 resin (Sterogene Bioseparations, Inc.) was rinsed with 1 M NaOH and incubated for 5 hours in 1 M NaOH at room temperature with agitation. The resin was washed and equilibrated with PBS, pH 7.2. Eighty mL of the filtered and equilibrated resin was added to 1063 mL of bikunin S200 pool (5460.11 mg), and agitated overnight at 2 - 8°C. The ETOX resin was then removed by filtration with 0.2 micron Nalgene flask filter.

*Results.* Table 10 shows the average yield afforded by each step.

**Table 10**

| Purification Step | Average Yield (%) |
|---|---|
| **SP-Sepharose** | 88.1 |
| **UF/DF plus filtration** | 81 |
| **Q-Sepharose** | 59 ± 14 |
| **Zn-IMAC** | 99.5 |
| **Sephacryl S-200** | 81 |
| **ETOX Resin** | 93 |

Overall yield of bikunin was about 30% with a purity of 95%. Mass spectroscopy data also suggested that in addition to full length Bikunin (1-170) molecules, species lacking three (G-S-K) and four (L-G-S-K) amino acids from the carboxy end of Bikunin (1-170) were present in the pure protein pool. The material produced was shown to be stable to degradation when exposed to 72-hours incubation at ambient temperature or at 37°C, neutral pH. N-terminal sequencing, gel electrophoresis, immunoblotting, and amino acid analysis indicated that the bikunin was substantially pure (no other sequences were detected). An additional reverse phase chromatography step revealed that the CHO-derived purified bikunin was still able to be fractionated into several species (Figure 30A). CHO bikunin (8.5 mg) was adjusted to pH 2.5 with trifluoroacetic acid (TFA, 0.1% final concentration) and subjected to chromatography on a C18 reverse-phase column (Vydac, 2.5 x 25 cm) equilibrated in 17.5% acetonitrile and 0.1% TFA at a flow rate of 2 ml/min. CHO bikunin was eluted with a linear gradient of 17.5-40% acetonitrile in 0.1% TFA over 60 min. Figure 30B shows the silver stained SDS-PAGE profile of these fractions (lane between 54 and 55 represents molecular size markers).

Preliminary carbohydrate analysis was performed on the glycosylated isoforms of CHO bikunin having a MW ranging from about 21 kDa to about 38 kDa. The total sugar content was found to be 7.5%. Both N-linked sites (Asn-30 and Asn-67) were found to be occupied with carbohydrate structures. Chromatographic and mass spectrometric analysis confirmed the presence of very heterogeneous and highly branched oligosaccharide structures contributing to the size heterogeneity observed for the purified bikunin. About 90% of the oligosaccharides were sialylated and the remaining structures were neutral. When treated with N-Glycosidase F, the glycosylated isoforms of CHO bikunin (Figure 30B) were converted to a single 18 kDa isoform (See Figure 31).

The sialic acid content of bikunin was analyzed by incubation with sialidase in 50 mM sodium acetate buffer, pH 5.0, for 18 hours in a capped microfuge tube. Sialic acids were separated on a Carbo Pac PA1 anion-exchange column using a buffer gradient of 20-250 mM sodium acetate in 100 mM NaOH for 50 minutes at a flow rate of 1 ml/min. Detection was done with a pulsed electrochemical detector and quantitated by comparing retention times and peak areas of samples to standard sialic acids (N-acetylneuraminic acid and N-glutarylneuraminic acid). The results are shown in Table 11.

**Table 11. Sialic acid composition of bikunin**

| Sialic acid | Contents (g/100g of bikunin) |
|---|---|
| N-Acetylneuraminic acid | 5.4 |
| N-Glutarylneuraminic acid | 0 |

**Example 18**

**Comparative Protease Inhibitor Activity of Placental Bikunin (1-170) expressed in Chinese Hamster Ovary (CHO) cells**

***General.*** The *in vitro* specificity of recombinant bikunin was determined using the materials and methods as described in Examples 3, 4, 7, and 10. Table 12 below shows the efficacy of recombinant bikunin as an inhibitor of various serine proteases in vitro. Data is shown using either recombinant bikunin or aprotinin.

***Proteases.*** Human plasmin and human plasma kallikrein quantitation was carried out by active site titration using p-nitrophenyl p'-guanidinobenzoate HCl as previously described (Chase,T., and Shaw, E., (1970) Methods Enzmol. 19, 20-27). Human tissue kallikrein (Bayer, Germany) was quantitated by active site titration using bovine aprotinin as a standard and PFR-AMC as a substrate assuming a 1:1 complex formation. The $K_m$ for GPK-AMC with plasmin under the conditions used was 726 μM; the $K_m$ for PFR-AMC with human plasma kallikrein was 457 μM; the $K_m$ for PFR-AMC with human tissue kallikrein was 5.7 μM.

***Inhibition Kinetics:*** The inhibition of human plasmin by CHO expressed placental bikunin (1-170) and aprotinin was determined with plasmin (50 pM) and CHO expressed placental bikunin (1-170) (0-2 nM) or aprotinin (0-4 nM) in buffer containing 50 mM Tris-HCl (pH 7.5), 0.1 M NaCl, and 0.02% triton x-100. After 30 min. incubation at 37°C, 25 μl of 20 mM GPK-AMC was added and the change in fluorescence monitored. The inhibition of human plasma kallikrein by CHO expressed placental bikunin (1-170) or aprotinin was determined using kallikrein (0.2 nM) and CHO expressed placental bikunin (1-170) (0-4 nM) or aprotinin (0-45 nM) in 50 mM Tris-HCl (pH 8.0), 50 mM NaCl, and 0.02% triton x-100. After 30 min. at 37°C, 5 μl of 20 mM PFR-AMC was added and the change in fluorescence monitored.

The inhibition of human tissue kallikrein by aprotinin or CHO expressed placental bikunin (1-170) was measured by the incubation of 0.35 nM human tissue kallikrein with CHO expressed placental bikunin (1-170) (0-10 nM) or aprotinin (0-0.5 nM) in a 1 ml reaction volume containing 50 mM Tris-HCl buffer pH 9.0, 50 mM NaCl, and 0.1% triton x-100. After 5 min. at 37°C, 5 ul of 2 mM PFR-AMC was added achieving 10 uM final concentration and the change in fluorescence monitored.

The inhibition of Factor XIa (from Enzyme Research Labs, South Bend, IN) was measured by incubating FXIa (0.1 nM) with either 0 to 40 nM CHO expressed placental bikunin (1-170) or 0 to 4 uM aprotinin in buffer containing 50 mM Hepes pH 7.5, 100 mM NaCl, 2 mM CaCl2, 0.01 % triton x-100, and 1% BSA in a total volume of 1 ml. After 30 min at 37 C, 10 ul of 40 mM Boc-Glu(OBzl)-Ala-Arg-AMC (Bachem Biosciences, King of Prussia, PA) was added and the change in fluorescence monitored.

The apparent inhibition constant $K_i$* was determined using the nonlinear regression data analysis program Enzfitter software (Biosoft, Cambridge, UK): The kinetic data from each experiment were analyzed in terms of the equation for a tight binding inhibitor:

$$V_i/V_o = 1 - (E_o + I_o + K_i^* - [(E_o + I_o + K_i^*)^2 - 4\,E_oI_o]^{1/2})/2E_o \qquad (2)$$

where $V_i/V_o$ is the fractional enzyme activity (inhibited vs. uninhibited rate), and $E_o$ and $I_o$ are the total concentrations of enzyme and inhibitor, respectively. Ki values were obtained by correcting for the effect of substrate according to the equation:

$$K_i = K_i^*/(1 + [S_o]/K_m) \quad (3)$$

(Boudier, C., and Bieth, J. G., (1989) Biochim Biophys Acta. 995: 36-41).
***Results:*** The Ki values are listed in Table 12 below.

**Table 12. Comparison of Ki values for the inhibition of various proteases by CHO Bikunin (1-170) or aprotinin**

| Protease | CHO bikunin (1-170) Ki (nM) | Aprotinin Ki (nM) |
|---|---|---|
| Human plasma kallikrein | 0.5 | 23.0 |
| Human tissue kallikrein | 0.3 | 0.004 |
| Human plasmin | 0.2 | 0.2 |
| Human FXIa | 1.9 | 270.0 |

The results show that recombinant bikunin can be expressed in CHO cells to yield an active protease inhibitor that is effective against at least three different serine proteases. Recombinant bikunin was more potent than aprotinin against human plasma kallikrein, and human FXIa. It was equipotent with aprotinin at inhibiting human plasmin.

**Example 19**

**Aprotinin decreases sodium current in cultured human cystic fibrosis bronchial epithelial cell short circuit current (Isc) in vitro**

HBE cells were isolated from CF patient lung transplant tissue and grown in collagen coated flasks for one week. The cells were then passaged and seeded onto collagen coated Costar Transwell filters (0.33 $cm^2$) and grown in DMEM/F12 media supplemented with 2% Ultroser G. Cells were grown at an air liquid interface and used 2 to 4 weeks after seeding:

Cells on the Transwell filters were mounted in modified Costar Ussing chambers and studied under Isc conditions. Baseline Isc values were recorded (0 to 10 minutes), and then aprotinin (1mg/mL in PBS) was added to the apical side. Isc was recorded for 100 minutes and then the apical bath fluid was exchanged with fresh buffer. Trypsin (100 BAEE units/mL) was added to the apical side and Isc recorded for 10 minutes. Amiloride (10 uM) was then added to the apical side and Isc was recorded for a further 10 minutes.

***Results***

As shown in Figure 21, aprotinin (1mg/mL in PBS) inhibited Isc in vitro in human CF bronchial epithelial cells over a 100 minute period. After washout, Isc was increased by treatment of the apical surface with the serine protease, trypsin. Finally, addition of amiloride (10 uM) demonstrated that the changes in Isc were the result of changes in sodium dependent current.

**Example 20**

**Assessment of activity of Bikunin (1-170) following nebulization**

The aim of this study was to assess the anti-protease activity of Bikunin (1-170) described in Example 17 following nebulization. All studies were performed with Bikunin formulated in phosphate buffered saline, pH 7.4 (137 mM NaCl, 3 mM KCl, 3mM $KH_2PO_4$, 8 mM $Na_2HPO_4$, 0.02 g/L Tween 80).

***Methods of nebulization***

Raindrop® Medication Nebulizer: Bikunin was aerosolized at concentrations of 1 and 3 mg/mL. The nebulizer cup was filled with 2.5 mL of Bikunin solution. Aerosolisation was performed at 7.35 L/ min or 35 psi.

Collison Nebulizer: Bikunin was aerosolised at a concentration of 4.7 mg/mL. The nebulizer cup was filled with 25 mL of Bikunin solution. Aerosolisation was performed at 35 psi.

***Collection of nebulised samples***

Aerosolised Bikunin was collected using a twin impinger (6.4 um mean aerodynamic particle cut-off size at 60 L/min through the system). The impinger works on the principle of liquid impingement and divided the aerosol into a non-respirable fraction (>6.4 um collected in Stage 1) and a respirable fraction (<6.4 um collected in Stage 2).

***Measurement of anti-protease activity***

Bikunin activity was measured in vitro by its inhibition of human plasma kallikrein.

***Results***

Raindrop® Medication Nebulizer: Activity (Ki) values for the pre- and post-nebulization samples were as follows: 1 mg/mL Bikunin: Ki values were 0.47($\pm$0.02) and 0.76($\pm$0.04) respectively; and for 3 mg/mL Bikunin(1-170) the Ki values were 0.52 ($\pm$0.03) and 0.62 ($\pm$0.03) respectively.

Collison Nebulizer: Activity (Ki) values for the pre- and post-nebulization samples were 0.27 ($\pm$0.03) and 0.45 ($\pm$0.03) respectively.

***Conclusion:***

Pre- and post-nebulized Bikunin samples from both the Raindrop and Collison nebulizers exhibited similar activities (similar Ki values within assay variability), indicating that Bikunin (1-170) was stable to aerosolization and retained its anti-protease activity following nebulization.

**Example 21**

**The effect of Bikunin on Tracheal Mucus Velocity in the Sheep**

The aim of this study was to investigate the effect of the Kunitz family serine protease inhibitor Bikunin (1-170) described in Example 17 on sheep trachcal mucus velocity over 8 hours after treatment. This agent was delivered by nebulised aerosol administration to the airways. The procedure used in this example is described in O'Riordan et al., J. Applied Physiol. 85(3), 1086-1091, 1998.

***Measurement of TMV***

Adult ewes were restrained in an upright position, with their heads immobilized, in a specialized body harness. They were nasally intubated with an endotracheal tube, with the cuff placed just below the vocal cords. The inspired air was warmed and humidified. To minimize possible impairment of TMV caused by inflated cuffs, the endotracheal tube cuff remained deflated throughout the study except for the period of aerosol administration

To measure TMV 5 to 10 radiopaque Teflon particles (approximately 1 mm in diameter, 0.8 mm thick, and weighing 1.5 to 2 mg) were insufflated into the trachea via a catheter placed within the endotracheal tube. The movement of the Teflon particles was then measured over a 1 minute period. The procedure used in this example is described in Russi et al., J. Applied Physiol. 59(5), 1416-1422,1985. A collar containing radiopaque markers of known length was applied to the exterior of the animals and used as a standard to convert distance traversed by the particles on the video screen to actual distance traveled. TMV was calculated from the average distance in a cephalad direction traveled per minute for 5 to 10 Teflon particles. Baseline TMV was measured immediately prior to administration of aerosol.

Test substances; PBS, 1 mg/mL Bikunin in PBS, or 3 mg/mL Bikunin in PBS; were delivered to the sheep airways as an aerosol (3mL) generated using a Raindrop jet nebuliser operated at a flow rate that produced droplets of mass median aerodynamic diameter of 3.6 um. TMV was measured immediately after administration of test substance (0 hours), then again at 0.5, 1, 2, 3, 4, 5, 6, 7, and at 8 hours.

***Results***

As shown in Figure 22, 9 mg Bikunin aerosol (3 mL of 3 mg/mL) delivered to sheep airways significantly increased TMV at 0, 0.5, 3, 4, 5, 6, 7, and 8 hours compared to the same time points for a group of animals receiving PBS vehicle aerosol. At 24 hours TMV had returned to baseline rates in both the Bikunin treatment and in the PBS vehicle groups.

At a lower dose (3mg Bikunin aerosol (3mL of 1 mg/mL)), no significant differences were observed in TMV between treatment and vehicle groups at any time point studied.

**Example 22**

**Bikunin (50 ug/mL) decreases sodium current in cultured guinea pig tracheal epithelial (GPTE) cell short circuit current (Isc)**

The aim of this study was to investigate the effect of Bikunin (1-170) (See Example 17) on Isc on GPTE cells in vitro. GPTE cells were seeded onto 1.2 cm diameter, 0.4 um pore size Snapwell ™ inserts (Costar UK). Cells were grown to confluence and mounted in modified Ussing chambers 2-4 days after placement on air-liquid interface. Inserts were immersed in Krebs buffer (KBR) solution and bubbled with 95% $O_2$/5%$CO_2$ warmed to 37° C.

After a 20 minute equilibration period, transepithelial potential difference was then clamped to 0 mV using a WPI EVC 4000 voltage clamp. Ag/ AgCl electrodes were used to monitor Isc. Once a stable baseline was achieved (typically 20-30 mins), cells were treated with amiloride (30uM). Once a response to amiloride was obtained, it was washed out with KBR solution. After return to baseline and equilibration, Bikunin (1-170) described in Example 17 (10 to 50 ug/mL) or PBS was added. 30 minutes following agent treatment amiloride (30 uM) was added.

***Results***

As shown in Figure 23, Bikunin (1-170) (50 ug/mL) inhibited sodium current in vitro in guinea pig tracheal epithelial cells over a 30 minute period.

**Example 23**

**Bikunin (1-170) (100 ug/mL) decreases sodium current in cultured ovine tracheal epithelial (OTE) cell short circuit current (Isc)**

The aim of this study was to investigate the effect of Bikunin (1-170) (See Example 17), on Isc in OTE cells *in vitro.* OTE cells were seeded onto 1.2 cm diameter, 0.4 um pore size Snapwell™ inserts (Costar UK). Cells were grown to confluence and mounted in modified Ussing chambers 3-5 days after placement on air-liquid interface. Inserts were immersed in Krebs buffer (KBR) solution and bubbled with 95%$O_2$/5%$CO_2$ warmed to 37° C.

After a 20 minute equilibration period, transepithelial potential difference was then clamped to 0 mV using a WPI EVC 4000 voltage clamp. Ag/AgCl electrodes were used to monitor Isc. Once a stable baseline was achieved (typically 30 mins), cells were treated with amiloride (10 uM). Once a response to amiloride was obtained, it was washed out with KBR solution. After return to baseline, Bikunin (1-170) described in Example 17 (25, 50 or 100 ug/mL) or PBS was added. 90 minutes following agent treatment amiloride (10uM) was again added.

***Results***

As shown in Figure 24, Bikunin (1-170) (100 ug/mL) significantly inhibited sodium current in vitro in ovine tracheal epithelial cells over a 90 minute period.

**Example 24**

**The effect of Bikunin (1-170) on tracheal potential difference in guinea pigs pretreated with LPS**

Polymorphonuclear (PMN) leukocyte (neutrophil) dominated airway inflammation is often a feature of CF lung disease. In the guinea pig, exposure to an aerosol of E. coli lipopolysaccharide (LPS) induces a marked PMN influx in the bronchoalveolar lavage fluid 24 hours post challenge. The aim of this study was to investigate the effect of Bikunin (1-170) described in Example 17 on tracheal potential difference in guinea pigs pre-exposed to an aerosol of LPS. Agents were delivered into the cephalad trachea by topical instillation. TPD was monitored for 60 minutes, 23 hours after exposure to LPS.

***Materials/Reagents***

Bikunin (1-170) was formulated in Hank's Balanced salt solution (HBSS). Amiloride was obtained from Sigma Chemicals and formulated in HBSS. Vehicle control was HBSS. Hypnorm (Fentanyl citrate 0.315 mg/mL and Fluanisone 10 mg/ mL) was obtained from Janssen Animal Health and Hypnovel (Midazolam 5 mg.mL) was obtained from Roche. Male Dunkin-Hartley guinea pigs (600-700 g) were supplied by Harlan UK. Thermistor probes were obtained from Kane-May Ltd, UK.

***Induction of PMN Influx***

Individual animals were exposed to an aerosol of 0.03 mg/mL LPS or PBS for 10 minutes.

***Preparation of guinea pig for measurement to tracheal potential difference***

23.4 hours following LPS treatment, guinea pigs were anaesthetised with Hypnorm and Hypnovel and immobilised in a supine position. A ventral midline incision was made from the lower jaw to the clavicles. Using blunt dissection a length of trachea was exposed and bisected at the upper edge of the sternum. The external jugular vein was exposed and cannulated. The caudal part of the trachea was then cannulated to allow the animal to spontaneously breath room air. The animal was then placed supine and body temperature was maintained at 37°C by manual adjustment of a heat lamp. Rectal temperature was monitored with a thermistor probe.

20 minutes following induction of anaesthesia, Bikunin (50 ug/mL) or amiloride (100 uM) was topically instilled into the cephalad trachea. The tracheal agar electrode was then inserted into the cephalad trachea and tracheal potential difference was measured for 60 minutes. The reference electrode was placed under the cephalad trachea in contact with the trachea cartilage. The wound site was covered to prevent drying.

***Results***

As shown in Figure 25(a), exposure to LPS caused a significant PMN influx. Bikunin significantly inhibited potential difference in guinea pigs pre-exposed to LPS, as shown in Figure 25(b).

**Example 25**

**The effect of Aprotinin double mutein on tracheal mucus velocity in the guinea pig**

The aim of this study was to investigate the effect of Aprotinin double mutein described in Example 16 on guinea pig tracheal mucus velocity 1.5 hours post treatment. This agent was delivered into the cephalad trachea by topical instillation. TMV was monitored 1.5 hours later for 60 minutes.

***Materials/Reagents***

Aprotinin double mutein (see Example 16) was obtained from Biotechnologie, Bayer AG, Germany USA and formulated in Hank's Balanced salt solution (HBSS). Hypnorm (Fentanyl citrate 0.315 mg/mL and Fluanisone 10 mg/mL) was obtained from Janssen Animal Health and Hypnovel (Midazolam 5 mg/mL) was obtained from Roche. Male Dunkin-Hartley guinea pigs (600-750 g) were supplied by Harlan UK. Thermistor probes were obtained from Kane-May Ltd., UK.

***Induction of anaesthesia:***

Animals were anaesthetised using halothane. Once a satisfactory level of anaesthesia was induced a small incision was made below the lower jaw. The trachea was exposed and 100 ul vehicle or Aprotinin double mutein (10 ug) was injected into the airway lumen using a needle and syringe. Once injected the skin overlying the tracheal injection site was repaired. The animals were then allowed to recover.

***Measurement of tracheal mucus velocity***

Tracheal mucus velocity (TMV) was monitored using a lead collimated minature Beta-particle detector probe arranged to detect the radioactivity emitted from an injected aliquot of phosphorus-32-labelled Saccharomyces cerevisiae as it was transported on the tracheal mucociliary layer of an anaesthetised guinea pig (Newton and Hall 1998). 70 minutes following instillation of test agent, guinea pigs were anaesthestised for a second time with hypnorm and hypnovel and immobilised in a supine position. The first TMV measurement was made 20 minutes afterwards.

***Results:***

As shown in Figure 26, Aprotinin double mutein (10 ug) increased TMV in vivo in the guinea pig, relative to HBSS, over a sustained period of 1.5 to 2.5 hours following administration.

**Example 26**

**Bikunin (1-170) decreases sodium current in cultured human cystic fibrosis bronchial epithelial cell short circuit current (Isc) in vitro**

HBE cells were isolated from CF patient lung transplant tissue and grown in collagen coated flasks for one week. The cells were then passaged and seeded onto collagen coated Costar Transwell filters (0.33 $cm^2$) and grown in DMEM/F12 media supplemented with 2% Ultroser G. Cells were grown at an air liquid interface and used 2 to 4 weeks after seeding.

Cells on the Transwell filters were mounted in modified Costar Ussing chambers and studied under Isc conditions. Baseline Isc values were recorded (0 to 20 minutes), and then bikunin (1-170) (see Example 17) (10 ug/mL in PBS) was added to the apical side. Isc was recorded for 90 minutes and then amiloride (10uM) was added and Isc was recorded for a further 10 minutes. The apical bath fluid was then exchanged with fresh buffer, and Isc was recorded for a further 15 minutes. Trypsin (1 uM) was added to the apical side and Isc recorded for 15 minutes. Amiloride (10 uM) was then added to the apical side and Isc was recorded for a further 10 minutes.

***Results***

As shown in Figure 28(a), bikunin (1-170)(10 ug/mL in PBS) inhibited Isc in vitro in human CF bronchial epithelial cells over a 90 minute period. Isc was further reduced by addition of amiloride (10 uM). On washout, Isc was increased back to the current achieved before the addition of amiloride. After a further 15 minutes, the apical surface was treated with trypsin (1 uM) and this further increased Isc to the baseline current level (i.e. that observed at 20 minutes). Finally, addition of amiloride (10 uM) inhibited the majority of the current, and demonstrated that the changes in Isc were the result of changes in sodium dependent current.

Figure 28(b) shows that bikunin (1-170) at 1, 5, and 10 ug/mL, and aprotinin at 20 ug/mL inhibited Isc at 90 minutes after apical application to human CF bronchial epithelial cells in vitro.

SEQUENCE LISTING FREE TEXT

<210> 9
<223> /note= "n at positions 622, 679, 707 is any nucleic acid"

<210> 10
<223> /note= "Xaa at positions 201, 226, and 233 are nonsence or stop codons"

<210> 11
<223> /note= "Xaa at positions 8, 17, 21-26, 40, 42, 45-47, 52, 64, 103, 112, 114, 116-121, 135, 137, 140-142, 147, and 159 is any amino acid residue"

<210> 12
<223> /note= "residue 361 is any nucleic acid"
<223> /note= "residue 367 is any nucleic acid"
<223> /note= "residue 384 is any nucleic acid"
<223> /note= "residue 390 is any nucleic acid"

<210> 13
<223> /label= signal peptide
<223> /note= "Xaa at positions 111, 120, 122, 128, and 130 represents a nonsense or stop codon"

<210> 14
<223> /note= "n at positions 425, 482, and 510 is any nucleic acid"

<210> 15
<223> /note= "Xaa at positions 2, 23, 132, 160, and 167 represent a nonsense or stop codon"

<210> 16
<223> /note= "n at positions 3, 11, 12, 17, 51 and 429 represent any nucleic acid"

<210> 17
<223> /note= "n at positions 6, 310 and 408 represent any nucleic acid"

<210> 18
<223> /note= "Tissue factor pathway inhibitor precursor 1"

<210> 19
<223> /note= "Tissue factor pathway inhibitor precursor 1"

<210> 20
<223> /note= "Tissue factor pathway inhibitor precursor"

<210> 21
<223> /note= "Tissue factor pathway inhibitor precursor 2"

<210> 22
<223> /note= "Tissue factor pathway inhibitor precursor 2"

<210> 23
<223> /note= "Amyloid Precursor Protein homologue"

<210> 24
<223> /note= "Aprotinin"

<210> 25
<223> /note= "Inter alpha-trypsin inhibitior precursor"

<210> 26
<223> /note= "Inter alpha-trypsin inhibitor precursor"

<210> 27
<223> /note= "Amyloid precursor protein"

<210> 28
<223> /note= "Collagen alpha-3 (VI) precursor"

<210> 29
<223> /note= "HKI-B9"

<210> 32
<223> /note= "cDNA of human Bikunin protein fragment"

<210> 45
<223> /label= signal peptide

<210> 47
<223> /label= signal peptide

<210> 49

<223> /label= signal peptide

<210> 50
<223> /note= "Human Bikunin protein fragment"

<210> 51
<223> /note= "Human Bikunin protein fragment"

<210> 52
<223> /note= "Human Bikunin protein fragment"

<210> 53
<223> /note= "Signal peptide of Human Bikunin protein"

<210> 54
<223> Human Bikunin protein fragment

<210> 55
<223> /note= "Oligomer for preparing expression construct"

<210> 56
<223> Oligomer for preparing expression construct

<210> 57
<223> /note= "Oligomer for preparing expression construct"

<210> 58
<223> /note= "Oligomer for preparing expression construct"

<210>
<223> /note= "Oligomer for preparing Bikunin expression construct"

<210> 62
<223> /note= "Oligomer for preparing Bikunin construct"

<210> 63
<223> /note= "Human Bikunin protein fragment"

<210> 64
<223> /note= "Human Bikunin protein fragment"

<210> 65
<223> /note= "Human Bikunin protein fragment"

<210> 66
<223> /note= "Human Bikunin protein fragment"

<210> 67
<223> /note= "Human Bikunin protein fragment"

<210> 68
<223> /note= "Human Bikunin protein fragment"

<210> 69
<223> /note= "Human Bikunin protein fragment"

<210> 70
<223> /note= "Human Bikunin protein fragment"

<210> 71
<223> /note= "Human Bikunin protein fragment"

## Claims

**1.** Use of a human Kunitz-type serine protease inhibitor for preparing a medicament for treating a lung disease associated with the retention and accumulation of mucus, selected from the group of diseases consisting of cystic fibrosis, chronic obstructive lung disease, chronic bronchitis, bronchietasis, chronic sinusitis and glue ear, the medicament comprising an effective amount of a human Kunitz-type serine protease inhibitor and physiologically acceptable carrier therefor, wherein the Kunitz-type serine protease inhibitor comprises the amino acid sequence:

```
                          MAQLCGL RRSRAFLALL GSLLLSGVLA  -1
ADRERSIHDF CLVSKVVGRC RASMPRWWYN VTDGSCQLFV YGGCDGNSNN 50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150
ACMLRCFRQQ ENPPLPLGSK VVVLAGLFVM VLILFLGASM VYLIRVARRN 200
QERALRTVWS SGDDKEQLVK NTYVL                            225
(SEQ ID NO.: 49),
```

```
                                  AGSFLAWL GSLLLSGVLA  -1
ADRERSIHDF CLVSKVVGRC RASMPRWWYN VTDGSCQLFV YGGCDGNSNN 50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150
ACMLRCFRQQ ENPPLPLGSK VVVLAGAVS                        179
(SEQ ID NO.: 2),
```

```
                           MLR AEADGVSRLL GSLLLSGVLA  -1
ADRERSIHDF CLVSKVVGRC RASMPRWWYN VTDGSCQLFV YGGCDGNSNN 50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150
ACMLRCFRQQ ENPPLPLGSK VVVLAGLFVM VLILFLGASM VYLIRVARRN 200
QERALRTVWS SGDDKEQLVK NTYVL                            225
(SEQ ID NO.: 45),
```

```
                          MAQLCGL RRSRAFLALL GSLLLSGVLA  -1
ADRERSIHDF CLVSKVVGRC RASMPRWWYN VTDGSCQLFV YGGCDGNSNN 50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150
ACMLRCFRQQ ENPPLPLGSK VVVLAGLFVM VLILFLGASM VYLIRVARRN 200
QERALRTVWS FGD                                         213
(SEQ ID NO.: 47),
```

ADRERSIHDF CLVSKVVGRC RASMPRWWYN VTDGSCQLFV YGGCDGNSNN 50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150
ACMLRCFRQQ ENPPLPLGSK VVVLAGLFVM VLILFLGASM VYLIRVARRN 200
QERALRTVWS SGDDKEQLVK NTYVL 225
(SEQ ID NO.: 70),

or

ADRERSIHDF CLVSKVVGRC RASMPRWWYN VTDGSCQLFV YGGCDGNSNN 50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150
ACMLRCFRQQ ENPPLPLGSK VVVLAGLFVM VLILFLGASM VYLIRVARRN 200
QERALRTVWS FGD 213
(SEQ ID NO.: 71),

IHDF CLVSKVVGRC RASMPRWWYN VTDGSCQLFV YGGCDGNSNN 50
YLTKEECLKK CATV 64
(SEQ ID NO.: 4),

CLVSKVVGRC RASMPRWWYN VTDGSCQLFV YGGCDGNSNN 50
YLTKEECLKK C 61
(SEQ ID NO.: 5),

YEEYCTANA VTGPCRASFP RWYFDVERNS CNNFIYGGCR GNKNSYRSEE 150
ACMLRCFRQ 159
(SEQ ID NO.: 6),

CTANAVTGPC RASFPRWYFD VERNSCNNFI YGGCRGNKNS YRSEE 150
ACMLRC 156
(SEQ ID NO.: 7),

IHDF CLVSKVVGRC RASMPRWWYN VTDGSCQLFV YGGCDGNSNN 50
YLTKEECLKK CATVTENATG DLATSRNAAD SSVPSAPRRQ DSEDHSSDMF 75
NYEEYCTANA VTGPCRASFP RWYFDVERNS CNNFIYGGCR GNKNSYRSEE 125
ACMLRCFRQ 159
(SEQ ID NO.: 3),

CLVSKVVGRC RASMPRWWYN VTDGSCQLFV YGGCDGNSNN 50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150
ACMLRC 156
(SEQ ID NO.: 50),

ADRERSIHDF CLVSKVVGRC RASMPRWWYN VTDGSCQLFV YGGCDGNSNN 25
YLTKEECLKK CATVTENATG DLATSRNAAD SSVPSAPRRQ DSEDHSSDMF 75
NYEEYCTANA VTGPCRASFP RWYFDVERNS CNNFIYGGCR GNKNSYRSEE 125
ACMLRCFRQQ ENPPLPLGSK VVVLAGAVS 179
(SEQ ID NO.: 1),

ADRERSIHDF CLVSKVVGRC RASMPRWWYN VTDGSCQLFV YGGCDGNSNN 50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150
ACMLRCFRQQ ENPPLPLGSK 170
(SEQ ID NO.: 52),

or

ADRERSIHDF CLVSKVVGRC RASMPRWWYN VTDGSCQLFV YGGCDGNSNN 50
YLTKEECLKK CATVTENATG DLATSRNAAD SSVPSAPRRQ DS 92
(SEQ ID NO.: 8).

2. A use as claimed in claim 1, wherein the disease is chronic obstructive lung disease.

3. Use according to claims 1 or 2, wherein the Kunitz-type serine protease inhibitor is glycosylated.

4. Use according to claims 1 or 2, wherein the Kunitz-type serine protease inhibitor contains at least one intra-chain cysteine-cysteine disulfide bond.

5. Use according to claims 1 or 2, wherein the Kunitz-type serine protease inhibitor contains at least one intra-chain cysteine-cysteine disulfide bond selected from the cysteine-cysteine paired groups consisting of CYS11-CYS61, CYS20-CYS44, CYS36-CYS57, CYS106-CYS156, CYS115-CYS139, and CYS131-CYS152, wherein the cysteine residues are numbered according to the amino acid sequence of native human placental bikunin.

6. Use according to claim 1, wherein the composition is to be administered to the lung airways.

7. Use according to claim 1, wherein said composition is to be administered directly by aerosolization.

8. Use according to claim 1, wherein said composition is to be administered directly as an aerosol suspension into the mammal's respiratory tract.

9. Use according to claim 8, wherein said aerosol suspension includes respirable particles ranging in size from about 1 to about 10 microns.

10. Use according to claim 8, wherein said aerosol suspension includes respirable particles ranging in size from about 1 to about 5 microns.

11. Use according to claim 8, wherein said aerosol suspension is to be delivered to said subject by a pressure driven nebulizer.

12. Use according to claim 8, wherein said aerosol suspension is to be delivered to said subject by an ultrasonic nebulizer.

13. Use according to claim 8, wherein said aerosol suspension is to be delivered to said subject by a non-toxic propellant.

14. Use according to claim 1, wherein said carrier is a member selected from the group consisting of a physiologically buffered solution, an isotonic saline, normal saline, and combinations thereof.

15. Use according to claim 8, wherein the aerosol suspension is to be delivered to said subject by dry power inhaler.

**Patentansprüche**

1. Verwendung eines humanen Serin-Protease-Inhibitors des Kunitz-Typs zur Herstellung eines Medikaments zur Behandlung einer Lungenerkrankung, die mit der Retention und Ansammlung von Schleim im Zusammenhang steht, ausgewählt aus der Gruppe von Krankheiten bestehend aus zystischer Fibrose, chronisch-obstruktiver Lungenerkrankung, chronischer Bronchitis, Bronchiektase, chronischer Sinusitis und Seromukotympanon, wobei das Medikament eine wirksame Menge eines humanen Serin-Protease-Inhibitors des Kunitz-Typs und einen physiologisch annehmbaren Träger dafür umfasst, wobei der Serin-Protease-Inhibitor des Kunitz-Typs die Aminosäure-Sequenz umfasst:

```
                                    MAQLCGL RRSRAFLALL GSLLLSGVLA  -1
ADRERSIHDF CLVSKVVGRC RASMPRWWYN VTDGSCQLFV YGGCDGNSNN  50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150
ACMLRCFRQQ ENPPLPLGSK VVVLAGLFVM VLILFLGASM VYLIRVARRN 200
QERALRTVWS SGDDKEQLVK NTYVL                            225
```

(SEQ ID NR.: 49),

```
                                    AGSFLAWL GSLLLSGVLA  -1
ADRERSIHDF CLVSKVVGRC RASMPRWWYN VTDGSCQLFV YGGCDGNSNN  50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150
ACMLRCFRQQ ENPPLPLGSK VVVLAGAVS                       179
```

(SEQ ID NR.: 2),

```
                               MLR AEADGVSRLL GSLLLSGVLA  -1
ADRERSIHDF CLVSKVVGRC RASMPRWWYN VTDGSCQLFV YGGCDGNSNN  50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150
ACMLRCFRQQ ENPPLPLGSK VVVLAGLFVM VLILFLGASM VYLIRVARRN 200
QERALRTVWS SGDDKEQLVK NTYVL                           225
```

(SEQ ID NR.: 45),

```
                      MAQLCGL RRSRAFLALL GSLLLSGVLA  -1
ADRERSIHDF CLVSKVVGRC RASMPRWWYN VTDGSCQLFV YGGCDGNSNN  50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150
ACMLRCFRQQ ENPPLPLGSK VVVLAGLFVM VLILFLGASM VYLIRVARRN 200
QERALRTVWS FGD                                        213
```

(SEQ ID NR.: 47),

```
ADRERSIHDF CLVSKVVGRC RASMPRWWYN VTDGSCQLFV YGGCDGNSNN  50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150
ACMLRCFRQQ ENPPLPLGSK VVVLAGLFVM VLILFLGASM VYLIRVARRN 200
QERALRTVWS SGDDKEQLVK NTYVL                           225
```

(SEQ ID NR.: 70),

ADRERSIHDF CLVSKVVGRC RASMPRWWYN VTDGSCQLFV YGGCDGNSNN 50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150
ACMLRCFRQQ ENPPLPLGSK VVVLAGLFVM VLILFLGASM VYLIRVARRN 200
QERALRTVWS FGD 213

(SEQ ID NR.: 71),

IHDF CLVSKVVGRC RASMPRWWYN VTDGSCQLFV YGGCDGNSNN 50
YLTKEECLKK CATV 64

(SEQ ID NR.: 4),

CLVSKVVGRC RASMPRWWYN VTDGSCQLFV YGGCDGNSNN 50
YLTKEECLKK C 61

(SEQ ID NR.: 5),

YEEYCTANA VTGPCRASFP RWYFDVERNS CNNFIYGGCR GNKNSYRSEE 150
ACMLRCFRQ 159

(SEQ ID NR.: 6),

CTANAVTGPC RASFPRWYFD VERNSCNNFI YGGCRGNKNS YRSEE 150
ACMLRC 156

(SEQ ID NR.: 7),

IHDF CLVSKVVGRC RASMPRWWYN VTDGSCQLFV YGGCDGNSNN 50
YLTKEECLKK CATVTENATG DLATSRNAAD SSVPSAPRRQ DSEDHSSDMF 75
NYEEYCTANA VTGPCRASFP RWYFDVERNS CNNFIYGGCR GNKNSYRSEE 125
ACMLRCFRQ 159

(SEQ ID NR.: 3),

CLVSKVVGRC RASMPRWWYN VTDGSCQLFV YGGCDGNSNN 50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150
ACMLRC 156

(SEQ ID NR.: 50),

ADRERSIHDF CLVSKVVGRC RASMPRWWYN VTDGSCQLFV YGGCDGNSNN 25
YLTKEECLKK CATVTENATG DLATSRNAAD SSVPSAPRRQ DSEDHSSDMF 75
NYEEYCTANA VTGPCRASFP RWYFDVERNS CNNFIYGGCR GNKNSYRSEE 125
ACMLRCFRQQ ENPPLPLGSK VVVLAGAVS 179

(SEQ ID NR.: 1),

ADRERSIHDF CLVSKVVGRC RASMPRWWYN VTDGSCQLFV YGGCDGNSNN 50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150
ACMLRCFRQQ ENPPLPLGSK 170

(SEQ ID NR.: 52),

oder

ADRERSIHDF CLVSKVVGRC RASMPRWWYN VTDGSCQLFV YGGCDGNSNN 50
YLTKEECLKK CATVTENATG DLATSRNAAD SSVPSAPRRQ DS 92

(SEQ ID NR.: 8).

**2.** Verwendung nach Anspruch 1, wobei die Krankheit chronisch-obstruktive Lungenerkrankung ist.

**3.** Verwendung nach Anspruch 1 oder 2, wobei der Serin-Protease-Inhibitor des Kunitz-Typs glykosyliert ist.

**4.** Verwendung nach Anspruch 1 oder 2, wobei der Serin-Protease-Inhibitor des Kunitz-Typs mindestens eine Cystein-Cystein-Disulfid-Bindung in der Kette enthält.

**5.** Verwendung nach Anspruch 1 oder 2, wobei der Serin-Protease-Inhibitor des Kunitz-Typs mindestens eine Cystein-Cystein-Disulfid-Bindung in der Kette enthält, die aus den Cystein-Cystein-gepaarten Gruppen bestehend aus CYS11-CYS61, CYS20-CYS44, CYS36-CYS57, CYS106-CYS156, CYS115-CYS139 und CYS131-CYS152 ausgewählt ist, wobei die Cystein-Reste gemäß der Aminosäure-Sequenz von nativem humanem plazentalem Bikunin nummeriert sind.

**6.** Verwendung nach Anspruch 1, wobei die Zusammensetzung zur Verabreichung in die Lungenatemwege vorgesehen ist.

**7.** Verwendung nach Anspruch 1, wobei die Zusammensetzung zur direkten Verabreichung durch Vernebeln vorgesehen ist.

**8.** Verwendung nach Anspruch 1, wobei die Zusammensetzung zur direkten Verabreichung als Aerosolsuspension in den Respirationstrakt des Säugers vorgesehen ist.

**9.** Verwendung nach Anspruch 8, wobei die Aerosolsuspension zum Einatmen geeignete Teilchen mit einer Größe im Bereich von ungefähr 1 bis ungefähr 10 $\mu$m umfasst.

**10.** Verwendung nach Anspruch 8, wobei die Aerosolsuspension zum Einatmen geeignete Teilchen mit einer Größe im Bereich von ungefähr 1 bis ungefähr 5 $\mu$m umfasst.

**11.** Verwendung nach Anspruch 8, wobei die Aerosolsuspension zur Verabreichung durch einen druckbetriebenen Vernebler an den Patienten vorgesehen ist.

**12.** Verwendung nach Anspruch 8, wobei die Aerosolsuspension zur Verabreichung durch einen Ultraschall-Vernebler an den Patienten vorgesehen ist.

**13.** Verwendung nach Anspruch 8, wobei die Aerosolsuspension zur Verabreichung durch ein nicht-toxisches Treibmittel an den Patienten vorgesehen ist.

**14.** Verwendung nach Anspruch 1, wobei der Träger ein Bestandteil ist, der aus der Gruppe bestehend aus einer physiologisch gepufferten Lösung, einer isotonischen Salzlösung, normaler Salzlösung und Kombinationen davon ausgewählt ist.

**15.** Verwendung nach Anspruch 8, wobei die Aerosolsuspension zur Verabreichung durch einen Trockenpulverinhalator an den Patienten vorgesehen ist.

## Revendications

**1.** Utilisation d'un inhibiteur de sérine protéase de type Kunitz humain pour fabriquer un médicament destiné à traiter une maladie pulmonaire associée à la rétention et à l'accumulation de mucus, choisie parmi le groupe de maladies constitué par la mucoviscidose, les maladies pulmonaires obstructives chroniques, la bronchite chronique, la bronchectasie, la sinusite chronique et l'otite muqueuse à tympan, le médicament comprenant une quantité efficace d'un inhibiteur d'une sérine protéase de type Kunitz humain et un support physiologiquement acceptable pour celui-ci, dans laquelle l'inhibiteur de sérine protéase de type Kunitz contient la séquence d'acides aminés suivante :

```
                                 MAQLCGL RRSRAFLALL GSLLLSGVLA  -1
ADRERSIHDF CLVSKVVGRC RASMPRWWYN VTDGSCQLFV YGGCDGNSNN 50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150
ACMLRCFRQQ ENPPLPLGSK VVVLAGLFVM VLILFLGASM VYLIRVARRN 200
QERALRTVWS SGDDKEQLVK NTYVL                            225
(SEQ ID NO.: 49),
```

AGSFLAWL GSLLLSGVLA -1
ADRERSIHDF CLVSKVVGRC RASMPRWWYN VTDGSCQLFV YGGCDGNSNN 50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150
ACMLRCFRQQ ENPPLPLGSK VVVLAGAVS 179
(SEQ ID NO.: 2),

MLR AEADGVSRLL GSLLLSGVLA -1
ADRERSIHDF CLVSKVVGRC RASMPRWWYN VTDGSCQLFV YGGCDGNSNN 50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150
ACMLRCFRQQ ENPPLPLGSK VVVLAGLFVM VLILFLGASM VYLIRVARRN 200
QERALRTVWS SGDDKEQLVK NTYVL 225
(SEQ ID NO.: 45),

MAQLCGL RRSRAFLALL GSLLLSGVLA -1
ADRERSIHDF CLVSKVVGRC RASMPRWWYN VTDGSCQLFV YGGCDGNSNN 50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150
ACMLRCFRQQ ENPPLPLGSK VVVLAGLFVM VLILFLGASM VYLIRVARRN 200
QERALRTVWS FGD 213
(SEQ ID NO.: 47),

ADRERSIHDF CLVSKVVGRC RASMPRWWYN VTDGSCQLFV YGGCDGNSNN 50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150
ACMLRCFRQQ ENPPLPLGSK VVVLAGLFVM VLILFLGASM VYLIRVARRN 200
QERALRTVWS SGDDKEQLVK NTYVL 225
(SEQ ID NO.: 70),

ou

ADRERSIHDF CLVSKVVGRC RASMPRWWYN VTDGSCQLFV YGGCDGNSNN 50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150
ACMLRCFRQQ ENPPLPLGSK VVVLAGLFVM VLILFLGASM VYLIRVARRN 200
QERALRTVWS FGD 213
(SEQ ID NO.: 71),

IHDF CLVSKVVGRC RASMPRWWYN VTDGSCQLFV YGGCDGNSNN 50
YLTKEECLKK CATV 64
(SEQ ID NO.: 4),

CLVSKVVGRC RASMPRWWYN VTDGSCQLFV YGGCDGNSNN 50
YLTKEECLKK C 61
(SEQ ID NO.: 5),

YEEYCTANA VTGPCRASFP RWYFDVERNS CNNFIYGGCR GNKNSYRSEE 150
ACMLRCFRQ 159
(SEQ ID NO.: 6),

CTANAVTGPC RASFPRWYFD VERNSCNNFI YGGCRGNKNS YRSEE 150
ACMLRC 156
(SEQ ID NO.: 7),

IHDF CLVSKVVGRC RASMPRWWYN VTDGSCQLFV YGGCDGNSNN 50
YLTKEECLKK CATVTENATG DLATSRNAAD SSVPSAPRRQ DSEDHSSDMF 75
NYEEYCTANA VTGPCRASFP RWYFDVERNS CNNFIYGGCR GNKNSYRSEE 125
ACMLRCFRQ 159
(SEQ ID NO.: 3),

```
CLVSKVVGRC RASMPRWWYN VTDGSCQLFV YGGCDGNSNN            50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150
ACMLRC                                                 156
(SEQ ID NO.: 50),
```

```
ADRERSIHDF CLVSKVVGRC RASMPRWWYN VTDGSCQLFV YGGCDGNSNN  25
YLTKEECLKK CATVTENATG DLATSRNAAD SSVPSAPRRQ DSEDHSSDMF  75
NYEEYCTANA VTGPCRASFP RWYFDVERNS CNNFIYGGCR GNKNSYRSEE 125
ACMLRCFRQQ ENPPLPLGSK VVVLAGAVS                        179
(SEQ ID NO.: 1),
```

```
ADRERSIHDF CLVSKVVGRC RASMPRWWYN VTDGSCQLFV YGGCDGNSNN 50
YLTKEECLKK CATVTENATG DLATSRNAAD SSVPSAPRRQ DSEDHSSDMF 100
NYEEYCTANA VTGPCRASFP RWYFDVERNS CNNFIYGGCR GNKNSYRSEE 150
ACMLRCFRQQ ENPPLPLGSK                                  170
(SEQ ID NO.: 52),
```

ou

```
ADRERSIHDF CLVSKVVGRC RASMPRWWYN VTDGSCQLFV YGGCDGNSNN 50
YLTKEECLKK CATVTENATG DLATSRNAAD SSVPSAPRRQ DS         92
(SEQ ID NO.: 8).
```

**2.** Utilisation selon la revendication 1, dans laquelle la maladie est une maladie pulmonaire obstructive chronique.

**3.** Utilisation selon les revendications 1 ou 2, dans laquelle l'inhibiteur de sérine protéase de type Kunitz est glycosylé.

**4.** Utilisation selon les revendications 1 ou 2, dans laquelle l'inhibiteur de sérine protéase de type Kunitz contient au moins un pont disulfure cystéine-cystéine intra-chaîne.

**5.** Utilisation selon les revendications 1 ou 2, dans laquelle l'inhibiteur de sérine protéase de type Kunitz contient au moins un pont disulfure cystéine-cystéine intra-chaîne choisi parmi les groupes appariés cystéine-cystéine constitués de CYS11-CYS61, CYS20-CYS44, CYS36-CYS57, CYS106-CYS156, CYS115-CYS139 et CYS131-CYS152, dans laquelle les radicaux cystéines sont numérotés selon la séquence d'acides aminés de la bikunine native du placenta humain.

**6.** Utilisation selon la revendication 1, dans laquelle la composition est destinée à être administrée aux voies respiratoires pulmonaires.

**7.** Utilisation selon la revendication 1, dans laquelle ladite composition est destinée à être administrée directement par aérosol.

8. Utilisation selon la revendication 1, dans laquelle ladite composition est destinée à être administrée directement sous forme d'un aérosol d'une suspension dans les voies respiratoires d'un mammifère.

9. Utilisation selon la revendication 8, dans laquelle ledit aérosol d'une suspension comprend des particules respirables présentant une taille dans la plage d'environ 1 à environ 10 microns.

10. Utilisation selon la revendication 8, dans laquelle ledit aérosol d'une suspension comprend des particules respirables présentant une taille dans la plage d'environ 1 à environ 5 microns.

11. Utilisation selon la revendication 8, dans laquelle ledit aérosol d'une suspension est destiné à être administré audit sujet par un nébuliseur sous pression.

12. Utilisation selon la revendication 8, dans laquelle ledit aérosol d'une suspension est destiné à être administré audit sujet par un nébuliseur à ultrasons.

13. Utilisation selon la revendication 8, dans laquelle ledit aérosol d'une suspension est destiné à être administré audit sujet par un gaz propulseur non toxique.

14. Utilisation selon la revendication 1, dans laquelle ledit support est un membre choisi dans le groupe constitué par une solution tamponnée physiologiquement, une solution saline isotonique, une solution saline normale et des combinaisons de celles-ci.

15. Utilisation selon la revendication 8, dans laquelle l'aérosol de suspension est destiné à être administré audit sujet par un inhalateur à puissance sans injection d'eau.

# FIG. 1

| | | |
|---|---|---|
| R35464 | GGCCGGGTCG TTTCTCGCCT GGCTGGGATC GCTGCTCCTC TCTGGGGTCC | 50 |
| ORF | P G R F S P G W D R C S S L G S | 16 |
| R35464 | TGGCCGGCCG ACCGAGAACG CAGCATCCAC GACTTCTGCC TGGTGTCGAA | 100 |
| ORF | W P A D R E R S I H D F C L V S K | 33 |
| R35464 | GGTGGTGGGC AGATTCCGGG CCTCCATGCC TAGGTGGTGG TACAATGTCA | 150 |
| ORF | V V G R F R A S M P R W W Y N V T | 50 |
| R35464 | CTGACGGATC CTGCCAGCTG TTTGTGTATG GGGGCTGTGA CGGAAACAGC | 200 |
| ORF | D G S C Q L F V Y G G C D G N S | 66 |
| R35464 | AATAATTACC TGACCAAGGA GGAGTGCCTC AAGAAATGTG CCACTGTCAC | 250 |
| ORF | N N Y L T K E E C L K K C A T V T | 83 |
| R35464 | AGAGAATGCC ACGGGTGACC TGGCCACCAG CAGGAATGCA GCGGATTCCT | 300 |
| ORF | E N A T G D L A T S R N A A D S S | 100 |
| R35464 | CTGTCCCAAG TGCTCCCAGA AGGCAGGATT CTTGAAGACC ACTTCAGCGA | 350 |
| ORF | V P S A P R R Q D S * R P L Q R | 116 |
| R35464 | TATGTTTCAA NTATTGNAAG AATAATTGCA CCGNCAACGN ATT------- | 393 |
| ORF | Y V S * I * R I I A P * T * | 130 |

KEY
R35464 = Nucleic acid sequence of EST R35464 (SEQ ID NO:12)
ORF = EST R35464 Open Reading Frame Translation (SEQ ID NO: 13)

# FIG. 2

```
R74593   GCAATAATTA CCTGACCAAG GAGGAGTGCC TCAAGAAATG TGCCACTGTC    50
ORF       Q  *  L   P  D  Q  G   G  V  P   Q  E  M   C  H  C  H   17

R74593   ACAGAGAATG CCACGGGTGA CCTGGCCACC AGCAGGAATG CAGCGGATTC   100
ORF        R  E  C   H  G  *   P  G  H  Q   Q  E  C   S  G  F      33

R74593   CTCTGTCCCA AGTCTCCCAG AAGGCAGGAT TCTGAAGACC ACTCCAGCGA   150
ORF      L  C  P  K   S  P  R   R  Q  D   S  E  D  H   S  S  D     50

R74593   TATGTTCAAC TATGAAGAAT ACTGCACCGC CAACGCAGTC ACTGGGCCTT   200
ORF       M  F  N   Y  E  E  Y   C  T  A   N  A  V   T  G  P  C     67

R74593   GCCGTGCATC CTTCCCACGC TGGTACTTTG ACGTGGAGAG GAACTCCTGC   250
ORF        R  A  S   F  P  R   W  Y  F  D   V  E  R   N  S  C        83

R74593   AATAACTTCA TCTATGGAGG CTGCCGGGGC AATAAGAACA GCTACCGCTC   300
ORF      N  N  F  I   Y  G  G   C  R  G   N  K  N  S   Y  R  S     100

R74593   TGAGGAGGCC TGCATGCTCC GCTGCTTCCG CCAGCAGGAG AATCCTCCCC   350
ORF        E  E  A   C  M  L  R   C  F  R   Q  Q  E   N  P  P  L  117

R74593   TGCCCCTTGG CTCAAAGGTG GTGGTTCTGG CCGGGGCTGT TTCGTGATGG   400
ORF       P  L  G   S  K  V   V  V  L  A   G  A  V   S  *  W       133

R74593   TGTTGATCCT TTTCCTGGGG AGCNTCCATG GTCTTACTGA TTCCGGGTGG   450
ORF      C  *  S  F   S  W  G   A  S  M   V  L  L  I   P  G  G     150

R74593   CAAGGAGGAA CCAGGAGCGT GCCCTGCGGA NCGTCTGGAG CTTCGGAGAT   500
ORF       K  E  E   P  G  A  C   P  A  X   R  L  E   L  R  R  *    167

R74593   GACAAGGGNT                                               510
ORF       Q  G                                                    169
```

KEY
R74593 = Nucleic acid sequence of EST R74593 (SEQ ID NO: 14)
ORF = EST R74593 Open Reading Frame Translation (SEQ ID NO: 15)

# FIG. 3

```
R35464        GGCCGGGTCGT TTCTCGCCTG GCTGGGA-TC GCTGCTCCTC TCTGGGGTCC    50
N39798                               TGGGANTC GCTGCTCCTC TCTGGGGTCC    28
H94519         GCNGCG-CGT TNNTCGCNT- GCTGGGA-TC GCTGCACCTC TCTGGGGTCG    47
R74593 corr.   ---------- ---------- ---------- ---------- ----------
Consensus     GGCCGGGTCGT TTCTCGCCTG GCTGGGA-TC GCTGCTCCTC TCTGGGGTCC    50
Translation     A  G  S  F   L  A  W   L  G  S   L  L  L   S  G  V     -3

R35464        TGGCCGGCCG ACCGAGAACG CAGCATCCAC GACTTCTGCC TGGTGTCGAA    100
N39798        TGG-CGGCCG ACCGAGAACG CAGCATCCAC GACTTCTGCC TGGTGTCGAA     77
H94519        NGG-CGGCCG ACCGAGAACG CAGCATCCAC GACTTCTGCC TGGTGTCGAA     96
R74593 corr.  ---------- ---------- ---------- ---------- ----------
Consensus     TGG-CGGCCG ACCGAGAACG CAGCATCCAC GACTTCTGCC TGGTGTCGAA     99
Translation   L  A  A  D   R  E  R   S  I  H   D  F  C  L   V  S  K     15

R35464        GGTGGTGGGC AGATTCCGGG CCTCCATGCC TAGGTGGTGG TACAATGTCA    150
N39798        GGTGGTGGGC AGATGCCGGG CCTCCATGCC TAGGTGGTGG TACAATGTCA    127
H94519        GGTGGTGGGC AGATGCCGGG CCTCCATGCC TAGGTGGTGG TACAATGTCA    146
R74593 corr.  ---------- ---------- ---------- ---------- ----------
Consensus     GGTGGTGGGC AGATGCCGGG CCTCCATGCC TAGGTGGTGG TACAATGTCA    149
Translation   V  V  G   R  C  R  A   S  M  P   R  W  W   Y  N  V  T     32

R35464        CTGACGGATC CTGCCAGCTG TTTGTGTATG GGGGCTGTGA CGGAAACAGC    200
N39798        CTGACGGATC CTGCCAGCTG TTTGTGTATG GGGGCTGTGA CGGAAACAGC    177
H94519        CTGACGGATC CTGCCAGCTG TTTGTGTATG GGGGCTGTGA CGGAAACAGC    196
R74593 corr.  ---------- ---------- ---------- ---------- --------GC      2
Consensus     CTGACGGATC CTGCCAGCTG TTTGTGTATG GGGGCTGTGA CGGAAACAGC    199
Translation    D  G  S   C  Q  L   F  V  Y  G   G  C  D   G  N  S       48

R35464        AATAATTACC TGACCAAGGA GGAGTGCCTC AAGAAATGTG CCACTGTCAC    250
N39798        AATAATTACC TGACCAAGGA GGAGTGCCTC AAGAAATGTG CCACTGTCAC    227
H94519        AATAATTACC TGACCAAGGA GGAGTGCCTC AAGAAATGTG CCACTGTCAC    246
R74593 corr.  AATAATTACC TGACCAAGGA GGAGTGCCTC AAGAAATGTG CCACTGTCAC     52
Consensus     AATAATTACC TGACCAAGGA GGAGTGCCTC AAGAAATGTG CCACTGTCAC    249
Translation   N  N  Y  L   T  K  E   E  C  L   K  K  C  A   T  V  T     65

R35464        AGAGAATGCC ACGGGTGACC TGGCCACCAG CAGGAATGCA GCGGATTCCT    300
N39798        AGAGAATGCC ACGGGTGACC TGGCCACCAG CAGGAATGCA GCGGATTCCT    277
H94519        AGAGAATGCC ACGGGTGACC TGGCCACCAG CAGGAATGCA GCGGATTCCT    296
R74593 corr.  AGAGAATGCC ACGGGTGACC TGGCCACCAG CAGGAATGCA GCGGATTCCT    102
Consensus     AGAGAATGCC ACGGGTGACC TGGCCACCAG CAGGAATGCA GCGGATTCCT    299
Translation    E  N  A   T  G  D  L   A  T  S   R  N  A   A  D  S  S     82

R35464        CTGTCCCAAG TGCTCCCAGA AGGCAGGATT CTTGAAGACC ACTTCAGCGA    350
N39798        CTGTCCCAAG TGCTCCCAGA AGGCAGGATT CT-GAAGACC ACTCCAGCGA    326
H94519        CTGTCCCAAG TGCTCCCAGA AGGCAGGATT CT-GAAGACC ACTCCAGCGA    345
R74593 corr.  CTGTCCCAAG TGCTCCCAGA AGGCAGGATT CT-GAAGACC ACTCCAGCGA    151
Consensus     CTGTCCCAAG TGCTCCCAGA AGGCAGGATT CT-GAAGACC ACTCCAGCGA    348
Translation    V  P  S   A  P  R   R  Q  D  S   E  D  H   S  S  D       98
```

# FIG. 3 (Cont.)

```
R35464         TATGTTTCAA NTATTGNAAG AATAATTGCA CCGNCAACGN ATT-------   393
N39798         TATGTT-CAA CTA-TG-AAG AATACT-GCA CCGCCAACGC AGTCACTGGG   372
H94519         TATGTT-CAA CTA-TG-AAG AATACTGGCA CCGCCAACGC ATTCACTGGG   392
R74593 corr.   TATGTT-CAA CTA-TG-AAG AATACT-GCA CCGCCAACGC AGTCACTGGG   197
Consensus      TATGTT-CAA CTA-TG-AAG AATACT-GCA CCGCCAACGC AGTCACTGGG   394
Translation     M  F  N   Y  E  E   Y  C  T   A  N  A   V  T  G      113

R35464         ---------- ---------- ---------- ---------- ----------
N39798         CCTTGC-GTG GAATCCTTTC CCACGCTGGN AATTTNGACG TTGAGAAGGA   421
H94519         CCT-GC-GTG -CATCCTT-C CCACGCTGGT ACTTT-GNCG ----------   427
R74593 corr.   CCTTGCCGTG -CATCCTT-C CCACGCTGGT ACTTT-GACG TGGAGA-GGA   243
Consensus      CCTTGCCGTG -CATCCTT-C CCACGCTGGT ACTTT-GACG TGGAGA-GGA   440
Translation     P  C  R  A    S  F   P  R  W  Y   F  D  V   E  R  N   129

R35464         ---------- ---------- ---------- ---------- ----------
N39798         AC-------- ---------- ---------- ---------- ----------   423
H94519         ---------- ---------- ---------- ---------- ----------
R74593 corr.   ACTCCTGCAA TAACTTCATC TATGGAGGCT GCCGGGGCAA TAAGAACAGC   293
Consensus      ACTCCTGCAA TAACTTCATC TATGGAGGCT GCCGGGGCAA TAAGAACAGC   490
Translation      S  C  N   N  F  I   Y  G  G  C   R  G  N   K  N  S   145

R35464         ---------- ---------- ---------- ---------- ----------
N39798         ---------- ---------- ---------- ---------- ----------
H94519         ---------- ---------- ---------- ---------- ----------
R74593 corr.   TACCGCTCTG AGGAGGCCTG CATGCTCCGC TGCTTCCGCC AGCAGGAGAA   343
Consensus      TACCGCTCTG AGGAGGCCTG CATGCTCCGC TGCTTCCGCC AGCAGGAGAA   540
Translation     Y  R  S  E   E  A  C   M  L  R   C  F  R  Q   Q  E  N  162

R35464         ---------- ---------- ---------- ---------- ----------
N39798         ---------- ---------- ---------- ---------- ----------
H94519         ---------- ---------- ---------- ---------- ----------
R74593 corr.   TCCTCCCCTG CCCCTTGGCT CAAAGGTGGT GGTTCTGGCC GGGGCTGTTT   393
Consensus      TCCTCCCCTG CCCCTTGGCT CAAAGGTGGT GGTTCTGGCC GGGGCTGTTT   590
Translation     P  P  L   P  L  G  S   K  V  V   V  L  A   G  A  V  S  179

R35464         ---------- ---------- ---------- ---------- ----------
N39798         ---------- ---------- ---------- ---------- ----------
H94519         ---------- ---------- ---------- ---------- ----------
R74593 corr.   CGTGATGGTG TTGATCCTTT TCCTGGGGAG CNTCCATGGT CTTACTGATT   443
Consensus      CGTGATGGTG TTGATCCTTT TCCTGGGGAG CNTCCATGGT CTTACTGATT   640
Translation     *  W  C   *  S  F   S  W  G  A   S  M  V   L  L  I   195

R35464         ---------- ---------- ---------- ---------- ----------
N39798         ---------- ---------- ---------- ---------- ----------
H94519         ---------- ---------- ---------- ---------- ----------
R74593 corr.   CCGGGTGGCA AGGAGGAACC AGGAGCGTGC CCTGCGGANC GTCTGGAGCT   493
Consensus      CCGGGTGGCA AGGAGGAACC AGGAGCGTGC CCTGCGGANC GTCTGGAGCT   690
Translation     P  G  G  K   E  E  P   G  A  C   P  A  *  R   L  E  L  212

R35464         ---------- --------
N39798         ---------- --------
H94519         ---------- --------
R74593 corr.   TCGGAGATGA CAAGGGNT                                      511
Consensus      TCGGAGATGA CAAGGGNT                                      708
Translation     R  R  *   Q  G                                          217
```

FIG. 3 (Cont.)

KEY
R35464 = Nucleic acid sequence of EST R35464 (SEQ ID NO.: 12)
N39798 = Nucleic acid sequence of EST N39798 (SEQ ID NO.: 17)
H94519 = Nucleic acid sequence of EST H94519 (SEQ ID NO.: 16)
R74593 corr = Corrected version of (SEQ ID NO.: 14) G at b.p. 114
Consensus = Nucleic acid sequence for human bikunin (SEQ ID NO. 9)
Translation = Amino acid Translation of Consensus (SEQ ID NO.: 10)

FIG. 4A

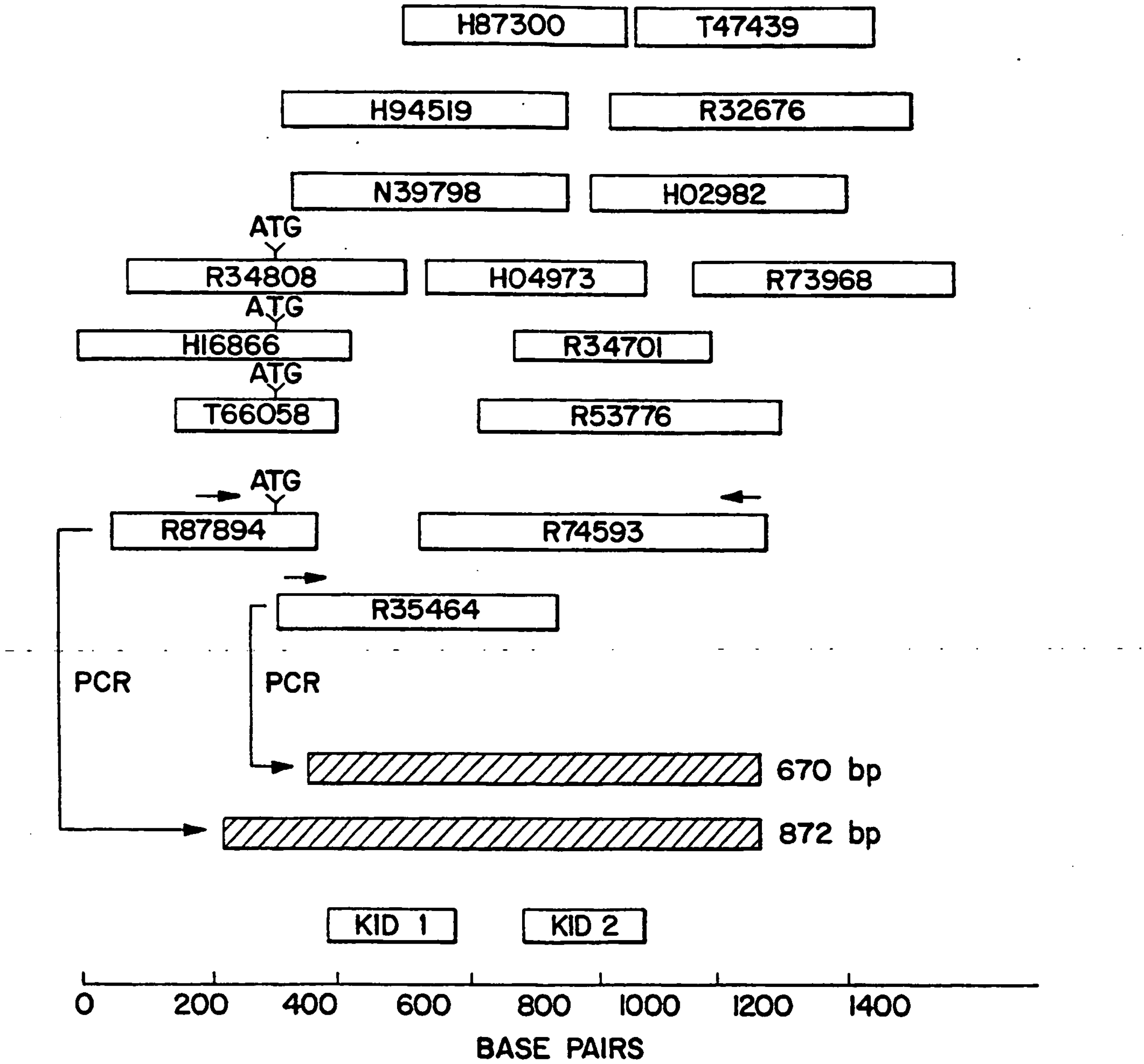
H87300
T47439
H94519
R32676
N39798
H02982
ATG
R34808
H04973
R73968
ATG
H16866
R34701
ATG
T66058
R53776
ATG
R87894
R74593
R35464
PCR
PCR
670 bp
872 bp
KID 1
KID 2
0
200
400
600
800
1000
1200
1400
BASE PAIRS

FIG. 4B

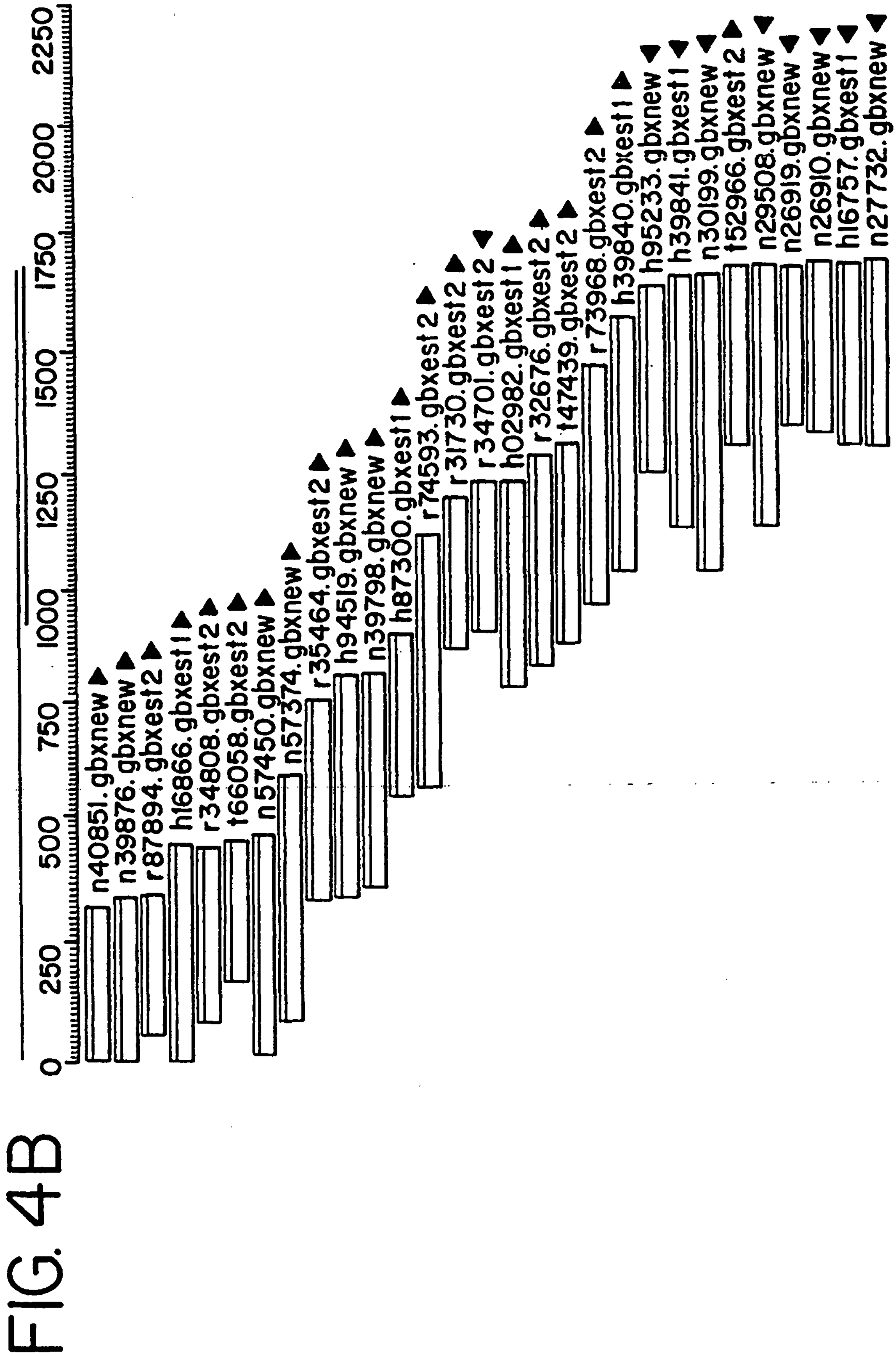
0
250
500
750
1000
1250
1500
1750
2000
2250
n40851.gbxnew
n39876.gbxnew
r87894.gbxest2
h16866.gbxest1
r34808.gbxest2
t66058.gbxest2
n57450.gbxnew
n57374.gbxnew
r35464.gbxest2
h94519.gbxnew
n39798.gbxnew
h87300.gbxest1
r74593.gbxest2
r31730.gbxest2
r34701.gbxest2
h02982.gbxest1
r32676.gbxest2
t47439.gbxest2
r73968.gbxest2
h39840.gbxest1
h95233.gbxnew
h39841.gbxest1
n30199.gbxnew
t52966.gbxest2
n29508.gbxnew
n26919.gbxnew
n26910.gbxnew
h16757.gbxest1
n27732.gbxnew

# FIG. 4C

```
           1                                                   50
Bikunin    ......GCGA CCTCCGCGCG TTGGGAGGTG TAGCGCGGCT CTGAACGCGT
 N40851    ......GCGA CCTCCGCGCG TTGGGAGGTG TAGCGCGGCT CTGAACGCGT
 N39876    ......GCGA CCTCCGCGCG TTGGGAGGTG TAGCGCGGCT CTGAACGCGT
 R87894    .......... .......... .......... .......... ..........
 H16866    .....GGCGA CCTCCGCGCG TTGGGAGGTG TAGCGCG.CT CTGAACGGGN
 R34808    .......... .......... .......... .......... ..........
 T66058    .......... .......... .......... .......... ..........
 N57450    .......... .......... .........T TAGCGCGGCT CTGAACGCNA
 N57374    .......... .......... .......... .......... ..........
 R35464    .......... .......... .......... .......... ..........
 H94519    .......... .......... .......... .......... ..........
 N39798    .......... .......... .......... .......... ..........
 H87300    .......... .......... .......... .......... ..........
 R74593    .......... .......... .......... .......... ..........
 R31730    .......... .......... .......... .......... ..........
 R34701    .......... .......... .......... .......... ..........
 H02982    .......... .......... .......... .......... ..........
 R32676    .......... .......... .......... .......... ..........
 T47439    .......... .......... .......... .......... ..........
 R73968    .......... .......... .......... .......... ..........
 H39840    .......... .......... .......... .......... ..........
 H95233    .......... .......... .......... .......... ..........
 H39841    .......... .......... .......... .......... ..........
 N30199    .......... .......... .......... .......... ..........
 T52966    .......... .......... .......... .......... ..........
 N29508    .......... .......... .......... .......... ..........
 N26919    .......... .......... .......... .......... ..........
 N26910    .......... .......... .......... .......... ..........
 H16757    .......... .......... .......... .......... ..........
 N27732    .......... .......... .......... .......... ..........
```

# FIG. 4C (Cont.)

| | 51 | | | | 100 |
|---|---|---|---|---|---|
| Bikunin | GNA GGGCCG | TTGAGTGTCG | CAGGCGGCGA | GGGCGCGAGT | GAGGAGCAGA |
| N40851 | NGAGNGGCCG | TTGAGTGTCG | CAGGCGGCGA | GGGCGCGAGT | GAGGAGCAGA |
| N39876 | GCA.GGGCCG | TTGAGTGTCG | CAGGCGGCGA | GGGCGCGAGT | GAGGAGCAGA |
| R87894 | .......... | TTGAGTGTNG | NAGGCGGCGA | GGGCGCGAGT | GAGGAGCAGA |
| H16866 | ..ANGGGCCG | TTGAGTGTCG | CAGGCGGC.A | GGGCN.GAGT | GAGGAGCAGA |
| R34808 | .......... | .......... | .......... | .........G | GAGGAGCAGA |
| T66058 | .......... | .......... | .......... | .......... | .......... |
| N57450 | GAAGNGGCCG | TTGAGTGTCG | CAGGCGGCGA | GGGCGCGAGT | GAGGAGCAGA |
| N57374 | .......... | .......... | .......... | .......... | .......AGA |
| R35464 | .......... | .......... | .......... | .......... | .......... |
| H94519 | .......... | .......... | .......... | .......... | .......... |
| N39798 | .......... | .......... | .......... | .......... | .......... |
| H87300 | .......... | .......... | .......... | .......... | .......... |
| R74593 | .......... | .......... | .......... | .......... | .......... |
| R31730 | .......... | .......... | .......... | .......... | .......... |
| R34701 | .......... | .......... | .......... | .......... | .......... |
| H02982 | .......... | .......... | .......... | .......... | .......... |
| R32676 | .......... | .......... | .......... | .......... | .......... |
| T47439 | .......... | .......... | .......... | .......... | .......... |
| R73968 | .......... | .......... | .......... | .......... | .......... |
| H39840 | .......... | .......... | .......... | .......... | .......... |
| H95233 | .......... | .......... | .......... | .......... | .......... |
| H39841 | .......... | .......... | .......... | .......... | .......... |
| N30199 | .......... | .......... | .......... | .......... | .......... |
| T52966 | .......... | .......... | .......... | .......... | .......... |
| N29508 | .......... | .......... | .......... | .......... | .......... |
| N26919 | .......... | .......... | .......... | .......... | .......... |
| N26910 | .......... | .......... | .......... | .......... | .......... |
| H16757 | .......... | .......... | .......... | .......... | .......... |
| N27732 | .......... | .......... | .......... | .......... | .......... |

# FIG. 4C (Cont.)

| | 101 | | | | 150 |
|---|---|---|---|---|---|
| Bikunin | CCCAGGCATC | GCGCGCCGAG | AAGNC GGGC | GTCCCCACAC | TGAAGGTCCG |
| N40851 | CCCAGGCATC | GCGCGCCGAG | AAGNC.GGGC | GTCCCCACAC | TGAAGGTCCG |
| N39876 | CCCAGGCATC | GCGCGCCGAG | AAGNC.GGGC | NTCCCCACAC | TGAAGGTCCG |
| R87894 | CCCAGGCATC | GCGCGCCGAG | AAGGCCGGGC | GTCCCCACAC | TGAAGGTCCG |
| H16866 | CCCAGGCATC | GCGCGCCGAG | AAGNC.GGGC | GTCCCCACAC | TGAAGGTCCG |
| R34808 | CCCAGGCATC | GCGCGCCGAG | AAGNC.GGGC | GTCCCCACAC | TGAAGGTCCG |
| T66058 | .......... | .......... | .......... | .......... | .......... |
| N57450 | CCCAGGCATC | GCGCGCCGAG | AAGNC.GGGC | GTCCCCACAC | TGAAGGTCCG |
| N57374 | CCCAGGCATC | GCGCGCCGAG | AAGNC.GGGC | GTCCCCACAC | TGAAGGTCCG |
| R35464 | .......... | .......... | .......... | .......... | .......... |
| H94519 | .......... | .......... | .......... | .......... | .......... |
| N39798 | .......... | .......... | .......... | .......... | .......... |
| H87300 | .......... | .......... | .......... | .......... | .......... |
| R74593 | .......... | .......... | .......... | .......... | .......... |
| R31730 | .......... | .......... | .......... | .......... | .......... |
| R34701 | .......... | .......... | .......... | .......... | .......... |
| H02982 | .......... | .......... | .......... | .......... | .......... |
| R32676 | .......... | .......... | .......... | .......... | .......... |
| T47439 | .......... | .......... | .......... | .......... | .......... |
| R73968 | .......... | .......... | .......... | .......... | .......... |
| H39840 | .......... | .......... | .......... | .......... | .......... |
| H95233 | .......... | .......... | .......... | .......... | .......... |
| H39841 | .......... | .......... | .......... | .......... | .......... |
| N30199 | .......... | .......... | .......... | .......... | .......... |
| T52966 | .......... | .......... | .......... | .......... | .......... |
| N29508 | .......... | .......... | .......... | .......... | .......... |
| N26919 | .......... | .......... | .......... | .......... | .......... |
| N26910 | .......... | .......... | .......... | .......... | .......... |
| H16757 | .......... | .......... | .......... | .......... | .......... |
| N27732 | .......... | .......... | .......... | .......... | .......... |

FIG. 4C (Cont.)

| | 151 | | | | 200 |
|---|---|---|---|---|---|
| Bikunin | GAAAGGCGAC | TTCCGGGGGC | TTTGGCACCT | GGCGGACCCT | CCCGGAGCGT |
| N40851 | GAAAGGCGAC | TTCCGGGGGC | TTTGGCACCT | GGCGGACCCT | CCCGGAGCGT |
| N39876 | GAAAGGCGAC | TTCCGGGGGC | TTTGGCACCT | GGCGGACCCT | CCCGGAGCGT |
| R87894 | GAAAGGCGAC | TTCCGGGGGC | TTTGGCACCT | GGCGGACCCT | CCCGGAGCGT |
| H16866 | GAAAGGCGAC | TTCCGGGGGC | TTTGGCACCT | GGCGGACG.T | CCCGGAGCN. |
| R34808 | GAAAGGCGAC | TTCCGGGGGC | TTTGGCACCT | GGCGGACCCT | CCCGGAGCGT |
| T66058 | .......... | .......... | .......... | ...GGACCCT | CCCGGAGCGT |
| N57450 | GAAAGGCGAC | TTCCGGGGGC | TTTGGCACCT | GGCGGACCCT | CCCGGAGCGT |
| N57374 | GAAAGGCGAC | TTCCGGGGGC | TTTGGCACCT | GGCGGACCCT | CCCGGAGCGT |
| R35464 | .......... | .......... | .......... | .......... | .......... |
| H94519 | .......... | .......... | .......... | .......... | .......... |
| N39798 | .......... | .......... | .......... | .......... | .......... |
| H87300 | .......... | .......... | .......... | .......... | .......... |
| R74593 | .......... | .......... | .......... | .......... | .......... |
| R31730 | .......... | .......... | .......... | .......... | .......... |
| R34701 | .......... | .......... | .......... | .......... | .......... |
| H02982 | .......... | .......... | .......... | .......... | .......... |
| R32676 | .......... | .......... | .......... | .......... | .......... |
| T47439 | .......... | .......... | .......... | .......... | .......... |
| R73968 | .......... | .......... | .......... | .......... | .......... |
| H39840 | .......... | .......... | .......... | .......... | .......... |
| H95233 | .......... | .......... | .......... | .......... | .......... |
| H39841 | .......... | .......... | .......... | .......... | .......... |
| N30199 | .......... | .......... | .......... | .......... | .......... |
| T52966 | .......... | .......... | .......... | .......... | .......... |
| N29508 | .......... | .......... | .......... | .......... | .......... |
| N26919 | .......... | .......... | .......... | .......... | .......... |
| N26910 | .......... | .......... | .......... | .......... | .......... |
| H16757 | .......... | .......... | .......... | .......... | .......... |
| N27732 | .......... | .......... | .......... | .......... | .......... |

# FIG. 4C (Cont.)

| | 201 | | | | 250 |
|---|---|---|---|---|---|
| Bikunin | CGGCACCTGA | ACGCGAGGCG | CTCCATTGCG | CGTGCGTTTG | .AGGGGCTTC |
| N40851 | CGGCACCTGA | ACGCGAGGCG | CTCCATTGCG | CGTGCGTNTG | .AGGGGCTTC |
| N39876 | CGGCACCTGA | ACGCGAGGCG | CTCCATTGCG | CGTGCGTTTG | .AGGGGCTTC |
| R87894 | CGGCACCTGA | ACGCGAGGCG | CTCCATTGCG | CGTGCGTTTG | .AGGGGCTTC |
| H16866 | .GGCACCTGA | ACGCGAGGCG | CTCCATTGCG | CGTGCGTTTG | .AGGGGCTTC |
| R34808 | CGGCACCTGA | ACGCGAGGCG | CTCCATTGCG | CGTGCGTNTG | GAGGGGCTTC |
| T66058 | CGGCACCTGA | ACGCGAGGC. | CTCCATTGCG | .GTGCGTGTG | NAGGGGCTTC |
| N57450 | CGGCACCTGA | ACGCGAGGCG | CTCCATTGCG | CGTGCGTTTG | .AGGGGCTTC |
| N57374 | CGGCACCTGA | ACGCGAGGC. | CTCCATTGC. | CGTGCGTTNG | .AGGGGCTTC |
| R35464 | .......... | .......... | .......... | .......... | .......... |
| H94519 | .......... | .......... | .......... | .......... | .......... |
| N39798 | .......... | .......... | .......... | .......... | .......... |
| H87300 | .......... | .......... | .......... | .......... | .......... |
| R74593 | .......... | .......... | .......... | .......... | .......... |
| R31730 | .......... | .......... | .......... | .......... | .......... |
| R34701 | .......... | .......... | .......... | .......... | .......... |
| H02982 | .......... | .......... | .......... | .......... | .......... |
| R32676 | .......... | .......... | .......... | .......... | .......... |
| T47439 | .......... | .......... | .......... | .......... | .......... |
| R73968 | .......... | .......... | .......... | .......... | .......... |
| H39840 | .......... | .......... | .......... | .......... | .......... |
| H95233 | .......... | .......... | .......... | .......... | .......... |
| H39841 | .......... | .......... | .......... | .......... | .......... |
| N30199 | .......... | .......... | .......... | .......... | .......... |
| T52966 | .......... | .......... | .......... | .......... | .......... |
| N29508 | .......... | .......... | .......... | .......... | .......... |
| N26919 | .......... | .......... | .......... | .......... | .......... |
| N26910 | .......... | .......... | .......... | .......... | .......... |
| H16757 | .......... | .......... | .......... | .......... | .......... |
| N27732 | .......... | .......... | .......... | .......... | .......... |

# FIG. 4C (Cont.)

```
         251                                                 300
Bikunin  CCGCACCT G ATCGCGAGAC CCCAACGGCT GGTGG CGTC GC TG CGCG
 N40851  CCGCACCT.G ATCGCGAGAC CCCAACGGCT GGTGG.CGTC GCCTG.CGCG
 N39876  CCGCACCT.G ATCGCGAGAC CCCAACGGCT GGTGG.CGTC GCCTG.CGCG
 R87894  CCGCACCT.G ATCGCGAGAC CCCAACGGCT GGTNG.CGTC GC.TN.CGCG
 H16866  CCGCACCT.G ATCGCGAGAC CCCAACGGCT GGTNG.CGTC GC.TGGCGCG
 R34808  CCGCACCT.G ATCGCGAGAC CCCAACGGCT GGTGGGCGTC GC.TG.CGCG
 T66058  CCGCACCT.G ATCGCGAGAC CCCAACGGCT GGTGG.CGTC GC.TG.CGCG
 N57450  CCGCACCT.G ATCGCGAGAC CCCAACGGCT GGTGG.CGTC GCCTG.CGCG
 N57374  CCGGAACTTG ATCGCGAGAC CCCAACGGCT GGTGG.CGTC GC.TG.CGCG
 R35464  .......... .......... .......... .......... ..........
 H94519  .......... .......... .......... .......... ..........
 N39798  .......... .......... .......... .......... ..........
 H87300  .......... .......... .......... .......... ..........
 R74593  .......... .......... .......... .......... ..........
 R31730  .......... .......... .......... .......... ..........
 R34701  .......... .......... .......... .......... ..........
 H02982  .......... .......... .......... .......... ..........
 R32676  .......... .......... .......... .......... ..........
 T47439  .......... .......... .......... .......... ..........
 R73968  .......... .......... .......... .......... ..........
 H39840  .......... .......... .......... .......... ..........
 H95233  .......... .......... .......... .......... ..........
 H39841  .......... .......... .......... .......... ..........
 N30199  .......... .......... .......... .......... ..........
 T52966  .......... .......... .......... .......... ..........
 N29508  .......... .......... .......... .......... ..........
 N26919  .......... .......... .......... .......... ..........
 N26910  .......... .......... .......... .......... ..........
 H16757  .......... .......... .......... .......... ..........
 N27732  .......... .......... .......... .......... ..........
```

# FIG. 4C (Cont.)

```
         301                                                350
Bikunin  TC TCGGCTG AGCT GGCCA TGGCGCANT  GTTGC GGGC T GAGGC GG
 N40851  TC.TCGGCTG AGCT.GGNCA TGTCG
 N39876  TC.TCGGCTG AGCT.GGCCA TGGCGCACT. G.TGCGGNGC T.GAGGC.G
 R87894  TC.TCGGCTG AGCTTGGCCA TGGCGCANT. GTTNC.GGGC T.NAGGC.GG
 H16866  TTCTCGGCTG AGCT.GGCCA TGGCGCANT. GTTGC.GNGC T.GAGGC.GG
 R34808  TCTTCGGCTG AGCTGGGCCA TGGCGCANTT GTTGC.GGGC T.GAGGC.GG
 T66058  TC.TCGGCTG AGCT.GGCCA TGGCGCANT. GTTGC.GNGC T.GAGGC.GG
 N57450  TC.TCGGCTG AGCT.GGCCA TGGCGCANT. GGTGC.GGGC TTGAGGC.GG
 N57374  TCCTCGGCTG AGCT.GGCCA TGGCGCANT. GGTGCCGNGC T.GAGGCCGG
 R35464  .......... .......... .......... .......... ....GGCCGG
 H94519  .......... .......... .......... .......... ..........
 N39798  .......... .......... .......... .......... ..........
 H87300  .......... .......... .......... .......... ..........
 R74593  .......... .......... .......... .......... ..........
 R31730  .......... .......... .......... .......... ..........
 R34701  .......... .......... .......... .......... ..........
 H02982  .......... .......... .......... .......... ..........
 R32676  .......... .......... .......... .......... ..........
 T47439  .......... .......... .......... .......... ..........
 R73968  .......... .......... .......... .......... ..........
 H39840  .......... .......... .......... .......... ..........
 H95233  .......... .......... .......... .......... ..........
 H39841  .......... .......... .......... .......... ..........
 N30199  .......... .......... .......... .......... ..........
 T52966  .......... .......... .......... .......... ..........
 N29508  .......... .......... .......... .......... ..........
 N26919  .......... .......... .......... .......... ..........
 N26910  .......... .......... .......... .......... ..........
 H16757  .......... .......... .......... .......... ..........
 N27732  .......... .......... .......... .......... ..........
```

# FIG. 4C (Cont.)

```
         351                                              400
Bikunin  AC  GG CG    TTTCTCG  CC TGCTGGG A TCGCT GC T CCTCTCT
 R87894  ACG.
 H16866  AC..CGNCGT TTTTCTTCG. CCTTGCTGGG ATTCGCTTGC TTCCTNTCTG
 R34808  ACGCGGNCG. .TTTTTTCGN CCTTGCTGGG ATTCG.TTG. TTNCTCTCTN
 T66058  ...CGGNCG. .TTTTCTCG. CC.TGCTGGG A.TCGCT.GC T.CCTCTCT.
 N57450  ANN.NGCCG. ..TTTCTCG. CC.TGCTGGG A.TCGCT.GC T.CCTCTCT.
 N57374  AG..GGCCGG ..TTTCTCG. CCTTGCTGGG A.TCGCT.GC T.CCTCTCTG
 R35464  .....GTCG. ..TTTCTCG. CCTGGCTGGG A.TCGCT.GC T.CCTCTCT.
 H94519  .GCNGCGCG. ..TTNNTCG. CN.TGCTGGG A.TCGCT.GC A.CCTCTCT.
 N39798  .......... .......... .....CTGGG ANTCGCT.GC T.CCTCTCT.
 H87300  .......... .......... .......... .......... ..........
 R74593  .......... .......... .......... .......... ..........
 R31730  .......... .......... .......... .......... ..........
 R34701  .......... .......... .......... .......... ..........
 H02982  .......... .......... .......... .......... ..........
 R32676  .......... .......... .......... .......... ..........
 T47439  .......... .......... .......... .......... ..........
 R73968  .......... .......... .......... .......... ..........
 H39840  .......... .......... .......... .......... ..........
 H95233  .......... .......... .......... .......... ..........
 H39841  .......... .......... .......... .......... ..........
 N30199  .......... .......... .......... .......... ..........
 T52966  .......... .......... .......... .......... ..........
 N29508  .......... .......... .......... .......... ..........
 N26919  .......... .......... .......... .......... ..........
 N26910  .......... .......... .......... .......... ..........
 H16757  .......... .......... .......... .......... ..........
 N27732  .......... .......... .......... .......... ..........
```

# FIG. 4C (Cont.)

```
         401                                                450
Bikunin  GGGG TCCTG G  CGGCCGA CCGA GAACG CA GCA TCC ACGACTT CT
 H16866  GGGGTTCCTG GG.CGGCCGA CCGA.GAACG CA.GCA.TCC AAGAATTTTT
 R34808  GGGGTTC.TG GGGNGGCCGA NCGA.GAACG CAAGCA.TTC ACGA.TTT
 T66058  GGGG.TCCTG G..CGGCCGA CCGA.GAACG CA.GCA.TCC ACGANTT.CT
 N57450  GGGG.TCCTG G..CGGCCGA CCGA.GAACG CA.GCA.TCC ACGACTT.CT
 N57374  GGGG.TCCTG G..CGGCCGA NCGAAGAANG CA.GCAATCC ANGAATTNCT
 R35464  GGGG.TCCTG G.CCGGCCGA CCGA.GAACG CA.GCA.TCC ACGACTT.CT
 H94519  GGGG.TCGNG G..CGGCCGA CCGA.GAACG CA.GCA.TCC ACGACTT.CT
 N39798  GGGG.TCCTG G..CGGCCGA CCGA.GAACG CA.GCA.TCC ACGACTT.CT
 H87300  .......... .......... .......... .......... ..........
 R74593  .......... .......... .......... .......... ..........
 R31730  .......... .......... .......... .......... ..........
 R34701  .......... .......... .......... .......... ..........
 H02982  .......... .......... .......... .......... ..........
 R32676  .......... .......... .......... .......... ..........
 T47439  .......... .......... .......... .......... ..........
 R73968  .......... .......... .......... .......... ..........
 H39840  .......... .......... .......... .......... ..........
 H95233  .......... .......... .......... .......... ..........
 H39841  .......... .......... .......... .......... ..........
 N30199  .......... .......... .......... .......... ..........
 T52966  .......... .......... .......... .......... ..........
 N29508  .......... .......... .......... .......... ..........
 N26919  .......... .......... .......... .......... ..........
 N26910  .......... .......... .......... .......... ..........
 H16757  .......... .......... .......... .......... ..........
 N27732  .......... .......... .......... .......... ..........
```

# FIG. 4C (Cont.)

| | | | | | |
|---|---|---|---|---|---|
| | 451 | | | | 500 |
| Bikunin | GCCTGGTGT | CGAAGGT GG | TGGGCAGATG | CCGGG CCTC | CATGCCTA G |
| H16866 | GCC | | | | |
| T66058 | TCCTGGTGTT | CGAAGG | | | |
| N57450 | GCCTGGTGT. | CGAAGGT.GG | TGGGCAG | | |
| N57374 | GCCTGGTGTT | CGAAAGTTGG | TGGGCANATT | CCGGGGCCTT | CATGNCTAAG |
| R35464 | GCCTGGTGT. | CGAAGGT.GG | TGGGCAGATT | CCGGG.CCTC | CATGCCTA.G |
| H94519 | GCCTGGTGT. | CGAAGGT.GG | TGGGCAGATG | CCGGG.CCTC | CATGCCTA.G |
| N39798 | GCCTGGTGT. | CGAAGGT.GG | TGGGCAGATG | CCGGG.CCTC | CATGCCTA.G |
| H87300 | .......... | .......... | .......... | .......... | .......... |
| R74593 | .......... | .......... | .......... | .......... | .......... |
| R31730 | .......... | .......... | .......... | .......... | .......... |
| R34701 | .......... | .......... | .......... | .......... | .......... |
| H02982 | .......... | .......... | .......... | .......... | .......... |
| R32676 | .......... | .......... | .......... | .......... | .......... |
| T47439 | .......... | .......... | .......... | .......... | .......... |
| R73968 | .......... | .......... | .......... | .......... | .......... |
| H39840 | .......... | .......... | .......... | .......... | .......... |
| H95233 | .......... | .......... | .......... | .......... | .......... |
| H39841 | .......... | .......... | .......... | .......... | .......... |
| N30199 | .......... | .......... | .......... | .......... | .......... |
| T52966 | .......... | .......... | .......... | .......... | .......... |
| N29508 | .......... | .......... | .......... | .......... | .......... |
| N26919 | .......... | .......... | .......... | .......... | .......... |
| N26910 | .......... | .......... | .......... | .......... | .......... |
| H16757 | .......... | .......... | .......... | .......... | .......... |
| N27732 | .......... | .......... | .......... | .......... | .......... |

# FIG. 4C (Cont.)

```
         501                                                  550
Bikunin  G TGGT GGT ACAATGTCAC TGACGGATCC TGCCAGCTGT TTGTGT ATG
 N57374  GTTGGTTGGT ANAATGTNAA TTAANGATTC TTGCAACTGT TTGTGTNATT
 R35464  G.TGGT.GGT ACAATGTCAC TGACGGATCC TGCCAGCTGT TTGTGT.ATG
 H94519  G.TGGT.GGT ACAATGTCAC TGACGGATCC TGCCAGCTGT TTGTGT.ATG
 N39798  G.TGGT.GGT ACAATGTCAC TGACGGATCC TGCCAGCTGT TTGTGT.ATG
 H87300  .......... .......... .......... .......... ..........
 R74593  .......... .......... .......... .......... ..........
 R31730  .......... .......... .......... .......... ..........
 R34701  .......... .......... .......... .......... ..........
 H02982  .......... .......... .......... .......... ..........
 R32676  .......... .......... .......... .......... ..........
 T47439  .......... .......... .......... .......... ..........
 R73968  .......... .......... .......... .......... ..........
 H39840  .......... .......... .......... .......... ..........
 H95233  .......... .......... .......... .......... ..........
 H39841  .......... .......... .......... .......... ..........
 N30199  .......... .......... .......... .......... ..........
 T52966  .......... .......... .......... .......... ..........
 N29508  .......... .......... .......... .......... ..........
 N26919  .......... .......... .......... .......... ..........
 N26910  .......... .......... .......... .......... ..........
 H16757  .......... .......... .......... .......... ..........
 N27732  .......... .......... .......... .......... ..........

         551                                                  600
Bikunin  GGGGCTGTGA   CGGAAACA GCAATAATTA CCTGACCAAG GA GGAGTGC
 N57374  GGGGCTNTTA AACGGAAANA .CAATAATNA CCTGACCAAA GAAGNAAT..
 R35464  GGGGCTGTGA ..CGGAAACA GCAATAATTA CCTGACCAAG GA.GGAGTGC
 H94519  GGGGCTGTGA ..CGGAAACA GCAATAATTA CCTGACCAAG GA.GGAGTGC
 N39798  GGGGCTGTGA ..CGGAAACA GCAATAATTA CCTGACCAAG GA.GGAGTGC
 H87300  GATTCGGCAC AGGGGAAACA GCAATAATTA CCTGACCAAG GA.GGAGTNC
 R74593  .......... .......... GCAATAATTA CCTGACCAAG GA.GGAGTGC
 R31730  .......... .......... .......... .......... ..........
 R34701  .......... .......... .......... .......... ..........
 H02982  .......... .......... .......... .......... ..........
 R32676  .......... .......... .......... .......... ..........
 T47439  .......... .......... .......... .......... ..........
 R73968  .......... .......... .......... .......... ..........
 H39840  .......... .......... .......... .......... ..........
 H95233  .......... .......... .......... .......... ..........
 H39841  .......... .......... .......... .......... ..........
 N30199  .......... .......... .......... .......... ..........
 T52966  .......... .......... .......... .......... ..........
 N29508  .......... .......... .......... .......... ..........
 N26919  .......... .......... .......... .......... ..........
 N26910  .......... .......... .......... .......... ..........
 H16757  .......... .......... .......... .......... ..........
 N27732  .......... .......... .......... .......... ..........
```

# FIG. 4C (Cont.)

| | 601 | | | | 650 |
|---|---|---|---|---|---|
| Bikunin | CTCAAGAAAT | GTGCCACTGT | CACAGAGAAT | GCCACGGGTG | ACCTGGCCAC |
| R35464 | CTCAAGAAAT | GTGCCACTGT | CACAGAGAAT | GCCACGGGTG | ACCTGGCCAC |
| H94519 | CTCAAGAAAT | GTGCCACTGT | CACAGAGAAT | GCCACGGGTG | ACCTGGCCAC |
| N39798 | CTCAAGAAAT | GTGCCACTGT | CACAGAGAAT | GCCACGGGTG | ACCTGGCCAC |
| H87300 | CTCAAGAAAT | GTNCCACTGT | CACAGAGAAT | GCCACGGGTG | ACCTGGCCAC |
| R74593 | CTCAAGAAAT | GTGCCACTGT | CACAGAGAAT | GCCACGGGTG | ACCTGGCCAC |
| R31730 | .......... | .......... | .......... | .......... | .......... |
| R34701 | .......... | .......... | .......... | .......... | .......... |
| H02982 | .......... | .......... | .......... | .......... | .......... |
| R32676 | .......... | .......... | .......... | .......... | .......... |
| T47439 | .......... | .......... | .......... | .......... | .......... |
| R73968 | .......... | .......... | .......... | .......... | .......... |
| H39840 | .......... | .......... | .......... | .......... | .......... |
| H95233 | .......... | .......... | .......... | .......... | .......... |
| H39841 | .......... | .......... | .......... | .......... | .......... |
| N30199 | .......... | .......... | .......... | .......... | .......... |
| T52966 | .......... | .......... | .......... | .......... | .......... |
| N29508 | .......... | .......... | .......... | .......... | .......... |
| N26919 | .......... | .......... | .......... | .......... | .......... |
| N26910 | .......... | .......... | .......... | .......... | .......... |
| H16757 | .......... | .......... | .......... | .......... | .......... |
| N27732 | .......... | .......... | .......... | .......... | .......... |

| | 651 | | | | 700 |
|---|---|---|---|---|---|
| Bikunin | CAGCAGGAAT | GCAGCGGATT | CCTCTGTCCC | AAGTGCTCCC | AGAAGGCAGG |
| R35464 | CAGCAGGAAT | GCAGCGGATT | CCTCTGTCCC | AAGTGCTCCC | AGAAGGCAGG |
| H94519 | CAGCAGGAAT | GCAGCGGATT | CCTCTGTCCC | AAGTGCTCCC | AGAAGGCAGG |
| N39798 | CAGCAGGAAT | GCAGCGGATT | CCTCTGTCCC | AAGTGCTCCC | AGAAGGCAGG |
| H87300 | CAGCAGGAAT | GCAGCGGATT | CCTCTGTCCC | AAGTGCTCCC | AGAAGGCAGG |
| R74593 | CAGCAGGAAT | GCAGCGGATT | CCTCTGTCCC | AAGT.CTCCC | AGAAGGCAGG |
| R31730 | .......... | .......... | .......... | .......... | .......... |
| R34701 | .......... | .......... | .......... | .......... | .......... |
| H02982 | .......... | .......... | .......... | .......... | .......... |
| R32676 | .......... | .......... | .......... | .......... | .......... |
| T47439 | .......... | .......... | .......... | .......... | .......... |
| R73968 | .......... | .......... | .......... | .......... | .......... |
| H39840 | .......... | .......... | .......... | .......... | .......... |
| H95233 | .......... | .......... | .......... | .......... | .......... |
| H39841 | .......... | .......... | .......... | .......... | .......... |
| N30199 | .......... | .......... | .......... | .......... | .......... |
| T52966 | .......... | .......... | .......... | .......... | .......... |
| N29508 | .......... | .......... | .......... | .......... | .......... |
| N26919 | .......... | .......... | .......... | .......... | .......... |
| N26910 | .......... | .......... | .......... | .......... | .......... |
| H16757 | .......... | .......... | .......... | .......... | .......... |
| N27732 | .......... | .......... | .......... | .......... | .......... |

# FIG. 4C (Cont.)

```
         701                                                750
Bikunin  ATTCT GAAG ACCACTCCAG CGATATGTT  CAACTAT  G AAGAATACTG
 R35464  ATTCTTGAAG ACCACTTCAG CGATATGTTT CAANTATTGN AAGAATAATT
 H94519  ATTCT.GAAG ACCACTCCAG CGATATGTT. CAACTAT..G AAGAATACTG
 N39798  ATTCT.GAAG ACCACTCCAG CGATATGTT. CAACTAT..G AAGAATACTG
 H87300  ATTCT.GAAG ACCACTCCAG CGATATGTT. CAACTAT..G AAGAATACTG
 R74593  ATTCT.GAAG ACCACTCCAG CGATATGTT. CAACTAT..G AAGAATACTG
 R31730  .......... .......... .......... .......... ..........
 R34701  .......... .......... .......... .......... ..........
 H02982  .......... .......... .......... .......... ..........
 R32676  .......... .......... .......... .......... ..........
 T47439  .......... .......... .......... .......... ..........
 R73968  .......... .......... .......... .......... ..........
 H39840  .......... .......... .......... .......... ..........
 H95233  .......... .......... .......... .......... ..........
 H39841  .......... .......... .......... .......... ..........
 N30199  .......... .......... .......... .......... ..........
 T52966  .......... .......... .......... .......... ..........
 N29508  .......... .......... .......... .......... ..........
 N26919  .......... .......... .......... .......... ..........
 N26910  .......... .......... .......... .......... ..........
 H16757  .......... .......... .......... .......... ..........
 N27732  .......... .......... .......... .......... ..........

         751                                                800
Bikunin   CACCGCCAA CGCAGT CAC TGGGCC TTG CCGTG CAT  CCTT CCCAC
 R35464  GCACCGNCAA CGNATT
 H94519  GCACCGCCAA CGCATT.CAC TGGGCC..TG C.GTG.CAT. CCTT.CCCAC
 N39798  .CACCGCCAA CGCAGT.CAC TGGGGCCTTG C.GTGGAAT. CCTTTCCCAC
 H87300  .CACCGCCAA CGCAGTNCAC TGGGCC.TTG C.GTGGCATN CCTT.CCCAC
 R74593  .CACCGCCAA CGCAGT.CAC TGGGCC.TTG CCGTG.CAT. CCTT.CCCAC
 R31730  .......... .......... .......... .......... ..........
 R34701  .......... .......... .......... .......... ..........
 H02982  .......... .......... .......... .......... ..........
 R32676  .......... .......... .......... .......... ..........
 T47439  .......... .......... .......... .......... ..........
 R73968  .......... .......... .......... .......... ..........
 H39840  .......... .......... .......... .......... ..........
 H95233  .......... .......... .......... .......... ..........
 H39841  .......... .......... .......... .......... ..........
 N30199  .......... .......... .......... .......... ..........
 T52966  .......... .......... .......... .......... ..........
 N29508  .......... .......... .......... .......... ..........
 N26919  .......... .......... .......... .......... ..........
 N26910  .......... .......... .......... .......... ..........
 H16757  .......... .......... .......... .......... ..........
 N27732  .......... .......... .......... .......... ..........
```

# FIG. 4C (Cont.)

```
         801                                                850
Bikunin  GCTGGTACTT T GACGTGGA GA GGAACTC CTG CAATAA CTTCATCTAT
 H94519  GCTGGTACTT T.GNCGT
 N39798  GCTGGNAATT TNGACGTTGA GAAGGAAC
 H87300  GCTNGTACTT T.GACGTGGA GA.GGAACTC CTGGCAATAA CTTCATCTAT
 R74593  GCTGGTACTT T.GACGTGGA GA.GGAACTC CTG.CAATAA CTTCATCTAT
 R31730  .......... .......... .......... .......... ..........
 R34701  .......... .......... .......... .......... ..........
 H02982  .......... ........GA GA.GGAACTC CTG.CAATAA CTTCATCTAT
 R32676  .......... .......... .......... .........G ATTC..GGAA
 T47439  .......... .......... .......... .......... ..........
 R73968  .......... .......... .......... .......... ..........
 H39840  .......... .......... .......... .......... ..........
 H95233  .......... .......... .......... .......... ..........
 H39841  .......... .......... .......... .......... ..........
 N30199  .......... .......... .......... .......... ..........
 T52966  .......... .......... .......... .......... ..........
 N29508  .......... .......... .......... .......... ..........
 N26919  .......... .......... .......... .......... ..........
 N26910  .......... .......... .......... .......... ..........
 H16757  .......... .......... .......... .......... ..........
 N27732  .......... .......... .......... .......... ..........

         851                                                900
Bikunin  GGAGGCT GC CGGGGCAAT  AAGAACAG C TACCGCTC T GAGGAGGCCT
 H87300  GGAGGCTTGC CGGGGCAATN AAGAACAGNT TACCGCTCTT TAGGAGGCCT
 R74593  GGAGGCT.GC CGGGGCAAT. AAGAACAG.C TACCGCTC.T GAGGAGGCCT
 R31730  .......... .......... .......G.C TACCGCTC.T GAGGAGGCCT
 R34701  .......... .......... .......... .......... ..........
 H02982  GGNGGCT.GC CGGGG.AAT. AAGAACA.NC TACCGCTC.T GAGGAGGCCT
 R32676  CGAGGA..GC CGGGGCAAT. AAGAACAG.C TACCGCTC.T GAGGAGGCCT
 T47439  .......... .......... .......... .......... ....NGGCCT
 R73968  .......... .......... .......... .......... ..........
 H39840  .......... .......... .......... .......... ..........
 H95233  .......... .......... .......... .......... ..........
 H39841  .......... .......... .......... .......... ..........
 N30199  .......... .......... .......... .......... ..........
 T52966  .......... .......... .......... .......... ..........
 N29508  .......... .......... .......... .......... ..........
 N26919  .......... .......... .......... .......... ..........
 N26910  .......... .......... .......... .......... ..........
 H16757  .......... .......... .......... .......... ..........
 N27732  .......... .......... .......... .......... ..........
```

# FIG. 4C (Cont.)

```
         901                                                   950
Bikunin   GCA TGCTC CGCTGCTTCC GC                        CA GCAGGA
 H87300  .GCA.T.... .
 R74593  .GCA.TGCTC CGCTGCTTCC GC........ ........... .CA.GCAGGA
 R31730  .GCA.TGCTC CGCTGCTTCC GC........ ........... .CA.GCAGGA
 R34701  .......... ......TTCC GC........ ........... .CAAGCAGGA
 H02982  .GCG.TGCTC CGCTGCTTCC GCTGTGTGTT CTCTTCCAGG CCA.GCAGGA
 R32676  .GCA.TGCTC CGCTGCTTCC GC........ ........... .CA.GCAGGA
 T47439  TGCAGTGCTC CGCTGCTTCC GC........ ........... .CA.GCAGGA
 R73968  .......... .......... .......... .......... ..........
 H39840  .......... .......... .......... .......... ..........
 H95233  .......... .......... .......... .......... ..........
 H39841  .......... .......... .......... .......... ..........
 N30199  .......... .......... .......... .......... ..........
 T52966  .......... .......... .......... .......... ..........
 N29508  .......... .......... .......... .......... ..........
 N26919  .......... .......... .......... .......... ..........
 N26910  .......... .......... .......... .......... ..........
 H16757  .......... .......... .......... .......... ..........
 N27732  .......... .......... .......... .......... ..........

         951                                                  1000
Bikunin  GAA TCCTCC CCTGCCCCTT GGCTCAAAGG TGGTGGTTC  TGG CGGGGC
 R74593  GAA.TCCTCC CCTGCCCCTT GGCTCAAAGG TGGTGGTTC. TGGCCGGGGC
 R31730  GAA.TCCTCC CCTGCCCCTT GGCTCAAAGG TGGTGGTTC. TGG.CGGGGC
 R34701  AAANTCCTCC CCTCCCCCTT GGCTCAAAGG TGGTGGTTCC TGG.CGGGGC
 H02982  GAA.TCCTCC CCTGCCCCTT GGCTCAAAGG TGGTGGTTC. TGG.CGGGGC
 R32676  GAA.TCCTCC CCTGCCCCTT GGCTCAAAGG TGGTGGTTC. TGG.CGGGGC
 T47439  GAA.TCCTCC CCTGCCCCTT GGCTCAAAGG TGGTGGTTC. TGG.CGGGGC
 R73968  .......... .......... .......... .......... ....CGGGGC
 H39840  .......... .......... .......... .......... ..........
 H95233  .......... .......... .......... .......... ..........
 H39841  .......... .......... .......... .......... ..........
 N30199  .......... .......... .......... .......... ..........
 T52966  .......... .......... .......... .......... ..........
 N29508  .......... .......... .......... .......... ..........
 N26919  .......... .......... .......... .......... ..........
 N26910  .......... .......... .......... .......... ..........
 H16757  .......... .......... .......... .......... ..........
 N27732  .......... .......... .......... .......... ..........
```

# FIG. 4C (Cont.)

| | 1001 | | | | 1050 |
|---|---|---|---|---|---|
| Bikunin | TGTT CGTGA | TGGTGTTGAT | CC T CTTCC | TGGG AGCCT | CC ATGGTC |
| R74593 | TGTTTCGTGA | TGGTGTTGAT | CCTT..TTCC | TGGGGAGCNT | CC.ATGGTCT |
| R31730 | TGTT.CGTGA | TGGTGTTGAT | CC.T.CTTCC | TGGGGAGCCT | CC.ATGGTC. |
| R34701 | TGTT.CGTGA | TGGTGTTGAT | CCCTCCTTCC | CGGG.AGCCT | CCCATGGTCC |
| H02982 | TGTT.CGTGA | TGGTGTTGAT | CC.T.CTTCC | TGGG.AGCCT | CC.ATGGTN. |
| R32676 | TGTT.CGTGA | TGGTGTTGAT | CC.T.CTTCC | TGGG.AGCCT | CC.ATGGTC. |
| T47439 | TGTT.CGTGA | TGGTGTTGAT | CC.T.CTTCC | TGGG.AGCCT | CC.ATGGTC. |
| R73968 | TGTT.CGTGA | TGGTGTTGAT | CC.T.CTTCC | TGGG.AGCCT | CC.ATGGTC. |
| H39840 | .......... | .......... | .......... | .......... | .......... |
| H95233 | .......... | .......... | .......... | .......... | .......... |
| H39841 | .......... | .......... | .......... | .......... | .......... |
| N30199 | .......... | .......... | .......... | .......... | .......... |
| T52966 | .......... | .......... | .......... | .......... | .......... |
| N29508 | .......... | .......... | .......... | .......... | .......... |
| N26919 | .......... | .......... | .......... | .......... | .......... |
| N26910 | .......... | .......... | .......... | .......... | .......... |
| H16757 | .......... | .......... | .......... | .......... | .......... |
| N27732 | .......... | .......... | .......... | .......... | .......... |

| | 1051 | | | | 1100 |
|---|---|---|---|---|---|
| Bikunin | TACC TGAT | CCGGGTGGCA | CGGAGG AAC | C AGG AGCG | TGCCCTGCGC |
| R74593 | TAC..TGATT | CCGGGTGGCA | AGGAGG.AAC | C.AGG.AGCG | TGCCCTGCGG |
| R31730 | TACC.TGAT. | CCGGGTGGCA | CGGAGGGAAC | C.AGGGAGCG | TGCCCTGCGC |
| R34701 | TACCCTGAT. | CCGGGTGGCA | CGGAGG.AAC | CCAGG.ANCG | TGCCCTGCGC |
| H02982 | TACC.TGAT. | CCGGGTNGCA | CGGAGG.AAC | C.AGGGAGCG | TGCCCTGCGN |
| R32676 | TACC.TGAT. | CCGGGTGGCA | CGGAGG.AAC | C.AGGGAGCG | TGCCCTGCGC |
| T47439 | TACC.TGAT. | CCGGGTNGCA | CGGAGG.AAC | C.AGG.AGCG | TGCCCTGCGC |
| R73968 | TACC.TGAT. | CCGGGTGGCA | CGGAGG.AAC | C.AGG.AGCG | TGCCCTGCGC |
| H39840 | .......... | .......... | ...GGG.AAC | C.AGG.AGCG | TGCCCTGCGC |
| H95233 | .......... | .......... | .......... | .......... | .......... |
| H39841 | .......... | .......... | .......... | .......... | .......... |
| N30199 | .......... | .......... | ..GAGGAACC | C.ANG.AGCT | TCCCCTGCGC |
| T52966 | .......... | .......... | .......... | .......... | .......... |
| N29508 | .......... | .......... | .......... | .......... | .......... |
| N26919 | .......... | .......... | .......... | .......... | .......... |
| N26910 | .......... | .......... | .......... | .......... | .......... |
| H16757 | .......... | .......... | .......... | .......... | .......... |
| N27732 | .......... | .......... | .......... | .......... | .......... |

# FIG. 4C (Cont.)

```
         1101                                                 1150
Bikunin  ACCG TCT G GAGCTCCGGA GATGACAAGG  AGCAGCTGG  TGAAGAAC
R74593   ANCG.TCT.G GAGCTTCGGA GATGACAAGG GNT
R31730   ACCG.TCTGG GAGCTCCGGA GATGACAAGG GAGCAGCTGG GTGAAGAAC.
R34701   ACCG.TCT.G GAGCTCCGGA GATGACAAGG .AGCAGCTGG .TGAAGAAC.
H02982   ACCG.TCTNG GAGCTCCGGA GATGACAAGG .AGCAGCTGG .TGAAGAAC.
R32676   ACCG.TCTGG GAGCTCCGGA GATGACAAGG GAGCAGCTGG .TGAAGAAC.
T47439   ACCG.TCT.G GAGCTCCGGA GATGACAAGG .AGCAGCTGG .TGAAGAAC.
R73968   ACCG.TCT.G GAGCTCCGGA GATGACAAGG .AGCAGCTGG .TGAAGAAC.
H39840   ACCGGTCT.G GAGCTCCGGA GATGACAAGG .AGCAGCTGG .TGAAGAAC.
H95233   .......... .......... .......... .......... ..........
H39841   .......... .......... .......... .......... ..........
N30199   ACCG.TCT.G GAGCTCCGGA GATNACAANG .AGCAGCTGN .TGAAGAACC
T52966   .......... .......... .......... .......... ..........
N29508   .......... .......... .......... .......... ..........
N26919   .......... .......... .......... .......... ..........
N26910   .......... .......... .......... .......... ..........
H16757   .......... .......... .......... .......... ..........
N27732   .......... .......... .......... .......... ..........

         1151                                                 1200
Bikunin  ACATATGT C CTGT GACCG CCCTGT CGC C AAGAGG A CT GGGGAA
R31730   ACATATGTTC CTGTTGACCG NCCTGTTCGC C.AAGAGG.A TTGGGGGAA.
R34701   ACATATGT.C CTGT.GACCG CCCTGT.CGC C.AAGAGG.A CT.GGGGAA.
H02982   ACATATGT.C CTGT.GACCG NCCTGTTCGN C.AAGAGG.A CTNGGGGAAA
R32676   ACATATGTTC CTGTTGACCG CCCTGTTCGC C.AAGAGGGA NTGGGGGAA.
T47439   ACATATGT.C CTGT.GACCG CCCTGT.CGC C.AAGAGG.A CT.GGGGAA.
R73968   ACATATGT.C CTGT.GACCG CCCTGT.CGC C.AAGAGG.A CT.GGGGAA.
H39840   ACATATGT.C CTGT.GACCG CCCTGT.CGC C.AAGAGG.A CT.NGGGAA.
H95233   .......... .......... .......... .......... ..........
H39841   .......... ........C. CCCTGT.CGC CCAAAAGG.A CT.GGGGAA.
N30199   ACATATGT.C CTGT.GACCG CCCTNT.CGC C.AAGAGG.A CT.GGGNAAA
T52966   .......... .......... .......... .......... ..........
N29508   .......... .......CC. CCCTNT.CGC C.AAGAGG.A CT.GGG.AA.
N26919   .......... .......... .......... .......... ..........
N26910   .......... .......... .......... .......... ..........
H16757   .......... .......... .......... .......... ..........
N27732   .......... .......... .......... .......... ..........
```

# FIG. 4C (Cont.)

```
         1201                                                  1250
Bikunin  GGGAGGGG   AGACTAT G  TGT GA GCT  TTTTTT   AA A TAGA   GG
R31730   .GGGAGGGGG A
R34701   .GGGAGGGG. AGACTAT.G. TGT.GA.GCT TTTTTT..AA A.TA
H02982   GGGGAGGGG. AGATTAT.G. TGTTGA.GTT TTTTTT..AA ANTAG
R32676   GGGGAGGGGG AGANTATTGT TGTTGA.GNT TTTTTTTAAA ATTAGGAGGG
T47439   .GGGAGGGG. AGACTAT.G. TGT.GA.GCT TTTTTT..AA A.TAGA..GG
R73968   .GGGAGGGG. AGACTAT.G. TGT.GA.GCT TTTTTT..AA A.TAGA..GG
H39840   .GGGAGGGG. AGACTAT.G. TGT.GA.GCT TTTTTT..AA A.TAGA..GG
H95233   .......... .......... .......... .......... ..........
H39841   .GGGAGGGGA AAACNAT.G. TGT.GAACCT TTTTTT.AAA A.TAGA..GG
N30199   .GGGAGGNG. AGACTAT.G. TGT.AA.GCT TTTTTT..AA A.TAGA..GG
T52966   .......... .......... .......... .......... ..........
N29508   .GGGAGGGG. AGACTA..G. TGT.GA.GCT TTTTTT..AA A.TAGA..GG
N26919   .......... .......... .......... .......... ..........
N26910   .......... .......... .......... .......... ..........
H16757   .......... .......... .......... .......... ..........
N27732   .......... .......... .......... .......... ..........

         1251                                                  1300
Bikunin  GATTGACTC   GGATTTG A GT GATC A  TTAGGG  CT GAGGTCTGTT
R32676   GNTTGANTTC GGGNTTTTNA GTTGATCCAT TTAGGGGGNT GAG
T47439   GATTGACTC. .GGATTTG.A GT.GATC.A. TTAGGG..CT GAGGTCTNTT
R73968   GATTGACTC. .GGATTTG.A GT.GATC.A. TTAGGG..CT GAGGTCTGTT
H39840   GATTGACTC. .GGATTTG.A GT.GATC.A. TTAGGG..CT GAGGTCTGTT
H95233   .......... .......... .........A. TTAGGG..CT GAGGTCTGTT
H39841   GATTGACTC. .GGATTTG.A GT.GATC.A. TTAGGG..CT GAGGTCTGTT
N30199   GATTGACTC. .GGATTTGGA GT.GATC.A. TTAGGG..CT GAGGTCTGTT
T52966   .......... .......... .......... .......... ..........
N29508   GATTGACTC. .GGATTTG.A GT.GATCNA. TTAGGG..CT GAGGTCTGTT
N26919   .......... .......... .......... .......... ..........
N26910   .......... .......... .......... .......... ..........
H16757   .......... .......... .......... .......... ..........
N27732   .......... .......... .......... .......... ..........

         1301                                                  1350
Bikunin  TCTCTGGGAG GTAGGACGGC TGCTTCC TG G TC TGGCA  GGGATGGG
T47439   TCTCTNGGAG GTAGGACGA
R73968   TCTCTGGGAG GTAGGACGGC TGCTTCC.TG GGTCTTGGCA .GGGATGGGG
H39840   TCTCTGGGAG GTAGGACGGC TGCTTCC.TG G.TC.TGGCA .GGGATGGG.
H95233   NCTCTGGGAG NTAGGACGGC TGCCTTCCTG G.TC.TGGCA .GGGATGGG.
H39841   TCNCTGGGAG GTAGGACGGC TGCTCCCCTG G.TC.TGGCA .GGGATGGG.
N30199   TCTCTGGGAG GTAGGACGGC TGCTTCC.TG G.TC.TGGCA .GGGATGGG.
T52966   .......... .......... .......... ..TC.TGGCA .GGGATGGG.
N29508   TCTCTGGGAG GTAGGACGGC TGCTTCA.TG G.TC.TGGCA .GGGATGGG.
N26919   .......... .......... .......... .......... ..........
N26910   .......... .......... .......... .......... ..........
H16757   .......... .......... .........G G.TC.TGGCA .GGGATGGG.
N27732   .......... .......... .....CCCTG GGTCCTGNCA AGGNATGGGG
```

# FIG. 4C (Cont.)

```
         1351                                                1400
Bikunin  TTTG CTTTG G AAATCCTC T AGGAGGCT CCTCCT CGC ATGG CC TG
 R73968  TTTG.CTTTG GGAAATCCTC TTNGGAGGCT CCTCCTTCGC ATGGGCCTTG
 H39840  TTTG.CTTTG GAGAATCCTC T.ANGAGGCT CCTCCT.CGC ATGG.CC.TG
 H95233  TTTG.CTTTG G.AAATCCTC T.AGGAGGCT CCTCCT.CGC ATGG.CC.TG
 H39841  TTTG.CTTTG G.AAANCCNC T.AGGAGGCT CCTCCT.CGC ATGG.CC.TG
 N30199  TTTG.CTTTG G.AAATCCTC T.AGGAGGCT CCTCCTTCGC ATGG.CC.TG
 T52966  TTTG.CTTTG G.AAATCCTC T.AGGAGGCT CCTCCT.CGC ATGG.CC.TG
 N29508  TTTG.CTTTG G.AAATCCTC T.AGGAGGCT CCTCCT.CGC ATGG.CC.TG
 N26919  .......... .......... ....GAGGCT CCTCCT.CGC ATGG.CC.TG
 N26910  .....CTTTT GNAAATCCTC T.AGGAGGCT CCTCCT.CGC ATGG.CC.TG
 H16757  TTTGCCTTTG G.AAANCCTC T.AGGAGGCT CCTCCT.CGC ATGG.CC.TG
 N27732  TTTG.CTTTG G.AAATCCTC TTAGGAGGCT CCTCCT.CGC ATGG.CC.TG

         1401                                                1450
Bikunin  CAGT CT GG CAGCAG CCC CGAGTTGTTT  CC TCGCTG ATC GATTTC
 R73968  CAGT.CTNGG CAGCANCCCC CGAGTTTTTT TCCTTCGCTG ATCCGATTTC
 H39840  CAGT.CT.GG CAGCAG.CCC CGAGTTGTTT .CC.TCGCTG ATC.GATTTC
 H95233  CAGTTCT..G CAGCAG.CCC CGAGTTGTTT .CC.TCGCTG ATC.GATTTC
 H39841  CAGT.CT.GG CAGCAG.CCC CGAGTTGTTN .CC.TCGCTG ATC.GATNTC
 N30199  CAGT.CT.GG CAGCAG.CCC CGAGTTGTTT .CC.TCGCTG ATC.GATTTC
 T52966  CAGT.CT.GG CAGCAG..CC CGAGTTGTTT .CC.TCGCTG ATC.GATTTC
 N29508  CAGT.CT..G CAGCAG.CCC CGAGTTGTTT .CC.TCGCTG ATC.GATTTC
 N26919  CAGT.CTTGG CAGCAG.CCC CGAGTTGTTT .CC.TCGCTG ANC.GATTTC
 N26910  CAGT.CT..G CAGCAG.CCC CGAGTTGTTT .CC.TCGCTG ATCGGATTTC
 H16757  CAGTNCT.GG CAGCAGACCC CGAGTTGTTT .CC.TCGCTG ATC.GATTTC
 N27732  CAGT.CT.GG CAGCAG.CCC CGAGTTGTTT .CC.TCGCTG ANC.GATTTC

         1451                                                1500
Bikunin  TTT CCTCCA GGTAG  AGT TTTC TTTG  CTTATGTTGA ATTCCATTGC
 R73968  TTTTCCTCCA GGTAAGAATT TTTCTTTT
 H39840  TTT.CCTCCA GGTAG..AGT TTTC.TTTG. CTTATGTTGA ATTCCATTGC
 H95233  TTT.CCTCCA GGTAG..AGT TTTC.TTTG. CTTATGTTGA ATTCCATTGC
 H39841  TTT.CCCCCA GGTAG..AGT TTTC.TTTG. CTTATGTTGA ANTCCATTGC
 N30199  TTT.CCTCCA GGTAG..AGT TTTC.TTTG. CTTATGTTGA ATTCCATTGC
 T52966  TTT.CCTCCA GGTAG..AGT TTTC.TTTG. CTTATGTTGA ATTCCATTGC
 N29508  TTT.CCTCCA GGTAG..AGT TTTC.TTTG. CTTATGTTGA ATTCCATTGC
 N26919  TTT.CCNCCA GGTAG..AGT TTTC.TTTG. CTTATGTTGA ATTCCATTGC
 N26910  TTT.CCTCCA GGTAG..AGT TTTC.TTTG. CTTATGTTGA ATTCCATTGC
 H16757  TTTACCCCCA GGTAG..AGT TTTCCTTTGN CTTATGTTGA ATTCCATTGC
 N27732  TTT.CCTCCA GGTAG..AGT TTTC.TTTG. CTTATGTTGA ATTCCATTGC
```

# FIG. 4C (Cont.)

```
         1501                                            1550
Bikunin  CTCTTTT CT CATCACAGAA GTGATGTTGG AATCGTTTCT TTTGTTT GT
 H39840  CTCTTTT.CT CATCACAGAA GTGATGTTGG AATCGTTTCT TTTGTTTTGT
 H95233  CTCTTTT.CT CATCACAGAA GTGATGTTGG AATCGTTTCT TTTGTTT.GT
 H39841  CTCTTTT.CT CATCACAGAA GTGATGTTGG AATCGTTTCT TTTGTTT.GT
 N30199  CTCTTTT.CT CATCACAGAA GTGATGTTGG AATCGTTTCT TTTGTTT.GT
 T52966  CTCTTTT.CT CATCACAGAA GTGATGTTGG AATCGTTTCT TTTGTTT.GT
 N29508  CTCTTTT.CT CATCACAGAA GTGATGTTGG AATCGTTTCT TTTGTTT.GT
 N26919  CTCTTTT.CN CATCACAGAA GTGATGTTGG AATCGTTTCT TTTGTTT.GT
 N26910  CTCTTTT.CT CATCACAGAA GTGATGTTGG AATCGTTTCT TTTGTTT.GT
 H16757  CTCTTTTACT CATCACAGAA GTGATGTTGG AATCGTTTCT TTTGTTT.GT
 N27732  CTCTTTT.CT CATCACAGAA GTGATGTTGG AATCGTTTCT TTTGTTT.GT

         1551                                            1600
Bikunin  CTGATTTATG G  TTTTTTT AAGTATAAAC AAAAGTTTTT TATTAGCATT
 H39840  CTGATTTATG GGTTTTTTTT AAGTAT
 H95233  CTGATTTATG G..TTTTTTT AAGTATAAAC AAAAGTTTTT TATTAGCATT
 H39841  CTGATTTATG G..TTTTTTT AAGTATAAAC AAAAGTTTTT TATTAGCATT
 N30199  CTGATTTATG G..TTTTTTT AAGTATAAAC AAAAGTTTTT TATTAGCATT
 T52966  CTGATTTATG G..TTTTTTT AAGTATAAAC AAAAGTTTTT TATTAGCATT
 N29508  CTGATTTATG G..TTTTTTT AAGTATAAAC AAAAGTTTTT TATTAGCATT
 N26919  CTGATTTATG G..TTTTTTT AAGTNTAAAC AAAAGTTTTT TATTAGCATT
 N26910  CTGATTTATG G..TTTTTTT AAGTATAAAC AAAAGTTTTT TATTAGCATT
 H16757  CTGATTTATG G..TTTTTTT AAGTATAAAC AAAAGTTTTT TATTAGCATT
 N27732  CTGATTTATG G..TTTTTTT AAGTATAAAC AAAAGTTTTT TATTAGCATT

         1601                                            1650
Bikunin  CTGAAAGAAG GAAAGTAAAA TGTACAAGTT TAATAAAAAG GGGCCTTCCC
 H95233  CTGAAAGAAG GAAAGTAAAA TGTACAAGTT TAATAAA
 H39841  CTGAAAGAAG GAAAGTAAAN TGTACAAGTT TAATAAAAAG GGGCCTTCCC
 N30199  CTGAAAGAAG GAAAGTAAAA TGTACAAGTT TAATAAAAAG GGGCCTTCCC
 T52966  CTGAAAGAAG GAAAGTAAAA TGTACAAGTT TAATAAAAAG GGGCCTTCCC
 N29508  CTGAAAGAAG GAAAGTAAAA TGTACAAGTT TAATAAAAAG GGGCCTTCCC
 N26919  CTGAAAGAAG GAAAGTAAAA TGTACAAGTT TAATAAAAAG GGGCCTTCCC
 N26910  CTGAAAGAAG GAAAGTAAAA TGTACAAGTT TAATAAAAAG GGGCCTTCCC
 H16757  CTGAAAGAAG GAAAGTAAAA TGTACAAGTT TAATAAAAAG GGGCCTTCCC
 N27732  CTGAAAGAAG GAAAGTAAAA TGTACAAGTT TAATAAAAAG GGGCCTTCCC

         1651                                 1689
Bikunin  CTTTAG AAT AAAAAAAAAA AAAAAAAAAA AAAAAAAAA
 H39841  CTTTAA.
 N30199  CTTTAG.AAT AAA
 T52966  CTTTAGGAAT NAAAANAAAA AAGGGTG
 N29508  CTTTAG.AAT AAATTTCAGC ATGTGCTTTC AA
 N26919  CTTTAG.AAT AAAAAAAAAA AAAAAAAAAA A
 N26910  CTTTAG.AAT AAATTTCAGC ATGTGCTTTC AAAAAA
 H16757  CTTTAG.AAT AAAAAAAAAA AAAAAAAAAA AAAAAA
 N27732  CTTTAG.AAT AAAAAAAAAA AAAAAAAAAA AAAAAAAAA
```

FIG. 4D

EST consens MLRAEADGVS RLLGSLLLSG VLAADRERSI HDFCLVSKVV GRCRASMPRW 50

EST consens WYNVTDGSCQ LFVYGGCDGN SNNYLTKEEC LKKCATVTEN ATGDLATSRN 100

EST consens AADSSVPSAP RRQDSEDHSS DMFNYEEYCT ANAVTGPCRA SFPRWYFDVE 150

EST consens RNSCNNFIYG GCRGNKNSYR SEEACMLRCF RQQENPPLPL GSKVVVLAGL 200

EST consens FVMVLILFLG ASMVYLIRVA RRNQERALRT VWSSGDDKEQ LVKNTYVL 248

# FIG. 4E

```
cDNA                                                                  ACC   3
translation                                                           T   -47

cDNA        TGATCGCGAG ACCCCAACGG CTGGTGGCGT CGCCTGCGCG TCTCGGCTGA  53
translation . S R D   P N G   W W R   R L R V   S A E  -30

cDNA        GCTGGCCATG GCGCAGCTGT GCGGGCTGAG GCGGAGCCGG GCGTTTCTCG 103
translation L A M   A Q L C   G L R   R S R   A F L A-13

cDNA        CCCTGCTGGG ATCGCTGCTC CTCTCTGGGG TCCTGGCGGC CGACCGAGAA 153
translation  L L G   S L L   L S G V   L A A   D R E    4

cDNA        CGCAGCATCC ACGACTTCTG CCTGGTGTCG AAGGTGGTGG GCAGATGCCG 203
translation R S I H   D F C   L V S   K V V G   R C R   21

cDNA        GGCCTCCATG CCTAGGTGGT GGTACAATGT CACTGACGGA TCCTGCCAGC 253
translation  A S M   P R W W   Y N V   T D G   S C Q L  38

cDNA        TGTTTGTGTA TGGGGGCTGT GACGGAAACA GCAATAATTA CCTGACCAAG 303
translation  F V Y   G G C   D G N S   N N Y   L T K    54

cDNA        GAGGAGTGCC TCAAGAAATG TGCCACTGTC ACAGAGAATG CCACGGGTGA 353
translation E E C L   K K C   A T V   T E N A   T G D   71

cDNA        CCTGGCCACC AGCAGGAATG CAGCGGATTC CTCTGTCCCA AGTGCTCCCA 403
translation  L A T   S R N A   A D S   S V P   S A P R  88

cDNA        GAAGGCAGGA TTCTGAAGAC CACTCCAGCG ATATGTTCAA CTATGAAGAA 453
translation  R Q D   S E D   H S S D   M F N   Y E E   104

cDNA        TACTGCACCG CCAACGCAGT CACTGGGCCT TGCCGTGCAT CCTTCCCACG 503
translation Y C T A   N A V   T G P   C R A S   F P R  121

cDNA        CTGGTACTTT GACGTGGAGA GGAACTCCTG CAATAACTTC ATCTATGGAG 553
translation  W Y F   D V E R   N S C   N N F   I Y G G 138

cDNA        GCTGCCGGGG CAATAAGAAC AGCTACCGCT CTGAGGAGGC CTGCATGCTC 603
translation  C R G   N K N   S Y R S   E E A   C M L   154

cDNA        CGCTGCTTCC GCCAGCAGGA GAATCCTCCC CTGCCCCTTG GCTCAAAGGT 653
translation R C F R   Q Q E   N P P   L P L G   S K V  171

cDNA        GGTGGTTCTG GCGGGGCTGT TCGTGATGGT GTTGATCCTC TTCCTGGGAG 703
translation  V V L   A G L F   V M V   L I L   F L G A 188

cDNA        CCTCCATGGT CTACCTGATC CGGGTGGCAC GGAGGAACCA GGAGCGTGCC 753
translation  S M V   Y L I   R V A R   R N Q   E R A   204

cDNA        CTGCGCACCG TCTGGAGCTT CGGAGATGA                        782
translation L R T V   W S F   G D                                  213
```

# FIG. 4F

```
cDNA        GCACGAGTTG GGAGGTGTAG CGCGGCTCTG AACGCGCTGA GGGCCGTTGA   50
cDNA        GTGTCGCAGG CGGCGAGGGC GCGAGTGAGG AGCAGACCCA GGCATCGCGC  100
cDNA        GCCGAGAAGG CCGGGCGTCC CCACACTGAA GGTCCGGAAA GGCGACTTCC  150
cDNA        GGGGGCTTTG GCACCTGGCG GACCCTCCCG GAGCGTCGGC ACCTGAACGC  200
cDNA        GAGGCGCTCC ATTGCGCGTG CGCGTTGAGG GGCTTCCCGC ACCTGATCGC  250
cDNA        GAGACCCCAA CGGCTGGTGG CGTCGCCTGC GCGTCTCGGC TGAGCTGGCC  300
cDNA        ATGGCGCAGC TGTGCGGGCT GAGGCGGAGC CGGGCGTTTC TCGCCCTGCT  350
translation M A Q L    C G L    R R S    R A F L   A L L   -11

cDNA        GGGATCGCTG CTCCTCTCTG GGGTCCTGGC GGCCGACCGA GAACGCAGCA  400
translation  G S L    L L S G    V L A    A D R    E R S I     7

cDNA        TCCACGACTT CTGCCTGGTG TCGAAGGTGG TGGGCAGATG CCGGGCCTCC  450
translation  H D F    C L V    S K V V    G R C    R A S      23

cDNA        ATGCCTAGGT GGTGGTACAA TGTCACTGAC GGATCCTGCC AGCTGTTTGT  500
translation M P R W    W Y N    V T D    G S C Q    L F V      40

cDNA        GTATGGGGGC TGTGACGGAA ACAGCAATAA TTACCTGACC AAGGAGGAGT  550
translation  Y G G    C D G N    S N N    Y L T    K E E C     57

cDNA        GCCTCAAGAA ATGTGCCACT GTCACAGAGA ATGCCACGGG TGACCTGGCC  600
translation  L K K    C A T    V T E N    A T G    D L A      73

cDNA        ACCAGCAGGA ATGCAGCGGA TTCCTCTGTC CCAAGTGCTC CCAGAAGGCA  650
translation T S R N    A A D    S S V    P S A P    R R Q      90

cDNA        GGATTCTGAA GACCACTCCA GCGATATGTT CAACTATGAA GAATACTGCA  700
translation  D S E    D H S S    D M F    N Y E    E Y C T    107

cDNA        CCGCCAACGC AGTCACTGGG CCTTGCCGTG CATCCTTCCC ACGCTGGTAC  750
translation  A N A    V T G    P C R A    S F P    R W Y     123

cDNA        TTTGACGTGG AGAGGAACTC CTGCAATAAC TTCATCTATG GAGGCTGCCG  800
translation F D V E    R N S    C N N    F I Y G    G C R     140

cDNA        GGGCAATAAG AACAGCTACC GCTCTGAGGA GGCCTGCATG CTCCGCTGCT  850
translation  G N K    N S Y R    S E E    A C M    L R C F    157

cDNA        TCCGCCAGCA GGAGAATCCT CCCCTGCCCC TTGGCTCAAA GGTGGTGGTT  900
translation  R Q Q    E N P    P L P L    G S K    V V V      173

cDNA        CTGGCGGGGC TGTTCGTGAT GGTGTTGATC CTCTTCCTGG GAGCCTCCAT  950
translation L A G L    F V M    V L I    L F L G    A S M      190

cDNA        GGTCTACCTG ATCCGGGTGG CACGGAGGAA CCAGGAGCGT GCCCTGCGCA 1000
translation  V Y L    I R V A    R R N    Q E R    A L R T    207

cDNA        CCGTCTGGAG CTCCGGAGAT GACAAGGAGC AGCTGGTGAA GAACACATAT 1050
translation  V W S    S G D    D K E Q    L V K    N T Y     223

cDNA        GTCCTGTGAC CGCCCTGTCG CCAAGAGGAC TGGGGAAGGG AGGGGAGACT 1100
translation V L *                                                225
```

# FIG. 4F (Cont.)

| | | | | | | |
|---|---|---|---|---|---|---|
| cDNA | ATGTGTGAGC | TTTTTTTAAA | TAGAGGGATT | GACTCGGATT | TGAGTGATCA | 1150 |
| cDNA | TTAGGGCTGA | GGTCTGTTTC | TCTGGGAGGT | AGGACGGCTG | CTTCCTGGTC | 1200 |
| cDNA | TGGCAGGGAT | GGGTTTGCTT | TGGAAATCCT | CTAGGAGGCT | CCTCCTCGCA | 1250 |
| cDNA | TGGCCTGCAG | TCTGGCAGCA | GCCCCGAGTT | GTTTCCTCGC | TGATCGATTT | 1300 |
| cDNA | CTTTCCTCCA | GGTAGAGTTT | TCTTTGCTTA | TGTTGAATTC | CATTGCCTCC | 1350 |
| cDNA | TTTTCTCNAT | CACAGAAGTG | ATGTTGGAAT | CGTTTCTTTT | GTTTGTCTGA | 1400 |
| cDNA | TTTATGGTTT | TTTTAAGTAT | AAACAAAAGT | TTTTTATTAG | CATTCTGAAA | 1450 |
| cDNA | GAAGGAAAGT | AAAATGTACA | AGTTTAATAA | AAAGGGGCCT | TCCCCTTTAG | 1500 |
| cDNA | AATAAATTTC | CAGCATGTTG | CTTTCAAAAA | AAAAAAAAAA | AAAA | 1550 |

# FIG. 4G

```
EST consens                                       MLR AEADGVSRLL GSLLLSGVLA  -1
PCR clone                                     MAQLCGL RRSRAFLALL GSLLLSGVLA  -1
λcDNA clone                                   MAQLCGL RRSRAFLALL GSLLLSGVLA  -1

EST consens ADRERSIHDF CLVSKVVGRC RASMPRWWYN VTDGSCQLFV YGGCDGNSNN  50
PCR clone   ADRERSIHDF CLVSKVVGRC RASMPRWWYN VTDGSCQLFV YGGCDGNSNN  50
λcDNA clone ADRERSIHDF CLVSKVVGRC RASMPRWWYN VTDGSCQLFV YGGCDGNSNN  50

EST consens YLTKEECLKK CATVTENATG DLATSRNAAD SSVPSAPRRQ DSEDHSSDMF 100
PCR clone   YLTKEECLKK CATVTENATG DLATSRNAAD SSVPSAPRRQ DSEDHSSDMF 100
λcDNA clone YLTKEECLKK CATVTENATG DLATSRNAAD SSVPSAPRRQ DSEDHSSDMF 100

EST consens NYEEYCTANA VTGPCRASFP RWYFDVERNS CNNFIYGGCR GNKNSYRSEE 150
PCR clone   NYEEYCTANA VTGPCRASFP RWYFDVERNS CNNFIYGGCR GNKNSYRSEE 150
λcDNA clone NYEEYCTANA VTGPCRASFP RWYFDVERNS CNNFIYGGCR GNKNSYRSEE 150

EST consens ACMLRCFRQQ ENPPLPLGSK VVVLAGLFVM VLILFLGASM VYLIRVARRN 200
PCR clone   ACMLRCFRQQ ENPPLPLGSK VVVLAGLFVM VLILFLGASM VYLIRVARRN 200
λcDNA clone ACMLRCFRQQ ENPPLPLGSK VVVLAGLFVM VLILFLGASM VYLIRVARRN 200

EST consens QERALRTVWS SGDDKEQLVK NTYVL                            225
PCR clone   QERALRTVWS FGD                                        213
λcDNA clone QERALRTVWS SGDDKEQLVK NTYVL                            225
```

FIG. 5

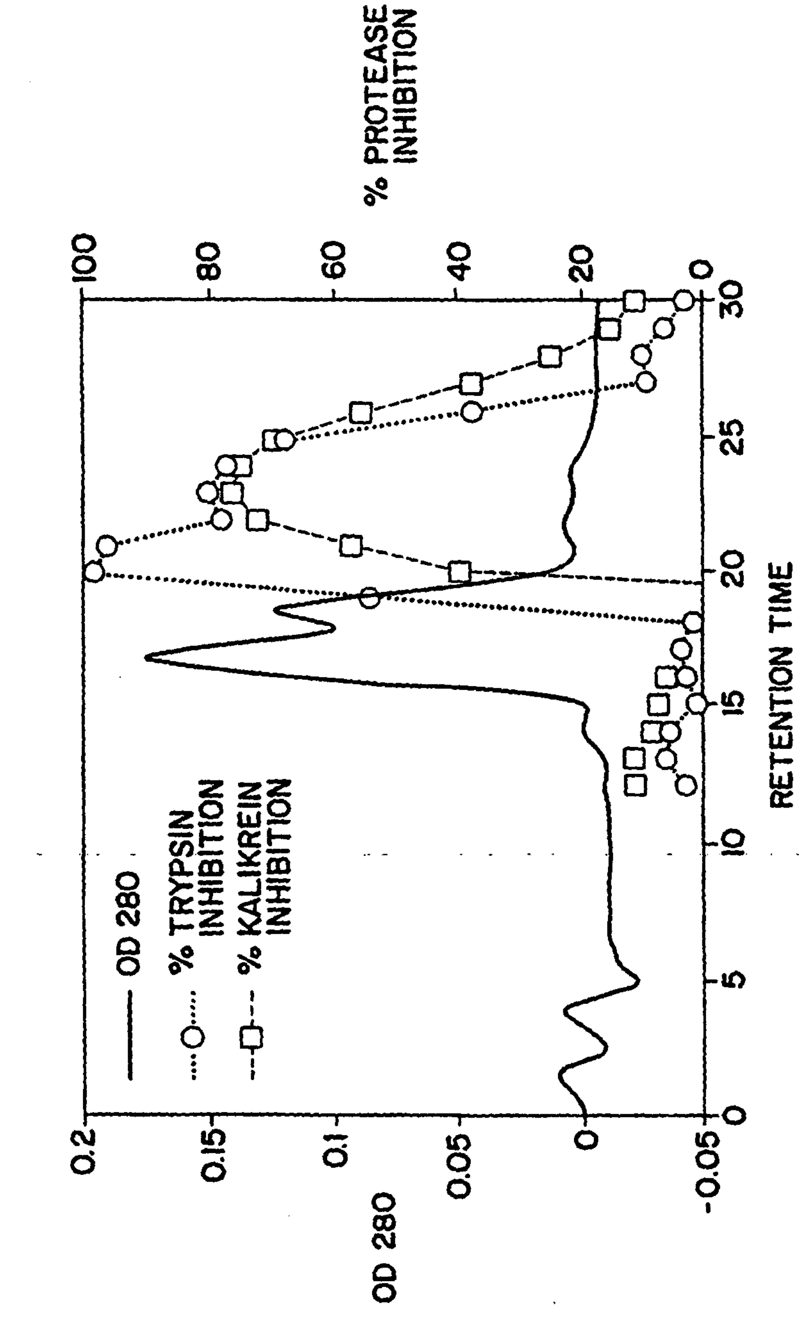
OD 280
% TRYPSIN INHIBITION
% KALIKREIN INHIBITION
% PROTEASE INHIBITION
OD 280
RETENTION TIME
0.2
0.15
0.1
0.05
0
-0.05
100
80
60
40
20
0
0
5
10
15
20
25
30

FIG. 6

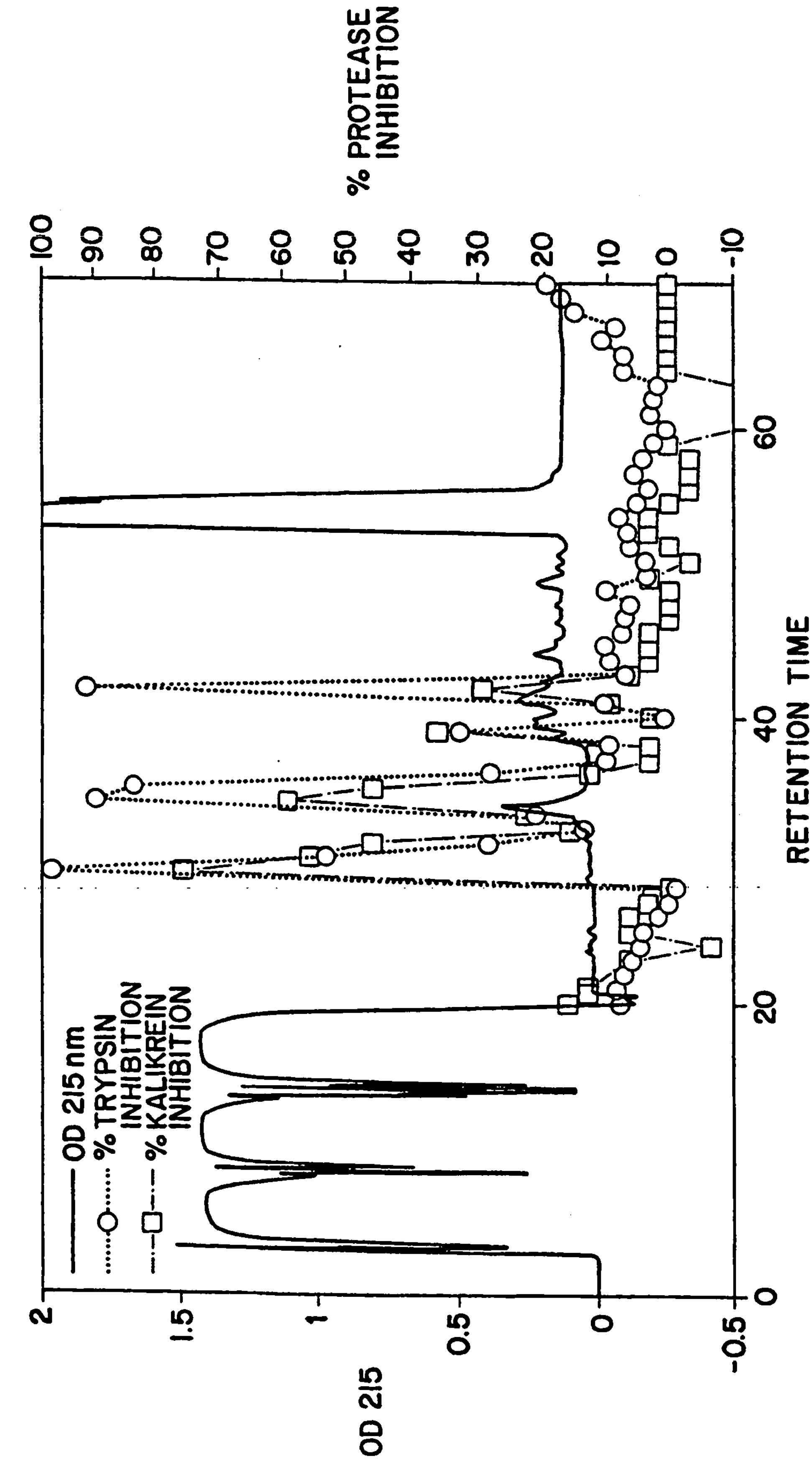
OD 215 nm
% TRYPSIN INHIBITION
% KALIKREIN INHIBITION
OD 215
2
1.5
1
0.5
0
-0.5
% PROTEASE INHIBITION
100
90
80
70
60
50
40
30
20
10
0
-10
0
20
40
60
RETENTION TIME

FIG. 7

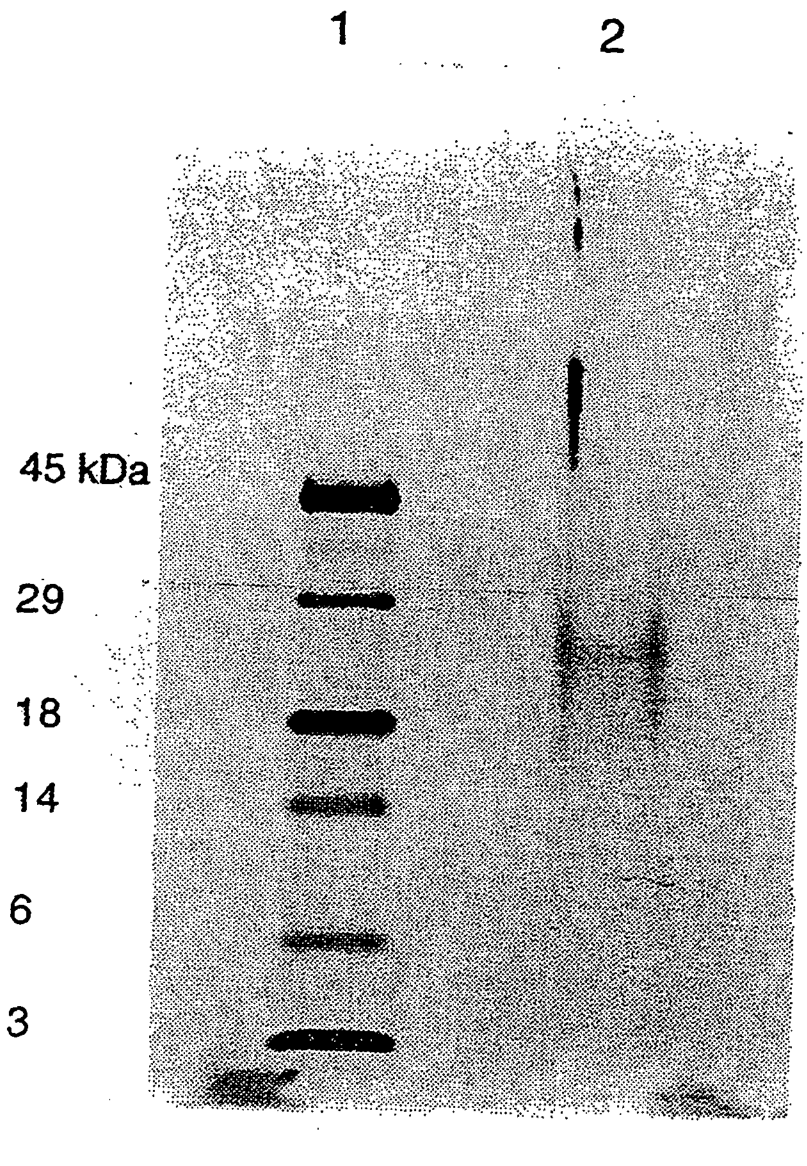

FIG. 8A

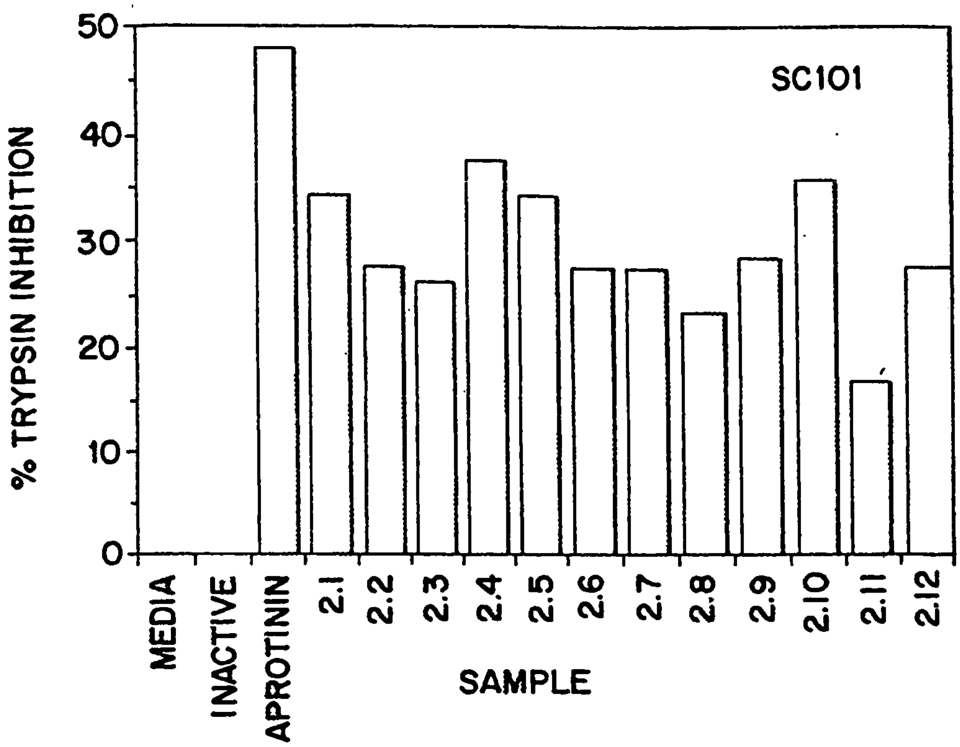
50
40
30
20
10
0
% TRYPSIN INHIBITION
SC101
MEDIA
INACTIVE
APROTININ
2.1
2.2
2.3
2.4
2.5
2.6
2.7
2.8
2.9
2.10
2.11
2.12
SAMPLE

FIG. 8B

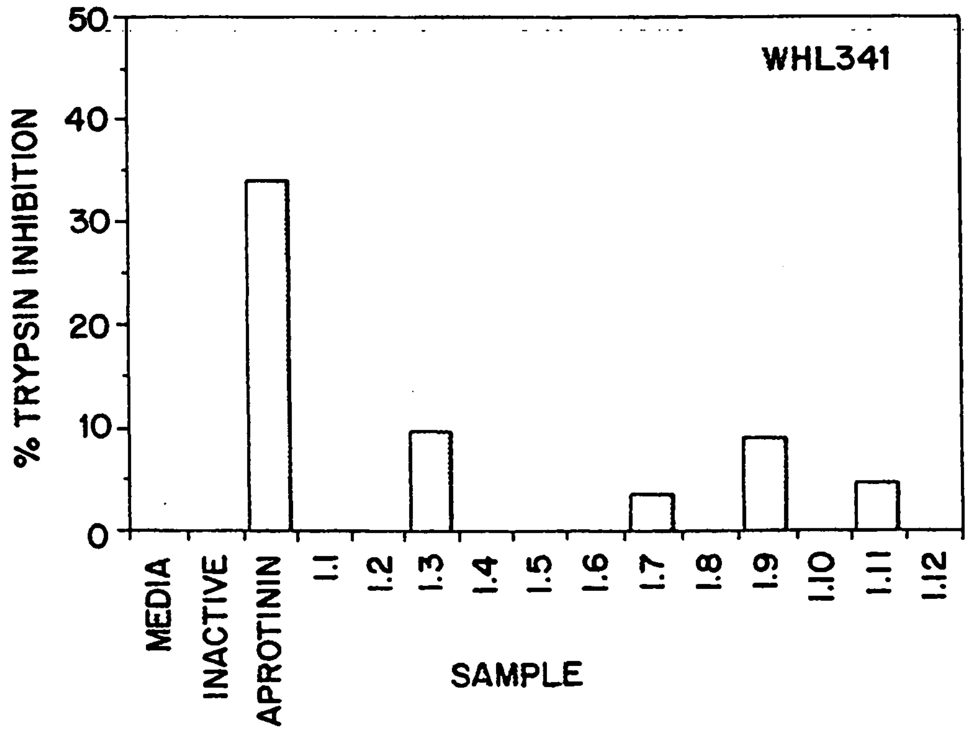
50
40
30
20
10
0
% TRYPSIN INHIBITION
WHL341
MEDIA
INACTIVE
APROTININ
1.1
1.2
1.3
1.4
1.5
1.6
1.7
1.8
1.9
1.10
1.11
1.12
SAMPLE

FIG. 9A

SDS-PAGE

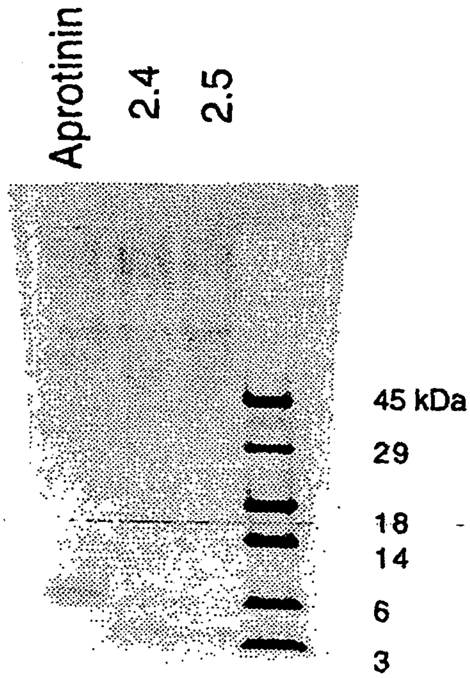


FIG. 9B

Western

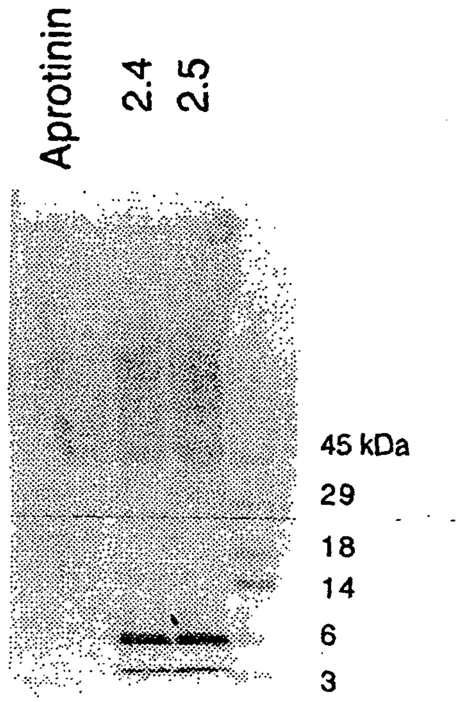

FIG. 10

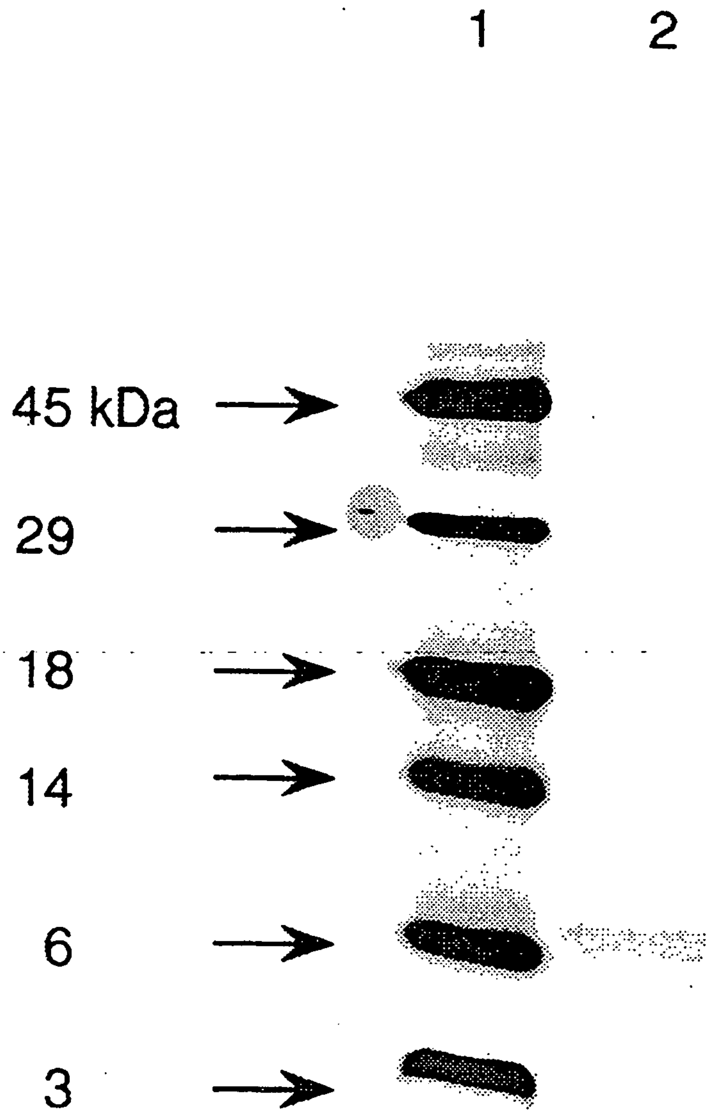

FIG. 11A

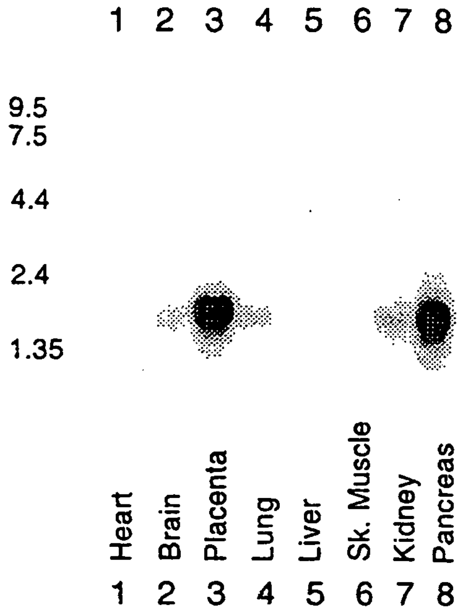


FIG. 11B

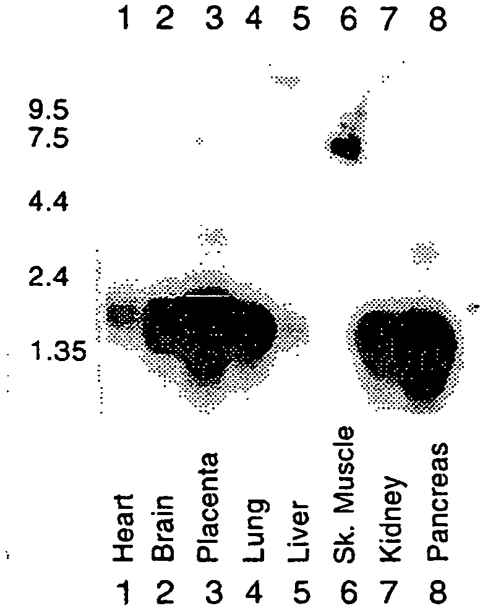


FIG. 12A

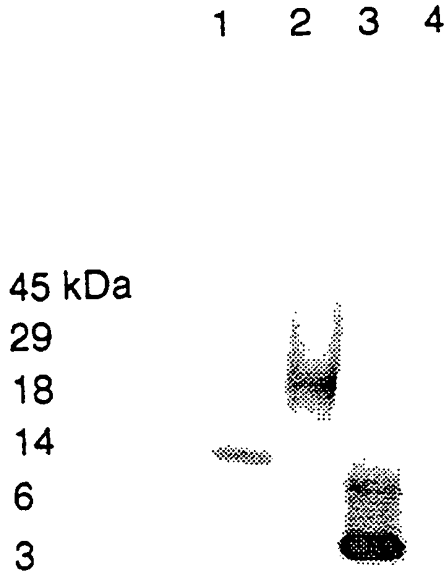


FIG. 12B

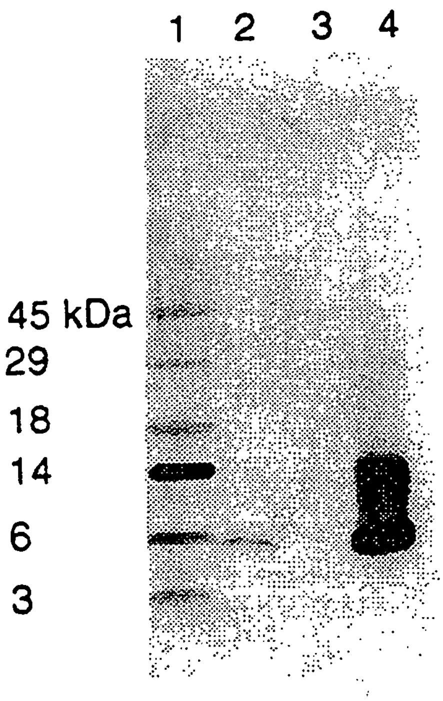

FIG. 13

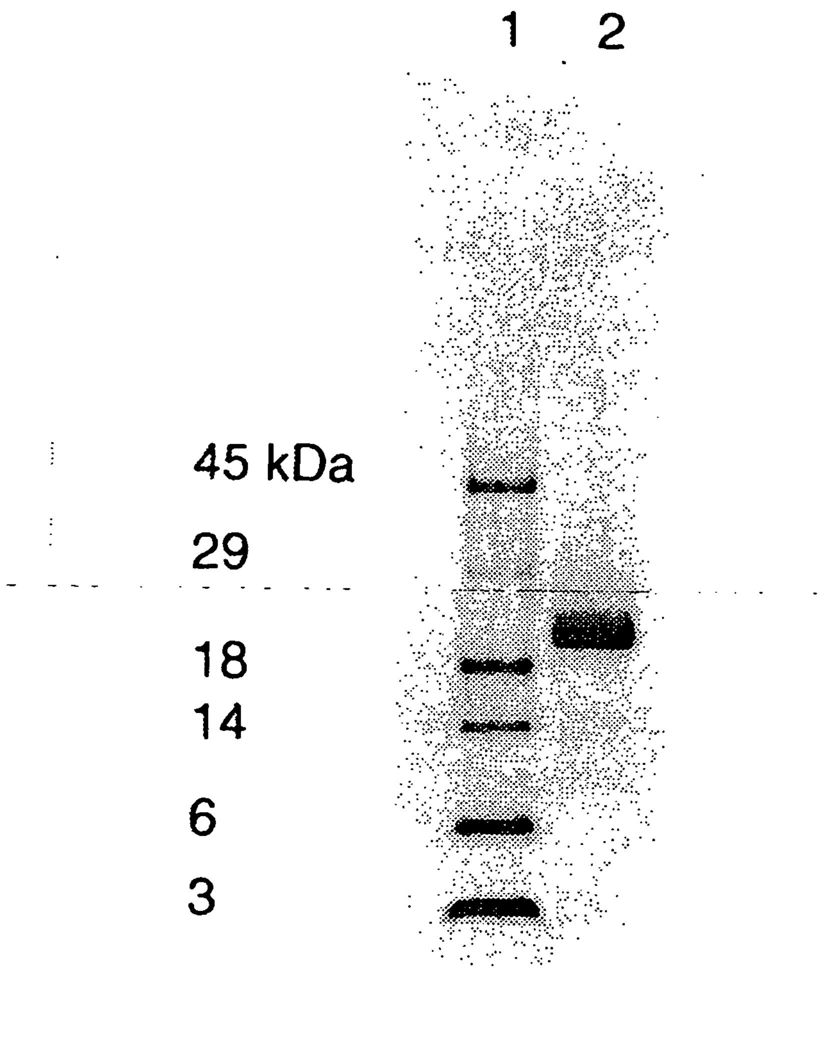

FIG. 14

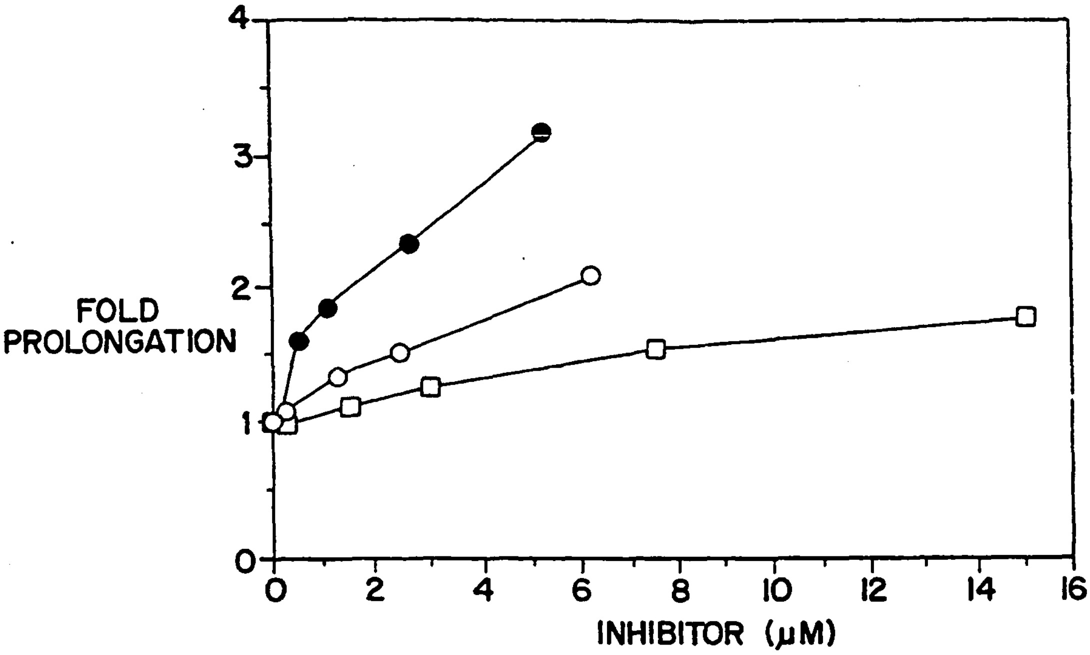
FOLD PROLONGATION
4
3
2
1
0
0
2
4
6
8
10
12
14
16
INHIBITOR (μM)

FIG. 15

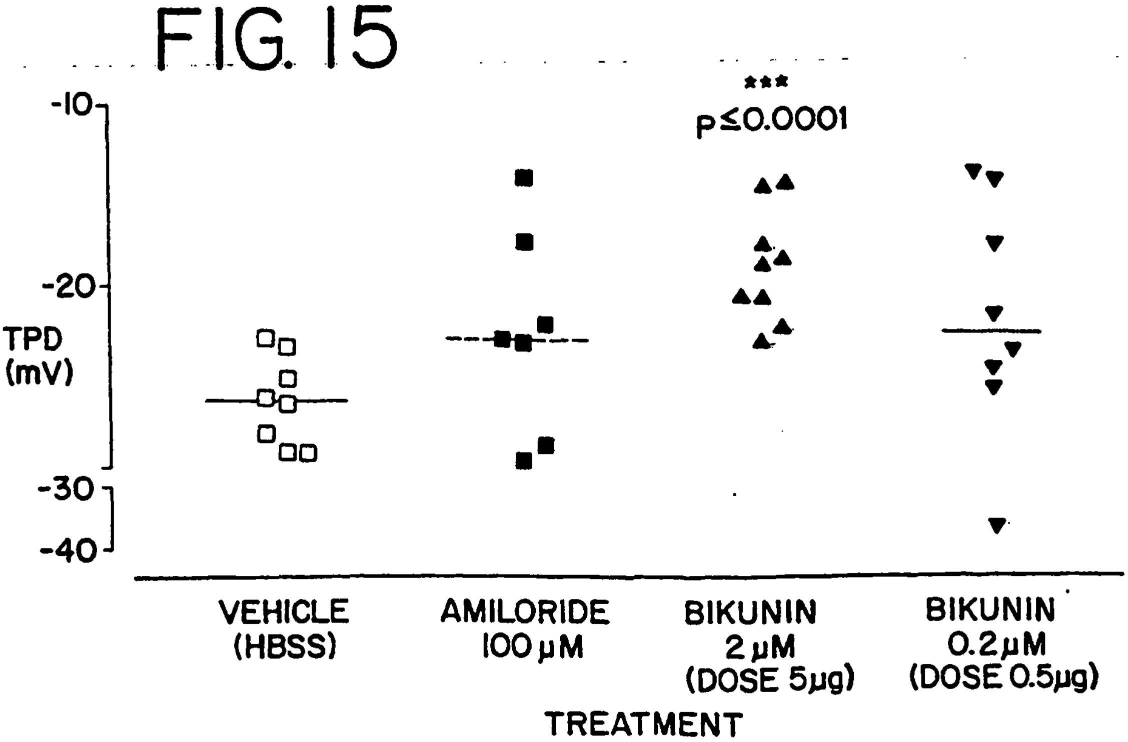
***
p≤0.0001
-10
-20
-30
-40
TPD (mV)
VEHICLE (HBSS)
AMILORIDE 100 μM
BIKUNIN 2 μM (DOSE 5μg)
BIKUNIN 0.2 μM (DOSE 0.5μg)
TREATMENT

FIG. 16A

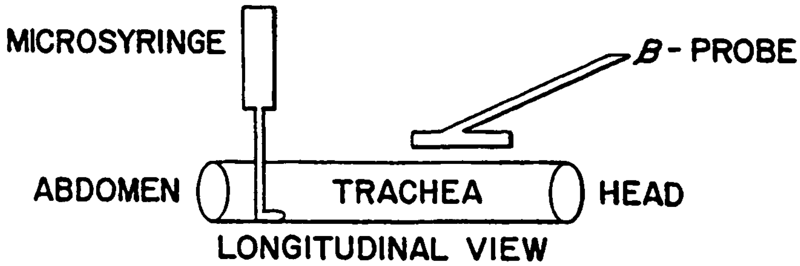


FIG. 16B

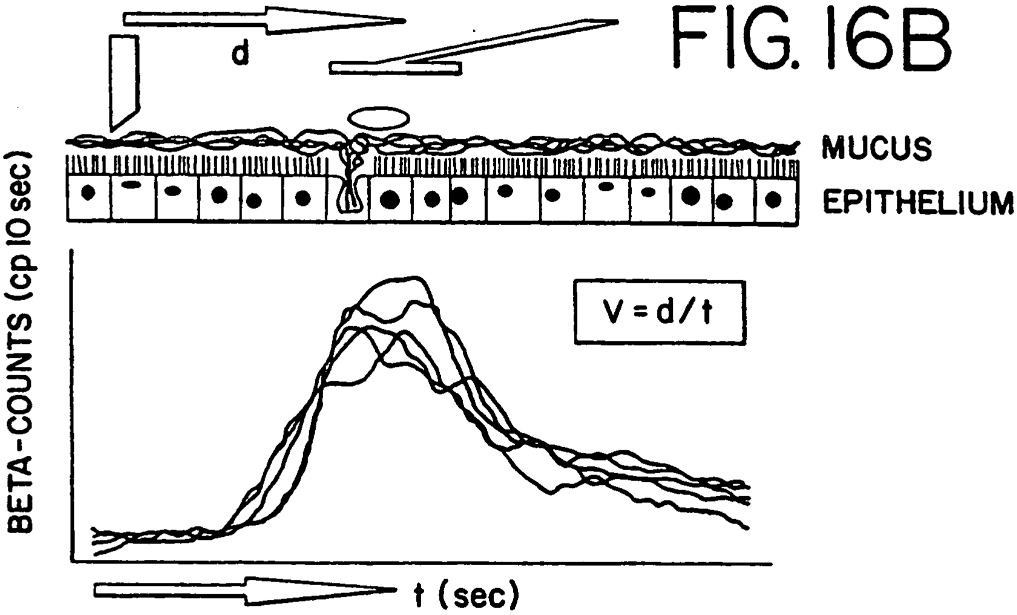


FIG. 16C

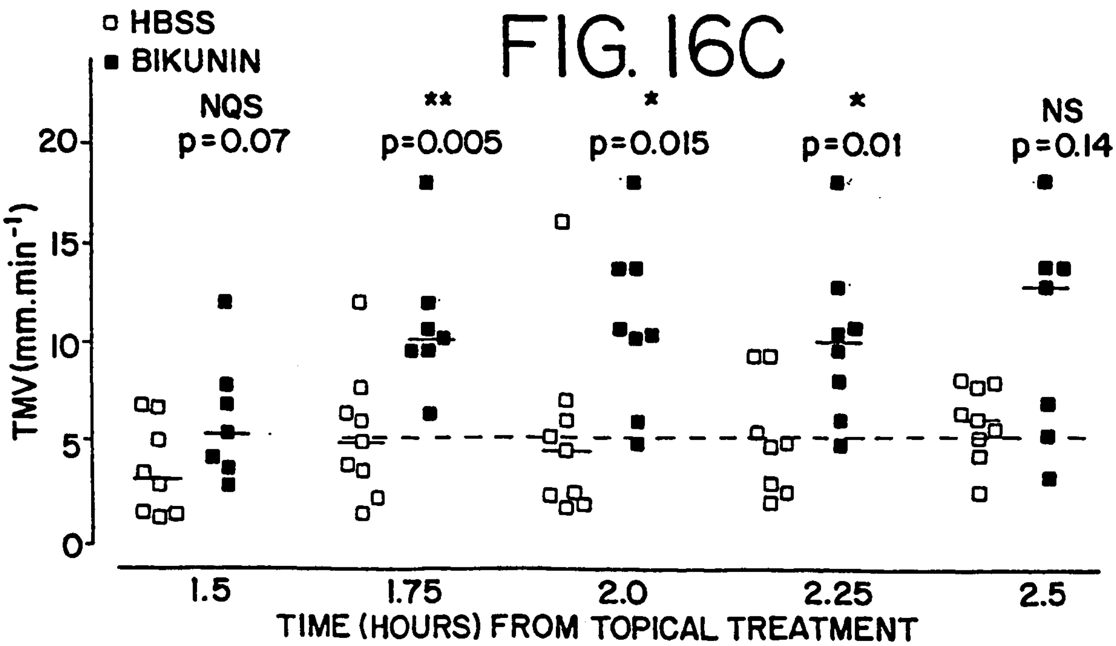

FIG. 17

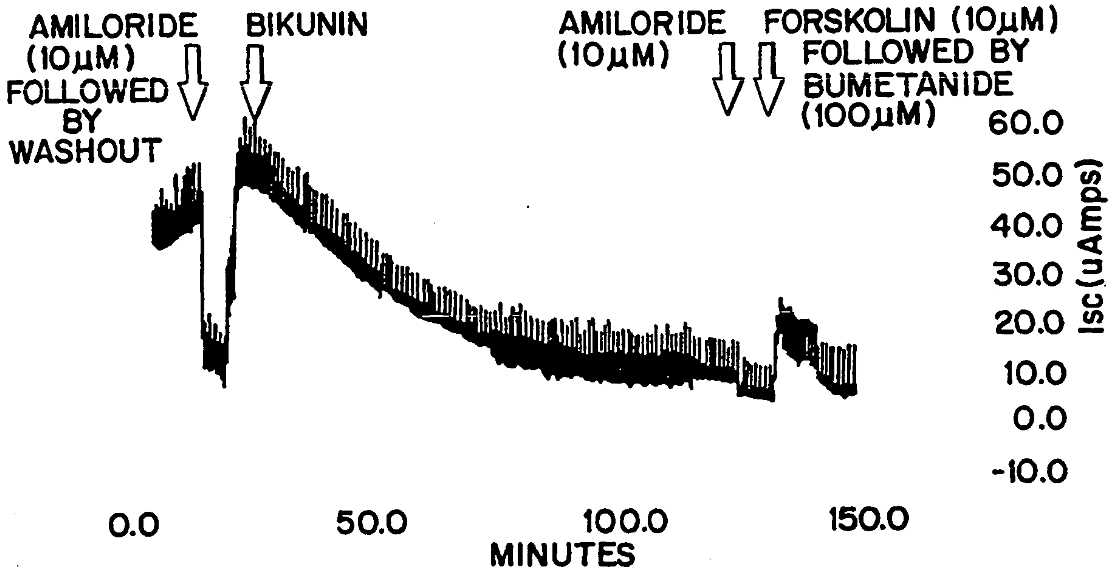
AMILORIDE (10µM) FOLLOWED BY WASHOUT
BIKUNIN
AMILORIDE (10µM)
FORSKOLIN (10µM) FOLLOWED BY BUMETANIDE (100µM)
60.0
50.0
40.0
30.0
20.0
10.0
0.0
-10.0
Isc(uAmps)
0.0
50.0
100.0
150.0
MINUTES

FIG. 18

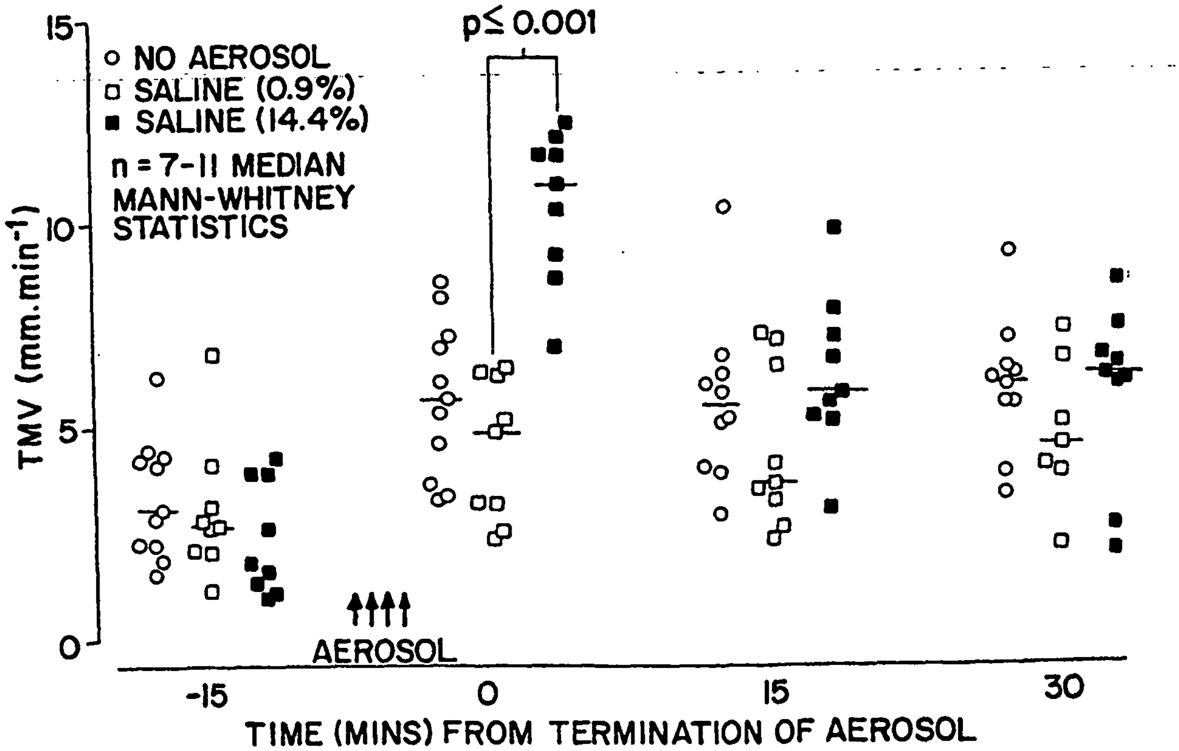
15
10
5
0
TMV (mm.min$^{-1}$)
p≤ 0.001
o NO AEROSOL
□ SALINE (0.9%)
■ SALINE (14.4%)
n = 7-11 MEDIAN
MANN-WHITNEY
STATISTICS
AEROSOL
-15
0
15
30
TIME (MINS) FROM TERMINATION OF AEROSOL

FIG. 19

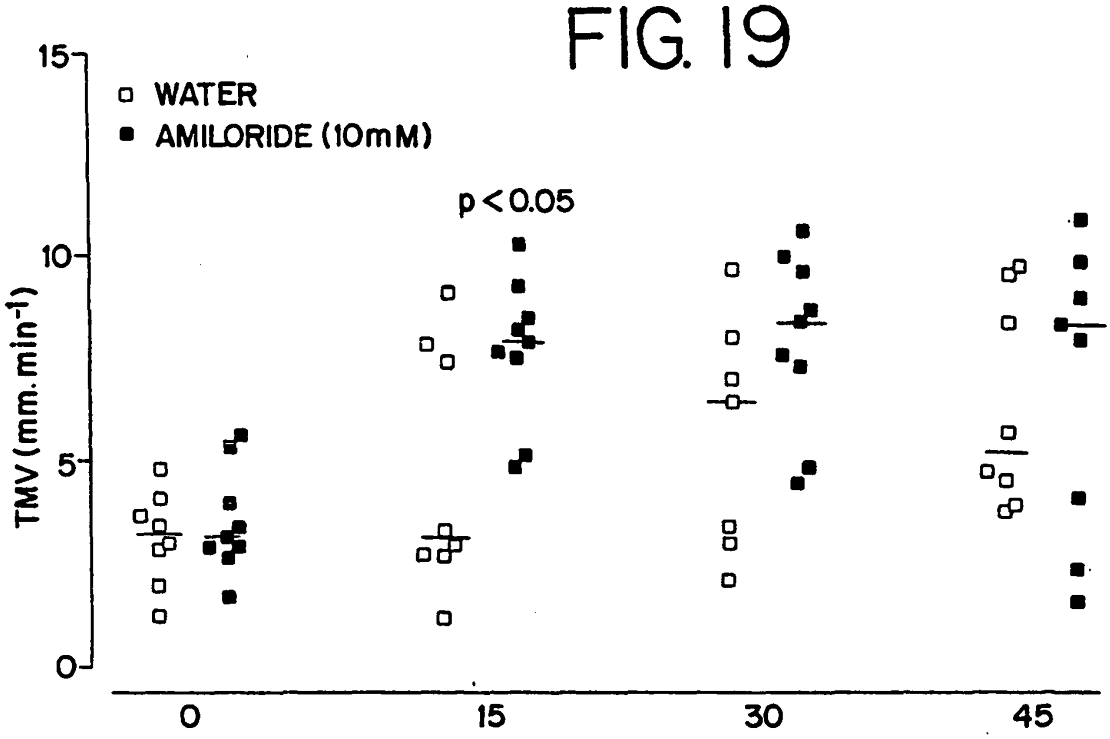
15
10
5
0
TMV (mm.min-1)
WATER
AMILORIDE (10mM)
p<0.05
0
15
30
45

FIG. 20

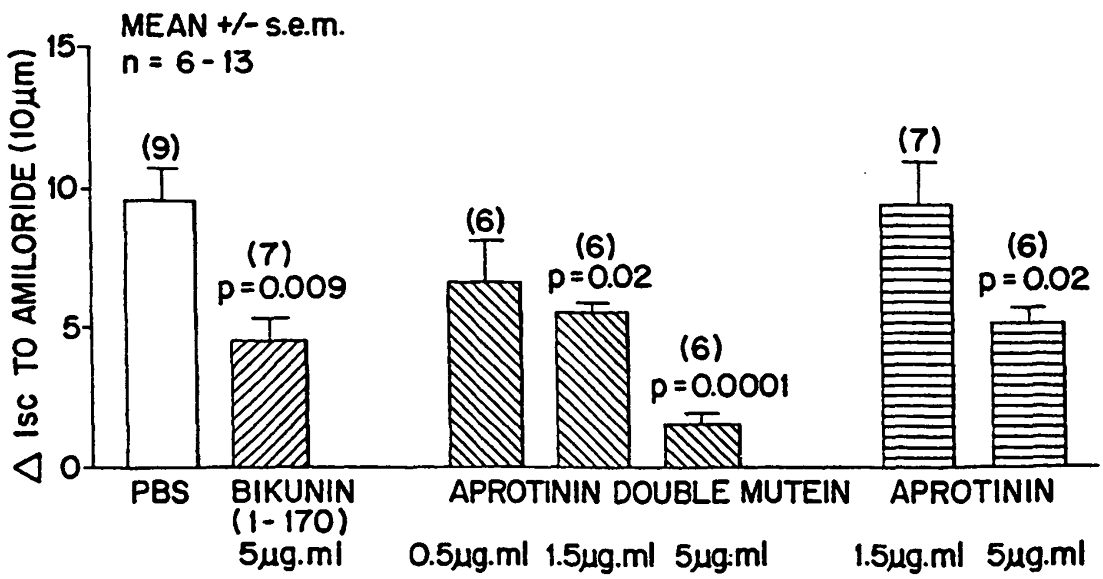
MEAN +/- s.e.m.
n = 6 - 13
Δ Isc TO AMILORIDE (10μm)
15
10
5
0
(9)
(7)
p=0.009
(6)
(6)
p=0.02
(6)
p=0.0001
(7)
(6)
p=0.02
PBS
BIKUNIN (1-170) 5μg.ml
APROTININ DOUBLE MUTEIN
0.5μg.ml
1.5μg.ml
5μg:ml
APROTININ
1.5μg.ml
5μg.ml

FIG. 21

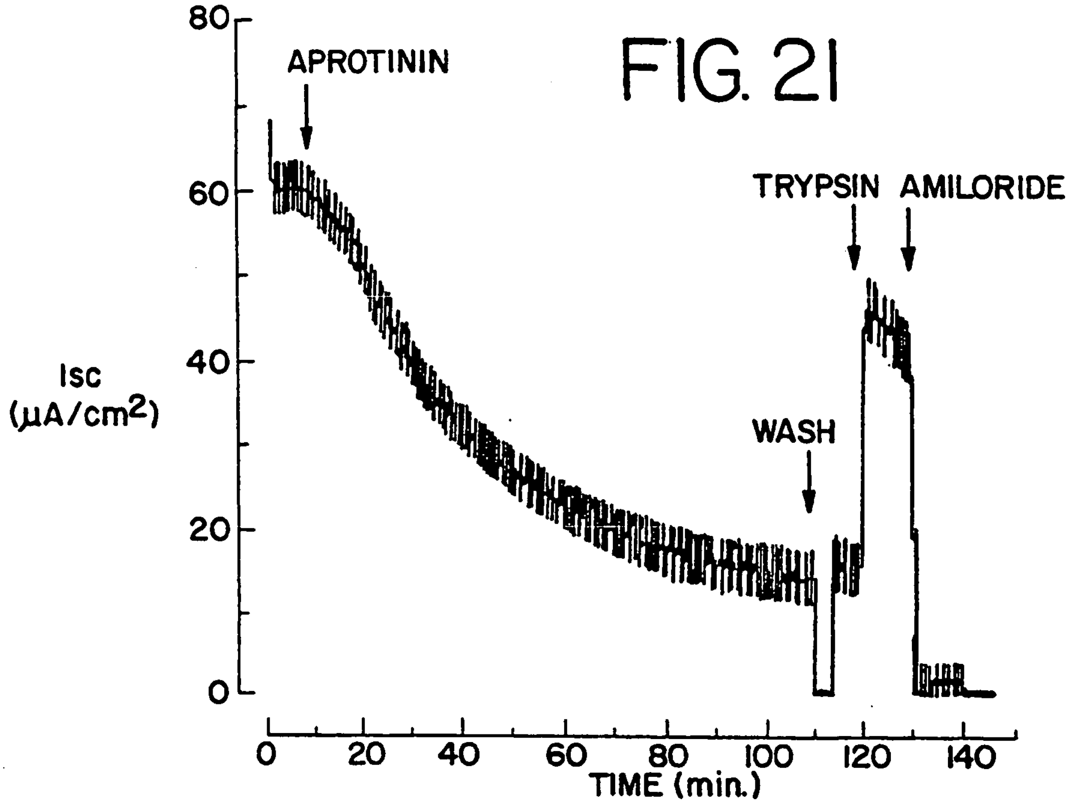
APROTININ
TRYPSIN
AMILORIDE
WASH
Isc (μA/cm2)
0
20
40
60
80
0
20
40
60
80
100
120
140
TIME (min.)

FIG. 22

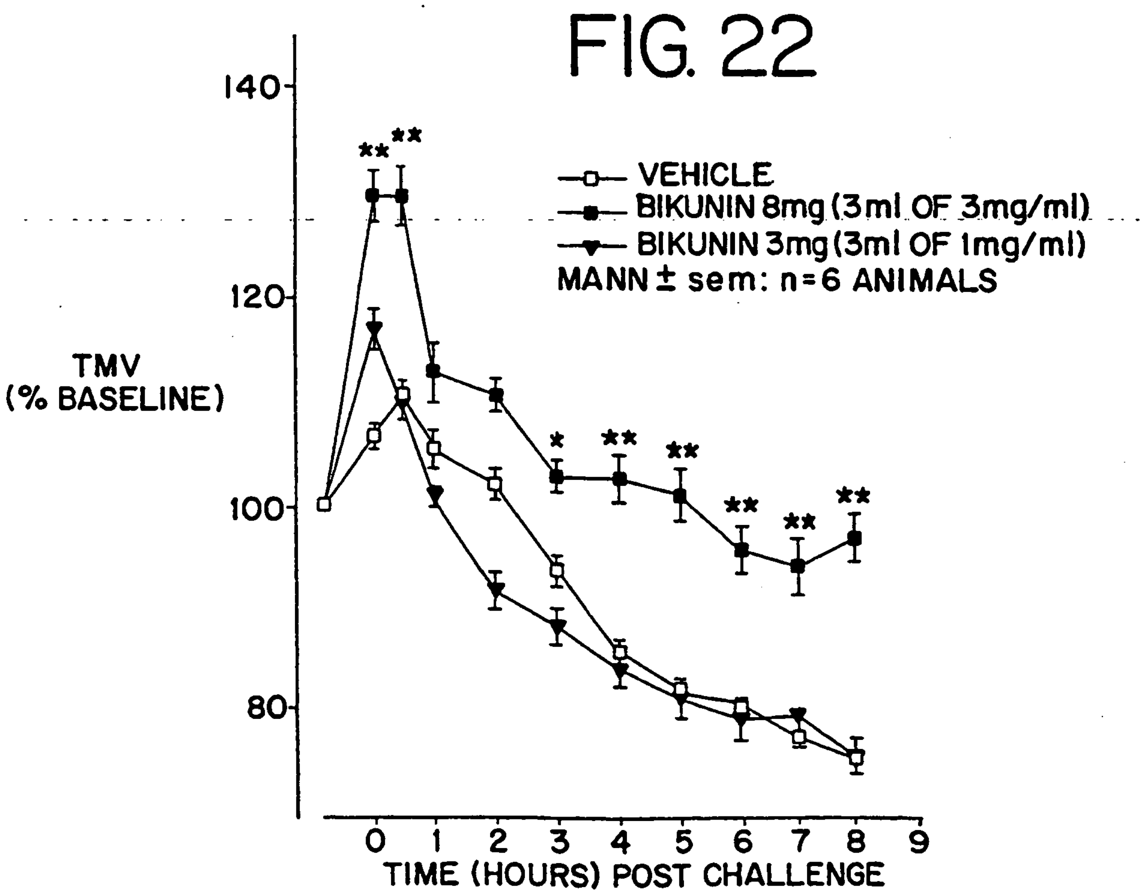
VEHICLE
BIKUNIN 8mg (3ml OF 3mg/ml)
BIKUNIN 3mg (3ml OF 1mg/ml)
MANN ± sem: n=6 ANIMALS
TMV (% BASELINE)
80
100
120
140
0
1
2
3
4
5
6
7
8
9
TIME (HOURS) POST CHALLENGE

FIG. 23

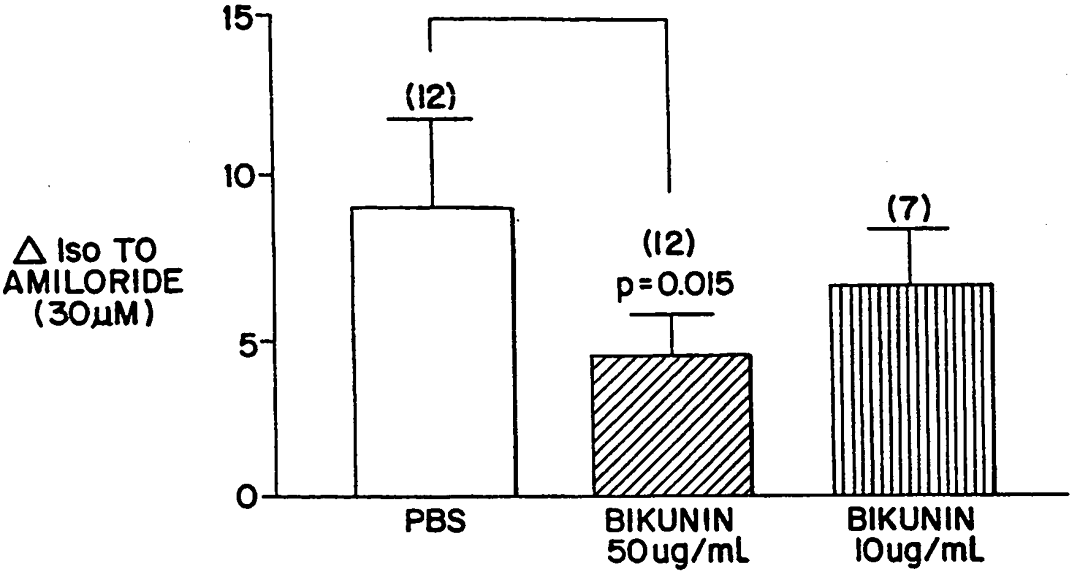
15
10
5
0
Δ Iso TO
AMILORIDE
(30μM)
(12)
(12)
p=0.015
(7)
PBS
BIKUNIN
50ug/mL
BIKUNIN
10ug/mL

FIG. 24

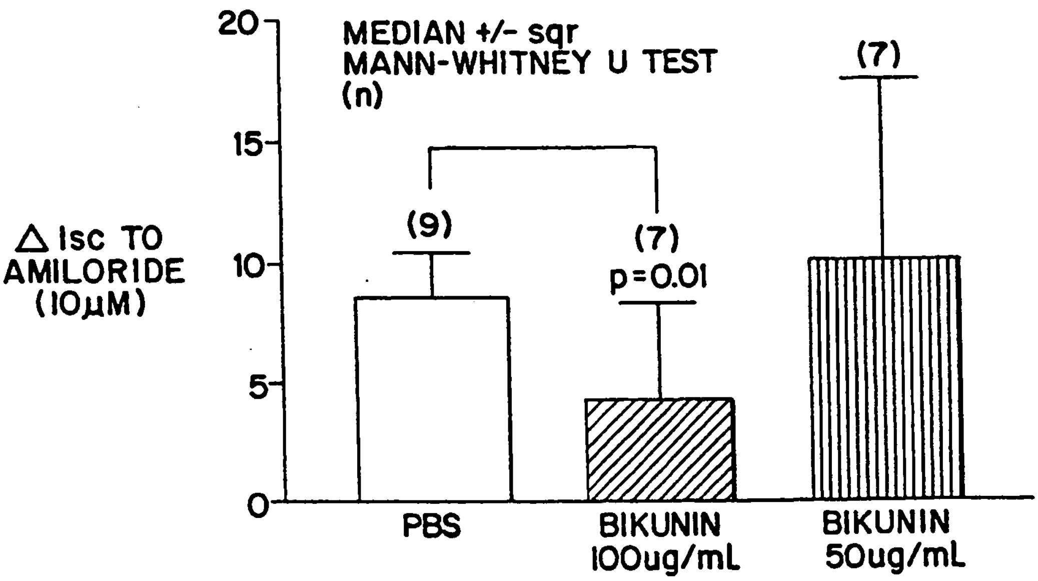
20
15
10
5
0
MEDIAN +/- sqr
MANN-WHITNEY U TEST
(n)
Δ Isc TO
AMILORIDE
(10μM)
(9)
(7)
p=0.01
(7)
PBS
BIKUNIN
100ug/mL
BIKUNIN
50ug/mL

FIG. 25A

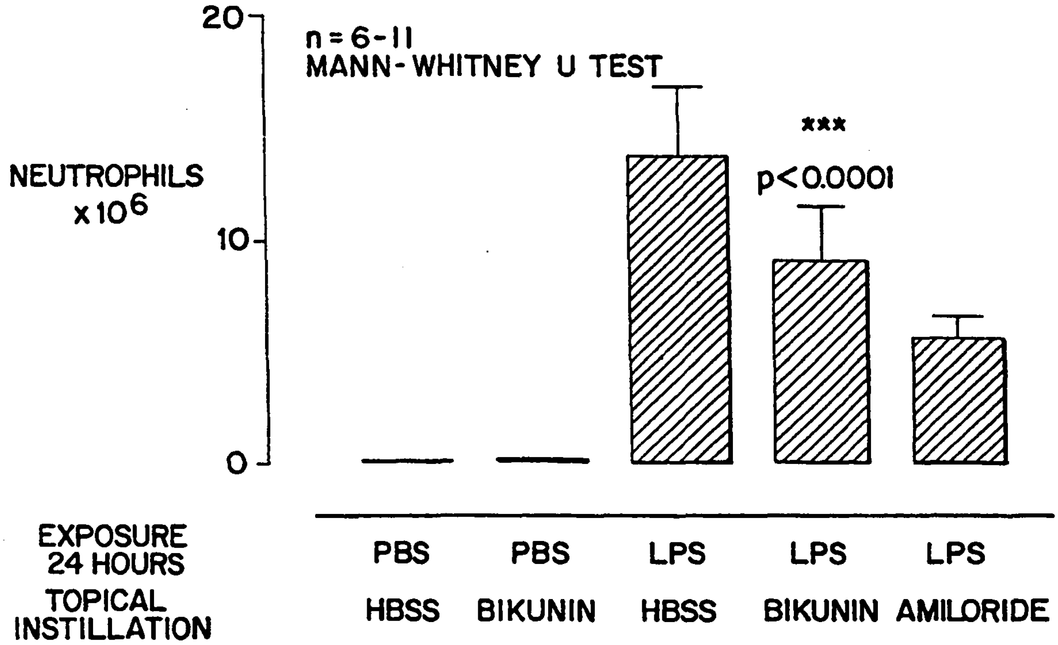


FIG. 25B

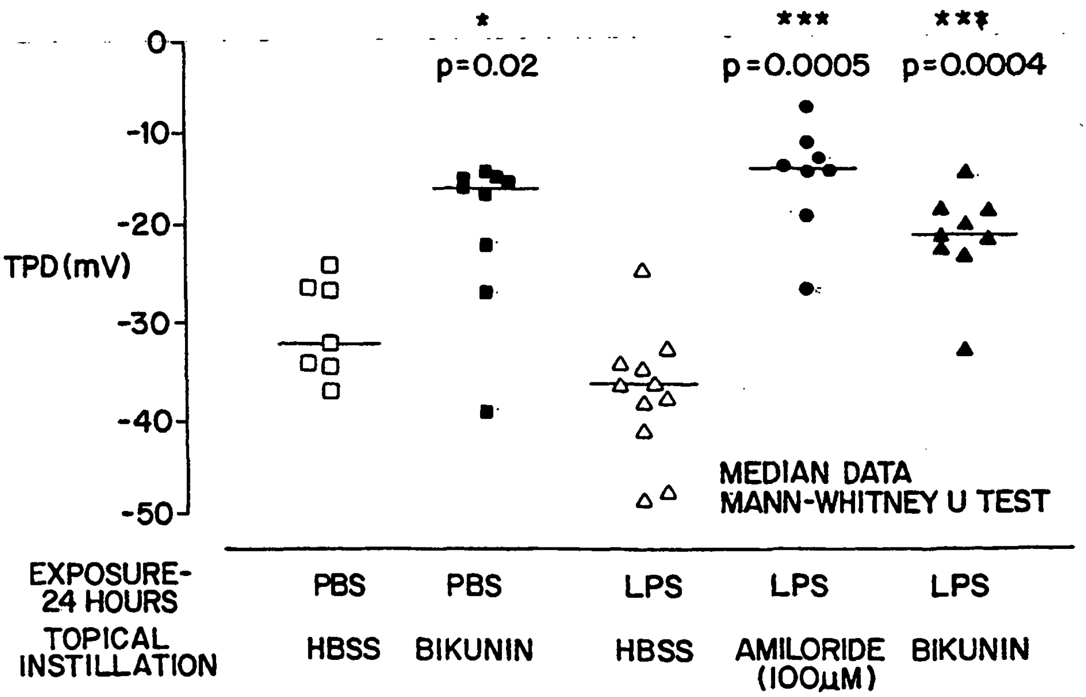

FIG. 26

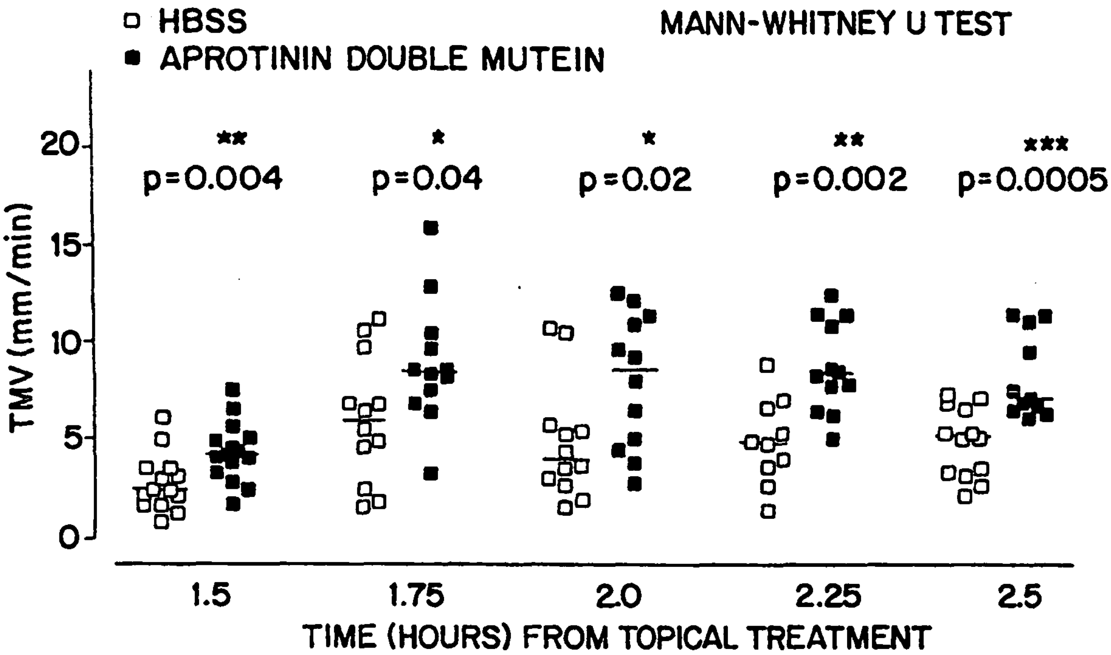
□ HBSS
■ APROTININ DOUBLE MUTEIN
MANN-WHITNEY U TEST
** p=0.004
* p=0.04
* p=0.02
** p=0.002
*** p=0.0005
TMV (mm/min)
0
5
10
15
20
1.5
1.75
2.0
2.25
2.5
TIME (HOURS) FROM TOPICAL TREATMENT

FIG. 27

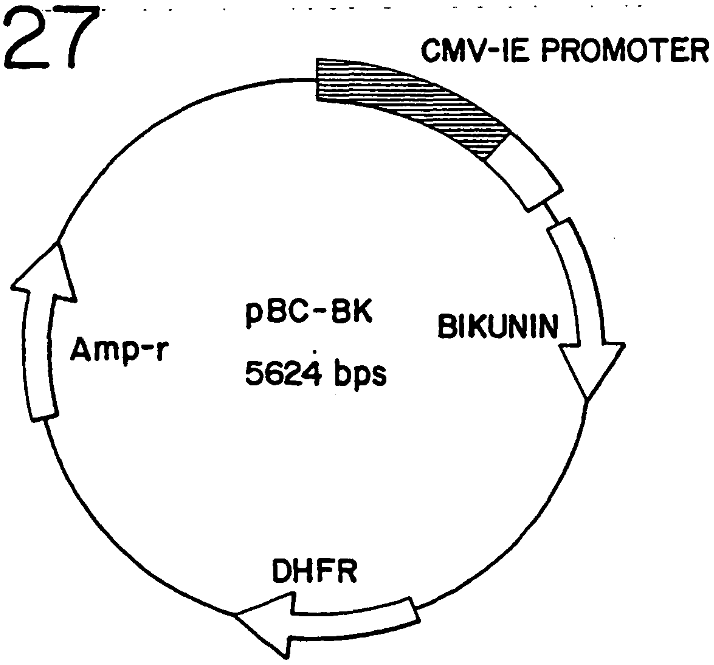
CMV-IE PROMOTER
BIKUNIN
DHFR
Amp-r
pBC-BK
5624 bps

FIG. 28A

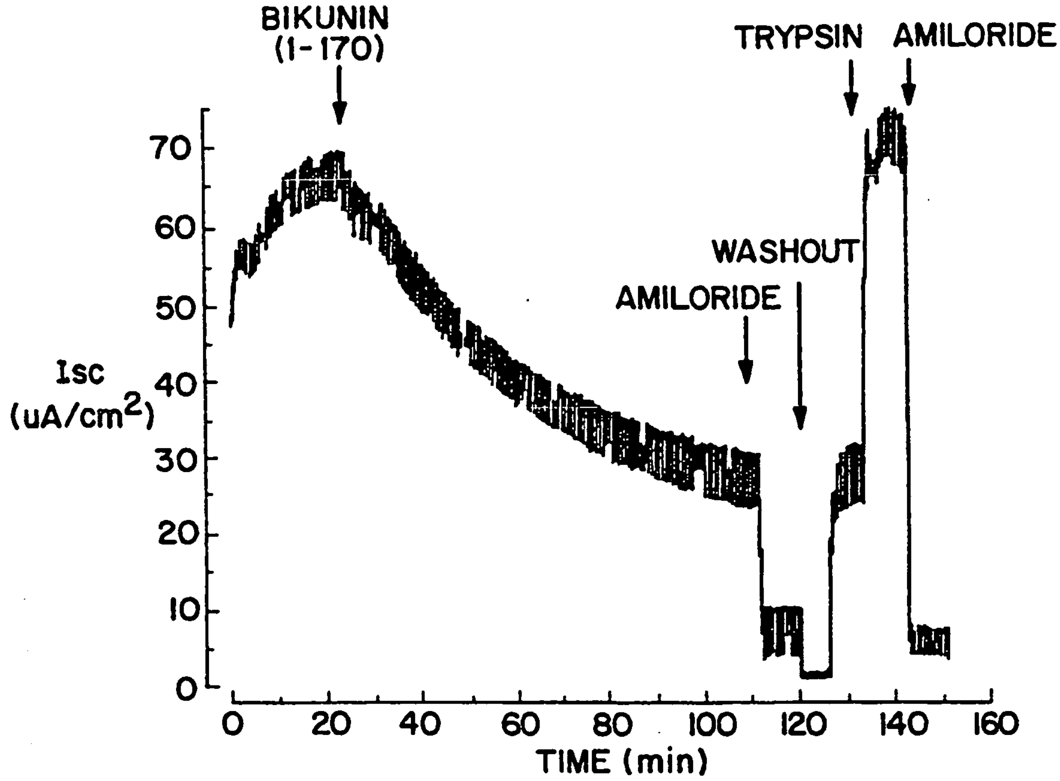


FIG. 28B

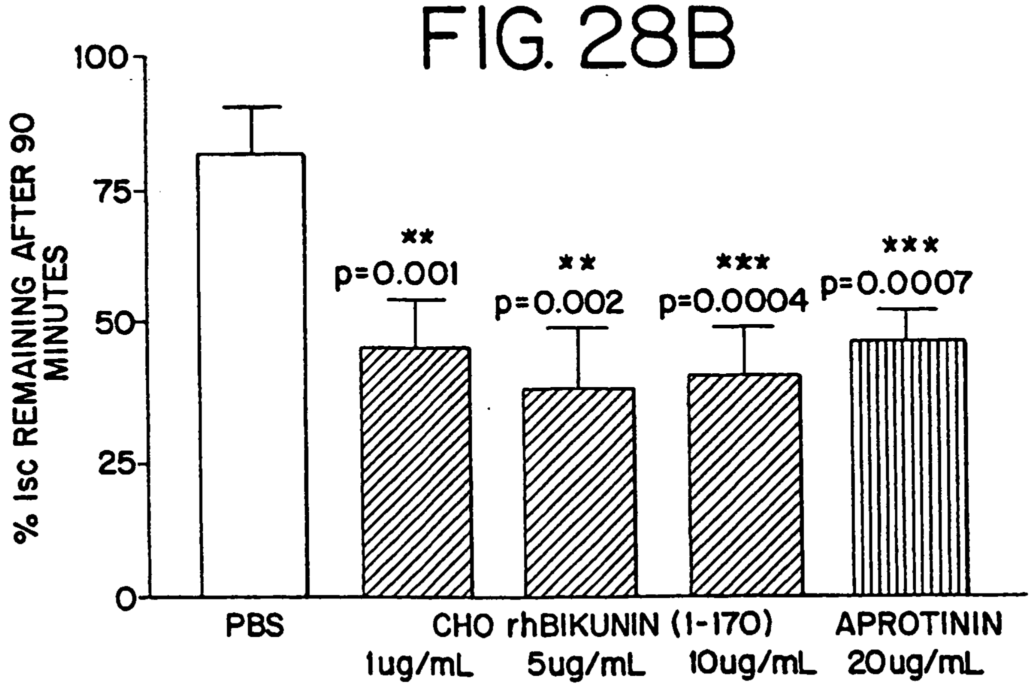

FIG. 29

FLOW CHART OF CHO rhBIKUNIN PURIFICATION PROCESS TRAIN

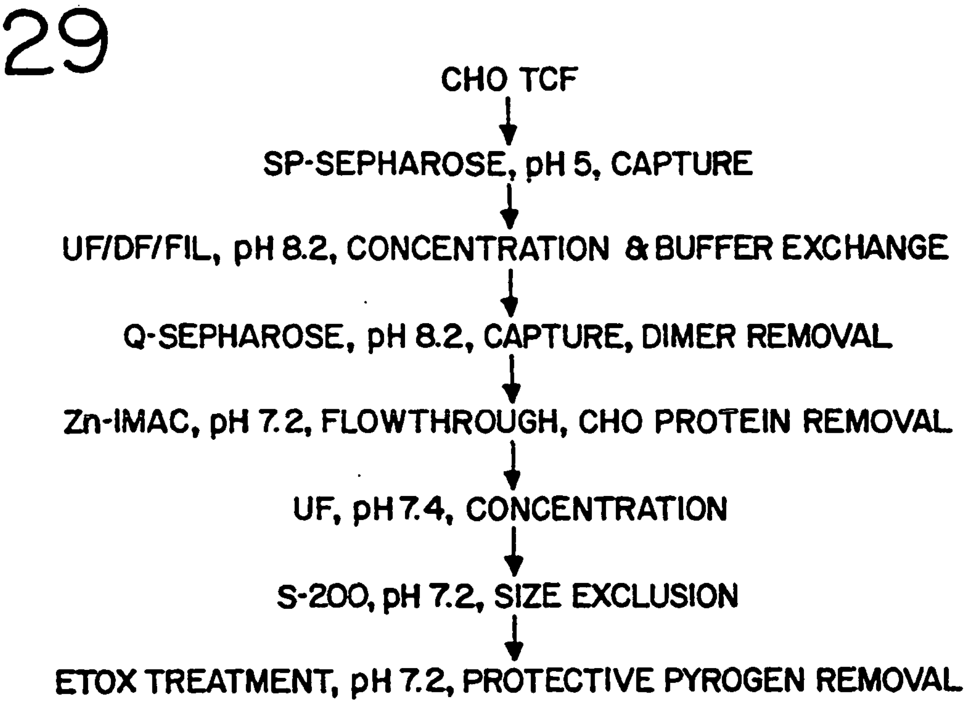


FIG. 30A

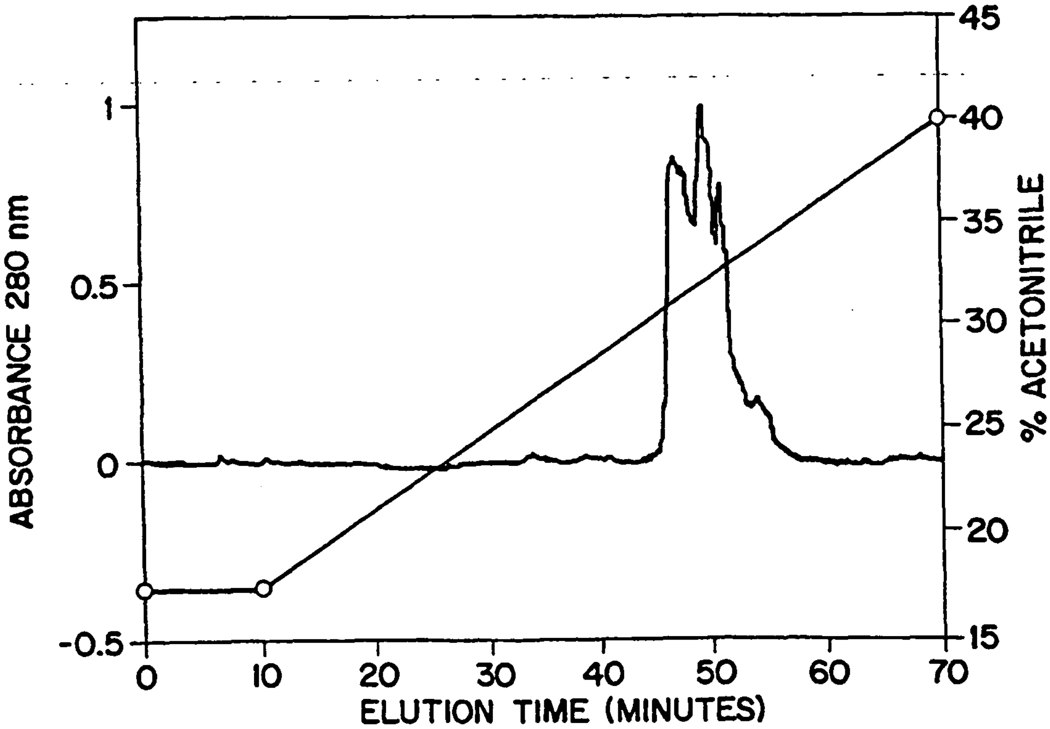

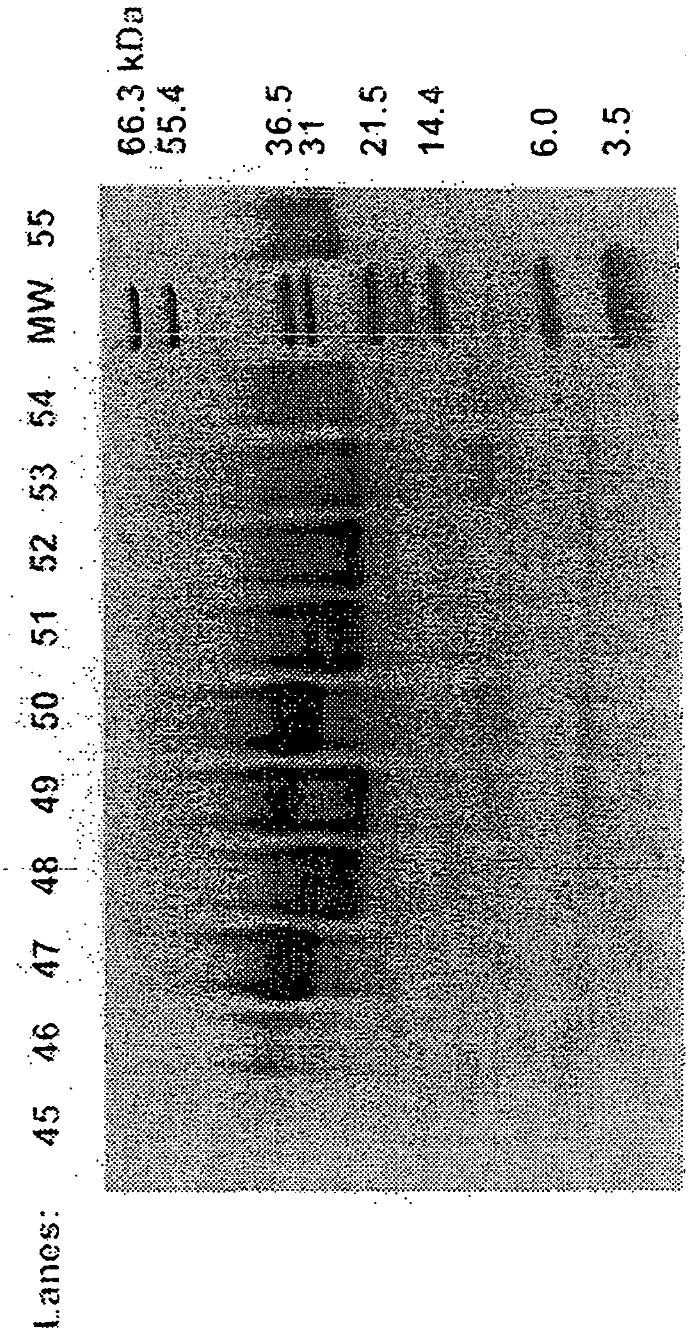
Lanes:
45
46
47
48
49
50
51
52
53
54
MW
55
66.3 kDa
55.4
36.5
31
21.5
14.4
6.0
3.5

FIG. 30B

FIG. 31

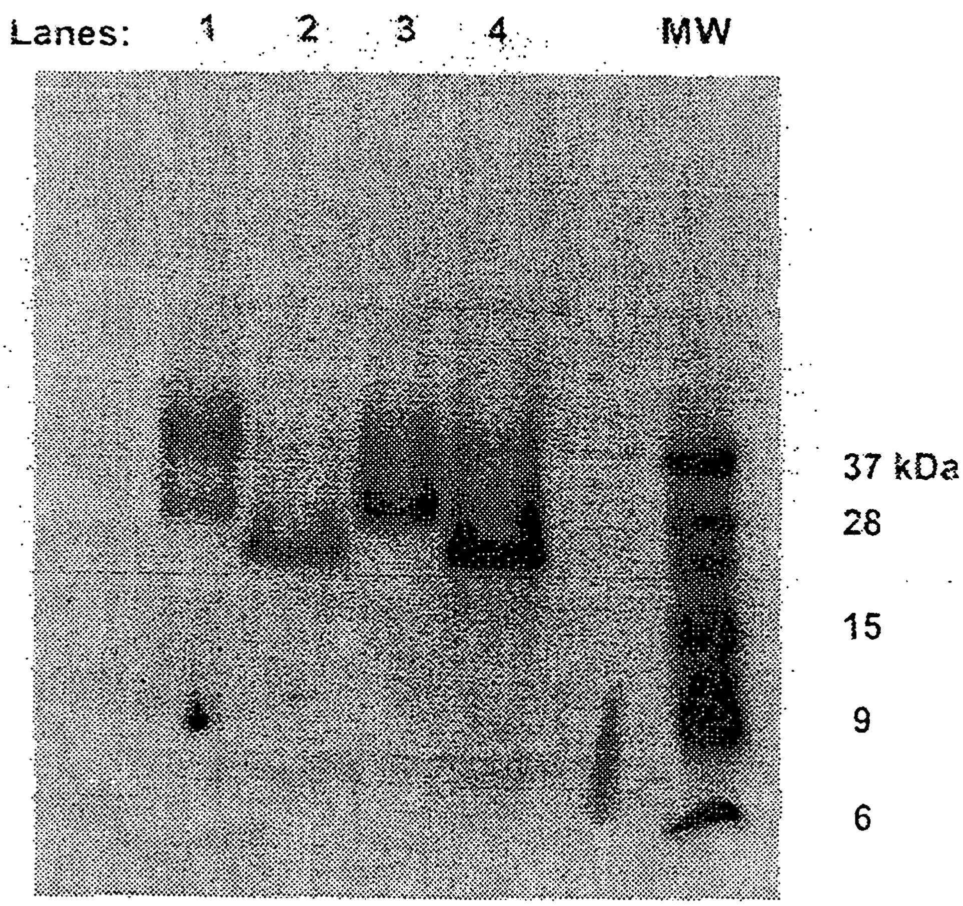

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 21891398 A
- US 5830436 A
- WO 9733996 A
- US 5407915 A
- US 4501729 A
- WO 9303709 A
- WO 9312240 A
- WO 9526356 A
- US 5164482 A
- US 5032573 A
- EP 821007 A
- US SN09441654 A
- GB 9904381 W
- US 09441966 B
- US 09218913 B


### Non-patent literature cited in the description


- **BRAGA.** Drugs in Bronchial Mucology. Raven Press, 1989
- **LETHEM et al.** *Am Rev. Respir. Dis.,* 1990, vol. 142, 1053-1058
- **DI SANTAGRESE et al.** *Am J. Med.,* 1979, vol. 66, 121-132
- **CF. KNOWLES.** *Clin. Chest. Med.,* 1986, vol. 11, 75
- **BEDROSSIAN et al.** *Human Pathol.,* 1976, vol. 7, 195-204
- **WILLS et al.** *J. Clin. Invest.,* 1995, vol. 97 (1), 9-13
- **WILLS et al.** *J. Resp. Crit. Care Med.,* 1997, vol. 151 (4), 1255-1258
- **MATTHEWS et al.** *Am. Rev. Resp. Dis.,* 1963, vol. 88, 199-204
- **POTTER et al.** *Am. Rev. Resp. Dis.,* 1967, vol. 67 (1), 83-87
- **TOMKIEWICZ et al.** *Am. Rev. Resp. Dis.,* 1993, vol. 148 (1), 1002-1007
- **ROBINSON et al.** *Thorax,* 1997, vol. 52 (10), 900-903
- **APP et al.** *Am. Rev. Resp. Dis.,* 1990, vol. 141, 605-612
- **KEREM et al.** *New England J. Med.,* 1999, vol. 341, 156-162
- **VALLET et al.** *Nature,* 1997, vol. 389 (6651), 607-610
- **CHRAIBI et al.** *J. Gen. Physio.,* 1998, vol. 111 (1), 127-138
- **DELARIA et al.** *J. Biol. Chem.,* 1997, vol. 272 (18), 12209-12214
- **MARLOR et al.** *J. Biol. Chem.,* 1997, vol. 272 (18), 12202-12208
- **MCAULAY et al.** *Ped. Pulm.,* 1998, S17
- **O'RIORDAN et al.** *Am. J. Resp. Crit. Care Med.,* 1997, vol. 97 (5), 1522-1528
- **O'RIORDAN et al.** *J. App. Physio.,* 1998, vol. 85 (3), 1086-1091
- **LASKOWSKE ; KATO.** *Ann. Rev. Biochem,* 1980, vol. 49, 593-626
- **ALTSCHUL.** *J. Mol Biol,* 1990, vol. 215 (00), 403-410
- **SANGER F. et al.** *Proc. Natl. Acad. Sci (USA,* 1977, vol. 74, 5463-5467
- **ESCH F. et al.** *Science,* 1990, vol. 248, 1122-1124
- **WANG R. et al.** *J. Biol Chem,* 1991, vol. 266, 16960-16964
- **PEARSON ; UPMAN.** *Proc. Natl. Acad. Sci. USA,* 1988, vol. 85, 2444-2448
- **MERRIFIELD R.B. ; BARANY G. et al.** The peptides, Analysis, Synthesis, Biology. Academic Press, 1980
- **CARPINO L.A. ; HAN G.Y.** *J. Amer Chem Soc.,* 1970, vol. 92, 5748-5749
- **BEAUCAGE S.L. ; CARUTHERS M.H.** *Tetrahedron Lett,* 1981, vol. 22, 1859-1862
- **MATTEUCCI M.D ; CARUTHERS M.H.** *J. Am. Chem. Soc.,* 1981, vol. 103, 3185
- **SAMBROOK et al.** Molecular Cloning: A laboratory Manual. Cold Spring Harbor, 1989
- **AUSUBEL F.M et al.** Current Protocols in Molecular Biology. John Wiley & Sons, 1995
- **KUNKEL T.A.** *Proc. Natl. Acad. Sci USA,* 1985, vol. 82, 488-492
- **TAM et al.** *J. Am. Chem. Soc.,* 1991, vol. 113, 6657-62
- **TENSTAD et al.** *Acta Physiol. Scand.,* 1994, vol. 152, 33-50
- **CHASE,T. ; SHAW, E.** *Methods Enzmol.,* 1970, vol. 19, 20-27
- **CHASE, T. ; SHAW, E.** *Methods Enzmol.,* 1970, vol. 19, 20-27

- **BOUDIER, C. ; BIETH, J. G.** *Biochim Biophys Acta,* 1989, vol. 995, 36-41
- **NEWTON et al.** Cilia, Mucus and Mucociliary Interactions. Marcel Dekker, 1998
- **NEWTON et al.** *Ped. Pulm.,* 1998, S17
- **NEWTON et al.** Cila, Mucus and Mucociliary Interactions. Marcel Dekker, 1998
- **NEWTON et al.** *Ped. Pulm.,* 1998, vol. S17
- **NEWTON et al.** Cilia. Mucus and Mucociliary Interactions. Marcel Dekker, 1990
- **NEWTON et al.** *Pediatric Pulmonology,* 1998, vol. S17
- **NEWTON et al.** Cilia, Mucus and Mucociliary Interactions. Marcel Dekker, 1990
- **CHASE,T. ; SHAW, E.** Methods Enzmol. 1970, vol. 19, 20-27
- **O'RIORDAN et al.** *J. Applied Physiol.,* 1998, vol. 85 (3), 1086-1091
- **RUSSI et al.** *J. Applied Physiol.,* 1985, vol. 59 (5), 1416-1422